# EUROPEAN PATENT SPECIFICATION

(11) **EP 0 677 039 B1**
(45) Date of publication and mention of the grant of the patent: **10.03.1999**
(21) Application number: 94931987.5
(22) Date of filing: 20.10.1994
(51) Int. Cl.: C07C 233/60, C07C 323/40, C07D 257/04, A61K 31/16, A61K 31/41

(54) **CYCLOBUTANE DERIVATIVES AS INHIBITORS OF SQUALENE SYNTHETASE AND PROTEIN FARNESYLTRANSFERASE**
CYCLOBUTAN-DERIVATE ALS INHIBITOREN DER SQUALEN-SYNTHETASE UND DER PROTEIN-FARNESYLTRANSFERASE
DERIVES DE CYCLOBUTANE UTILISES EN TANT QU'INHIBITEURS DE LA SQUALENE-SYNTHETASE ET DE LA FARNESYLTRANSFERASE PROTEIQUE

(30) Priority: 04.11.1993 US 147708; 09.09.1994 US 289711
(43) Date of publication of application: 18.10.1995
(73) Proprietor: Abbott Laboratories, Abbott Park, Illinois 60064-3500 (US)
(72) Inventor: BAKER, William, R., Bellevue, WA 98006 (US); ROCKWAY, Todd, W., Garyslake, IL 60030 (US); DONNER, B., Gregory, Mundelein, IL 60060 (US); SHEN, Wang, Skokie, IL 60077 (US); ROSENBERG, Saul, H., Grayslake, IL 60030 (US); FAKHOURY, Stephen, A., Mundelein, IL 60060 (US); O'CONNOR, Stephen, J., Wilmette, IL 60091 (US); STOUT, David, M., Mettawa, IL 60048 (US); FUNG, Anthony, K., L., Gurnee, IL 60031 (US); GARVEY, David, S., Waltham, MA 02154 (US); PRASAD, Rajnandan, Vernon Hills, IL 60061 (US); SULLIVAN, Gerard, M., Round Lake Beach, IL 60073 (US)
(74) Representative: Modiano, Guido, Dr.-Ing.
(86) International application number: US9412132
(87) International publication number: WO9512572

(56) References cited:
- US-A- 4 340 715

## Description

The present invention relates to new cyclobutane dicarboxylic acid compounds which are useful in inhibiting *de novo* squalene production or inhibiting protein farnesyltransferase and the farnesylation of the oncogene protein Ras or inhibiting fungal growth and to chemotherapeutic, antifungal, hypolipidaemic and antiatherosclerotic compositions containing such compounds and to a method of using such compounds for inhibiting cholesterol biosynthesis and atherosclerosis, for inhibiting protein farnesyl-transferase and the farnesylation of the oncogene protein Ras and as antifungals.

### Background of the Invention

Squalene synthetase is a microsomal enzyme which catalyzes the reductive dimerization of two molcules of farnesyl pyrophosphate (FPP) in the presence of nicotinamide adenine dinucleotide phosphate, reduced form, (NADPH) to form squalene (Poulter, C. D., Rilling, H. C., in "Biosynthesis of Isoprenoid Compounds", Vol. I, Chapter 8, pp. 413-441, J. Wiley and Sons, 1981 and references therein). This enzyme is the first committed step of the *de novo* chlolesterol biosynthetic pathway. Thus inhibition of squalene synthetase will lead to inhibition of cholesterol biosynthesis and thus will act as a hypocholesterolemic. Thus squalene synthetase inhibitors ultimately should be useful for the treatment and prevention of hyperlipidaemia or atherosclerosis or other disorders resulting from an excess of cholesterol.

Transformed protein Ras is involved in the proliferation of cancer cells. The Ras must be farnesylated before this proliferation can occur. Farnesylation of Ras by farnesyl pyrophosphate (FPP) is effected by protein famesyltransferase. Inhibition of protein famesyltransferase and, thereby, of farnesylation of the Ras protein, blocks the ability of transformed cells to proliferate.

Activation of Ras also partially mediates smooth muscle cell proliferation ((Circulation, 1-3: 88 (1993). Inhibition of protein famesyltransferase and, thereby, of farnesylation of the Ras protein, would aid in the prevention of restenosis.

Inhibition of squalene synthetase also results in the inhibition of fungal growth.

### Disclosure of the Invention

In accordance with the present invention there are provided substituted cyclobutanes of formula (I): 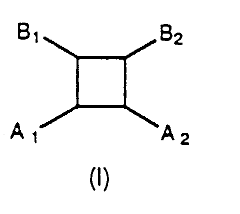 wherein
A₁ and A₂ are independently selected from
   (1) -X-C(O)-G or -X-C(S)-G wherein at each occurrence X is independently selected from (a) a covalent bond, (b) -CH₂-, (c) -O-, (d) -S- and (e)-N(Rₐ)- wherein Rₐ is hydrogen, loweralkyl, cycloalkyl or cycloalkylalkyl and at each occurrence G is independently selected from -R₂, -N(R₁)(R₂), -OR₂ and -SR₂ wherein at each occurrence R₁ is independently selected from (a) hydrogen, (b) loweralkyl, (c) alkenyl, (d) alkynyl, (e) cycloalkyl, (f) cycloalkylalkyl, (g) alkoxycarbonylalkyl, (h) alkoxyalkyl, (i) thioalkoxyalkyl, (j) haloalkyl, (k) aryl, (I) heterocyclic, (m) arylalkyl, (n) aryl-substituted cydoalkylalkyl (o) (heterocyclic)alkyl, (p) heterocyclic-substituted cycloalkylalkyl and (q) aryl, heterocyclic, arylalkyl, aryl-substituted cycloalkylalkyl, (heterocyclic)alkyl or heterocyclic-substituted cycloalkylalkyl wherein the aryl group, the aryl part of the arylalkyl group, the aryl part of the aryl-substituted cycloalkylalkyl group, the heterocyclic group, the heterocyclic part of the (heterocyclic)alkyl group or the heterocyclic part ot the heterocyclic-substituted cycloalkylalkyl group is substituted with -Y-R₃ wherein at each occurrence Y is independently selected from (i) a covalent bond, (ii) -C(O)-, (iii) -CH₂-, (iv) -O-, (v) -S(O)ₘ- wherein m is 0, 1 or 2, (vi) -N(R_{b})- wherein R_{b} is hydrogen or loweralkyl, (vii) -CH₂O-, (viii) -CH₂S(O)ₘ- wherein m is 0, 1 or 2, and (ix) -CH₂N(R_{b})- wherein R_{b} is hydrogen or loweralkyl and at each occurrence R₃ is independently selected from (i) aryl, (ii) arylalkyl, (iii) cycloalkyl, (iv) cycloalkylalkyl, (v) heterocyclic and (vi) (heterocyclic)alkyl and at each occurrence R₂ is independently selected from (i) alkenyl, (ii) alkynyl, (iii) aryl, (iv) arylalkyl, (v) arylalkenyl, (vi) heterocyclic, (vii) (heterocyclic)alkyl and (vi) aryl, heterocyclic, arylalkyl or (heterocyclic)alkyl wherein the aryl group, the aryl part of the arylalkyl group, the heterocyclic group or the heterocyclic part of the (heterocyclic)alkyl group is substituted with -Z-R₄ wherein at each occurrence Z is independently selected from (i) a covalent bond, (ii) -C(O)-, (iii) -CH₂-, (iv) -O-, (v) -S(O)ₚ- wherein p is 0, 1 or 2, (vi) -N(R_{c})- wherein R_{c} is hydrogen or loweralkyl, (vii) -CH₂O-, (viii) -CH₂S(O)ₚ- wherein p is 0, 1 or 2 and (ix) -CH₂N(R_{c})- wherein R_{c} is hydrogen or loweralkyl and at each occurrence R₄ is independently selected from (i) aryl, (ii) arylalkyl, (iii) cycloalkyl, (iv) cycloalkylalkyl, (v) heterocyclic and (vi) (heterocyclic)alkyl and
   (2) -(CH₂)_{q}-N(R₁)(R₂) wherein q is 0, 1 or 2 and at each occurrence R₁ and R₂ are independently defined as above; and
B₁ and B₂ are independently selected from
   (1) -CH₂OH,
   (2) -CH=NOH,
   (3) -W-R₅ wherein at each occurrence W is independently selected from (a) a covalent bond, (b) alkylene, (c) alkenylene, (d) -C(O)NH- and (e) -NHC(O)NH- and R₅ is independently selected from
      (a) 5-tetrazolyl,
      (b) 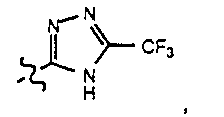
      (c) 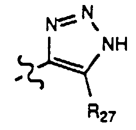 wherein R₂₇ is -CN, -NO₂, or -CO₂R₂₈ wherein R₂₈ is hydrogen, aryl or loweralkyl,
      (d) 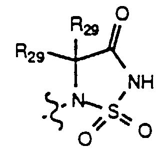 wherein at each occurrence R₂₉ is selected from hydrogen and loweralkyl,
      (e) 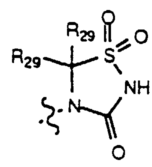 wherein R₂₉ is as defined above,
      (f) 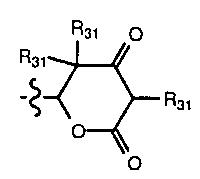 wherein at each occurrence R₃₁ is selected from hydrogen, loweralkyl, alkenyl, alkoxyalkyl and benzyl,
      (g) 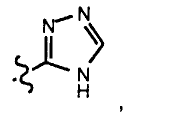
      (h) 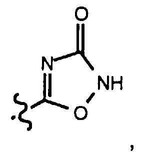
      (i) 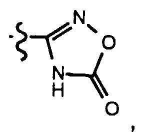
      (j) 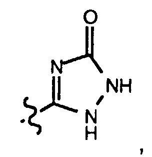
      (k) 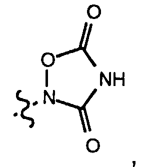
      (l) 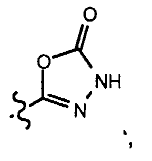
      (m) 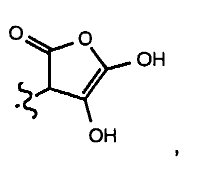 and
      (n) 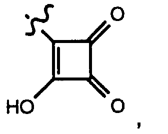
   (4) -Q-C(O)R₆ wherein at each occurrence Q is independently selected from (a) a covalent bond, (b) alkylene, (c) alkenylene, (d) -CH(OH)- and (e) -NHC(O)(CH₂)ᵣ- wherein r is 0 to 4 and at each occurrence R₆ is independently selected from (a) -OR₇ wherein R₇ is hydrogen or a carboxy-protecting group, (b) -NH₂, (c) -NHOH, (d) -NHSO₂CF₃ (e) an alpha-amino acid or a beta-amino acid which is bonded via the alpha- or beta-amino group and (f) a di-, tri- or tetra-peptide which is bonded via the amino terminal amino group,
   (5) -CH₂-N(OH)-C(O)-R₂₅ wherein R₂₅ is hydrogen, methyl or trifluoromethyl, and
   (6) -C(O)-NH-S(O)₂-R₂₆ wherein R₂₆ is aryl, heterocyclic, arylalkyl, (heterocyclic)alkyl, C₃-C₇-cycloalkyl, C₁-C₈-alkyl or perfluoro-C₁-C₄-alkyl,
or a pharmaceutically acceptable salt thereof.

Preferred compounds of the invention are compounds of formula (II): 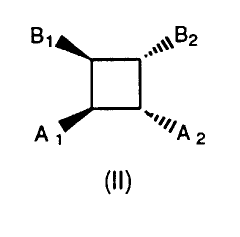 wherein A₁, A₂, B₁ and B₂ are defined as above;
or a pharmaceutically acceptable salt thereof.

Preferred compounds of the invention are compounds of formula (I) or (II) wherein A₁ and A₂ are independently selected from -C(O)-G wherein G is defined as above and B₁ and B₂ are independently selected from (a) -W-R₅ wherein W is a covalent bond or alkylene and R₅ is 5-tetrazolyl and (b) -Q-C(O)-R₆ wherein at each occurrence Q is independently selected from a covalent bond and alkylene and at each occurrence R₆ is independently selected from (1) -OR₇ wherein R₇ is hydrogen or a carboxy-protecting group, (2) an alpha-amino acid or a beta-amino acid which is bonded via the alpha- or beta-amino group and (3) a di-, tri- or tetra-peptide which is bonded via the amino terminal amino group.

More preferred compounds of the invention are compounds of formula (I) or (II) wherein A₁ and A₂ are independently selected from -C(O)-G wherein G is -N(R₁)(R₂) wherein R₁ and R₂ are as defined above and B₁ and B₂ are independently selected from (a) -W-R₅ wherein W is a covalent bond or alkylene and R₅ is 5-tetrazolyl and (b) -Q-C(O)-R₆ wherein at each occurrence Q is independently selected from a covalent bond and alkylene and at each occurrence R₆ is independently selected from (1) -OR₇ wherein R₇ is hydrogen or a carboxy-protecting group, (2) an alpha-amino acid or a beta-amino acid which is bonded via the alpha- or beta-amino group and (3) a di-, tri- or tetra-peptide which is bonded via the amino terminal amino group.

Even more preferred compounds of the invention are compounds of formula (I) or (II) wherein A₁ and A₂ are independently selected from -C(O)-G wherein G is -N(R₁)(R₂) wherein at each occurrence R₁ is independently selected from (a) hydrogen, (b) loweralkyl, (c) cycloalkyl, (d) cycloalkylalkyl, (e) alkoxyalkyl, (f) thioalkoxyalkyl, (g) aryl, (h) heterocyclic, (i) arylalkyl, (j) (heterocyclic)alkyl, (k) aryl-substituted cycloalkylalkyl, (I) heterocyclic-substituted cycloalkylalkyl and (m) aryl, heterocyclic, arylalkyl, (heterocyclic)alkyl, aryl-substituted cycloalkylalkyl or heterocyclic-substituted cycloalkylalkyl wherein the aryl group, the aryl part of the arylalkyl group, the aryl part of the aryl-substituted cycloalkylalkyl group, the heterocyclic group, the heterocyclic part of the (heterocyclic)alkyl group or the heterocyclic part of the heterocyclic-substituted cycloalkylalkyl group is substituted with -Y-R₃ wherein at each occurrence Y is independently selected from (i) -O-, (ii) -S(O)ₘ- wherein m is 0, 1 or 2 and (iii) -N(R_{b})- wherein R_{b} is hydrogen or loweralkyl and at each occurrence R₃ is independently selected from (i) aryl, (ii) arylalkyl, (iii) cycloalkyl, (iv) cycloalkylalkyl, (v) heterocyclic and (vi) (heterocyclic)alkyl and at each occurrence R₂ is independently selected from aryl, heterocyclic, arylalkyl and (heterocyclic)alkyl wherein the aryl group, aryl part of the arylalkyl group, heterocyclic group or heterocyclic part of the (heterocyclic)alkyl group is substituted with -Z-R₄ wherein at each occurrence Z is independently selected from (i) -O-, (ii) -S(O)ₚ- wherein p is 0, 1 or 2 and (iii) -N(R_{c})- wherein R_{c} is hydrogen or loweralkyl and at each occurrence R₄ is independently selected from (i) aryl, (ii) arylalkyl, (iii) cycloalkyl, (iv) cycloalkylalkyl, (v) heterocyclic and (vi) (heterocyclic)alkyl and B₁ and B₂ are independently selected from (a) -W-R₅ wherein W is a covalent bond or alkylene and R₅ is 5-tetrazolyl and (b) -Q-C(O)-R₆ wherein at each occurrence Q is independently selected from a covalent bond and alkylene and at each occurrence R₆ is independently selected from (1) -OR₇ wherein R₇ is hydrogen or a carboxy-protecting group, (2) an alpha-amino acid or a beta-amino acid which is bonded via the alpha- or beta-amino group and (3) a di-, tri- or tetra-peptide which is bonded via the amino terminal amino group.

Yet even more preferred compounds of the invention are compounds of formula (I) or (II) wherein A₁ and A₂ are independently selected from -C(O)-G wherein G is -N(R₁)(R₂) wherein at each occurrence R₁ is independently selected from (a) loweralkyl, (b) cycloalkyl and (c) cycloalkylalkyl and at each occurrence R₂ is independently selected from aryl and arylalkyl wherein the aryl group or aryl part of the arylalkyl group is substituted with -Z-R₄ wherein at each occurrence Z is independently selected from (i) -O- and (ii) -S- and at each occurrence R₄ is independently selected from (i) aryl, (ii) arylalkyl, (iii) cycloalkyl, (iv) cycloalkylalkyl, (v) heterocyclic and (vi) (heterocyclic)alkyl and B₁ and B₂ are independently selected from -Q-C(O)-R₆ and -W-R₅ wherein at each occurrence Q and W are independently selected from a covalent bond and alkylene, R₆ is -OR₇ wherein R₇ is hydrogen or a carboxy-protecting group and R₅ is 5-tetrazolyl.

Most preferred compounds of the invention are compounds of formula (I) or (II) wherein A₁ and A₂ are independently selected from -C(O)-G wherein G is -N(R₁)(R₂) wherein at each occurrence R₁ is independently selected from (a) loweralkyl, (b) cycloalkyl and (c) cycloalkylalkyl and at each occurrence R₂ is independently selected from phenyl and benzyl wherein the phenyl group or the phenyl ring of the benzyl group is substituted with -Z-R₄ wherein at each occurrence Z is independently selected from (i) -O- and (ii) -S- and at each occurrence R₄ is independently selected from (i) aryl, (ii) arylalkyl, (iii) heterocyclic and (iv) (heterocyclic)alkyl and B₁ and B₂ are independently selected from -Q-C(O)-R₆ and -W-R₅ wherein at each occurrence Q and W are independently selected from a covalent bond and alkylene, R₆ is -OR₇ wherein R₇ is hydrogen or a carboxy-protecting group and R₅ is 5-tetrazolyl.

Most highly preferred compounds of the invention are compounds of formula (I) or (II) wherein A₁ and A₂ are independently selected from -C(O)-G wherein G is -N(R₁)(R₂) wherein at each occurrence R₁ is independently selected from (a) loweralkyl, (b) cycloalkyl and (c) cycloalkylalkyl and R₂ is benzyl wherein the phenyl ring of the benzyl group is substituted with -Z-R₄ wherein at each occurrence Z is independently selected from (i) -O- and (ii) -S- and R₄ is aryl and B₁ and B₂ are independently selected from -Q-C(O)-R₆ and -W-R₅ wherein at each occurrence Q and W are independently selected from a covalent bond and alkylene, R₆ is -OR₇ wherein R₇ is hydrogen or a carboxy-protecting group and R₅ is 5-tetrazolyl.

Other most highly preferred compounds of the invention are compounds of formula (I) or (II) wherein A₁ and A₂ are independently selected from -C(O)-G wherein G is -N(R₁)(R₂) wherein at each occurrence R₁ is (a) loweralkyl, (b) cycloalkyl and (c) cycloalkylalkyl and R₂ is benzyl wherein the phenyl ring of the benzyl group is substituted with -Z-R₄ wherein at each occurrence Z is independently selected from (i) -O- and (ii) -S- and R₄ is heterocyclic and B₁ and B₂ are independently selected from -Q-C(O)-R₆ and -W-R₅ wherein at each occurrence Q and W are independently selected from a covalent bond and alkylene, R₆ is -OR₇ wherein R₇ is hydrogen or a carboxy-protecting group and R₅ is 5-tetrazolyl.

Another aspect of this invention relates to the use of compounds of the formula: 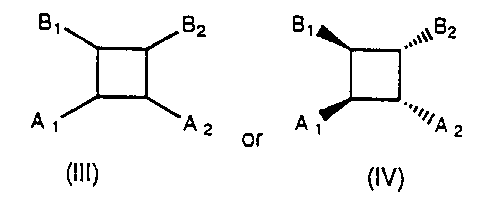 wherein
A₁ and A₂ are independently selected from -X-C(O)-G or -X-C(S)-G wherein at each occurrence X is independently selected from (a) a covalent bond, (b) -CH₂-, (c) -O-, (d) -S- and (e) -N(Rₐ)- wherein Rₐ is hydrogen, loweralkyl, cycloalkyl or cycloalkylalkyl and at each occurrence G is independently selected from -R₂, -N(R₁)(R₂), -OR₂ and -SR₂ wherein at each occurrence R₁ is independently selected from (a) hydrogen, (b) loweralkyl, (c) alkenyl, (d) alkynyl, (e) cycloalkyl, (f) cycloalkylalkyl, (g) alkoxycarbonylalkyl, (h) alkoxyalkyl, (i) thioalkoxyalkyl, (j) haloalkyl, (k) aryl, (I)heterocyclic,(m)arylalkyl, (n)aryl-substituted cycloalkylalkyl, (o) (heterocyclic)alkyl, (p) heterocyclic-substituted cycloalkylalkyl and (q) aryl, heterocyclic, arylalkyl, aryl-substituted cycloalkylalkyl, (heterocyclic)alkyl or heterocyclic-substituted cycloalkylalkyl wherein the aryl group, the aryl part of the arylalkyl group, the aryl part of the aryl-substituted cycloalkylalkyl group, the heterocyclic group, the heterocyclic part of the (heterocyclic)alkyl group or the heterocyclic part of the heterocyclic-substituted cycloalkylalkyl group is substituted with -Y-R₃ wherein at each occurrence Y is independently selected from (i) a covalent bond, (ii) -C(O)-, (iii) -CH₂-, (iv) -O-, (v) -S(O)ₘ- wherein m is 0, 1 or 2, (vi) -N(R_{b})- wherein R_{b} is hydrogen or loweralkyl, (vii) -CH₂O-, (viii) -CH₂S(O)ₘ- wherein m is 0, 1 or 2, and (ix) -CH₂N(R_{b})- wherein R_{b} is hydrogen or loweralkyl and at each occurrence R₃ is independently selected from (i) aryl, (ii) arylalkyl, (iii) cycloalkyl, (iv) cycloalkylalkyl, (v) heterocyclic and (vi) (heterocyclic)alkyl and at each occurrence R₂ is independently selected from (i) alkenyl, (ii) alkynyl, (iii) aryl, (iv) arylalkyl, (v) arylalkenyl, (vi) heterocyclic, (vii) (heterocyclic)alkyl and (vi) aryl, heterocyclic, arylalkyl or (heterocyclic)alkyl wherein the aryl group, the aryl part of the arylalkyl group, the heterocyclic group or the heterocyclic part of the (heterocyclic)alkyl group is substituted with -Z-R₄ wherein at each occurrence Z is independently selected from (i) a covalent bond, (ii) -C(O)-, (iii) -CH₂-, (iv) -O-, (v) -S(O)ₚ- wherein p is 0, 1 or 2, (vi) -N(R_{c})- wherein R_{c} is hydrogen or loweralkyl, (vii) -CH₂O-, (viii) -CH₂S(O)ₚ- wherein p is 0, 1 or 2 and (ix) -CH₂N(R_{c})- wherein R_{c} is hydrogen or loweralkyl and at each occurrence R₄ is independently selected from (i) aryl, (ii) arylalkyl, (iii) cycloalkyl, (iv) cycloalkylalkyl, (v) heterocyclic and (vi) (heterocyclic)alky; and
B₁ and B₂ are independently selected from
   (1) -CH₂OH,
   (2) -CH=NOH,
   (3) -W-R₅ wherein at each occurrence W is independently selected from (a) a covalent bond, (b) alkylene, (c) alkenylene, (d) -C(O)NH- and (e) -NHC(O)NH- and R₅ is independently selected from
      (a) 5-tetrazolyl,
      (b) 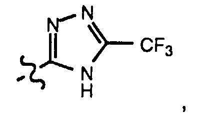
      (c) 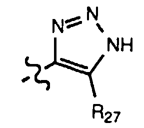 wherein R₂₇ is -CN, -NO₂, or -CO₂R₂₈ wherein R₂₈ is hydrogen, aryl or loweralkyl,
      (d) 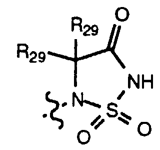 wherein at each occurrence R₂₉ is selected from hydrogen and loweralkyl,
      (e) 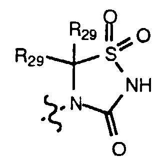 wherein R₂₉ is as defined above,
      (f) 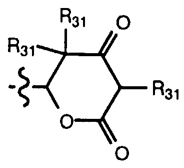 wherein at each occurrence R₃₁ is selected from hydrogen, loweralkyl, alkenyl, alkoxyalkyl and benzyl,
      (g) 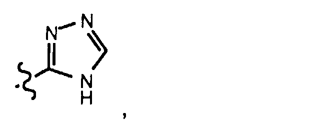
      (h) 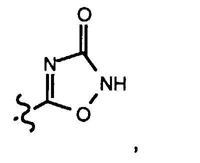
      (i) 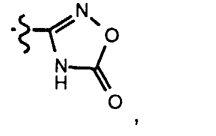
      (j) 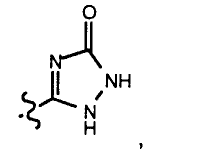
      (k) 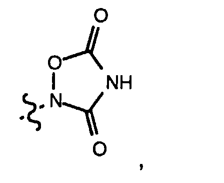
      (l) 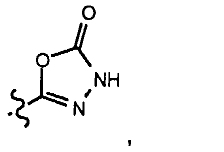
      (m) 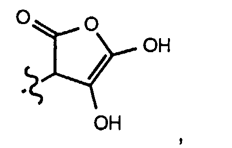 and
      (n) 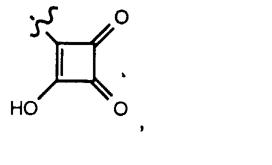
   (4) -Q-C(O)R₆ wherein at each occurrence Q is independently selected from (a) a covalent bond, (b) alkylene, (c) alkenylene, (d) -CH(OH)- and (e) -NHC(O)(CH₂)ᵣ- wherein r is 0 to 4 and at each occurrence R₆ is independently selected from (a) -OR₇ wherein R₇ is hydrogen or a carboxy-protecting group, (b) -NH₂, (c) -NHOH, (d) -NHSO₂CF₃ (e) an alpha-amino acid or a beta-amino acid which is bonded via the alpha- or beta-amino group and (f) a di-, tri- or tetra-peptide which is bonded via the amino terminal amino group,
   (5) -CH₂-N(OH)-C(O)-R₂₅ wherein R₂₅ is hydrogen, methyl or trifluoromethyl, and
   (6) -C(O)-NH-S(O)₂-R₂₆ wherein R₂₆ is aryl, heterocyclic, arylalkyl, (heterocydic)alkyl, C₃-C₇-cycloalkyl, C₁-C₈-alkyl or perfluoro-C₁-C₄-alkyl,
or a pharmaceutically acceptable salt thereof;
as inhibitors of protein farnesyltransferase.

Preferred inhibitors of protein famesyltransferase are compounds of formula (III) or (IV) wherein
A₁ and A₂ are independently -C(O)-NR₁R₂ wherein at each occurrence R₁ is independently selected from (k) aryl, (I) heterocyclic, (m) arylalkyl, (n) aryl-substituted cycloalkylalkyl, (o) (heterocyclic)alkyl, (p) heterocyclic-substituted cycloalkylalkyl and (q) aryl, heterocyclic, arylalkyl, aryl-substituted cycloalkylalkyl, (heterocyclic)alkyl or heterocyclic-substituted cycloalkylalkyl wherein the aryl group, the aryl part of the arylalkyl group, the aryl part of the aryl-substituted cycloalkylalkyl group, the heterocyclic group, the heterocyclic part of the (heterocyclic)alkyl group or the heterocyclic part of the heterocyclic-substituted cycloalkylalkyl group is substituted with -Y-R₃ wherein at each occurrence Y is independently selected from (i) a covalent bond, (ii) -C(O)-, (iii) -CH₂-, (iv) -O-, (v) -S(O)ₘ- wherein m is 0, 1 or 2, (vi) -N(R_{b})- wherein R_{b} is hydrogen or loweralkyl, (vii) -CH₂O-, (viii) -CH₂S(O)ₘ- wherein m is 0, 1 or 2, and (ix) -CH₂N(R_{b})- wherein R_{b} is hydrogen or loweralkyl and at each occurrence R₃ is independently selected from (i) aryl, (ii) arylalkyl, (iii) cycloalkyl, (iv) cycloalkylalkyl, (v) heterocyclic and (vi) (heterocyclic)alkyl, and at each occurrence R₂ is independently selected from arylalkyl and (heterocyclic)alkyl; and
B₁ and B₂ are independently selected from
   (1) -W-R₅ wherein at each occurrence W is independently selected from (a) a covalent bond, (b) alkylene, (c) alkenylene, (d) -C(O)NH- and (e) -NHC(O)NH- and R₅ is 5-tetrazolyl,
   (2) -Q-C(O)R₆ wherein at each occurrence Q is independently selected from (a) a covalent bond, (b) alkylene, (c) alkenylene, (d) -CH(OH)- and (e) -NHC(O)(CH₂)ᵣ- wherein r is 0 to 4 and at each occurrence R₆ is independently selected from (a) -OR₇ wherein R₇ is hydrogen or a carboxy-protecting group, (b) -NH₂, (c) -NHOH, (d) -NHSO₂CF₃ (e) an alpha-amino acid or a beta-amino acid which is bonded via the alpha- or beta-amino group and (f) a di-, tri- or tetra-peptide which is bonded via the amino terminal amino group, and
   (3) -C(O)-NH-S(O)₂-R₂₆ wherein R₂₆ is aryl, heterocyclic, arylalkyl, (heterocyclic)alkyl, C₃-C₇-cycloalkyl, C₁-C₈-alkyl or perfluoro-C₁-C₄-alkyl,
or a pharmaceutically acceptable salt thereof.

The present invention also relates to processes for preparing the compounds of formula (I), (II), (III) or (IV) and to the synthetic intermediates useful in such processes.

In a further aspect of the present invention are disclosed pharmaceutical compositions which comprise a compound of the present invention in combination with a pharmaceutically acceptable carrier.

In yet another aspect of the present invention are disclosed pharmaceutical compositions which comprise a compound of the present invention in combination with another antihyperlipoproteinemic agent and/or with one or more other serum cholesterol lowering agents or HMG CoA reductase inhibitors and a pharmaceutically acceptable carrier.

In yet another aspect of the present invention are disclosed pharmaceutical compositions which comprise a compound of the present invention in combination with another chemotherapeutic agent and a pharmaceutically acceptable carrier.

In yet another aspect of the present invention is disclosed a method of inhibiting squalene synthase in a human or lower mammal, comprising administering to the patient a therapeutically effective amount of a compound of the invention.

In yet another aspect of the present invention is disclosed a method of inhibiting or treating atherosclerosis or inhibiting or treating hyperlipidemia which would inhibit the development of atherosclerosis in a human or lower mammal, comprising administering to the patient a therapeutically effective amount of a compound of the invention alone or in combination with another cardiovascular agent.

In yet another aspect of the present invention is disclosed a method of inhibiting protein farnesyltransferase in a human or lower mammal, comprising administering to the patient a therapeutically effective amount of a compound of the invention.

In yet another aspect of the present invention is disclosed a method of treating cancer in a human or lower mammal, comprising administering to the patient a therapeutically effective amount of a compound of the invention alone or in combination with another chemotherapeutic agent

Also disclosed is a method of treating fungal infections in a human or lower mammal, comprising administering to the patient a therapeutically effective amount of a compound of the invention.

The compounds of the invention comprise asymmetrically substituted carbon atoms. As a result, all stereoisomers of the compounds of the invention are meant to be included in the invention, including racemic mixtures, mixtures of diastereomers, as well as single diastereomers of the compounds of the invention. The terms "S" and "R" configuration, as used herein, are as defined by the IUPAC 1974 Recommendations for Section E, Fundamental Stereochemistry, Pure Appl. Chem. (1976) 45, 13-30.

The terms α and β are employed to describe relative orientation for ring substituents on cyclic compounds, i.e., substituted cyclobutanes in the present invention. The α-side of the reference plane (the plane formed by the cyclobutane ring) is that side on which the highest ranking substituent (according to the Cahn-lngold-Prelog Sequence Rule) lies at the lowest-numbered stereogenic carbon atom. All substituents lying on the same side of the reference plane as the highest-ranking substituent are assigned an α descriptor. Those substituents lying on the opposite side of the reference plane are assigned a β descriptor. It should be noted that this usage does not describe absolute configuration. The terms α and β configuration, as used herein, are as defined by the Chemical Abstracts Index Guide-Appendix IV (1987) ¶ 203.

The term "α-amino acid" or "alpha-amino acid" refers to an α-amino acid selected from the group consisting of alanine, arginine, asparagine, aspartic acid, cysteine, glutamine, glutamic acid, glycine, histidine, isoleucine, leucine, lysine, methionine, norleucine, ornithine, phenylalanine, proline, sarcosine, serine, threonine, tryptophan, tyrosine and valine. The stereochemistry at the asymmetric center can be of the D- or L- configuration.

The term "β-amino acid" or "beta-amino acid" refers to an amino acid wherein the amino group is β to the carboxylic acid functionality. Examples of β-amino acids include β-alanine, β-phenylalanine.

The term "dipeptide" as used herein refers to AA₁-AA₂ wherein AA₁ and AA₂ are independently selected from *α*- and β-amino acids as described above coupled together by an amide bond (-C(O)-NH-) between the carboxy terminus of AA₁ and the amino terminus of AA₂. Examples of dipeptides include H-Glycyl-Alanine-OH, H-Glycyl-β-Alanine-OH, H-Leucyl-Glycine-OH.

The term "tripeptide" as used herein refers to AA₁-AA₂-AA₃ wherein AA₁, AA₂ and AA₃ are independently selected from α- and β-amino acids as described above coupled together by amide bonds (-C(O)-NH-) between the carboxy terminus of AA₁ and the amino terminus of AA₂ and the carboxy terminus of AA₂ and the amino terminus of AA₃. Examples of tripeptides include H-Glycyl-Alanyl-Leucine-OH, H-Glycyl-β-Alanyl-Sarcosine-OH, H-Leucyl-Glycyl-Alanine-OH.

The term "tetrapeptide" as used herein refers to AA₁-AA₂-AA₃-AA₄ wherein AA₁, AA₂, AA₃ and AA₄ are independently selected from α- and β-amino acids as described above coupled together by amide bonds (-C(O)-NH-) between the carboxy terminus of AA₁ and the amino terminus of AA₂, the carboxy terminus of AA₂ and the amino terminus of AA₃, and the carboxy terminus of AA₃ and the amino terminus of AA₄.

The term "carboxy protecting group" as used herein refers to a carboxylic acid protecting ester group employed to block or protect the carboxylic acid functionality while the reactions involving other functional sites of the compound are carried out. Carboxy-protecting groups are disclosed in Greene, "Protective Groups in Organic Synthesis" pp. 152-186 (1981). In addition, a carboxy-protecting group can be used as a prodrug whereby the carboxy-protecting group can be readily cleaved *in vivo,* for example by enzymatic hydrolysis, to release the biologically active parent. T. Higuchi and V. Stella provide a thorough discussion of the prodrug concept in "Pro-drugs as Novel Delivery Systems", Vol 14 of the A.C.S. Symposium Series, American Chemical Society (1975). Such carboxy-protecting groups are well known to those skilled in the art, having been extensively used in the protection of carboxyl groups in the penicillin and cephalosporin fields, as described in U.S. Pat. No. 3,840,556 and 3,719,667. Examples of esters useful as prodrugs for compounds containing carboxyl groups can be found on pages 14-21 of "Bioreversible Carriers in Drug Design: Theory and Application", edited by E.B. Roche, Pergamon Press, New York (1987). Representative carboxy-protecting groups are C₁ to C₈ loweralkyl (e.g., methyl, ethyl or tertiary butyl); benzyl and substituted derivatives thereof such as alkoxybenzyl or nitrobenzyl groups; dialkylaminoalkyl (e.g., dimethylaminoethyl); alkanoyloxyalkyl groups such as pivaloyloxymethyl or propionyloxymethyl; aroyloxyalkyl, such as benzoyloxyethyl; alkoxycarbonylalkyl, such as methoxycarbonylmethyl, cyclohexyloxycarbonylmethyl; alkoxycarbonyloxyalkyl, such as t-butyloxycarbonyloxymethyl; alkoxycarbonylaminoalkyl, such as t-butyloxycarbonylaminomethyl; alkylaminocarbonylaminoalkyl, such as methylaminocarbonylaminomethyl; alkanoylaminoalkyl, such as acetylaminomethyl; heterocycliccarbonyloxyalkyl, such as 4-methylpiperazinylcarbonyloxymethyl; dialkylaminocarbonylalkyl, such as dimethylaminocarbonylmethyl; (5-(loweralkyl)-2-oxo-1,3-dioxolen-4-yl)alkyl, such as (5-t-butyl-2-oxo-1,3-dioxolen-4-yl)methyl; and (5-phenyl-2-oxo-1,3-dioxolen-4-yl)alkyl, such as (5-phenyl-2-oxo-1,3-dioxolen-4-yl)methyl.

The term "N-protecting group" or "N-protected" as used herein refers to those groups intended to protect the N-terminus of an amino acid or peptide or to protect an amino group against undersirable reactions during synthetic procedures. Commonly used N-protecting groups are disclosed in Greene, "Protective Groups In Organic Synthesis," (John Wiley & Sons, New York (1981)). N-protecting groups comprise acyl groups such as formyl, acetyl, propionyl, pivaloyl, t-butylacetyl, 2-chloroacetyl, 2-bromoacetyl, trifluoroacetyl, trichloroacetyl, phthalyl, o-nitrophenoxyacetyl, α-chlorobutyryl, benzoyl, 4-chlorobenzoyl, 4-bromobenzoyl, 4-nitrobenzoyl; sulfonyl groups such as benzenesulfonyl, p-toluenesulfonyl; carbamate forming groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyloxycarbonyl, 2,4-dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydryloxycarbonyl, t-butyloxycarbonyl, diisopropylmethoxycarbonyl, isopropyloxycarbonyl, ethoxycarbonyl, methoxycarbonyl, allyloxycarbonyl, 2,2,2,-trichloroethoxycarbonyl, phenoxycarbonyl, 4-nitrophenoxycarbonyl, fluorenyl-9-methoxycarbonyl, cyclopentyloxycarbonyl, adamantyloxycarbonyl, cyclohexyloxycarbonyl, phenylthiocarbonyl; alkyl groups such as benzyl, triphenylmethyl, benzyloxymethyl; and silyl groups such as trimethylsilyl. Preferred N-protecting groups are formyl, acetyl, benzoyl, pivaloyl, t-butylacetyl, phenylsulfonyl, benzyl, t-butyloxycarbonyl (Boc) and benzyloxycarbonyl (Cbz).

The term "loweralkyl" as used herein refers to branched or straight chain alkyl groups comprising one to ten carbon atoms, including methyl, ethyl, propyl, isopropyl, n-butyl, t-butyl, neopentyl.

The term "alkoxy" as used herein refers to RO- wherein R is loweralkyl as defined above. Representative examples of alkoxy groups include methoxy, ethoxy, t-butoxy.

The term "alkoxyalkoxy" as used herein refers to R₈₀O-R₈₁O- wherein R₈₀ is loweralkyl as defined above and R₈₁ is an alkylene group. Representative examples of alkoxyalkoxy groups include methoxymethoxy, ethoxymethoxy, t-butoxymethoxy.

The term "alkoxyalkyl" as used herein refers to an alkoxy group as previously defined appended to an alkyl group as previously defined. Examples of alkoxyalkyl include, but are not limited to, methoxymethyl, methoxyethyl, isopropoxymethyl.

The term "alkoxycarbonyl" as used herein refers to an alkoxy group as previously defined appended to the parent molecular moiety through a carbonyl group. Examples of alkoxycarbonyl include methoxycarbonyl, ethoxycarbonyl, isopropoxycarbonyl.

The term "alkoxycarbonylalkyl" as used herein refers to an alkoxylcarbonyl group as previously defined appended to a loweralkyl group. Examples of alkoxycarbonylalkyl include methoxycarbonylmethyl, 2-ethoxycarbonylethyl.

The term "alkylene" denotes a divalent group derived from a straight or branched chain saturated hydrocarbon having from 1 to 10 carbon atoms by the removal of two hydrogen atoms, for example methylene, 1,2-ethylene, 1,1-ethylene, 1,3-propylene, 2,2-dimethylpropylene.

The term "alkenyl" as used herein refers to a branched or straight hydrocarbon chain comprising two to twenty carbon atoms which also comprises one or more carbon-carbon double bonds. Representative alkenyl groups include 2-propenyl (i.e., allyl), 3-methyl-2-butenyl, 3,7-dimethyl-2,6-octadienyl, 4,8-dimethyl-3,7-nonadienyl, 3,7,11-trimethyl-2,6,10-dodecatrienyl.

The term "alkenylene" denotes a divalent group derived from a straight or branched chain hydrocarbon containing from 2 to 10 carbon atoms and also containing at least one carbon-carbon double bond. Examples of alkenylene include -CH=CH-, -CH₂CH=CH-, -C(CH₃)=CH-, -CH₂CH=CHCH₂-.

The term "alkynyl" as used herein refers to a branched or straight hydrocarbon chain comprising two to twenty carbon atoms which also comprises one or more carbon-carbon triple bonds. Representative alkynyl groups include ethynyl, 2-propynyl (propargyl), 1-propynyl.

The term "amino" as used herein refers to -NH₂.

The term "alkylamino" as used herein refers to R₅₁NH- wherein R₅₁ is a loweralkyl group, for example, methylamino, ethylamino, butylamino.

The term "dialkylamino" as used herein refers to R₅₆R₅₇N- wherein R₅₆ and R₅₇ are independently selected from loweralkyl, for example dimethylamino, diethylamino, methyl propylamino.

The term "aryl" as used herein refers to a mono- or bicyclic carbocyclic ring system having one or two aromatic rings including, but not limited to, phenyl, naphthyl, tetrahydronaphthyl, indanyl, indenyl. Aryl groups (including bicyclic aryl groups) can be unsubstituted or substituted with one, two or three substituents independently selected from loweralkyl, haloalkyl, alkoxy, thioalkoxy, amino, alkylamino, dialkylamino, hydroxy, halo, mercapto, nitro, carboxaldehyde, carboxy, alkoxycarbonyl and carboxamide. In addition, substituted aryl groups include tetrafluorophenyl and pentafluorophenyl.

The term "arylalkyl" as used herein refers to a loweralkyl radical to which is appended an aryl group. Representative arylalkyl groups include benzyl, phenylethyl, hydroxybenzyl, fluorobenzyl, fluorophenylethyl.

The term "arylalkenyl" as used herein refers to an aryl group as previously defined appended to an alkenyl group as previously defined. Examples of arylalkenyl include styryl (*i.e.,* 2-phenylethenyl), 2-(1-naphthyl)ethenyl.

The term "aryl-substituted cycloalkylalkyl" as used herein refers to a cycloalkylalkyl radical in which the alkyl portion of the radical is substituted with an aryl group. Examples of aryl-substituted cycloalkylalkyl include α-(cyclopropylmethyl)benzyl, α-(cyclobutylmethyl)benzyl.

The term "carboxaldehyde" as used herein refers to the group -C(O)H.

The term "carboxamide" as used herein refers to the group -C(O)NH₂.

The term "cycloalkyl" as used herein refers to an alicyclic group comprising from 3 to 7 carbon atoms including, but not limited to, cyclopropyl, cyclobutyl, cyclopentyl, cyclohexyl.

The term "cycloalkylalkyl" as used herein refers to a loweralkyl radical to which is appended a cycloalkyl group. Representative examples of cycloalkylalkyl include cyclopropylmethyl, cyclohexylmethyl, 2-(cyclopropyl)ethyl.

The term "1,2,3,4-cyclobutanetetracarboxylic dianhydride" as used herein refers to the (1.2/3.4) compound wherein the two anhydride rings are *trans* (i.e., on opposite sides of the plane formed by the cyclobutane ring) to one another. The absolute stereochemistry is as 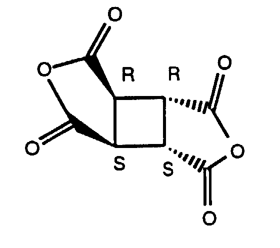 shown.

The term "halogen" or "halo" as used herein refers to I, Br, Cl or F.

The term "haloalkyl" as used herein refers to a lower alkyl radical, as defined above, bearing at least one halogen substituent, for example, chloromethyl, fluoroethyl or trifluoromethyl.

The term "heterocyclic ring" or "heterocyclic" or "heterocycle" as used herein refers to any 3- or 4-membered ring containing a heteroatom selected from oxygen, nitrogen and sulfur; or a 5-, 6- or 7-membered ring containing one, two or three nitrogen atoms; one oxygen atom; one sulfur atom; one nitrogen and one sulfur atom; one nitrogen and one oxygen atom; two oxygen atoms in non-adjacent positions; one oxygen and one sulfur atom in non-adjacent positions; or two sulfur atoms in non-adjacent positions. The 5-membered ring has 0-2 double bonds and the 6- and 7-membered rings have 0-3 double bonds. The nitrogen heteroatoms can be optionally quaternized. The term "heterocyclic" also includes bicyclic groups in which any of the above heterocyclic rings is fused to a benzene ring or a cyclohexane ring or another heterocyclic ring (for example, indolyl, quinolyl, isoquinolyl, tetrahydroquinolyl, benzofuryl or benzothienyl). Heterocyclics include: azetidinyl, pyrrolyl, pyrrolinyl, pyrrolidinyl, pyrazolyl, pyrazolinyl, pyrazolidinyl, imidazolyl, imidazolinyl, imidazolidinyl, pyridyl, piperidinyl, homopiperidinyl, pyrazinyl, piperazinyl, pyrimidinyl, pyridazinyl, oxazolyl, oxazolidinyl, isoxazolyl, isoxazolidinyl, morpholinyl, thiazolyl, thiazolidinyl, isothiazolyl, isothiazolidinyl, indolyl, quinolinyl, isoquinolinyl, benzimidazolyl, benzothiazolyl, benzoxazolyl, furyl, thienyl, thiazolidinyl, isothiazolyl, triazolyl, tetrazolyl, isoxazolyl, oxadiazolyl, thiadiazolyl, pyrrolyl, pyrimidyl and benzothienyl. Heterocyclics also include compounds of the formula 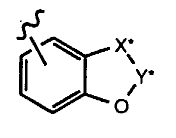 where X* is -CH₂- or -O- and Y* is -C(O)- or [-C(R")₂-]ᵥ where R" is hydrogen or C₁-C₄-alkyl and v is 1, 2 or 3 such as 1,3-benzodioxolyl, 1,4-benzodioxanyl.

Heterocyclics can be unsubstituted or monosubstituted or disubstituted with substituents independently selected from hydroxy, halo, oxo (=O), alkylimino (R*N= wherein R* is a loweralkyl group), amino, alkylamino, dialkylamino, alkoxy, alkoxyalkoxy, haloalkyl, cycloalkyl, aryl, arylalkyl, -COOH, -SO₃H and loweralkyl. In addition, nitrogen containing heterocycles can be N-protected.

The term "(heterocyclic)alkyl" as used herein refers to a heterocyclic group as defined above appended to a loweralkyl radical as defined above. Examples of heterocyclic alkyl include 2-pyridylmethyl, 4-pyridylmethyl, 4-quinolinylmethyl.

The term "heterocyclic-substituted cycloalkylalkyl" as used herein refers to a cycloalkylalkyl radical in which the alkyl portion of the radical is substituted with a heterocyclic group. Examples of heterocyclic-substituted cycloalkylalkyl include α-(cyclopropylmethyl)furan-2-ylmethyl, α-(cyclobutylmethyl)thien-2-ylmethyl.

The term "mercapto" as used herein refers to the group -SH.

The term "perfluoro-C₁-C₄-alkyl" as used herein refers to an alkyl radical of 1 to 4 carbon atoms in which all hydrogen atoms have been replaced with fluorine atoms. Examples of perfluoro-C₁-C₄-alkyl include trifluoromethyl, pentafluoroethyl.

The term "tetrazolyl" or "5-tetrazolyl" as used herein refers to a radical of the formula 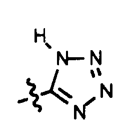 or a tautomer thereof.

The term "thioalkoxy" as used herein refers to R₇₀S- wherein R₇₀ is loweralkyl. Examples of thioalkoxy include, but are not limited to, methylthio, ethylthio.

The term "thioalkoxyalkyl" as used herein refers to a thioalkoxy group as previously defined appended to a loweralkyl group as previously defined. Examples of thioalkoxyalkyl include thiomethoxymethyl, 2-thiomethoxyethyl.

Representative compounds of the invention include:
(1α,2β,3β,4α)-1,2-Di[N-methyl-N-(4-phenoxybenzyl)aminocarbonyl]cyclobutane-3,4-dicarboxylic acid;
(1α,2β,3β,4α)-1,2-Di[N-methyl-N-(4-benzyloxybenzyl)aminocarbonyl]cyclobutane-3,4-dicarboxylic acid;
(1α,2β,3β,4α)-1,2-Di[N-benzyl-N-(4-phenoxybenzyl)aminocarbonyl]cyclobutane-3,4-dicarboxylic acid;
(1α,2β,3β,4α)-1,2-Di-N-ethyl-N-(4-phenoxybenzyl)aminocarbonyl]cyclobutane-3,4-dicarboxylic acid;
(1α,2β,3β,4α)-1,2-Di[N-propyl-N-(4-phenoxybenzyl)aminocarbonyl]cyclobutane-3,4-dicarboxylic acid;
(1α,2β,3β,4α)-1,2-Di[N-methyl-N-(3-phenoxybenzyl)aminocarbonyl]cyclobutane-3,4-dicarboxylic acid;
(1α,2β,3β,4α)-1,2-Di[N,N-di(4-phenoxybenzyl)aminocarbonyl]cyclobutane-3,4-dicarboxylic acid;
(1α,2β,3β,4α)-1,2-Di[N-methyl-N-(4-[4-fluorophenoxy]benzyl)aminocarbonyl]cyclobutane-3,4-dicarboxylic acid;
(1α,2β,3β,4α)-1,2-Di[N-methyl-N-(3-[4-fluorophenoxy]benzyl)aminocarbonyl]cyclobutane-3,4-dicarboxylic acid;
(1α,2β,3β,4α)-1,2-Di[N-methyl-N-biphenylamino-carbonyl]cyclobutane-2,4-dicarboxylic acid;
(1α,2β,3β,4α)-1,2-Di[N-isopropyl-N-biphenylaminocarbonyl]cyclobutane-3,4-dicarboxylic acid;
(1α,2β,3β,4α)-1,2-Di[N-isobutyl-N-(4-phenoxybenzyl)aminocarbonyl]cyclobutane-3,4-dicarboxylic acid;
(1α,2β,3β,4α)-1,2-Di[N-propyl-N-(4-benzylbenzyl)aminocarbonyl]cyclobutane-3,4-dicarboxylic acid;
(1α,2β,3β,4α)-1,2-Di[N-butyl-N-(4-phenoxybenzyl)aminocarbonyl]cyclobutane-3,4-dicarboxylic acid;
(1α,2β,3β,4α)-1,2-Di[N-propargyl-N-(4-phenoxybenzyl)aminocarbonyl]cyclobutane-3,4-dicarboxylic acid;
(1α,2β,3β,4α)-1,2-Di[N-pentyl-N-(4-phenoxybenzyl)aminocarbonyl]cyclobutane-3,4-dicarboxylic acid;
(1α,2β,3β,4α)-1,2-Di[N-allyl-N-(4-phenoxybenzyl)aminocarbonyl]cyclobutane-3,4-dicarboxylic acid;
(1α,2β,3β,4α)-1,2-Di[N-cyclopropyl-N-(4-phenoxybenzyl)aminocarbonyl]cyclobutane-3,4-dicarboxylic acid;
(1α,2β,3β,4α)-1,2-Di[N-cyclohexylmethyl-N-phenoxybenzyl)aminocarbonyl]cyclobutane-3,4- dicarboxylic acid;
(1α,2β,3β,4α)-1,2-Di[N-phenyl-N-phenoxybenzyl)aminocarbonyl]cyclobutane-3,4-dicarboxylic acid;
(1α,2β,3β,4α)-1,2-Di[N-4-methoxybenzyl-N-4-phenoxybenzyl)aminocarbonyl]cyclobutane-3,4-dicarboxylic acid;
(1α,2β,3β,4α)-1,2-Di[N-(*S*)-α-methylbenzyl-N-4-phenoxybenzyl)aminocarbonyl]cyclobutane-3,4- dicarboxylic acid;
(1α,2β,3β,4α)-1,2-Di[N-(R)-α-methylbenzyl-N-4-phenoxybenzyl)aminocarbonyl]cyclobutane-3,4- dicarboxylic acid;
(1α,2β,3β,4α)-1,2-Di[N-benzyl-N-(5-phenyl-2,4-pentadienyl)aminocarbonyl]cyclobutane-3,4-dicarboxylic acid;
(-)-(1α,2β,3β,4α)-1,2-Di[N-propyl-N-(phenoxybenzyl)aminocarbonyl]cyclobutane-3,4-dicarboxylic acid;
(1α,2β,3β,4α)-1,2-Di[N-propyl-N-(2-(4-phenoxyphenyl)ethyl)aminocarbonyl]cyclobutane-3,4-dicarboxylic acid;
(1α,2β,3β,4α)-1,2-Di[N-propyl-N-(4-phenoxyphenyl)aminocarbonyl]cyclobutane-3,4-dicarboxylic acid;
(1α,2β,3β,4α)-1,2-Di[N-(2-methoxyethyl)-N-(4-phenoxybenzyl)aminocarbonyl]cyclobutane-3,4-dicarboxylic acid;
(1α,2β,3β,4α)-1,2-Di[N-(2-methylthioethyl)-N-(4-phenoxybenzyl)aminocarbonyl]cyclobutane-3,4-dicarboxylic acid;
(1α,2β,3β,4α)-1,2-Di[N-(2-ethylthioethyl)-N-(4-phenoxybenzyl)aminocarbonyl]cyclobutane-3,4-dicarboxylic acid;
(1α,2β,3β,4α)-1,2-Di[N-(2-fluoroethyl)-N-(4-phenoxybenzyl)aminocarbonyl]cyclobutane-3,4-dicarboxylic acid;
(1α,2β,3β,4α)-1,2-Di[N-(furan-2-ylmethyl)-N-(4-phenoxybenzyl)aminocarbonyl]cyclobutane-3,4-dicarboxylic acid;
(1α,2β,3β,4α)-1,2-Di[N-(thien-2-ylmethyl)-N-(4-phenoxybenzyl)aminocarbonyl]cyclobutane-3,4-dicarboxylic acid;
(1α,2β,3β,4α)-1,2-Di[N-(2-ethylthioethyl)-N-(4-phenylthiobenzyl)aminocarbonyl]cyclobutane-3,4-dicarboxylic acid;
(1α,2β,3β,4α)-1,2-Di[N-(cyclopropylmethyl)-N-(4-phenoxybenzyl)aminocarbonyl]cyclobutane-3,4-dicarboxylic acid;
(1α,2β,3β,4α)-1,2-Di[N-cyclobutyl-N-(4-phenoxybenzyl)aminocarbonyl]cyclobutane-3,4-dicarboxylic acid;
(1α,2β,3β,4α)-1,2-Di-N-cyclopentyl-N-(4-phenoxybenzyl)aminocarbonyl]cyclobutane-3,4-dicarboxylic acid;
(1α,2β,3β,4α)-1,2-Di[N-cyclohexyl-N-(4-phenoxybenzyl)aminocarbonyl]cyclobutane-3,4-dicarboxylic acid;
(1α,2β,3β,4α)-1,2-Di[N-(cyclopentylmethyl)-N-(4-phenoxybenzyl)aminocarbonyl]cyclobutane-3,4-dicarboxylic acid;
(1α,2β,3β,4α)-1,2-Di[N-(cyclobutylmethyl)-N-(4-phenoxybenzyl)aminocarbonyl]cyclobutane-3,4-dicarboxylic acid;
(1α,2β,3β,4α)-1,2-Di[N-(4-fluorobenzyl)-N-(4-phenoxybenzyl)aminocarbonyl]cyclobutane-3,4-dicarboxylic acid;
(1α,2β,3β,4α)-1,2-Di[N-(4-phenoxybenzyl)-N-(3-methoxyphenethyl)aminocarbonyl]cyclobutane-3,4-dicarboxylic acid;
(1α,2β,3β,4α)-1,2-Di[N-(4-phenoxybenzyl)-N-(3,4-dimethoxyphenethyl)aminocarbonyl]cyclobutane-3,4-dicarboxylic acid;
(1α,2β,3β,4α)-1,2-Di[N-(4-phenoxybenzyl)-N-phenethylaminocarbonyl]cyclobutane-3,4-dicarboxylic acid;
(1α,2β,3β,4α)-1,2-Di[N-(p-phenoxybenzyl)-N-(3-phenyl-1-propyl)aminocarbonyl]cyclobutane-3,4-dicarboxylic acid;
(1α,2β,3β,4α)-1,2-Di[N-(4-phenoxybenzyl)-N-(4-phenyl-1-butyl)aminocarbonyl]cyclobutane-3,4-dicarboxylic acid;
(1α,2β,3β,4α)-1,2-Di[N-(4-phenoxybenzyl)-N-(methoxycarbonylmethyl)aminocarbonyl]cyclobutane-3,4-dicarboxylic acid;
(1α,2β,3β,4α)-1,2-Di[N-(4-phenoxybenzyl)-N-(ethoxycarbonylethyl)aminocarbonyl]cyclobutane-3,4-dicarboxylic acid;
(1α,2β,3β,4α)-1,2-Di[N-propyl-N-(4-cyclohexyloxybenzyl)aminocarbonyl]cyclobutane-3,4-dicarboxylic acid;
(1α,2β,3β,4α)-1-[N-Propyl-N-(4-phenoxybenzyl)aminocarbonyl]-2-[N-methyl-N-(homogeranyl)aminocarbonyl]cyclobutane-3,4-dicarboxylic acid;
(1α,2β,3β,4α)-1-[N-Propyl-N-(4-phenoxybenzyl)aminocarbonyl]-2-[N-benzyl-N-(4-phenoxybenzyl)aminocarbonyl]cyclobutane-3,4-dicarboxylic acid;
(1α,2β,3β,4α)-1-[N-Propyl-N-(4-phenoxybenzyl)aminocarbonyl]-2-[N-(4-phenoxybenzyl)aminocarbonyl]cyclobutane-3,4-dicarboxylic acid;
(1α,2β,3β,4α)-1,2-Di-(4-phenoxybenzyloxycarbonyl)-3,4-dicarboxylic acid;
(1α,2β,3β,4α)-1,2-Di[N-propyl-N-(4-phenylaminophenyl)aminocarbonyl]cyclobutane-3,4-dicarboxylic acid;
(1α,2β,3β,4α)-1,2-Di[N-propyl-N-(4-phenylaminobenzyl)aminocarbonyl]cyclobutane-3,4-dicarboxylic acid;
(1α,2β,3β,4α)-1,2-Di[N-propyl-N-(4-phenylthiobenzyl)aminocarbonyl]cyclobutane-3,4-dicarboxylic acid;
(1α,2β,3β,4α)-1,2-Di[N-propyl-N-[4-phenoxymethylbenzyl]aminocarbonyl]cyclobutane-3,4-dicarboxylic acid;
(1α,2β,3β,4α)-1,2-Di[N-propyl-N-(4-phenoxybenzyl)aminocarbonyl]-4-hydroxymethyl-cyclobutane-3-carboxylic acid;
(1α,2β,3β,4α)-1,2-Di[N-propyl-N-(4-phenoxybenzyl)aminocarbonyl]-4-[(hydroxyimino)methyl]-cyclobutane-3-carboxylic acid;
(1α,2β,3β,4α)-1,2-Di[N-propyl-N-(4-phenoxybenzyl)aminocarbonyl]-4-tetrazolyl-cyclobutane-3-carboxylic acid;
(1α,2β,3β,4α)-1,2-Di[N-propyl-N-(4-phenoxybenzyl)aminocarbonyl-4-tetrazolylmethyl-cyclobutane-3-carboxylic acid;
(1α,2β,3β,4α)-1,2-Di[N-propyl-N-(4-phenoxybenzyl)aminocarbonyl]-4-(carboxycarbonylamino)cyclobutane-3-carboxylic acid;
(1α,2β,3β,4α)-1,2-Di[N-propyl-N-(4-phenoxybenzyl)aminocarbonyl]-4-(3-carboxypropionylamino)cyclobutane-3-carboxylic acid;
(1α,2β,3β,4α)-1,2-Di[N-propyl-N-(4-phenoxybenzyl)aminocarbonyl-4-(*E*-2-carboxyethenyl-cyclobutane-3-carboxylic acid;
(1α,2β,3β,4α)-1,2-Di[N-propyl-N-(4-phenoxybenzyl)aminocarbonyl-4-(2-carboxyethyl)-cyclobutane-3-carboxylic acid;
(1α,2β,3β,4α)-1,2-Di[N-propyl-N-(4-phenoxybenzyl)aminocarbonyl]-4-(1-carboxy-1-hydroxymethyl)cyclobutane-3-carboxylic acid;
(1α,2β,3β,4α)-1,2-Di[N-propyl-N-(4-phenoxybenzyl)aminocarbonyl]-3,4-di[(hydroxyimino)methyl]-cyclobutane;
(1α,2β,3β,4α)-1,2-Di[N-methyl-N-(4-phenoxybenzyl)aminocarbonyl]cyclobutane-3,4-dicarboxylic acid dimethyl ester;
(1α,2β,3β,4α)-1,2-Di[N-propyl-N-(3-phenoxybenzyl)aminocarbonyl]cyclobutane-3,4-dicarboxylic acid;
(1α,2β,3β,4α)-1,2-Di[N-propyl-N-(5-phenoxyfurfuryl)aminocarbonyl}cyclobutane-3,4-dicarboxylic acid;
(1α,2β,3β,4α)-1,2-Di[N-propyl-N-(5-phenoxythien-2-ylmethyl)aminocarbonyl]cyclobutane-3,4-dicarboxylic acid;
(1α,2β,3β,4α)-1,2-Di[N-propyl-N-(4-(furan-2-yloxybenzyl)aminocarbonyl]cyclobutane-3,4-dicarboxylic acid;
(1α,2β,3β,4α)-1,2-Di[N-propyl-N-(4-(thiazol-2-yloxybenzyl)aminocarbonyl]cyclobutane-3,4-dicarboxylic acid;
(1α,2β,3β,4α)-1,2-Di[N-propyl-N-(4-(pyrrol-1-ylmethyl)benzyl)aminocarbonyl]cyclobutane-3,4-dicarboxylic acid;
(1α,2β,3β,4α)-1,2-Di[N-propyl-N-(3-methyl-4-phenoxybenzyl)aminocarbonyl]cyclobutane-3,4-dicarboxylic acid;
(1α,2β,3β,4α)-1,2-Di[N-propyl-N-(4-naphth-2-yloxybenzyl)aminocarbonyl]cyclobutane-3,4-dicarboxylic acid;
(1α,2β,3β,4α)-1,2-Di[N-propyl-N-(4-(3-methyl-1-phenoxy)benzyl)aminocarbonyl]cyclobutane-3,4-dicarboxylic acid;
(1α,2β,3β,4α)-1,2-Di[N-propyl-N-(4-(4-methyl-1-phenoxy)benzyl)aminocarbonyl]cyclobutane-3,4-dicarboxylic acid;
(1α,2β,3β,4α)-1,2-Di[N-propyl-N-(4-naphth-1-yloxybenzyl)aminocarbonyl]cyclobutane-3,4-dicarboxylic acid;
(1α,2β,3β,4α)-1,2-Di[N-propyl-N-(4-phenoxybenzyl)aminocarbonyl]cyclobutane-3-[N-(5-tetrazolyl)]carboxamide-4-carboxylic acid;
(1α,2β,3β,4α)-1,2-Di[N-propyl-N-(4-phenoxybenzyl)aminocarbonyl]cyclobutane-3-(N-(5-tetrazolyl)aminocarbonylamino)-4-carboxylic acid;
(1α,2β,3β,4α)-1,2-Di[N-propyl-N-(4-phenoxybenzyl)aminocarbonyl]cyclobutane-3,4-dicarboxylic acid dimethyl ester;
(1α,2β,3β,4α)-1,2-Di[N-propyl-N-(4-phenoxybenzyl)aminocarbonyl]-3-(methoxycarbonyl)cyclobutane-4-carboxylic acid;
(1α,2β,3α,4α)-1,2-Di[-N-propyl-N-(4-phenoxybenzyl)aminocarbonyl]cyclobutane-3,4-dicarboxylic acid;
(1α,2β,3α,4β)-1,2-Di[N-propyl-N-(4-phenoxybenzyl)aminocarbonyl]cyclobutane-3,4-dicarboxylic acid;
(1α,2β,3β,4α)-1,2-Di[N-propyl-N-(4-phenoxybenzyl)aminocarbonyl]-3-[(hydroxyamino)carbonyl]cyclobutane-4-carboxylic acid;
(1α,2β,3β,4α)-3-(Amino)carbonyl-1,2-Di[N-propyl-N-(4-phenoxybenzyl)aminocarbonyl]cyclobutane-4-carboxylic acid;
(1α,2β,3β,4α)-1,2-Di[N-propyl-N-(4-phenoxybenzyl)aminocarbonyl]-3-(trifluoromethanesulfonylamino)cyclobutane-4-carboxylic acid;
(1α,2β,3β,4α)-4-(Carboxy)methyl)-1,2-Di[N-propyl-N-(4-phenoxybenzyl)aminocarbonyl]cyclobutane-3-carboxylic acid;
(1α,2β,3β,4α)-3,4-Bis(diazoacetyl)-1,2-Di[N-propyl-N-(4-phenoxybenzyl)aminocarbonyl]cyclobutane;
(1α,2β,3β,4α)-1,2-Di[N-propyl-N-(4-phenoxybenzyl)aminocarbonyl]cyclobutane-3,4-diacetic acid;
(1α,2β,3β,4α)-1,2-Di[N-propyl-N-(4-phenoxybenzyl)aminocarbonyl]cyclobutane-3,4-dicarboxylic acid mono- Norleucine amide;
(1α,2β,3β,4α)-1,2-Di[N-propyl-N-(4-phenoxybenzyl)aminocarbonyl]cyclobutane-3-phenoxycarbonyl-4-dicarboxylic acid;
(1α,2β,3β,4α)-1,2-Di-[N-propyl-N-(4-phenoxybenzyl)aminocarbonyl]cyclobutane-3,4-dicarboxylic acid mono-Glycine amide;
(1α,2β,3β,4α)-1,2-Di[N-propyl-N-(4-phenoxybenzyl)aminocarbonyl]cyclobutane-3,4-dicarboxylic acid mono-*d,l*-Proline amide;
(1α,2β,3β,4α)-1,2-Di[N-propyl-N-(4-phenoxybenzyl)aminocarbonyl]cyclobutane-3,4-dicarboxylic acid mono- Sarcosine amide;
(1α,2β,3β,4α)-1,2-Di[N-propyl-N-(4-phenoxybenzyl)aminocarbonyl]cyclobutane-3,4-dicarboxylic acid mono- *d,l*-Aspartic acid amide;
(1α,2β,3β,4α)-1,2-Di[N-propyl-N-(4-phenoxybenzyl)aminocarbonyl]cyclobutane-3,4-dicarboxylic acid mono Serine amide;
(1α,2β,3β,4α)-1,2-Di[N-propyl-N-(4-phenoxybenzyl)aminocarbonyl]cyclobutane-3,4-dicarboxylic acid mono-β-Alanine amide;
(1α,2β,3β,4α)-1,2-Di[N-propyl-N-(4-phenoxybenzyl)aminocarbonyl]cyclobutane-3,4-dicarboxylic acid mono-*d*-Norleucine amide;
(1α,2β,3β,4α)-1,2-Di[N-propyl-N-(4-phenoxybenzyl)aminocarbonyl]cyclobutane-3,4-dicarboxylic acid mono-1-Norleucine amide;
(1α,2β,3β,4α)-1,2-Di[N-(4-pyridyl)methyl-N-(phenoxybenzyl)aminocarbonyl]cyclobutane-3,4-dicarboxylic acid;
(1α,2α,3β,4β)-1,2-Di[N-propyl-N-(phenoxybenzyl)aminocarbonyl]cyclobutane-3,4-dicarboxylic acid;
(1α,2α,3α,4α)-1,2-Di[N-propyl-N-(phenoxybenzyl)aminocarbonyl]cyclobutane-3,4-dicarboxylic acid;
(1α,2α,3α,4β)-1,2-Di[N-propyl-N-(4-phenoxybenzyl)aminocarbonyl]cyclobutane-3,4-dicarboxylic acid;
(1α,2β,3β,4α)-1,2-Di[N-propyl-N-(4-benzoylbenzyl)aminocarbonyl]cyclobutane-3,4-carboxylic acid;
(1α,2β,3β,4α)-1,2-Di[N-propyl-N-(4-(3,4-methylenedioxyphenoxy)benzyl)-aminocarbonyl]cyclobutane-3,4-carboxylic acid;
(1α,2β,3β,4α)-1,2-Di[N-methyl-N-(4-phenoxybenzyl)aminocarbonylamino]cyclobutane-3,4-dicarboxylic acid;
(1α,2β,3β,4α)-1,2-Di[N,N-dibenzylaminocarbonyl]cyclobutane-3,4-dicarboxylic acid;
(1α,2β,3β,4α)-1,2-Di(N-benzyl-N-(4-chlorobenzyl)aminocarbonyl)cyclobutane-3,4-dicarboxylic acid;
(1α,2β,3β,4α)-1,2-Di[(4-phenoxybenzyl)carbonylamino]cyclobutane-3,4-dicarboxylic acid;
(1α,2β,3β,4α)-1,2-Di[N-propyl-N-(4-phenoxybenzyl)carbonylamino]cyclobutane-3,4-dicarboxylic acid;
(1α,2β,3β,4α)-1,2-Di-[N-propyl-N-(4-phenoxybenzyl)aminocarbonyl]-3,4-bis(tetrazolylmethyl)cyclobutane;
(1α,2β,3β,4α)-1,2-Di-[N-propyl-N-(4-phenoxybenzyl)aminocarbonylmethyl]-cyclobutane-3,4-dicarboxylic acid; (1α,2β,3β,4α)-1,2-Di{N-benzyl-N-[(4-phenoxy)benzyl]aminocarbonyl}cyclobutane-3,4-diacetic acid;
(-)-(1α,2β,3β,4α)-1,2-Di[N-cyclopropylmethyl-N-(4-phenoxybenzyl)aminocarbonyl]cyclobutane-3,4-dicarboxylic acid;
(1α,2β,3β,4α)-1,2-Di[N-propyl-N-(4-phenoxybenzyl)aminothiocarbonyl]cyclobutane-3,4-dicarboxylic acid;
(1α,2β,3β,4α)-Di[N-(S)-α-(cyclopropylmethyl)benzyl-N-(4-phenoxybenzyl)-aminocarbonyl]cyclobutane-3,4-dicarboxylic acid;
(1α,2β,3β,4α)-Di[N-(R)-α-(cyclopropylmethyl)benzyl-N-(4-phenoxybenzyl)-aminocarbonyl]cyclobutane-3,4-dicarboxylic acid;
(1α,2β,3β,4α)-Di[N-propyl-N-(S)-α-(cyclopropylmethyl)-(4-phenoxybenzyl)-aminocarbonyl]cyclobutane-3,4-dicarboxylic acid;
(1α,2β,3β,4α)-Di[N-propyl-N-(R)-α-(cyclopropylmethyl)-(4-phenoxybenzyl)-aminocarbonyl]cyclobutane-3,4-dicarboxylic acid;
(1α,2β,3β,4α)-Di-N-(R)-α-ethylbenzyl-N-(4-phenoxyphenyl)aminocarbonyl]-cyclobutane-3,4-dicarboxylic acid;
(1α,2β,3β,4α)-Di[N-(S)-α-ethylbenzyl-N-(4phenoxyphenyl)aminocarbonyl]-cyclobutane-3,4-dicarboxylic acid;
(1α,2β,3β,4α)-Di[N-(R)-α-(cyclopropylmethyl)benzyl-N-(4-phenoxybenzyl)-aminocarbonyl]cyclobutane-3,4-dicarboxylic acid;
(1α,2β,3β,4α)-Di[N-(S)-α-(cyclopropylmethyl)benzyl-N-(4-phenoxybenzyl)-aminocarbonyl]cyclobutane-3,4-dicarboxylic acid;
(1α,2β,3β,4α)-Di[N-(R)-α-propyl-(4-phenoxybenzyl)aminocarbonyl]cyclobutane- 3,4-dicarboxylic acid;
(1α,2β,3β,4α)-Di[N-(S)-α-propyl-(4-phenoxybenzyl)aminocarbonyl]cyclobutane-3,4-dicarboxylic acid;
(1α,2β,3β,4α)-Di[N-propyl-N-(R)-α-propyl-(4-phenoxybenzyl)aminocarbonyl]-cyclobutane-3,4-dicarboxylic acid;
(1α,2β,3β,4α)-Di[N-propyl-N-(S)-α-propyl-(4-phenoxybenzyl)aminocarbonyl]-cyclobutane-3,4-dicarboxylic acid;
(1α,2β,3β,4α)-Di[N-benzyl-N-(R)-α-propyl-(4-phenoxybenzyl)aminocarbonyl]-cyclobutane-3,4-dicarboxylic acid;
(1α,2β,3β,4α)-Di[N-benzyl-N-(S)-α-propyl-(4-phenoxybenzyl)aminocarbonyl]-cyclobutane-3,4-dicarboxylic acid;
(1α,2β,3β,4α)-Di[N-(cyclopropylmethyl)-N-(R)-α-propyl-(4-phenoxybenzyl)-aminocarbonyl]cyclobutane-3,4-dicarboxylic acid;
(1α,2β,3β,4α)-Di[N-(cyclopropylmethyl)-N-(S)-α-propyl-(4-phenoxybenzyl)-aminocarbonyl]cyclobutane-3,4-dicarboxylic acid;
(1α,2β,3β,4α)-Di[N-(R)-α-ethyl-(4-phenoxybenzyl)aminocarbonyl]cyclobutane-3,4-dicarboxylic acid;
(1α,2β,3β,4α)-Di[N-(S)-α-ethyl-(4-phenoxybenzyl)aminocarbonyl]cyclobutane-3,4-dicarboxylic acid;
(1α,2β,3β,4α)-Di[N-propyl-N-(R)-α-ethyl-N-(4-phenoxybenzyl)-aminocarbonyl]-cyclobutane-3,4-dicarboxylic acid;
(1α,2β,3β,4α)-Di[N-propyl-N-(S)-α-ethyl-N-(4-phenoxybenzyl)aminocarbonyl]-cyclobutane-3,4-dicarboxylic acid;
(1α,2β,3β,4α)-Di[N-benzyl-N-(R)-α-ethyl-N-(4-phenoxybenzyl)aminocarbonyl]-cyclobutane-3,4-dicarboxylic acid;
(1α,2β,3β,4α)-Di[N-benzyl-N-(S)-α-ethyl-N-(4-phenoxybenzyl)aminocarbonyl]-cyclobutane-3,4-dicarboxylic acid;
(1α,2β,3β,4α)-Di[N-(cyclopropylmethyl)-N-(R)-α-ethyl-(4-phenoxybenzyl)-aminocarbonyl]cyclobutane-3,4-dicarboxylic acid;
(1α,2β,3β,4α)-Di[N-(cyclopropylmethyl)-N-(S)-α-ethyl-(4-phenoxybenzyl)-aminocarbonyl]cyclobutane-3,4-dicarboxylic acid;
(1α,2β,3β,4α)-Di[N-(R)-α-methyl-(4-phenoxybenzyl)aminocarbonyl]-cyclobutane-3,4-dicarboxylic acid;
(1α,2β,3β,4α)-Di[N-(S)-α-methyl-(4-phenoxybenzyl)aminocarbonyl]-cyclobutane-3,4-dicarboxylic acid;
(1α,2β,3β,4α)-Di[N-propyl-N-(R)-α-methyl-(4-phenoxybenzyl)aminocarbonyl]-cyclobutane-3,4-dicarboxylic acid;
(1α,2β,3β,4α)-Di[N-propyl-N-(S)-α-methyl-(4-phenoxybenzyl)aminocarbonyl]-cyclobutane-3,4-dicarboxylic acid;
(1α,2β,3β,4α)-Di[N-benzyl-N-(R)-α-methyl-(4-phenoxybenzy)aminocarbonyl]-cyclobutane-3,4-dicarboxylic acid;
(1α,2β,3β,4α)-Di[N-benzyl-N-(S)-α-methyl-(4-phenoxybenzyl)aminocarbonyl]-cyclobutane-3,4-dicarboxylic acid;
(1α,2β,3β,4α)-Di[N-(cyclopropylmethyl)-N-(R)-α-methyl-(4-phenoxybenzyl)-aminocarbonyl]cyclobutane-3,4-dicarboxylic acid;
(1α,2β,3β,4α)-Di[N-(cyclopropylmethyl)-N-(S)-α-methyl-(4-phenoxybenzyl)-aminocarbonyl]cyclobutane-3,4-dicarboxylic acid;
(1α,2β,2β,4α)-Di[N-(R)-α-(cyclopropylmethyl)-(4-phenoxybenzyl)amino-carbonyl]cyclobutane-3,4-dicarboxylic acid;
(1α,2β,3β,4α)-Di[N-(S)-α-(cyclopropylmethyl)-(4-phenoxybenzyl)amino-carbonyl]cyclobutane-3,4-dicarboxylic acid;
(1α,2β,3β,4α)-Di(N-propyl-N-(R)-α-(cyclopropylmethyl)-(4-phenoxybenzyl)-aminocarbonyl]cyclobutane-3,4-dicarboxylic acid;
(1α,2β,3β,4α)-Di[N-propyl-N-(S)-α-(cyclopropylmethyl)-(4-phenoxybenzyl)-aminocarbonyl]cyclobutane-3,4-dicarboxylic acid;
(1α,2β,3β,4α)-Di[N-benzyl-N-(R)-α-(cyclopropylmethyl)-(4-phenoxybenzyl)-aminocarbonyl]cyclobutane-3,4-dicarboxylic acid;
(1α,2β,3β,4α)-Di[N-benzyl-N-(S)-α-(cyclopropylmethyl)-(4-phenoxybenzyl)-aminocarbonyl]cyclobutane-3,4-dicarboxylic acid;
(1α,2β,3β,4α)-Di[N-(cyclopropylmethyl)-N-(R)-α-(cyclopropylmethyl)-(4-phenoxybenzyl)aminocarbonyl]cyclobutane-3,4-dicarboxylic acid; and
(1α,2β,3β,4α)-Di[N-(cyclopropylmethyl)-N-(S)-α-(cyclopropylmethyl)-(4-phenoxybenzyl)aminocarbonyl]cyclobutane-3,4-dicarboxylic acid;
or a pharmaceutically acceptable salt thereof.

Preferred compounds are selected from the group consisting of
(1α,2β,3β,4α)-1,2-Di[N-benzyl-N-(4-phenoxybenzyl)aminocarbonyl]cyclobutane-3,4-dicarboxylic acid;
(1α,2β,3β,4α)-1,2-Di[N-propyl-N-(4-phenoxybenzyl)aminocarbonyl]cyclobutane-3,4-dicarboxylic acid;
(-)-(1α,2β,3β,4α)-1,2-Di[N-propyl-N-(4-phenoxybenzyl)aminocarbonyl]cyclobutane-3,4-dicarboxylic acid;
(1α,2β,3β,4α)-1,2-Di[N-(2-ethylthioethyl)-N-(4-phenoxybenzyl)aminocarbonyl]cyclobutane-3,4-dicarboxylic acid;
(1α,2β,3β,4α)-1,2-Di[N-(cyclopropylmethyl)-N-(4-phenoxybenzyl)-aminocarbonyl]cyclobutane-3,4-dicarboxylic acid;
(1α,2β,3β,4α)-1-[N-Propyl-N-(4-phenoxybenzyl)aminocarbonyl]-2-[N-benzyl-N-(4-phenoxybenzyl)aminocarbonyl]cyclobutane-3,4-dicarboxylic acid;
(1α,2β,3β,4α)-1,2-Di[N-propyl-N-(4-phenylthiobenzyl)aminocarbonyl]cyclobutane-3,4-dicarboxylic acid;
(1α,2β,3β,4α)-1,2-Di[N-propyl-N-(4-phenoxybenzyl)aminocarbonyl-4-tetrazolylmethyl-cyclobutane-3-carboxylic acid;
(1α,2β,3β,4α)-1,2-Di[N-propyl-N-(4-phenoxybenzyl)aminocarbonyl]-4-(3-carboxypropionylamino)cyclobutane-3-carboxylic acid;
(1α,2β,3β,4α)-1,2-Di[N-propyl-N-(4-phenoxybenzyl)aminocarbonyl]-4-(1-carboxy-1-hydroxymethyl)cyclobutane-3-carboxylic acid;
(1α,2β,3β,4α)-1,2-Di[N-propyl-N-(4-phenoxybenzyl)aminocarbonyl]cyclobutane-3-[N-(5-tetrazolyl)]carboxamide-4-carboxylic acid;
(1α,2β,3β,4α)-4-(Carboxy)methyl)-1,2-di[N-propyl-N-(4-phenoxybenzyl)aminocarbonyl]cyclobutane-3-carboxylic acid; and
(1α,2β,3β,4α)-1,2-Di[-N-propyl-N-(4-phenoxybenzyl)aminocarbonyl]cyclobutane-3,4-diacetic acid;
or a pharmaceutically acceptable salt thereof.

In general, the compounds of the invention can be prepared by the processes illustrated in Schemes I-XV. According to reaction Scheme I, 1,2,3,4-cyclobutanetetracarboxylic dianhydride (where the two anhydrides are trans to one another) in an inert solvent such as dimethylformamide is treated with an appropriately substituted secondary amine (HNR₁R₂) in the presence of an aprotic base such as triethylamine to afford a mixture of the 1,2- and 1,3-diamides. (The isomeric diamides are separable by column chromatography.) The dicarboxylic acid (2) can be further elaborated, if desired, to its diester 4 (wherein R₁₀ is loweralkyl, benzyl, a carboxy protecting group or prodrug) by treatment with an alcohol such as methanol in the presence of concentrated sulfuric acid or with diazomethane.

Alternatively Scheme II illustrates the reaction of 1,2,3,4-cyclobutanetetracarboxylic dianhydride (where the two anhydrides are trans to one another) with an alcohol such as methanol to give a mixture of the diesters 6 and 7 (wherein R₂₀ is loweralkyl or benzyl) following the procedure described in Angew. Chem. International Ed. 8: 208 (1969). (The isomeric diesters are separable by column chromatography or crystallization.) Compound 6 is activated as an acid halide (for example by treatment with thionyl chloride or phosphorus oxychloride) or activated ester including esters or anhydrides derived from formic acid, acetic acid, alkoxycarbonyl halides, N-hydroxysuccinimide, N-hydroxyphthalimide, N-hydroxybenzotriazole, N-hydroxy-5-norbornene-2,3-dicarboxamide, 2,4,5-trichlorophenol and then reacted with a secondary amine (HNR₁R₂) to give compound 8. Hydrolysis of the esters, for example, with sodium hydroxide in methanol-water or lithium hydroxide in THF) affords the dicarboxylic acid 2. Alternatively, the diacid is treated with diphenylphosphoryl azide and triethylamine followed by treatment with a secondary amine to give bisurea diester 9. Ester hydrolysis or catalytic hydrogenation of 9 affords the diacid 10.

The preparation of optically active compounds of the invention is shown in Scheme III. (In a preferred embodiment, R₁ is propyl and R₂ is 4-(phenoxy)benzyl.) The dicarboxylic acid 2 is esterified with a chiral alcohol (such as (+) or (-) *sec*-phenethyl alcohol or (+) or (-) menthol and the like) to give a mixture of phenethyl esters (11) which are separable by silica gel chromatography to give a single diastereomer 12. Catalytic hydrogenation or hydrolysis affords the optically active product 13.

The carboxy functionalities of compound 2 can be elaborated in a number of ways. Scheme IV shows the replacement of one of the carboxy groups with tetrazolyl. The dicarboxylic acid diamide 2, prepared in Scheme I, is converted to a mono-ester 14 where R₃₀ is loweralkyl (for example, making the diester and hydrolyzing one of the esters with a stoichiometric amount of lithium hydroxide). The remaining carboxylic acid moiety is reduced (for example, via a mixed anhydride with sodium borohydride or with BH₃ and the like) to give the hydroxymethyl compound 15. The hydroxymethyl compound is oxidized (for example, using tetrapropylammonium perruthenate (TPAP) or oxalyl chloride in DMSO and the like) to give the aldehyde 16. The aldehyde is reacted with hydroxylamine to give the oxime 17. Treatment of the oxime 17 with trifluoroacetic anydride gives the cyano compound 18. The cyano compound is reacted by standard tetrazole forming methodology (for example, sodium azide and triethylamine hydrochloride in DMF) to give the tetrazolyl compound 19. Ester hydrolysis of 19 (for example, lithium hydroxide in THF) affords the tetrazolyl carboxylic acid 20.

Other modifications of the carboxy functionality are shown in Scheme V. To make the tetrazolylmethyl compound, the hydroxymethyl compound 15 where R₃₀ is loweralkyl, prepared in Scheme IV, is activated (for example, by reacting with methane sulfonyl chloride to give the methane sulfonate) and then reacted with potassium cyanide to give the cyanomethyl compound 21. The cyano compound is reacted by standard tetrazole forming methodology (for example, sodium azide and triethylamine hydrochloride in DMF) to give the tetrazolyl compound, which is hydrolyzed (for example, with lithium hydroxide in water and methanol) to give the carboxylic acid 22.

Starting from the carboxaldehyde 16 where R₃₀ is loweralkyl, prepared in Scheme IV, treatment with furan, n-butyl lithium and CuCN in an inert solvent such as THF, followed by acetylation with acetic anhydride affords the acetoxy furanyl methyl compound 23. Treatment of compound 23 with ruthenium oxide and sodium periodate converts the furan to a carboxylic acid; and then lithium hydroxide hydrolysis of the ester affords the dicarboxylic acid 24.

Starting from the mono-ester 14 where R₃₀ is loweralkyl, prepared in Scheme IV, the carboxylic acid is activated with isobutylchloroformate in the presence of 4-methylmorpholine and then reacted with diazomethane to give the diazoacetyl compound 25. Treatment of the diazoacetyl compound with silver benzoate in methanol affords the diester which is hydrolyzed to give the dicarboxylic acid 26.

Starting with the dicarboxylic acid 2, prepared in Scheme I, the carboxylic acids are activated with isobutylchloroformate in the presence of 4-methylmorpholine and then reacted with diazomethane to give the bis-diazoacetyl compound 27. Treatment of the diazoacetyl compound with silver benzoate in methanol affords the diester which is hydrolyzed to give the di-acetic acid 28.

Starting with the mono-ester 14 (wherein R₃₀ is loweralkyl), prepared in Scheme IV, under standard peptide coupling conditions (for example, using 1-hydroxybenzotriazole and N-methylmorpholine in DMF) plus a carboxy-protected amino acid (for example, the methyl ester of norleucine, β-alanine, sarcosine, glycine, proline and the like) affords the carboxy-protected mono-amino acid derivative (where AA represents an amino acid), which is hydrolyzed to give the dicarboxylic acid 29. Di-, tri- and tetra-peptide derivatives can be similarly prepared, using the appropriate carboxy-protected di-, tri- or tetra-peptide.

Scheme VI illustrates further modifications of the carboxy moiety. The mono-ester 14 (wherein R₃₀ is loweralkyl), prepared in Scheme IV, is reacted with diphenylphosphorylazide in the presence of triethylamine followed by benzyl alcohol to give the benzyloxycarbonyl protected amine 43 (Z is benzyloxycarbonyl). Catalytic hydrogenation removes the Z protecting group to give the 4-amino compound 44. The amine 44 is reacted with EtO₂C(CH₂)ᵣC(O)Cl (where r is 0 to 4) in the presence of 2,6-lutidine to give compound 45. Ester hydrolysis using lithium hydroxide in THF affords the dicarboxylic acid 46.

Aldehyde 16, prepared in Scheme IV, is reacted with methyl triphenylphoranylideneacetate to give compound 47 where E is R₃₀ where R₃₀ is lower alkyl. Lithium hydroxide hydrolysis of 47 in THF affords the diacid 48 where E is hydrogen.

Scheme VII illustrates the preparation of two other stereo-isomers encompassed by the present invention. The (1α,2β,3β,4α) isomer 2 results from the opening of 1,2,3,4-cyclobutanetetracarboxylic dianhydride 1 described in Scheme I. Another isomer is obtained by epimerization of one center on the cyclobutane ring. The mono-ester 14, prepared in Scheme IV, (wherein R₃₀ is loweralkyl) is dissolved in an inert solvent, such as THF or ether or dimethoxyethane cooled, and treated with a non-nucleophilic base (for example, with sodium hexamethyldisilazide or lithium diisopropylamide). Quenching with a protic source such as acetic acid, followed by ester hydrolysis affords the (1α,2β,3α,4α)-isomer as the dicarboxylic acid 30.

Another isomer is obtained by taking the (1α,2β,3β,4α) isomer of diester 8, prepared in Scheme II, (wherein R₃₀ is loweralkyl) and epimerizing with sodium methoxide in methanol to give the (1α,2β,3α,4β) isomer 49 (wherein J is R₃₀ where R₃₀ is lower alkyl). Sodium hydroxide hydrolysis in methanol-water gives the dicarboxylic acid 50 (wherein J is hydrogen).

The preparation of other isomers is shown in Scheme VIII. Furan-2-acrylic acid 31 is photodimerized resulting in two isomers (the (1α,2α,3β,4β)-isomer 32 and the (1α,2α,3α,4α)-isomer 33) which are separable by column chromatography. These photoadducts are then coupled to the appropriate amine (R₁R₂NH) under standard peptide coupling conditions (for example, bis(2-oxo-3-oxazolidinyl)phosphinic chloride in DMF) to give the diamides 34 and 35. The furan groups can be converted to carboxylic acids using the procedure described by Danishefsky et al., J. Amer. Chem. Soc., 110 (12), 3929 - 3940 (1988) to give (1α,2α,3β,4β) 36 and (1α,2α,3α,4α) 37.

The preparation of yet another isomer is shown in Scheme IX. The (1α,2α,3β,4β)-isomer 36 is converted to the mono-ester 38 where R₃₀ is loweralkyl (for example, by converting to the dimethyl ester with diazomethane and then hydrolyzing one of the ester functionalities with a stoichiometric amount of lithium hydroxide). The mono-ester 38 is epimerized with a non-nucleophilic base (for example, with sodium hexamethyldisilazide or lithium diisopropylamide) to give the (1α,2α,3α,4β)-isomer 39. The ester is then hydrolyzed to give the dicarboxylic acid 40.

The preparation of compounds where both amides are not the same is shown in Scheme X. Opening 1 ,2,3,4-cyclobutanetetracarboxylic dianhydride with a stoichiometric amount of an amine (R₁R₂NH) gives the mono-amide tricarboxylic acid 41. Using typical peptide coupling conditions (for example, treating with dicyclohexylcarbodiimide in a mixture of DMF and methylene chloride) and an appropriate amine (R₁*R₂*NH) gives the desired diamide 42 having different amide substituents.

An alternate method for preparing the compounds of the invention is shown in Scheme Xl. 1,2,3,4-Cyclobutanetetracarboxylic anhydride 1 is reacted with benzyl alcohol to give the 1,2-dibenzyl ester 51 as a solid obtained by crystallization, *i.e.* no chromatography is required to separate it from the 1,3-dibenzyl ester also obtained. Treatment of compound 51 with oxalyl chloride and the appropriate amine (R₁R₂NH) in the presence of Hunig's base affords the diamide 52. Catalytic hydrogenation (for example, using a palladium on carbon catalyst, hydrogen, and a methanol-ethyl acetate solvent system) to remove the benzyl protecting groups affords the desired diacid diamide 2.

A procedure for preparing reverse amides is shown in Scheme XII. A dicarboxylic acid where R₂₀ is loweralkyl or benzyl (for example, a diester 6 whose preparation was shown in Scheme II) is reacted with diphenylphosphoryl azide in an inert solvent (for example, in benzene or toluene and the like) in the presence of triethylamine followed by tert-butanol to give the bis(Boc-protected amine) 53. The protecting group is removed with trifluoroacetic acid to give the diamine 54 as its trifluoroacetate salt. This diamine is reacted with an acid (R₂COOH) 55 under amide coupling conditions (for example, 1-(3-dimethylaminopropyl)-3-ethylcarbodiimide·HCl, 1-hydroxybenzotriazole hydrate, and triethylamine in THF) to give the diamide 56. Removal of the carboxy protecting groups (for example, hydrolysis of esters using sodium hydroxide in methanol-water or catalytic hydrogenation of benzyl esters) affords the diacid di-reverse amide 57.

A procedure for preparing N-substituted reverse amides is shown in Scheme XIII. The diamine 54 where R₂₀ is loweralkyl or benzyl, prepared in Scheme XII, is reacted with an acid chloride (Rₐ*C(O)Cl wherein Rₐ* is loweralkyl, cycloalkyl or cycloalkylalkyl) in the presence of triethylamine to give diamide 58. Reduction of the amide functionality (for example, using borane-tetrahydrofuran complex) affords the substituted amine compound 59. Treatment of 59 with the acid chloride of R₂-C(O)OH in the presence of triethylamine affords the diester diamide 60. Removal of the carboxy protecting groups (for example, hydrolysis of esters using sodium hydroxide in methanol-water or catalytic hydrogenation of benzyl esters) affords the diacid di-substituted reverse amide 61.

An alternate procedure for preparing N-substituted reverse amides is shown in Scheme XIV. The diacid 6 is converted to the N-protected diamine 65 (for example, by reaction with oxalyl chloride, followed by reaction with sodium azide, followed by reaction with t-BuOH and CuCl). Reaction of 65 with Rₐ-L (Rₐ is loweralkyl, cycloalkyl or cycloalkylalkyl and L is a leaving group such as Cl, Br, I or a sulfonate) in the presence of a non-nucleophilic strong base (for example, NaH), followed by N-deprotection provides 66. Diamine 66 can be converted to 61 as described in Scheme XIII.

An alternative preparation of optically active compounds of the invention is shown in Scheme XV. The dibenzyl ester 51, prepared in Scheme Xl, forms a salt with (-)-norephedrine in ethanol, and the desired optically active (-)-isomer crystallizes from solution. Separation of the salt of the (-)-isomer, followed by acidification affords the desired optically active dicarboxylic acid 62. Compound 62 is reacted with R₁R₂NH to give the optically active diamide 63. Catalytic hydrogenation of compound 63 affords the optically active dicarboxylic acid 64.

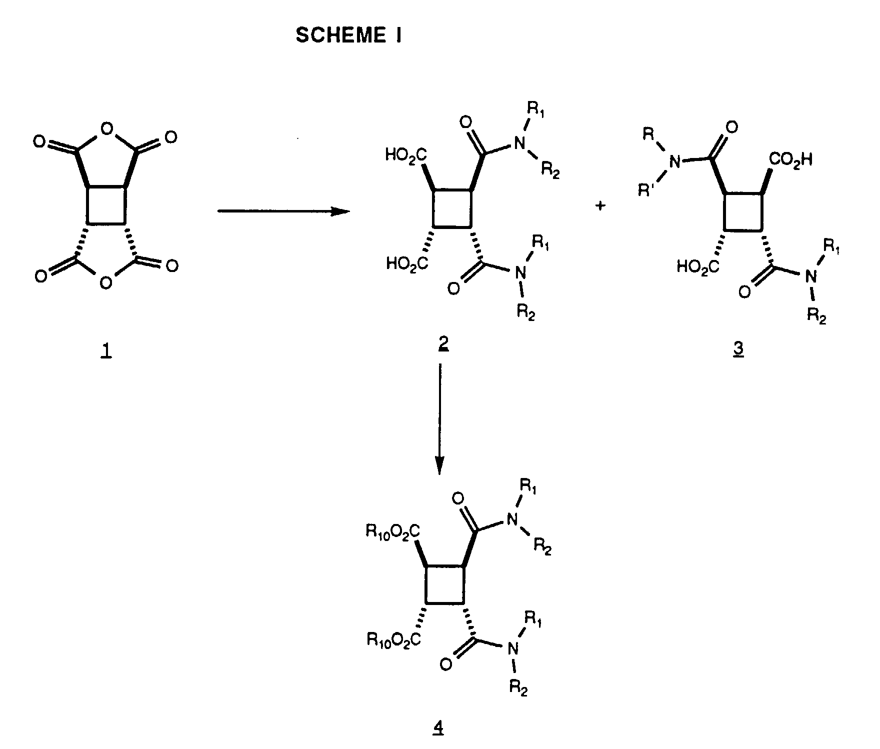

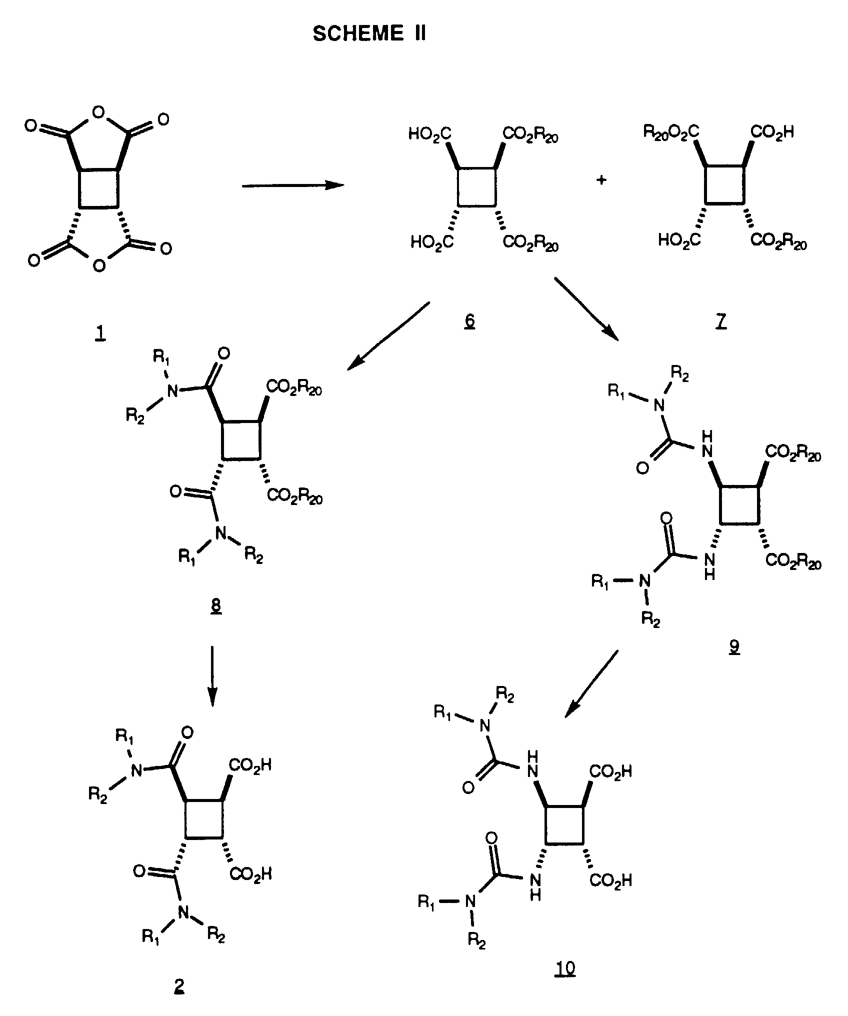

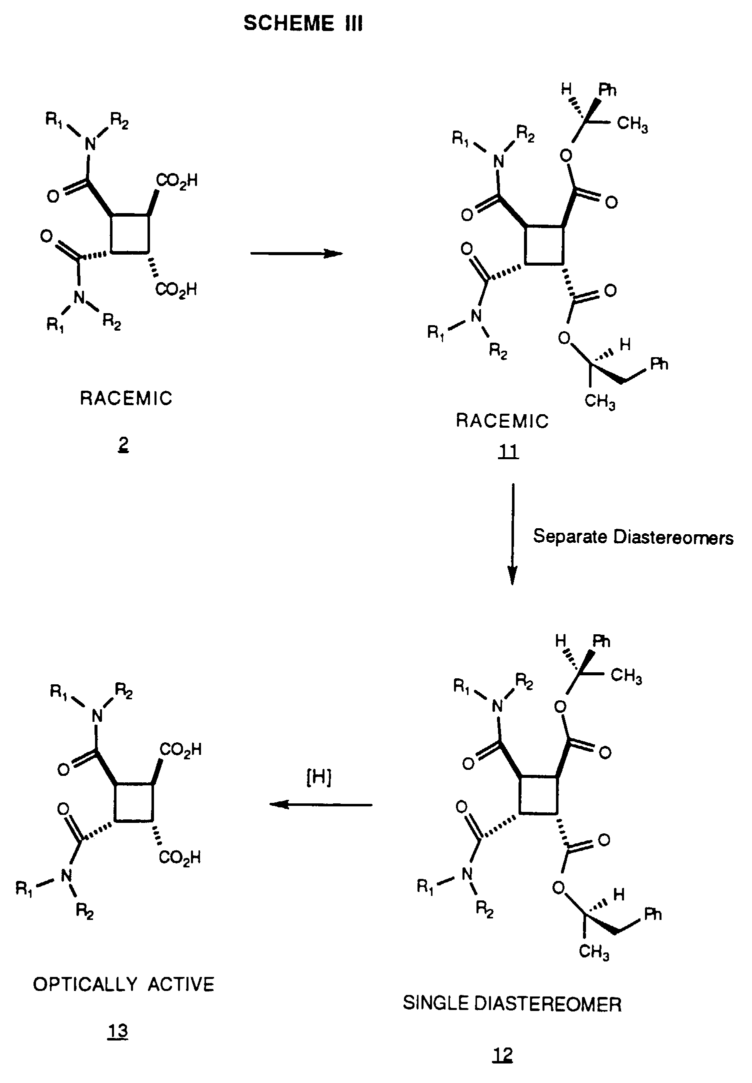

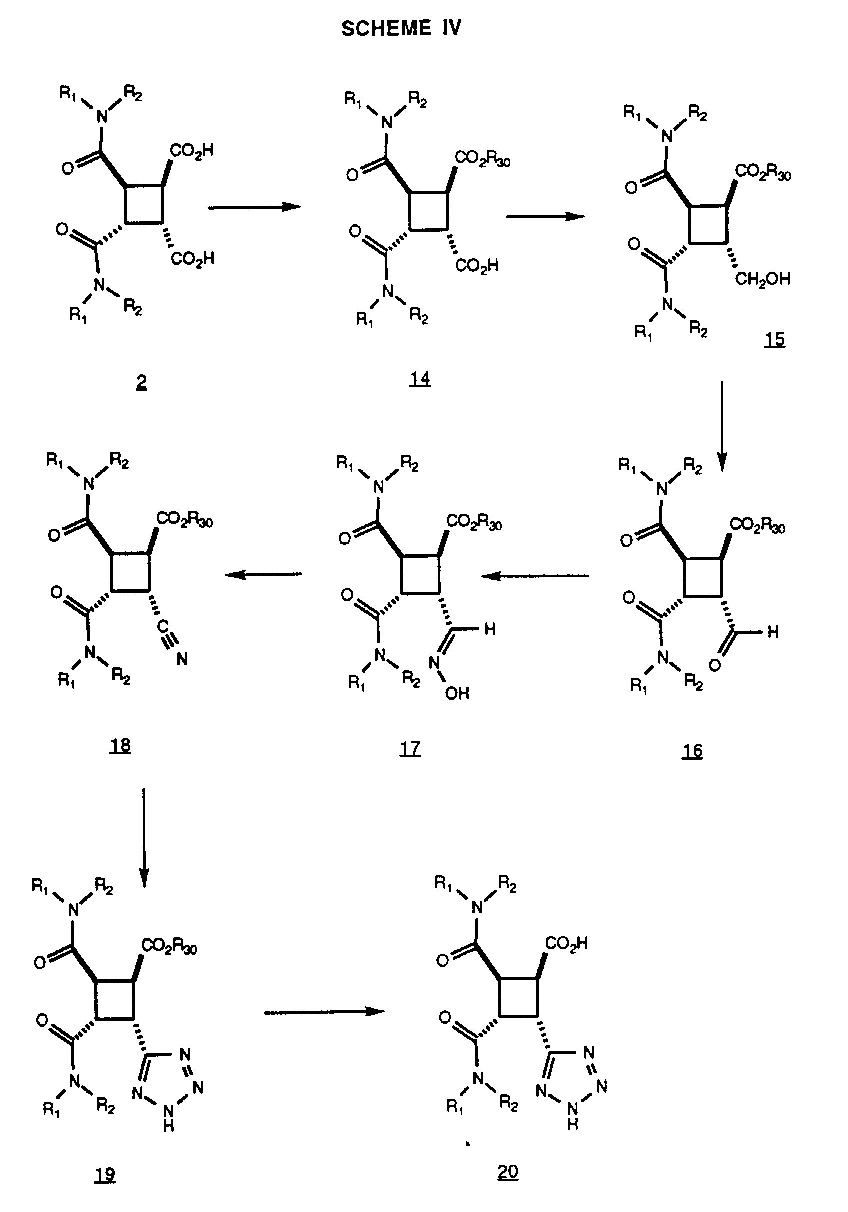

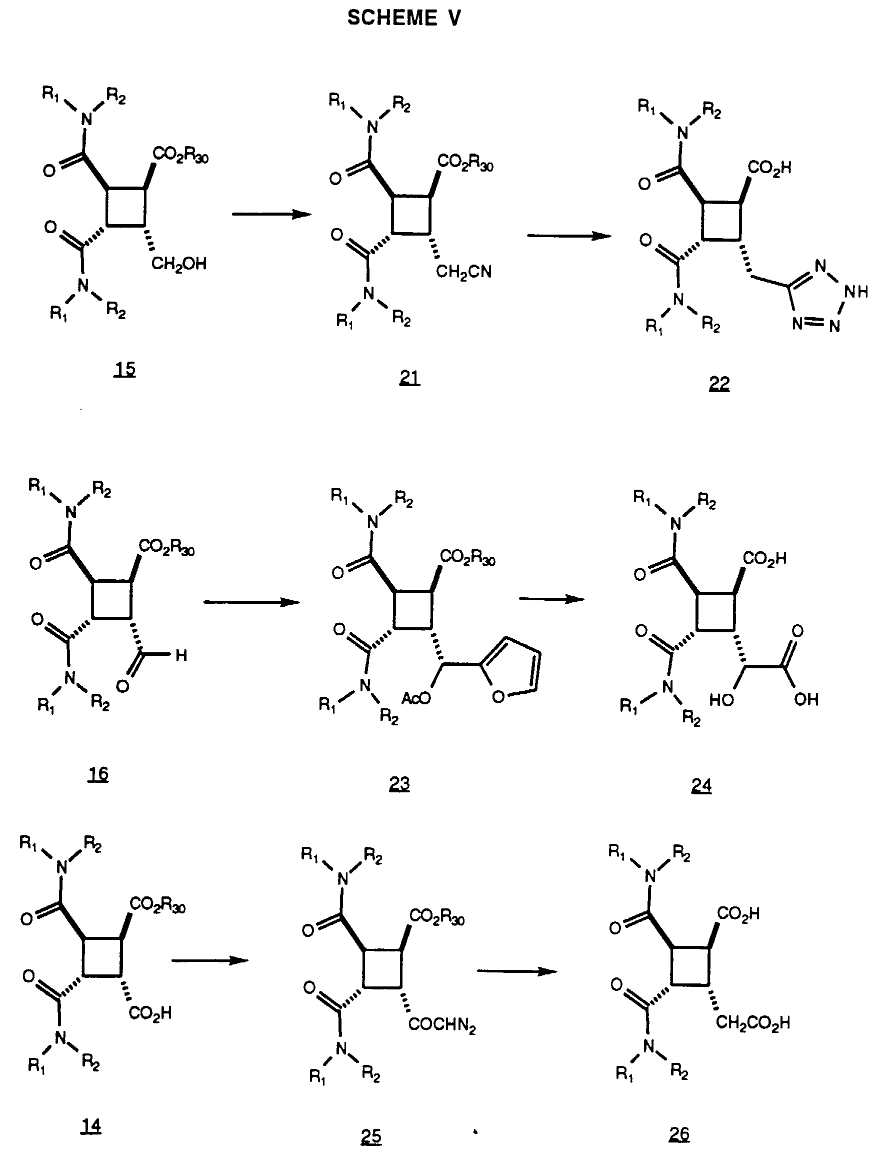

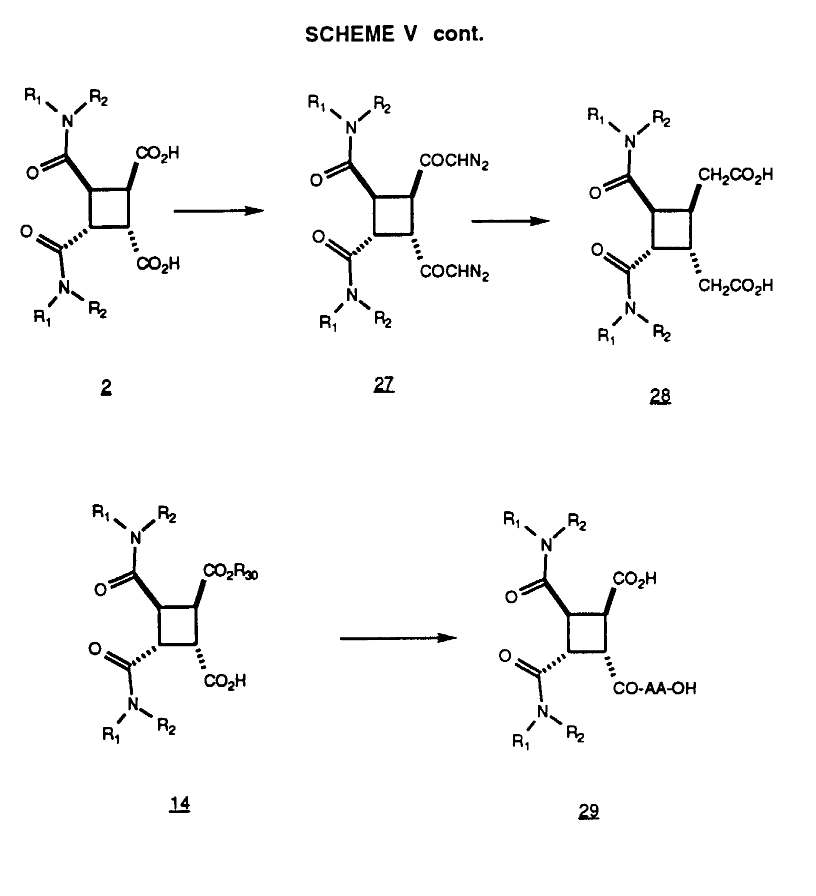

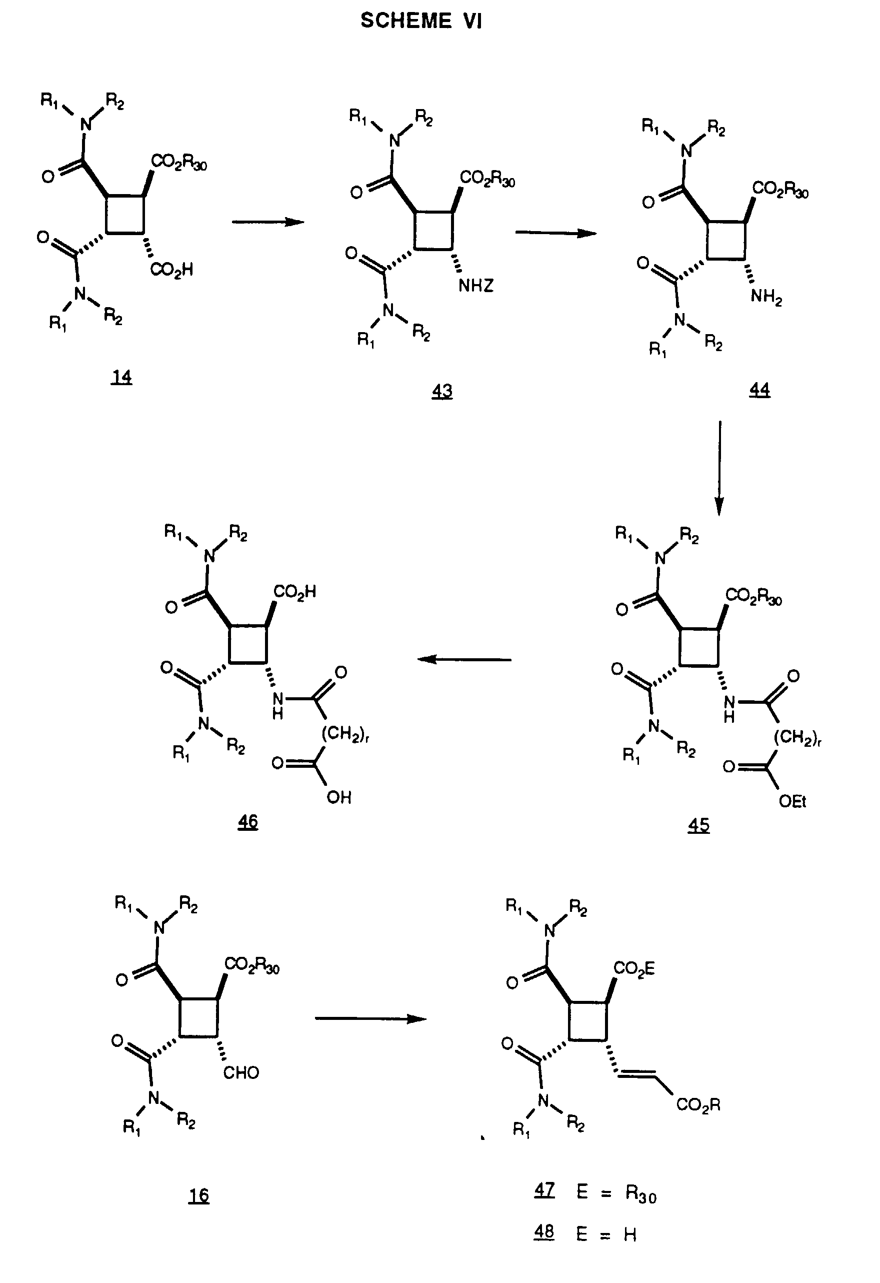

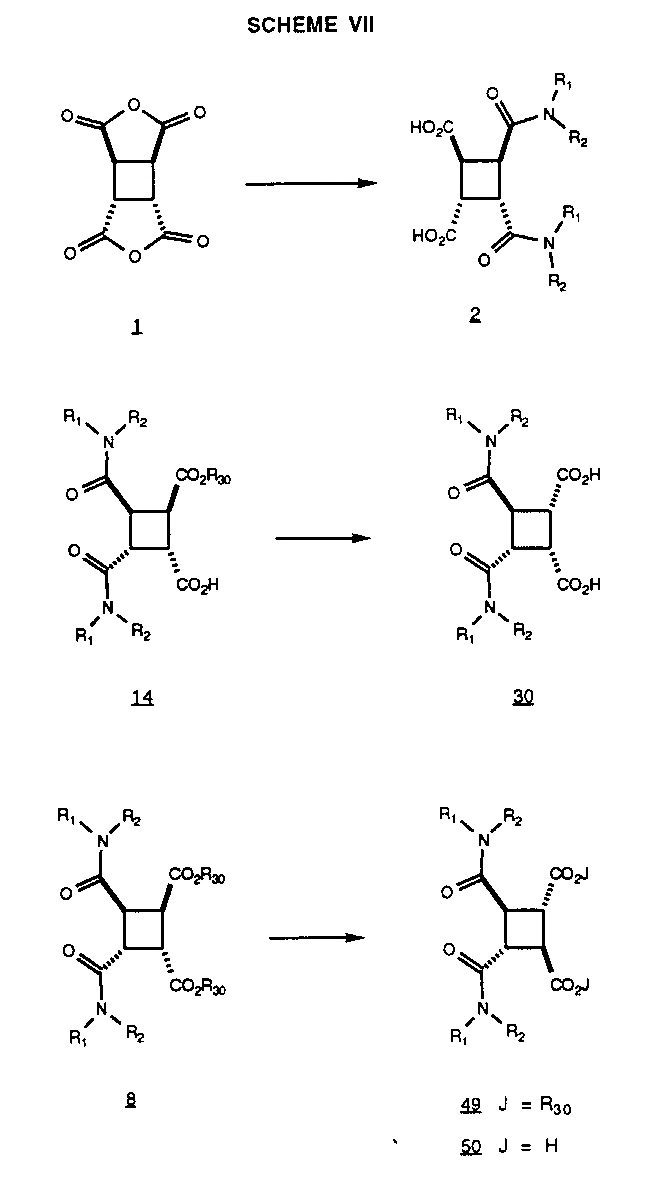

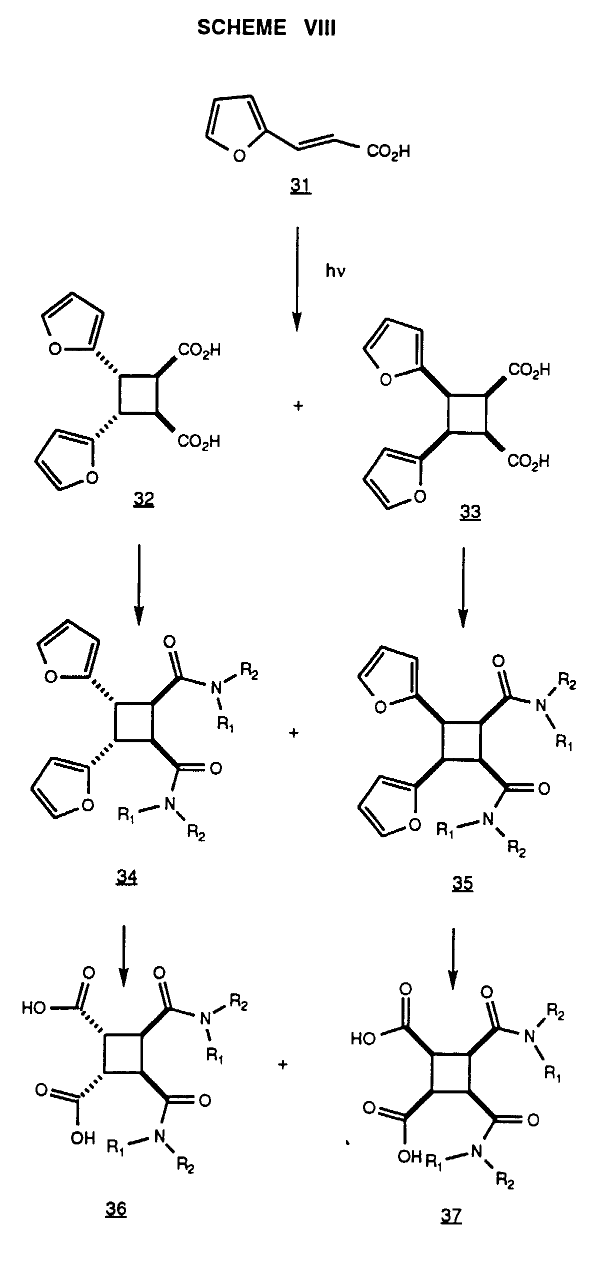

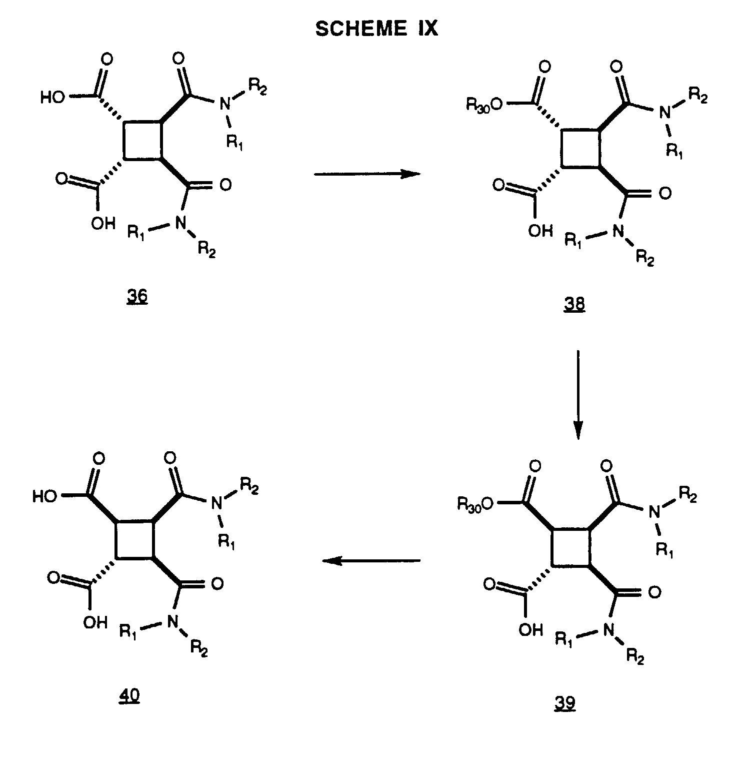

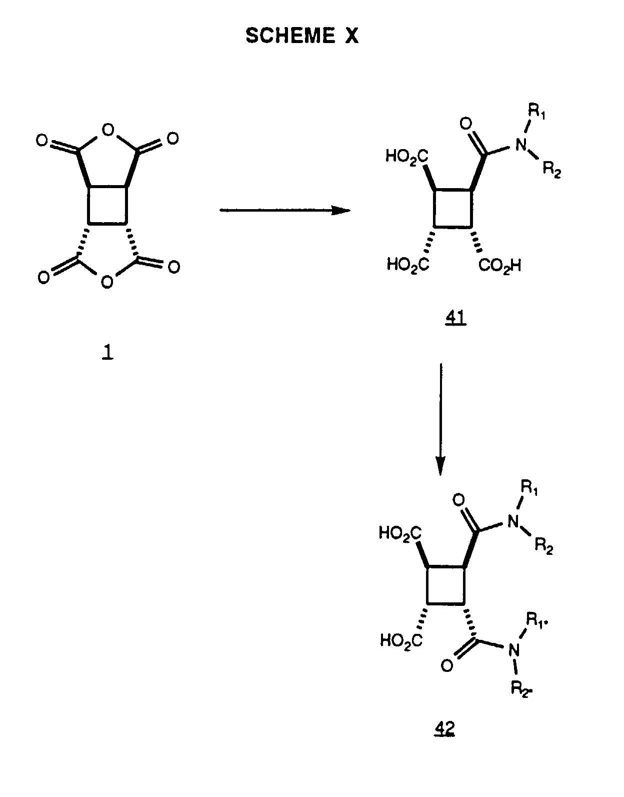

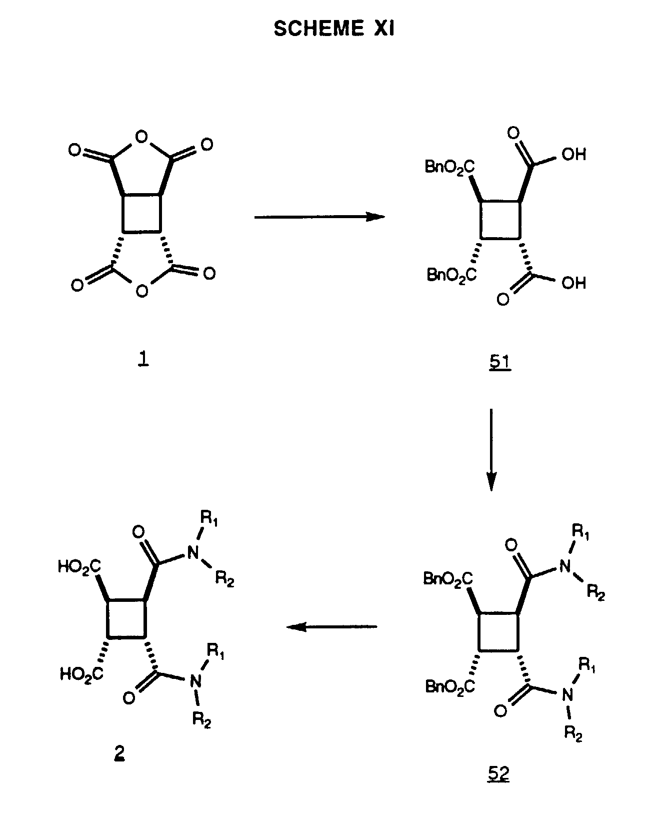

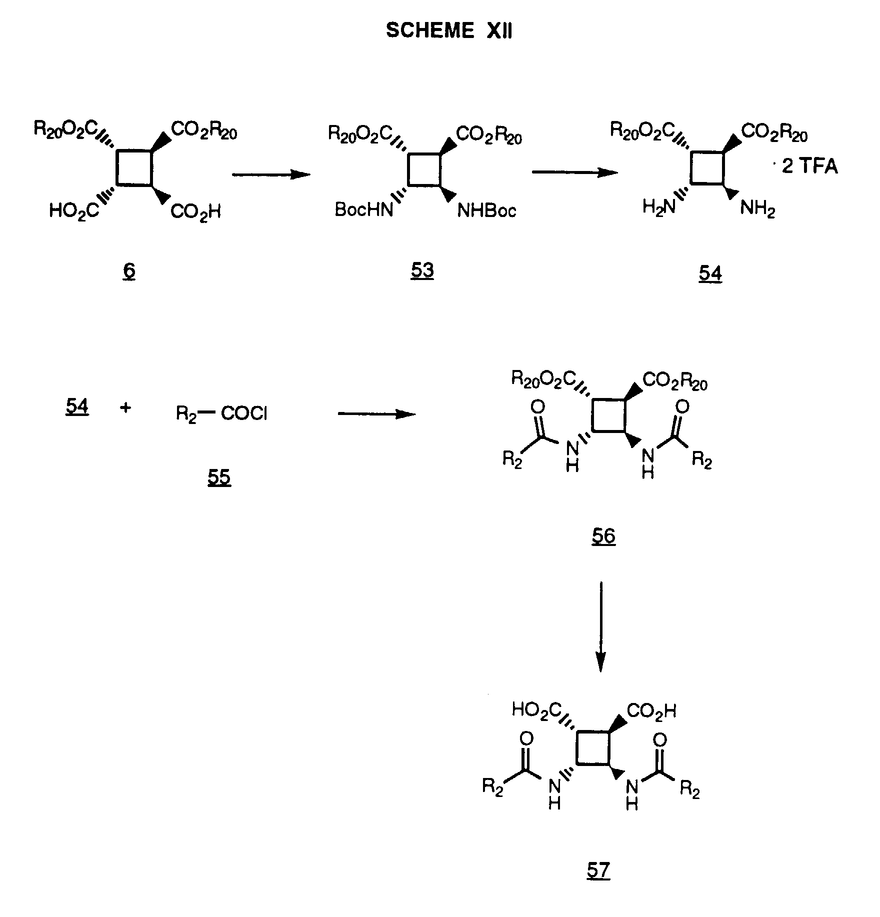

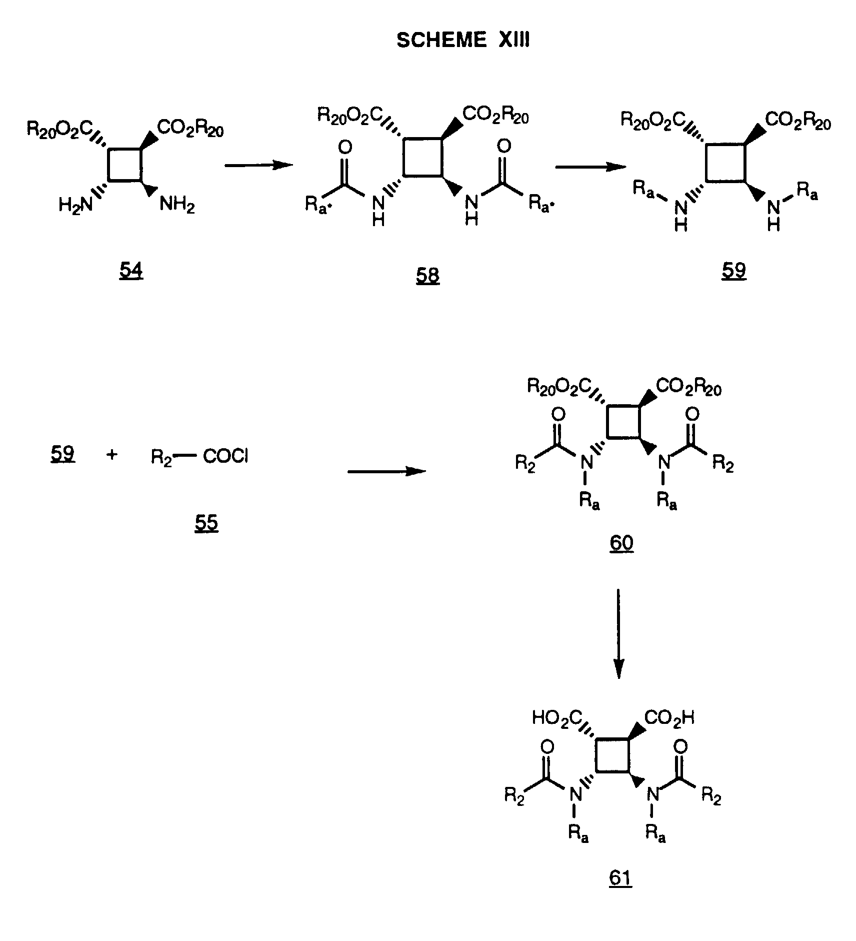

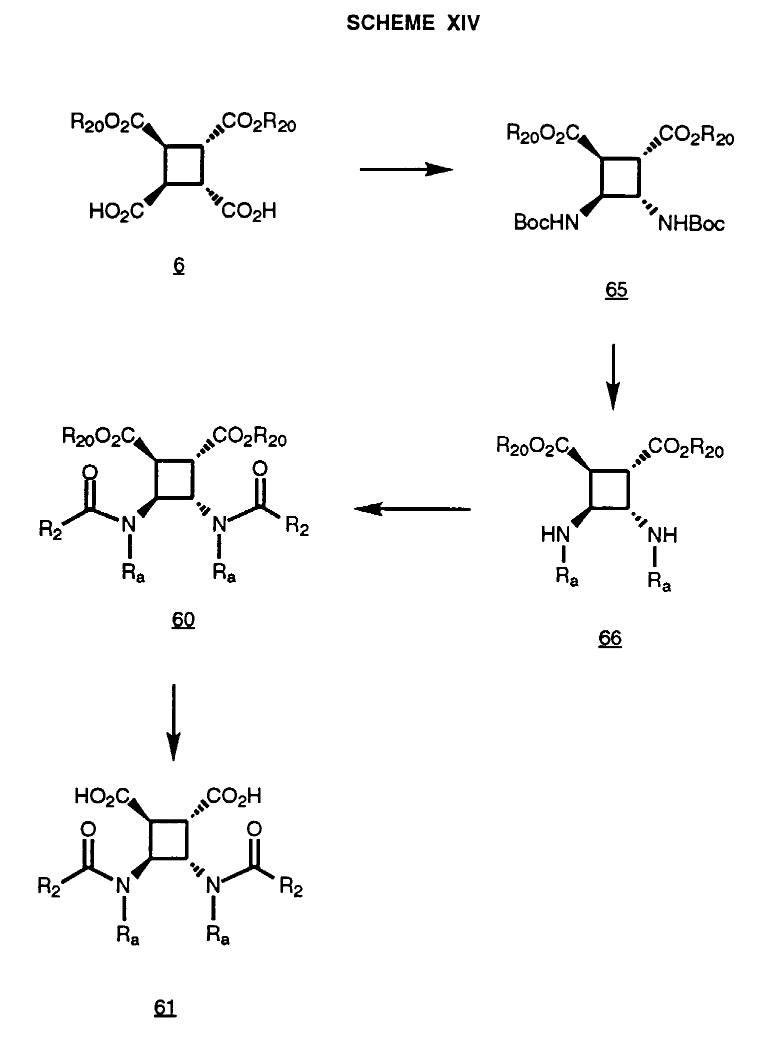

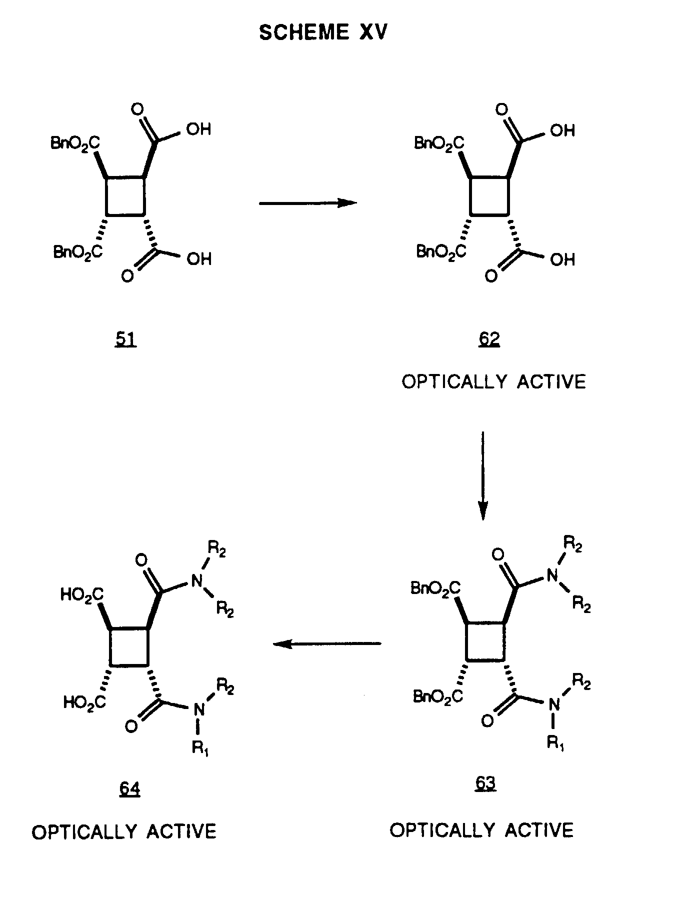

The foregoing may be better understood by reference to the following examples which are provided for illustration.

The following abbreviations were used AgOBn for silver benzoate, BOP-Cl for bis(2-oxo-3-oxazolidinyl)phosphinic chloride, n-BuLi for n-butyl lithium, DIBAL for diisobutylaluminum hydride, DMAP for dimethylaminopyridine, DME for dimethoxyethane, DMF for dimethylformamide, DMSO for dimethylsulfoxide, EDCI for 1-(3-dimethylaminopropyl)-3-ethylcarbodiimide hydrochloride, Et₃N for triethylamine, Et₂O for diethyl ether, EtOAc for ethyl acetate, EtOH for ethanol, HOAc for acetic acid, HOBT for 1-hydroxybenzotriazole, LAH for lithium aluminum hydride, LDA for lithium diisopropylamide, MeOH for methanol, Pd/C for palladium on carbon, THF for tetrahydrofuran, and TPAP for tetrapropylammonium perruthenate.

### Example 1A

### N-Methyl-N-(4-phenoxybenzyl)amine

4-Phenoxybenzaldehyde (10.0 g, 0.05 mol), excess methylamine and 1.5 g of 10% Pd/C in 200 mL of methanol were stirred under an atmosphere of hydrogen for 16 hours. After removal of the catalyst by filtration through Celite®, the filtrate was concentrated under reduced pressure to give the crude product as an oil. Chromatography on silica gel eluting with ethyl acetate gave the title compound in 80%.

### Example 1B

### N-i-Butyl-N-(4-phenoxybenzyl)amine

4-Phenoxybenzaldehyde (10.0 g, 0.05 mol), excess isobutylamine and 1.0 g of 10% Pd/C in 200 mL of ethanol were stirred under an inert atmosphere for 16 hours followed by an atmosphere of hydrogen for 16 hours. After removal of the catalyst by filtration through Celite®, the filtrate was concentrated under reduced pressure to give the crude product as an oil. The oil was dissolved in ether and precipitated by treatment with anhydrous HCI. The solid was filtered, washed with ether, and partitioned between ethyl acetate and 1M NaOH. The ethyl acetate solution was washed with brine, dried over Na₂SO₄, and evaporated to give the title compound in 93% yield.

### Examples 2-10

The following compounds were prepared by the procedures described in Examples 1A or 1B using the appropriate aldehydes and amines.

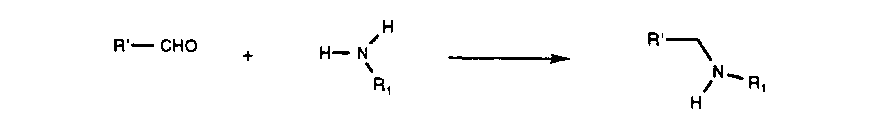

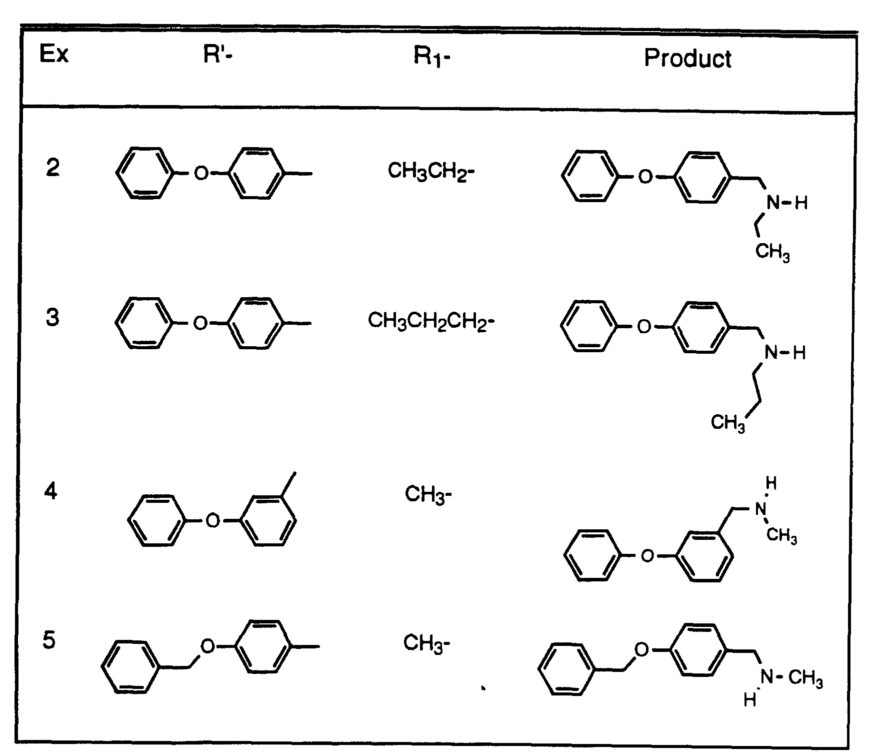

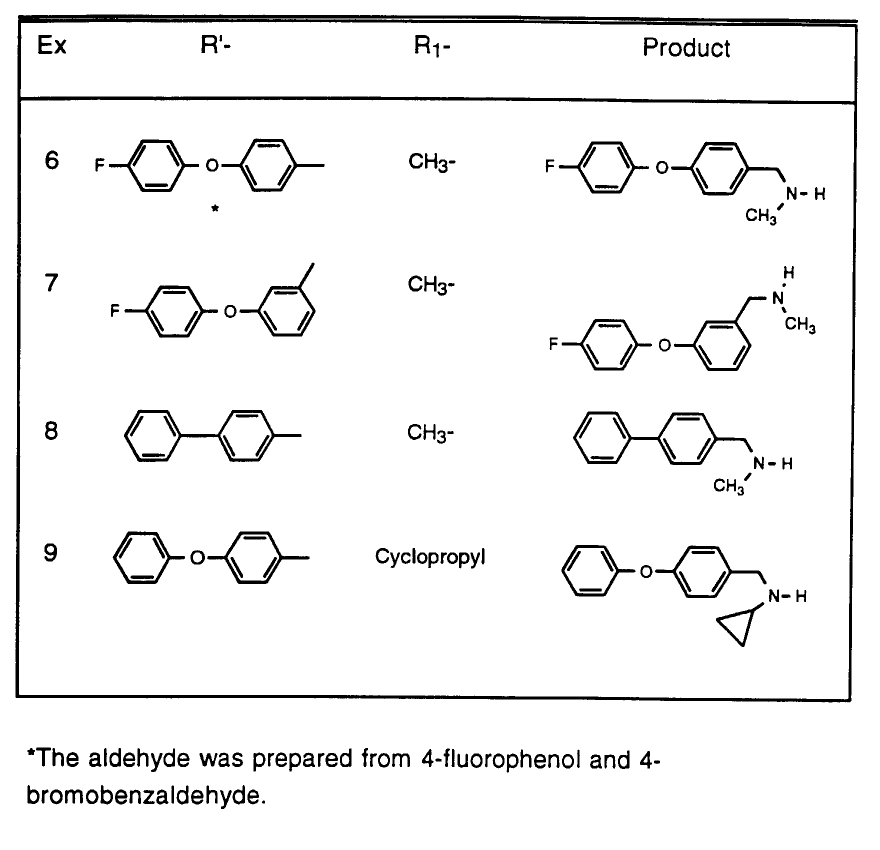

### Example 10

### N,N-Di(4-phenoxybenzyl)amine

4-Phenoxybenzaldehyde was reacted with ammonium acetate to give the title compound.

### Example 11

### N-Propargyl-N-(4-phenoxybenzyl)amine

4-Phenoxybenzaldehyde (5.0 g, 25.2 mmol) and propargylamine (1.46 g, 26.5 mmol) were dissolved in 100 mL of 1% acetic acid in methanol under an atmosphere of dry nitrogen. Sodium cyanoborohydride (1.66 g, 26.5 mmol) was added, and stirring was continued for 18 hours at which time the solvent was removed under reduced pressure. The residue was suspended in ether, washed with 5% NaHCO₃ and brine, and dried over Na₂SO₄ to give 5.6 g (93%) of the title compound.

### Example 12

### N-Benzyl-N-(4-phenoxybenzyl)amine

The title compound was prepared by the method described in Example 11 substituting benzylamine for propargylamine.

### Example 13

### (1α,2β,3β,4α)-1,2-Di[N-methyl-N-(4-phenoxybenzyl)aminocarbonyl]cyclobutane-3,4-dicarboxylic acid

To a solution cooled in an ice bath of 1,2,3,4-cyclobutanecarboxylic dianhydride (3.42 g, 17.4 mmol) in 30 mL of dimethylformamide was added triethylamine (3.51 g, 3.47 mmol). The mixture was then treated with N-methyl-(4-phenoxybenzyl)amine (10 g, 4.74 mmol) in 20 mL of dimethylformamide dropwise over 20 minutes. After stirring at ambient temperature overnight, the dimethylformamide was removed under reduced pressure to give a crude solid mixture. The crude solid was taken up in ethyl acetate, washed with dilute hydrochloric acid, water and saturated sodium chloride solution, dried over sodium sulfate, filtered and concentrated *in vacuo.* When solid began precipitating from solution, the mixture was cooled over an ice bath for 1 hour and then filtered. The combined filtrates were evaporated to give an oil product which was chromatographed on silica gel eluting with 94:5:1 chloroform-methanol-acetic acid to give 3 g (28%) of the title compound. m.p. 105-108 °C. ¹H NMR (DMSO-d₆, 300 MHz) δ 2.91 (s, 6H), 3.30 (d, 2H), 4.02 (d, 2H), 4.20 (d, 2H), 4.37 (d, 2H), 6.89-7.20 (m, 18H). MS m/e 623 (M+H)⁺.

### Example 14

### (1α,2β,3β,4α)-1,2-Di(N-methyl-N-(4-benzyloxybenzyl)aminocarbonyl]cyclobutane-3,4-dicarboxylic acid

To a solution of 1,2,3,4-cyclobutanecarboxylic dianhydride (0.44 g, 2.29 mmol) and triethylamine (0.63 mL, 4.58 mmol) in dimethylformamide (20 mL) at 0 °C under nitrogen was added the compound resulting from Example 5 (1.3 g, 5.75 mmol). The reaction was stirred at 0 °C for 2 hours and then allowed to warm to ambient temperature overnight. The DMF was removed under reduced pressure, and the oily residue was partitioned between ethyl acetate and water. The organic phase was washed with 1 N HCI, water and brine, dried over magnesium sulfate, filtered and evaporated under reduced pressure to give crude product. Flash silica gel chromatography eluting with 94:5:1 CHCl₃-MeOH-HOAC yielded 22% of the 1,2-di[N-methyl-N-(benzyloxybenzyl)aminocarbonyl]cyclobutane-3,4-dicarboxylic acid isomer. m.p. 210.212°C. MS m/e 651 (M+H)⁺.

### Example 15

### (1α,2β,3β,4α)-1,2-Di[N-benzyl-N-(4-phenoxybenzyl)aminocarbonyl]cyclobutane-3,4-dicarboxylic acid

To a solution of 1,2,3,4-cyclobutanetetracarboxylic dianhydride (0.5 g, 2.54 mmol) in THF (5 mL) was added N-benzyl-N-(4-phenoxybenzyl) amine (1.47 g, 5.08 mmol) in THF (5 mL) at 25 °C. The initial slurry became homogeneous after stirring at 25 °C for 25 minutes. The solution was stirred one additional hour, then poured into 100 mL of ethyl acetate. The organic layer was washed successively with 50 mL 1N H₃PO₄, 50 mL 10% NaHCO₃ and 50 mL 10% NaCI, then dried over anhydrous sodium sulfate, filtered and solvent removed *in vacuo* to afford 2.2 g of a white foamy solid. The crude product containing both isomers was purified by silica gel chromatography eluting with 94:5:1 CHCl₃-MeOH-HOAC. The slower moving product was isolated in 6% yield and characterized as 1,2-Di[N-benzyl-N-(4-phenoxybenzyl)aminocarbonyl]cyclobutane-3,4-dicarboxylic acid. ¹H NMR (CDCl₃ + CD₃OD, 500 MHz) δ 3.95-4.05 (m, 2H), 4.39-4.16 (m, 8H), 4.41-4.52 (m, 2H), 4.65-4.76 (m, 2H), 6.82-7.36 (m, 28H). MS (DC/NH₃) m/e 775 (M+H)⁺.

### Examples 16-30

The following examples were prepared using the general procedures described in Example 13.

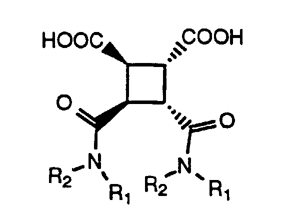

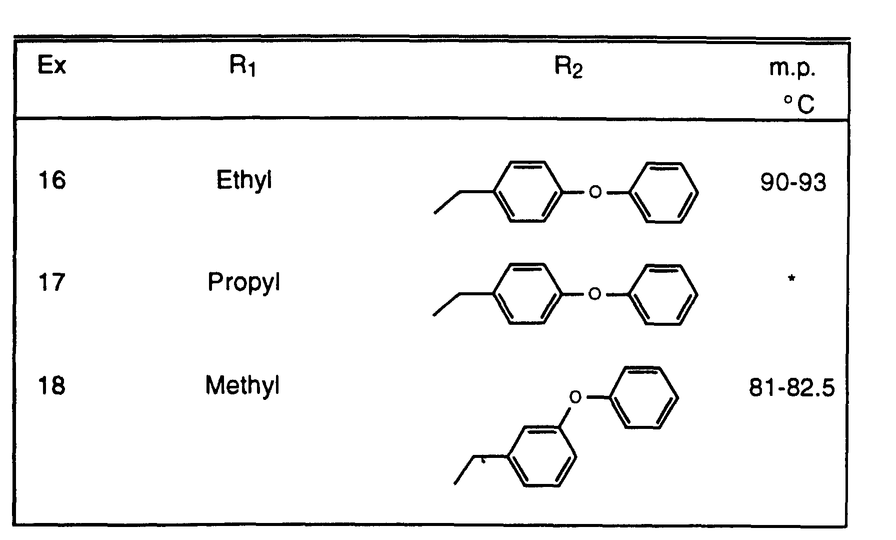

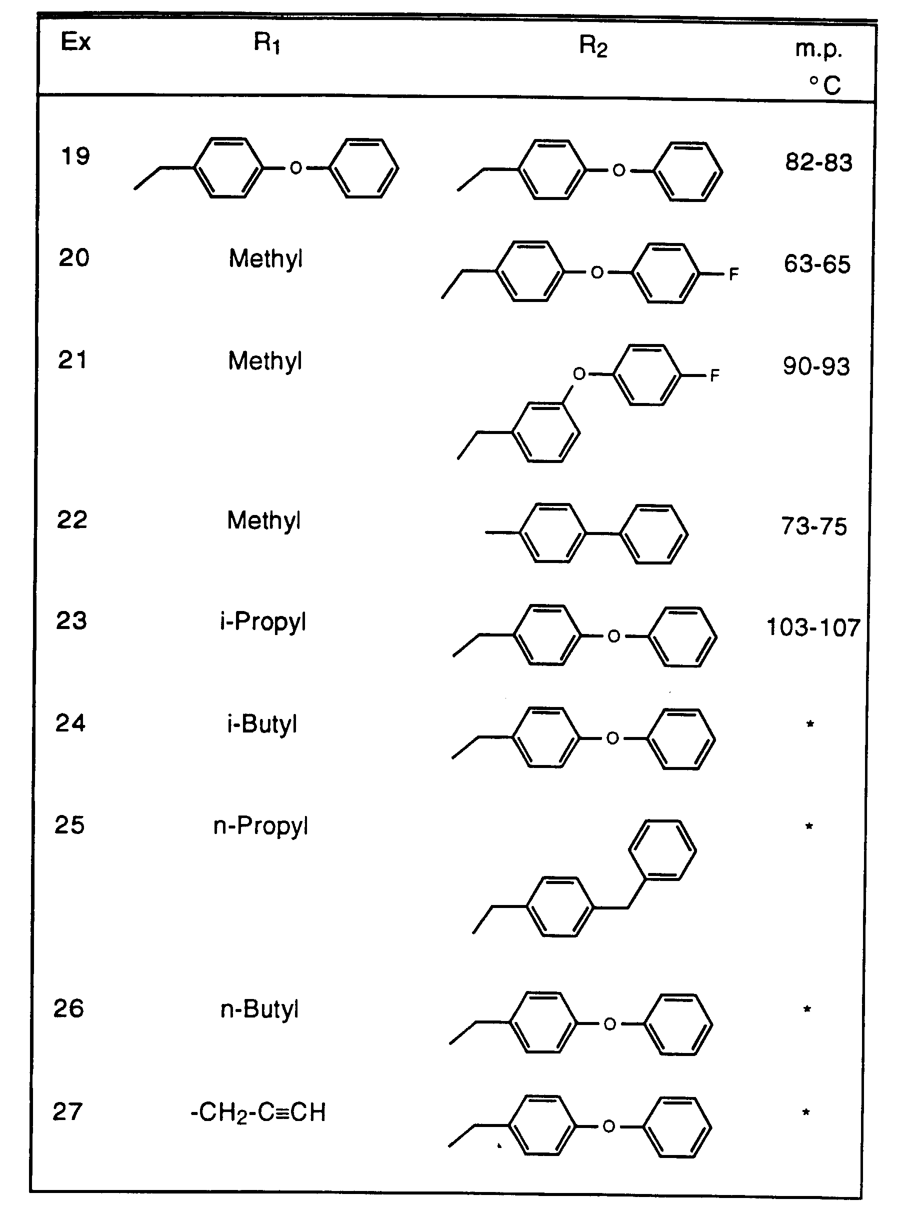

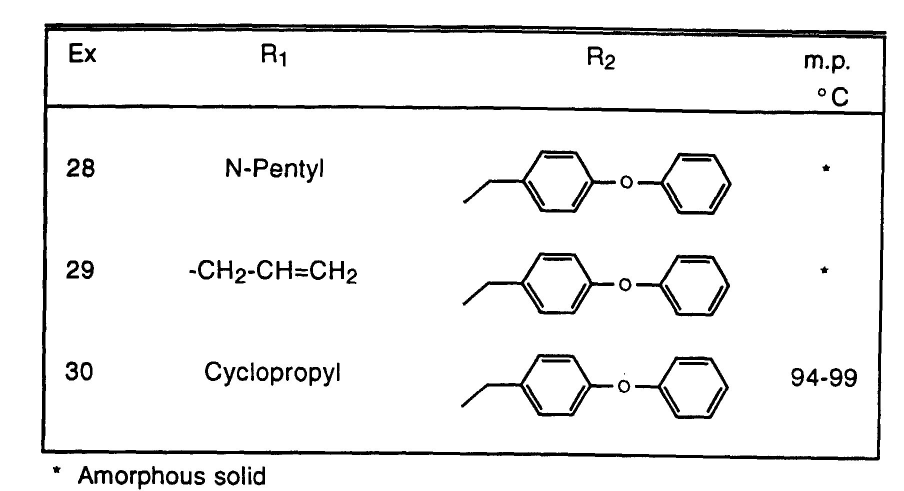

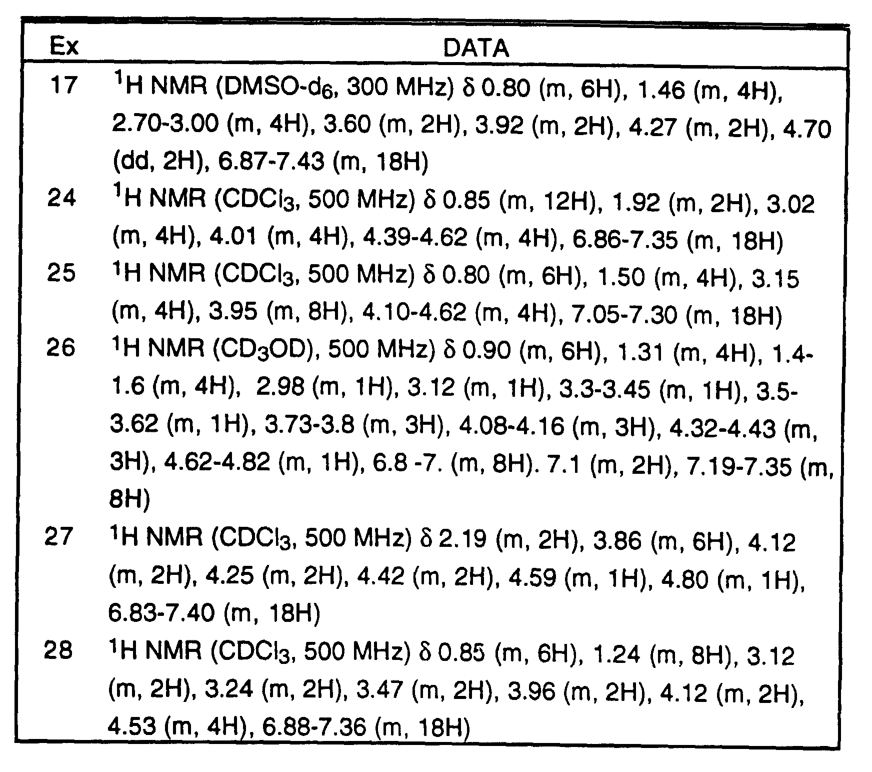

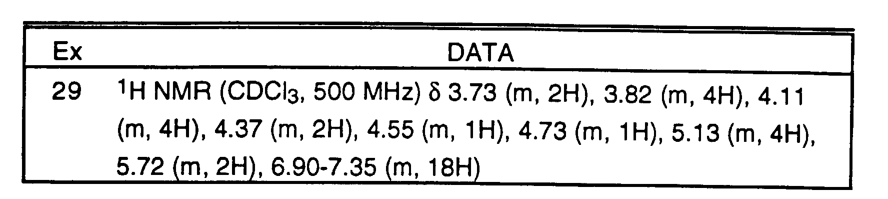

### Example 31

### (1α,2β,3β,4α)-1,2-Di[N-cyclohexylmethyl-N-phenoxybenzyl)aminocarbonyl]cyclobutane-3,4-dicarboxylic acid

To a slurry of 1,2,3,4-cyclobutanetetracarboxylic dianhydride (0.3 g, 1.5 mmol) in CH₃CN (1 mL) was added N-cyclohexylmethyl-N-phenoxybenzylamine (0.95 g, 3.21 mmol) in CH₃CN (10 mL). The slurry was stirred for 5 minutes at 20 °C resulting in a homogeneous solution. The solution was stirred for 20 hours at 20 °C then concentrated *in vacuo* to a white foam. The foam was dissolved in 100 mL of ethyl acetate and washed successively with 50 mL 1 N H₃PO₄ and 10% NaCI, then dried over anhydrous sodium sulfate, filtered and solvent removed *in vacuo* to afford 1.0 g of a white foamy solid. The crude product containing both isomers was purified by silica gel chromatography eluting with 94:5:1 CHCl₃-MeOH-HOAc. The slower moving product was isolated in 14% yield and characterized as 1,2-di[N-cyclohexylmethyl-N-phenoxybenzyl)aminocarbonyl]cyclobutane-3,4-dicarboxylic acid. ¹H NMR (CDCl₃, 500 MHz) δ 7.32-6.87 (m, 18H), 4.67-4.36 (m, 4H), 3.88-4.13 (m, 4H), 2.95-3.20 (m, 4H), 1.52-1.69 (m, 8H), 1.07-1.14 (m, 8H), 0.78-0.88 (m, 6H). MS (FAB⁺) m/e 787, (FAB⁻) 785.

### Example 32

### (1α,2β,3β-4a)-1,2-Di[N-phenyl-N-phenoxybenzyl)aminocarbonyl]cyclobutane-3,4-dicarboxylic acid

To a slurry of 1,2,3,4-cyclobutanetetracarboxylic dianhydride (0.5 g, 2.5 mmol) in DMF (5 mL) was added N-phenyl-N-phenoxybenzylamine HCI (1.6 g, 5.11 mmol), prepared by the procedures described in Example 11, in DMF (5 mL) followed by Et₃N (0.71 mL, 5.11 mmol). The slurry was stirred for 5 minutes at 20 °C resulting in a homogeneous solution. The solution was stirred 20 hours at 20 °C, then concentrated *in vacuo* to a white foam. The foam was dissolved in 100 mL of ethyl acetate and washed successively with 50 mL 1 N H₃PO₄ and 10% NaCI, then dried over anhydrous sodium sulfate, filtered and solvent removed *in vacuo* to afford 0.5 g of a clear oil. The crude product containing both isomers was purified by silica gel chromatography eluting with 94:5:1 CHCl₃-MeOH-HOAc. The slower moving product was isolated in 3% yield and characterized as 1,2-di[N-phenyl-N-phenoxybenzyl)aminocarbonyl]cyclobutane-3,4-dicarboxylic acid. ¹H NMR (DMSO-d₆, 300 MHz) δ 7.44.6.81 (m, 28H), 4.99-4.60 (m, 4H), 3.44-3.21 (m, 4H). MS (FAB⁺) m/e 747, (FAB⁻) 745.

### Example 33

### (1α,2β,3β,4α)-1,2-Di[N-4-methoxybenzyl-N-4-phenoxybenzyl)aminocarbonyl]cyclobutane-3,4-dicarboxylic acid

Following the procedures described in Example 31 and substituting N-4-methoxybenzyl-N-4-phenoxybenzylamine (1.02 g, 3.4 mmol), prepared by the procedures described in Example 11, for N-cyclohexylmethyl-N-phenoxybenzylamine afforded 1.2 g of crude product as a white foamy solid. The crude product containing both isomers was purified by silica gel chromatography eluting with 94:5:1 CHCl₃-MeOH-HOAc. The slower moving product was isolated in 27% yield and characterized as the title compound. ¹H NMR (CDCl₃, 500 MHz) δ 7.32.6.75 (m, 26H), 4.69-4.62 (m, 4H), 4.45-4.44 (m, 1H), 4.21-4.32 (m, 4H), 3.92-3.98 (m, 1H), 3.69-3.74 (m, 8H). MS (FAB⁺) m/e 835, (FAB⁻) 833.

### Example 34

### (1α,2β,3β,4α)-1,2-Di[N-(S)-α-methylbenzyl-N-4-phenoxybenzyl)aminocarbonyl]cyclobutane-3,4-dicarboxylic acid

To a slurry of 1,2,3,4-cyclobutanetetracarboxylic dianhydride (0.3 g, 1.5 mmol) in CH₃CN (6 mL) was added N-4-phenoxybenzyl-N-(*S*)-α-methylbenzylamine (1.02 g, 3.4 mmol), prepared by the procedures described in Example 11, in CH₃CN (3 mL) containing Et₃N (0.1 mL, 0.7 mmol). The slurry was stirred for 5 minutes at 20 °C, resulting in a homogeneous solution which was stirred 20 hours at 20 °C. The solution was diluted with 100 mL of ethyl acetate and washed successively with 50 mL 1 N H₃PO₄ and 10% NaCI, then dried over anhydrous sodium sulfate, filtered and solvent removed *in vacuo* to afford 0.9 g of a white foamy solid. The crude product containing both isomers was purified by silica gel chromatography eluting with 94:5:1 CHCl₃-MeOH-HOAc. The slower moving product was isolated in 12% yield and characterized as the title compound. ¹H NMR (DMSO-d₆, 300 MHz) δ 1.28.2.05 (m, 6H), 3.58-3.98 (m, 4H), 4.01-4.16 (m, 1H), 4.19-4.41 (m, 1H), 4.71-4.76 (m, 1H), 4.91-5.18 (m, 1H), 6.02-6.06 (m, 2H), 6.72-7.38 (m, 28H), 10.12 (bs, 2H). MS (FAB⁺) m/e 803, (FAB⁻) 801.

### Example 35

### (1α,2β,3β,4α)-1,2-Di[N-(R)-α-methylbenzyl-N-4-phenoxybenzyl)aminocarbonyl]cyclobutane-3,4-dicarboxylic acid

Following the procedures described in Example 34 and substituting N-4-phenoxybenzyl-N-(*R*)-α-methylbenzylamine (1.02 g, 3.4 mmol), prepared by the procedures described in Example 11, for N-4-phenoxybenzyl-N-(*S*)-α-methylbenzylamine afforded 1.0 g of crude product as a white foamy solid. The crude product containing both isomers was purified by silica gel chromatography eluting with 94:5:1 CHCl₃-MeOH-HOAc. The slower moving product was isolated in 17% yield and characterized as the title compound. ¹H NMR (CDCl₃, 500 MHz) δ 1.38.1.54 (m, 6H), 3.60-3.82 (m, 4H), 4.01-4.05 (m, 1H), 4.15-4.18 (m, 1H), 4.27-4.30 (m, 1H), 4.44-4.48 (m, 1H), 5.96-5.97 (m, 2H), 6.69-7.46 (m, 28H), 10.12 (bs, 2H). MS(FAB⁺) m/e 803, (FAB⁻) 801.

### Example 36

### (1α,2β,3β,4α)-1,2-Di[N-benzyl-N-(5-phenyl-2,4-pentadienyl)aminocarbonyl]cyclobutane-3,4-dicarboxylic acid

Following the procedures described in Example 34 and substituting N-(5-phenyl-2,4-pentadienyl)-N-benzylamine (1.02 g, 3.4 mmol), prepared by the procedures described in Example 11, for N-4-phenoxybenzyl-N-(*S*)-α-methylbenzylamine afforded 0.9 g of crude product as a white foamy solid. The crude product containing both isomers was purified by silica gel chromatography eluting with 94:5:1 CHCl₃-MeOH-HOAc. The slower moving product was isolated in 11% yield and characterized as the title compound. ¹H NMR (DMSO-d₆, 300 MHz) δ 3.35.3.72 (m, 4H), 3.88-4.16 (m, 4H), 4.19-4.41 (m, 2H), 4.67-4.87 (m, 2H), 5.30-5.40 (m, 2H), 5.70-5.83 (m, 2H), 6.26-6.43 (m, 2H), 6.53-6.75 (m, 2H), 6.81-6.98 (m, 2H), 7.21-7.44 (m, 18H), 12.55 (bs, 2H). MS(FAB⁺) m/e 695.

### Example 37

### (-)-(1α,2β,3β,4α)-1,2-Di[N-propyl-N-(4-phenoxybenzyl)aminocarbonyl]cyclobutane-3,4-dicarboxylic acid

### Example 37A

### (R)-sec-Phenethyl (1α,2β,3β,4α)-1,2-di[N-propyl-N-(4-phenoxybenzyl)aminocarbonyl]cyclobutane-3,4-dicarboxylate

A solution of 1,3-dicyclohexylcarbodiimide (1.26 g, 6.0 mmol), 4-dimethylaminopyridine (72 mg, 0.6 mmol), and 80 mL diethylether was added dropwise to a 0 °C solution of (+/-)-1,2-di[N-propyl-N-(phenoxybenzyl)aminocarbonyl]cyclobutane-3,4-dicarboxylic acid, the compound resulting from Example 17, (2.0 g, 2.9 mmol), *R*-(-)-*sec* phenethyl alcohol (0.73 g, 6.0 mmol), and 20 mL diethylether. The reaction mixture was stirred for 0.25 hour at 0 °C and for 18 hours at room temperature, then was diluted with diethylether and filtered. The filtrate was washed with 0.5 N HCI, H₂O, saturated aqueous NaHCO_{3,} and was dried over MgSO₄, filtered, and solvent evaporated under reduced pressure to afford 2.6 g of crude product as a white foam. The crude product was chromatagraphed on silica gel, eluting with 20% diethylether in hexanes to afford 0.22 g of the desired product as a single diastereomer. ¹H NMR (CDCl₃, 300MHz) δ 0.70.0.81 (m, 6H), 1.20-1.31 (m, 2H), 1.35-1.46 (m, 2H), 1.50-1.65 (m, 6H), 2.75-2.95 (m, 2H), 3.10-3.25 (m, 2H), 3.82-4.18 (m, 6H), 4.31-4.48 (m, 2H), 5.72-5.83 (m, 1H), 5.85-5.96 (m, 1H), 6.84-7.02 (m, 10H), 7.02-7.15 (m, 4H), 7.22-7.40 (m, 14H). MS (FAB) m/e 925 (m+K)⁺.

### Example 37B

### (-)-(1α,2β,3β,4α)-1,2-Di[N-propyl-N-(4-phenoxybenzyl)aminocarbonyl]cyclobutane-3,4-dicarboxylic acid

The compound resulting from Example 37A (0.22 g, 0.2 mmol) was dissolved in 100 mL of EtOAc and hydrogenated at 4 atmospheres of hydrogen at room temperature over Pd/C (0.15 g, anhydrous) for 23 hours. The reaction mixture was filtered and solvent evaporated *in vacuo* to afford 0.14 g of crude product as a light yellow solid. The crude product was triturated with hexane/ether to afford 0.13 g as a colorless glass. The glass was lyophilized to afford 0.11 g of the title compound as a white powder. ¹H NMR (CD₃OD, 300 MHz) δ 0.82-0.97 (m, 6H), 1.48-1.68 (m, 4H), 2.88-3.00 (m, 1H), 3.02-3.20 (m, 1H), 3.29-3.34 (m, 3H), 3.45-3.68 (m, 1H), 3.72-3.85 (m, 2H), 4.08-4.22 (m, 2H), 4.31-4.44 (m, 2H), 4.65-4.81 (m , 2H), 6.86-7.00 (m, 8H), 7.05-7.14 (m, 2H), 7.18-7.38 (m, 8H). MS (FAB) m/e 701 (m+Na)⁺. Anal calcd for C₄₀H₄₂N₂O₈ · 1.5 H₂O: C, 68.07; H, 6.43; N, 3.97. Found: C, 68.27; H, 6.01; N, 3.92. [α]_{D} = -77.4° (c=0.90, MeOH).

### Example 38

### (1α,2β,3β,4α)-1,2-Di[N-propyl-N-(2-(4-phenoxyphenyl)ethyl)aminocarbonyl]cyclobutane-3,4-dicarboxylic acid

### Example 38A

### 4-Phenoxystyrene

To a suspension of methyltriphenylphosphonium bromide (7.85 g, 22 mmol) in THF (10 mL) was added potassium *tert*-butoxide (1.0 M solution in THF, 22 mL). After 30 minutes, 4-phenoxybenzaldehyde (3.96 g, 20 mmol) was added to the above mixture. The reaction was diluted with equal volume of hexane after 20 minutes and filtered through silica gel (80 g). The residue was rinsed and washed with 20% ether in hexane. Concentration of the filtrate *in vacuo* gave the crude product as an off white solid which was used without further purification.

### Example 38B

### 2-(4-Phenoxyphenyl)ethyl alcohol

To the compound resulting from Example 38A in THF (40 mL) was added borane-methyl sulfide solution (10 M, 1.6 mL, 16 mmol). After 3 hours, the reaction was cooled with an ice-water bath, and anhydrous ethanol (5 mL) was added carefully to destroy excess borane. Aqueous sodium hydroxide (15%, 4 mL) was added, followed by 30% hydrogen peroxide (4 mL). The resulting mixture was refluxed for 1 hour, cooled to room temperature, then extracted with ether (80 mL). The organic phase was then washed with water (20 mL x 2), brine (20 mL), dried with anhydrous magnesium sulfate, filtered, and concentrated *in vacuo.* The residue was then purified by column chromatography eluting with 20% ethyl acetate in hexane, followed by 100% ether to give the title compound as the second fraction (2.32 g, 54%, 2 steps). ¹H NMR (300 MHz, CDCl₃) δ 7.37-6.94 (m, 9H), 3.87 (q, 2H), 2.86 (t, 2H), 1.37 (t, 1H).

### Example 38C

### 4-(2-Chloroethyl)phenyl phenyl ether

To a -78°C solution of the compound resulting from Example 38B (3.64 g, 17.0 mmol) in anhydrous methylene chloride (40 mL) was slowly added phosphorus trichloride (2.61 g, 19.0 mmol). The cold bath was then removed, and the reaction was allowed to warm to room temperature over 1 hour. The reaction mixture was then slowly poured into an ice-cooled saturated sodium bicarbonate solution (50 mL), and extracted with ether (100 mL). The organic phase was washed with water (30 mL x 2) and brine (20 mL), dried over anhydrous magnesium sulfate, filtered through silica gel, and concentrated *in vacuo.* The crude product was used without further purification for the next step. ¹H NMR (300 MHz, CDCl₃) δ 7.72-6.93 (m, 9H), 3.56 (t, 2H), 3.15 (t, 2H).

### Example 38D

### 2-(4-Phenoxyphenyl)ethylphthalimide

A solution of the product resulting from Example 38C and potassium phthalimide (3.78g, 20.4 mmol) in THF (40 mL) was refluxed for 16 hours, then concentrated to near dryness. The residue was titraturated with 1:1 ether-hexane (5 mL), and the solid was filtered and air dried. The crude product was used directly for the next step. ¹H NMR (300 MHz, CDCl₃) δ 7.85 (m, 2H), 7.72 (m, 2H), 7.33-6.92 (m, 9H), 3.92 (t, 2H), 2.98 (t, 2H).

### Example 38E

### 2-(4-Phenoxyphenyl)ethylamine

A suspension of the crude compound resulting from Example 38D in ethanol (20 mL) was refluxed with hydrazine (0.65 mL, 20.4 mmol). The reaction mixture became homogeneous after about 10 minutes, and a white precipitate started to form in a short time. After 16 hours of refluxing, the reaction mixture was cooled to room temperature, filtered, and washed with ethanol (5 mL x 3). The filtrate was diluted with ether (100 mL) and washed with 10% sodium carbonate (20 mL), water (30 mL) and brine (20 mL), dried over anhydrous potassium carbonate, filtered and concentrated *in vacuo.* The crude amine was used directly for the next step. ¹H NMR (300 MHz, CDCl₃) δ 7.35-6.94 (m, 9H), 2.98 (t, 2H), 2.72 9t, 2H)

### Example 38F

### N-(2-(4-Phenoxy)phenyl)ethylpropionamide

To the solution of crude amine resulting from Example 38E in methylene chloride (20 mL) and pyridine (5 mL) was added propionyl chloride (3.0 mL, 34 mmol). After 6 hours, the reaction was diluted with ethyl acetate (80 mL), washed with water (20 mL), 10% aqueous hydrochloric acid (20 mL), water (20 mL), saturated copper sulfate (20 mL), and brine (20 mL), dried over anhydrous magnesium sulfate, filtered, and concentrated *in vacuo.* The residue was purified by column chromatography eluting with 50% ethyl acetate in hexane to give the title compound (1.23 g, 27% for 4 steps). ¹H NMR (300 MHz, CDCl₃): δ 7.36-6.95 (m, 9H), 5.46 (bs, 1H), 3.51 (q, 2H), 2.80 (t, 2H), 2.19 (q, 2H), 1.15 (t, 3H).

### Example 38G

### N-Ethyl-N-(2-(4-phenoxy)phenylethyl)amine

To a solution of the amide resulting from Example 38F (1.23 g, 4.57 mmol) in THF (10 mL) was slowly added lithium aluminum hydride (1.0 M in THF, 4.6 mL). After the resulting mixture was refluxed for 3 hours, it was cooled to 0 °C, and water (0.18 mL), 15% aqueous NaOH (0.18 mL), and water (0.54 mL) were carefully added. White precipitate formed, and the mixture was filtered through celite, washed with ether, and concentrated *in vacuo* to afford pure title compound (1.05 g, 88%). ¹H NMR (300 MHz, CDCl₃) δ 7.35.6.92 (m, 9H), 3.30 (bs, 1H), 2.83 (m, 2H), 2.60 (t, 2H), 2.37 (dd, 2H), 1.49 (m, 2H), 0.92 (t, 3H).

### Example 38H

### (1α,2β,3β,4α)-1,2-Di[N-propyl-N-(2-(4-phenoxyphenyl)ethyl)aminocarbonyl]cyclobutane-3,4-dicarboxylic acid

A mixture of the amine resulting from Example 38G (0.942 g, 3.69 mmol), 1,2,3,4-cyclobutanetetracarboxylic dianhydride (0.290 g, 1.48 mmol), triethylamine (0.62 mL, 4.4 mmol) and DMAP (50 mg) in acetonitrile (10 mL) was stirred overnight. The reaction mixture was concentrated *in vacuo* and partitioned between 20% aqueous HCl and 3 portions of ethyl acetate. The combined organic extracts were washed with brine, dried over magnesium sulfate, filtered and concentrated *in vacuo.* The residue was purified by column eluting with 1:1 CHCl₃-EtOAc, followed by 50:50:2:1 CHCI₃-EtOAc-MeOH-AcOH to give 1,3-diamide-2,4-diacid as the first fraction (0.436 g, 42%) followed by the title compound (0.477 g, 46%). ¹H NMR (500 MHz, DMSO) δ 7.40-6.91 (m, 18H), 3.04 & 2.95 (2 m's, 8H), 2.81 & 2.39 (m, 4H), 2.93 & 2.82 (2 m's, 4H), 1.64 & 1.46 (2 m's, 4H), 0.91 & 0.82 (2 t's, 6H). MS (FAB⁻) m/e 705 (M-H).

### Example 39

### (1α,2β,3β,4α)-1,2-Di[N-propyl-N-(4-phenoxyphenyl)aminocarbonyl]cyclobutane-3,4-dicarboxylic acid

### Example 39A

### N-(4-Phenoxy)phenylpropionamide

To a solution of 4-phenoxyaniline (4.62 g, 25 mmol) in methylene chloride (50 mL) and pyridine (10 mL) was added propionyl chloride (3.3 mL, 37.5 mmol). After 1 hour, the reaction mixture was diluted with ether (150 mL), washed with water (50 mL), 10% sodium carbonate (50 mL), 10% HCI (50 mL), saturated copper sulfate (50 mL), water (50 mL) and brine (30 mL), dried over anhydrous magnesium sulfate, filtered, and concentrated *in vacuo.* The title compound amide was used without further purification. ¹H NMR (300 MHz, CDCI₃) δ 7.49 9d, 2H), 7.31 (t, 2H), 7.11-6.95 (m, 5H), 2.40 (q, 2H), 1.25 (t, 3H).

### Example 39B

### N-Propyl-(4-phenoxy)aniline

To a solution of the crude amide resulting from Example 39A in THF (30 mL) was added lithium aluminum hydride (1.0 M in THF, 20 mL). After 14 hours, the reaction was cooled in an ice bath, and water (0.80 mL), 15% NaOH (0.80 mL) and water (2.4 mL) were added sequentially. The resulting mixture was filtered through celite, washed with ether, and concentrated *in vacuo* to give the title compound (5.16 g, 91 % for 2 steps). ¹H NMR (300 MHz, CDCl₃) δ 7.28 (m, 2H), 6.99 9t, 1H), 6.92 (m, 4H), 6.60 (d, 2H), 3.55 (bs, 1H), 3.06 (t, 2H), 1.65 (sextet, 2H), 1.01 (t, 3H).

### Example 39C

### (1α,2β,3β,4α)-1,2-Di[N-propyl-N-(4-phenoxyphenyl)aminocarbonyl]cyclobutane-3,4-dicarboxylic acid

A mixture of the compound resulting from Example 39B (0.908 g, 4.0 mmol), 1,2,3,4-cyclobutanetetracarboxylic dianhydride (0.314 g, 1.6 mmol), triethylamine (0.69 mL, 4.8 mmol) and DMAP (20 mg) in acetonitrile (10 mL) were reacted, worked up and purified by the procedures described in Example 38H to give the 1,3-diamide-2,4-diacid as the first fraction (0.372 g, 36%) and the title compound as the second fraction (0.514 g, 49%). ¹H NMR (500 MHz, CDCl₃) δ 7.40-6.98 (m, 18H), 3.80-3.36 (m, 8H), 1.48 (m, 4H), 0.82 (t, 6H). MS (Cl) m/e 651 (M+H).

### Example 40

### (1α,2β,3β,4α)-1,2-Di[N-(2-methoxyethyl)-N-(4-phenoxybenzyl)aminocarbonyl]cyclobutane-3,4-dicarboxylic acid

### Example 40A

### N-(2-Methoxy)ethyl-N-(4-phenoxy)benzylamine

A mixture of 4-phenoxybenzaldehyde (1.98 g, 10 mmol) and 2-methoxyethylamine (0.751 g, 10 mmol) in ethanol (10 mL) was stirred for 1 hour. Acetic acid (1 mL) and sodium cyanoborohydride (10 mmol) were added, and the reaction was stirred an additional 14 hours. The reaction mixture was then partitioned between ether and 10% aqueous sodium hydroxide solution. The organic layer was further washed with water and brine, dried over anhydrous potassium carbonate, filtered and concentrated *in vacuo* to give the title compound (2.48 g, 97%). ¹H NMR (300 MHz, CDCl₃) δ 7.30 (m, 4H), 7.09 (t, 1H), 6.98 (m, 4H), 3.80 (s, 2H), 3.51 (t, 2H), 3.37 9s, 3H), 2.51 (t, 2H).

### Example 40B

### (1α,2β,3β,4α)-1,2-Di[N-(2-methoxyethyl)-N-(4-phenoxybenzyl)aminocarbonyl]cyclobutane-3,4-dicarboxylic acid

A mixture of the compound resulting from Example 40A (1.38 g, 5.36 mmol), 1,2,3,4-cyclobutanetetracarboxylic dianhydride (0.421 g, 2.15 mmol), triethylamine (0.90 mL, 6.5 mmol), and DMAP (50 mg) in acetonitrile (15 mL) were reacted, worked up and purified by the procedures described in Example 38H to give the 1,3-diamide-2,4-diacid as the first fraction (0.468 g, 31%) and the title compound as the second fraction (0.807 g, 53%). ¹H NMR (500 MHz, DMSO) δ 7.40-6.97 (m, 18H), 4.87-4.66 (m, 2H), 4.45-4.26 (m, 2H), 4.13 - 3.88(m, 2H), 3.67-3.48 (m, 4H), 3.47-3.30 (m, 4H), 3.19-3.13 (4 s's, 6H), 3.20-3.01 (m, 2H). MS (FAB⁺) m/e 711 (M+H).

### Example 41

### (1α,2β,3β,4α)-1,2-Di[N-(2-methylthioethyl)-N-(4-phenoxybenzyl)aminocarbonyl]cyclobutane-3,4-dicarboxylic acid

### Example 41A

### N-(2-Methylthio)ethyl-N-(4-phenoxy)benzylamine

Following the procedures described in Example 40A, 4-phenoxybenzaldehyde (1.98 g, 10 mmol) and 2-(methylthio)ethylamine (0.912 g, 10 mmol) were combined to give the title compound (2.53 g, 93%). ¹H NMR (300 MHz, CDCl₃) δ 7.32 (m, 4H), 7.09 (t, 1H), 6.97 (m, 4H), 3.80 (s, 2H), 2.84 9t, 2H), 2.68 (t, 2H), 2.10 (s, 3H).

### Example 41B

### (1α,2β,3β,4α)-1,2-Di[N-(2-methylthioethyl)-N-(4-phenoxybenzyl)aminocarbonyl]cyclobutane-3,4-dicarboxylic acid

A mixture of the compound resulting from Example 41A (0.682 g, 2.5 mmol), 1,2,3,4-cyclobutanetetracarboxylic dianhydride (0.196 g, 1.0 mmol), triethylamine (0.42 mL, 3.0 mmol) and DMAP (12 mg) in acetonitrile (10 mL) were reacted, worked up and purified by the procedures described in Example 38H to give the 1,3-diamide-2,4-diacid as the first fraction (0.214 g, 29%) and the title compound as the second fraction (0.274 g, 37%). ¹H NMR (500 MHz, CDCI₃) δ 7.32-6.87 (m, 18H), 4.70-4.14 (m, 6H), 3.97-3.84 (m, 2H), 3.76-3.23 (m, 4H), 2.53 (m, 4H), 2.02 (4 s's, 6H). MS (FAB⁺) m/e 743 (M+H).

### Example 42

### (1α,2β,3β,4α)-1,2-Di[N-(2-ethylthioethyl)-N-(4-phenoxybenzyl)aminocarbonyl]cyclobutane-3,4-dicarboxylic acid

Following the procedures described in Example 40A, 4-phenoxybenzaldehyde (1.98 g, 10 mmol), 2-(ethylthio)ethylamine hydrogen chloride (1.42 g, 10 mmol) and 20 mmol of sodium acetate were combined to give N-(2-ethylthio)ethyl-N-(4-phenoxy)benzylamine (2.72 g, 95%). ¹H NMR (300 MHz, CDCl₃): δ 7.33 (m, 4H), 7.09 (t, 1H), 6.98 (m, 4H), 3.78 (s, 2H), 2.84 (t, 2H), 2.71 (t, 2H), 2.54 (q, 2H), 1.26 (t, 3H).

A mixture of the amine prepared above (0.718 g, 2.5 mmol), 1,2,3,4-cyclobutanetetracarboxylic dianhydride (0.196 g, 1.0 mmol), triethylamine (0.42 mL, 3.0 mmol) and DMAP (24 mg) in acetonitrile (10 mL) were reacted, worked up and purified by the procedures described in Example 38H to give the 1,3-diamide-2,4-diacid as the first fraction (0.207 g, 27%) and the title compound as the second fraction (0.398 g, 52 %). ¹H NMR (500 MHz, CDCI₃) δ 7.32-6.87 (m, 18H), 4.73-4.12 (m, 6H), 3.98-3.86 (m, 2H), 3.78-3.25 (m, 4H), 2.59 (m, 4H), 2.46 (m, 4H), 1.17 (m, 6H). MS (FAB⁺) m/e 771 (M+H).

### Example 43

### (1α,2β,3β,4α)-1,2-Di[N-(2-fluoroethyl)-N-(4-phenoxybenzyl)aminocarbonyl]cyclobutane-3,4-dicarboxylic acid

Following the procedures described in Example 40A, 4-phenoxybenzaldehyde (1.98 g, 10 mmol), 2-fluoroethylamine hydrogen chloride (0.995 g, 10 mmol) and 20 mmol of sodium acetate were combined to give N-(2-fluoro)ethyl-N-(4-phenoxy)benzylamine (2.15 g, 88%). ¹H NMR (300 MHz, CDCI₃) δ 7.33 (m, 4H), 7.09 (t, 1H), 6.97 (m, 4H), 4.57 (dt, 2H), 3.73 (s, 2H), 2.94 (dt, 2H).

A mixture of the amine prepared above (0.613 g, 2.5 mmol), 1,2,3,4-cyclobutanetetracarboxylic dianhydride (0.196 g, 1.0 mmol), triethylamine (0.42 mL, 3.0 mmol) and DMAP (12 mg) in acetonitrile (10 mL) were reacted, worked up and purified by the procedures described in Example 38H to give the 1,3-diamide-2,4-diacid as the first fraction (0.195 g, 28%) and the title compound as the second fraction (0.377 g, 55 %). ¹H NMR (500 MHz, DMSO) δ 7.39.6.90 (m, 18H), 4.88-4.73 (m, 2H), 4.65-4.15 (m, 6H), 4.06-3.90 (m, 2H), 3.85-3.61 (m, 4H), 3.25 (m, 2H). MS (FAB⁺) m/e 687 (M+H).

### Example 44

### (1α,2β,3β,4α)-1,2-Di[N-(furan-2-ylmethyl)-N-(4-phenoxybenzyl)aminocarbonyl)cyclobutane-3,4-dicarboxylic acid

Following the procedures described in Example 40A, 4-phenoxybenzaldehyde (1.98 g, 10 mmol) and furfurylamine (0.971 g, 10 mmol) were combined to give N-(furan-2-yl)methyl-N-(4-phenoxy)benzylamine (2.43 g, 87%). ¹H NMR (300 MHz, CDCl₃) δ 7.30 (m, 5H), 7.09 (t, 1H), 6.98 (m, 4H), 6.34 (m, 1H), 6.19 (m, 1H), 3.80 (s, 2H), 3.77 (s, 2H).

A mixture of the amine prepared above (0.698 g, 2.5 mmol), 1,2,3,4-cyclobutanetetracarboxylic dianhydride (0.196 g, 1.0 mmol), triethylamine (0.42 mL, 3.0 mmol) and DMAP (12 mg) in acetonitrile (10 mL) were reacted, worked up and purified by the procedures described in Example 38H to give the 1,3-diamide-2,4-diacid as the first fraction (0.251g, 34%) and the title compound as the second fraction (0.438 g, 58 %). ¹H NMR (500 MHz, CDCl₃) δ 7.39.6.90 (m, 20H), 6.27-6.13 (m, 4H), 4.89-3.92 (m, 12H).. MS (DCI) m/e 755 (M+H).

### Example 45

### (1α,2β,3β,4α)-1,2-Di[N-(thien-2-ylmethyl)-N-(4-phenoxybenzyl)aminocarbonyl]cyclobutane-3,4-dicarboxylic acid

Following the procedures described in Example 40A, 4-phenoxybenzaldehyde (1.98 g, 10 mmol) and 2-thienylmethylamine (1.13 g, 10 mmol) were combined to give N-(thien-2-yl)methyl-N-(4-phenoxy)benzylamine (2.87 g, 97%). ¹H NMR (300 MHz, CDCl₃) δ 7.35-6.92 (m, 12H), 4.01 (s, 2H), 3.81 (s, 2H).

A mixture of the amine prepared above (0.738 g, 2.5 mmol), 1,2,3,4-cyclobutanetetracarboxylic dianhydride (0.196 g, 1.0 mmol), triethylamine (0.42 mL, 3.0 mmol) and DMAP (12 mg) in acetonitrile (10 mL) were reacted, worked up and purified by the procedures described in Example 38H to give the 1,3-diamide-2,4-diacid as the first fraction (0.193g, 25%) and the title compound as the second fraction (0.382 g, 49 %). ¹H NMR (500 MHz, CDCl₃) δ 7.37-6.82 (m, 24H), 4.89-3.97 (m, 12H). MS(DCl) m/e 787 (M+H).

### Example 46

### (1α,2β,3β,4α)-1,2-Di[N-(2-ethylthioethyl)-N-(4-phenylthiobenzyl)aminocarbonyl]cyclobutane-3,4-dicarboxylic acid

Following the procedures described in Example 40A, 4-phenylthiobenzaldehyde, 2-(ethylthio)ethylamine hydrochloride, sodium acetate and sodium cyanoborohydride are reacted to give N-(2-ethylthio)ethyl-N-(4-phenylthio)benzylamine.

A mixture of the amine prepared above, 1,2,3,4-cyclobutanetetracarboxylic dianhydride, triethylamine and DMAP in acetonitrile are reacted, worked up and purified by the procedures described in Example 38H to give the title compound.

### Example 47

### (1α,2β,3β,4α)-1,2-Di[N-(cyclopropylmethyl)-N-(4-phenoxybenzyl)aminocarbonyl]cyclobutane-3,4-dicarboxylic acid

### Example 47A

### N-(cyclopropylmethyl)-N-(4-phenoxybenzyl)amine

4-Phenoxybenzaldehyde (3.0 g, 15.1 mmol) and cyclopropylmethylamine (1.1 g, 15.1 mmol) were dissolved in methanol (85 mL) under nitrogen at room temperature. Sodium cyanoborohydride (0.95 g, 15.1 mmol) was added, and stirring was continued for 48 hours. The solvent was evaporated under reduced pressure, and the residue was suspended in ether, washed with brine, and dried over Na₂SO₄. The ether was evaporated, and the crude product was chromatographed on silica gel eluting with 3% methanol-methylene chloride to provide 3.0 g (78%) of the title compound as a colorless oil. ¹H NMR (CDCl₃, 300 MHz) δ 0.08.0.15 (m, 2H), 0.45-0.55 (m, 2H), 0.93-1.05 (m, 1H), 1.45 (br s, 1H), 2.50 (d, J=7.5 Hz, 2H), 3.78 (s, 2H), 6.95-7.03 (m, 4H), 7.05-7.13 (m, 1H), 7.25-7.38 (m, 4H). MS (DCI/NH₃) m/e 254 (M+H)⁺.

### Example 47B

### (1α,2β,3β,4α)-1,2-Di[N-(cyclopropylmethyl)-N-(4-phenoxybenzyl)aminocarbonyl]cyclobutane-3,4-dicarboxylic acid

A slurry of 1,2,3,4-cyclobutanetetracarboxylic dianhydride (1.0 g, 5.0 mmol) in acetonitrile (17 mL) under nitrogen at room temperature was treated with a solution of the compound resulting from Example 47A (2.5 g, 10.0 mmol) in acetonitrile (20 mL). The resulting suspension was refluxed for 15 hours, the solvent was evaporated, and the residue was chromatographed on silica gel eluting with 98:1:1 chloroform-methanol-acetic acid to provide a wet foam. The foam was dissolved in acetonitrile (7 mL), triturated with water, and lyophilized to provide 1.2 g (34%) of the title compound as a white powder. m.p. 89-91 °C. ¹H NMR (DMSO-d₆, 300 MHz) δ 0.01-1.25 (m, 4H), 0.27-0.52 (m, 4H), 0.80-0.96 (m, 2H), 2.68-2.92 (m, 2H), 3.20-3.55 (m, 2H), 3.56-3.68 (m, 2H), 3.89-4.98 (m, 1H), 4.00-4.10 (m, 1H), 4.28-4.58 (m, 2H), 4.65-4.89 (m, 2H), 6.85-7.04 (m, 8H), 7.08-7.30 (m, 6H), 7.33-7.43 (m, 4H), 12.11-12.20 (br s, 2H). MS (FAB) m/e 703 (M+H)⁺.

### Example 48

### (1α,2β,3β,4α)-1,2-Di[N-cyclobutyl-N-(4-phenoxybenzyl)aminocarbonyl]cyclobutane-3,4-dicarboxylic acid

Following the procedures described in Example 47A and substituting cyclobutylamine (1.8 g, 25.0 mmol) for cyclopropylmethylamine afforded 5.63 g (89%) of N-cyclobutyl-N-(4-phenoxybenzyl)amine as a colorless oil. ¹H NMR (CDCl₃, 300 MHz) δ 1.56-1.80 (m, 5H), 2.15-2.30 (m, 2H), 3.25-3.39 (m, 1H), 3.68 (s, 2H), 6.94-7.04 (m, 4H), 7.05-7.15 (tt, J=7.5, 1.5 Hz, 1H), 7.25-7.38 (m, 4H). MS (DCl/NH₃) m/e 254 (M+H)⁺.

Following the procedures described in Example 47B and using the amine prepared above (5.2 g, 20.0 mmol) provided the title compound as a wet foam after chromatography. The foam was dissolved in acetonitrile (15 mL), triturated with water, and lyophilized to provide 2.3 g (33%) of the title compound as a white powder. m.p. 110-112 °C. ¹H NMR (DMSO-d₆, 300 MHz) δ 1.44-1.64 (m, 4H), 1.80-2.19 (m, 10H), 3.48-3.57 (m, H), 3.60-3.80 (m, 2H), 3.90-4.50 (m, 1H), 4.20-4.75 (m, 4H), 6.84-7.02 (m, 8H), 7.04-7.28 (m, 6H), 7.30-7.44 (m, 4H) 12.20-12.31 (br s, 2H). MS (FAB) m/e 703 (M+H)⁺.

### Example 49

### (1α-2β,3β,4α)-1,2-Di[N-cyclopentyl-N-(4-phenoxybenzyl)aminocarbonyl]cyclobutane-3,4,dicarboxylic acid

Following the procedures described in Example 47A and substituting cyclopentylamine (2.5 g, 25.0 mmol) for cyclopropylmethylamine afforded 5.5 g (83%) of N-cyclopentyl-N-(4-phenoxybenzyl)amine as a colorless oil. ¹H NMR (CDCl₃, 300 MHz) δ 1.31-1.45 (m, 2H), 1.49-1.65 (m, 2H), 1.65-1.80 (m, 3H), 1.81-1.95 (m, 2H), 3.15 (p, J=7.5 Hz, 1H), 3.75 (s, 2H), 6.94-7.04 (m, 4H), 7.05-7.14 (m, 1H), 7.25-7.40 (m, 4H). MS (DCl/NH₃) m/e 268 (M+H)⁺.

Following the procedures described in Example 47B and using the amine prepared above (5.4 g, 20.0 mmol) provided the title compound as a wet foam after chromatography. The foam was dissolved in acetonitrile (15 mL), triturated with water, and lyophilized to provide 2.19 g (30%) of the title compound as a white powder. m.p. 115-118 °C. ¹H NMR (DMSO-d₆, 300 MHz) δ 1.35.1.85 (br m, 18H), 3.48-3.58 (m, 1H), 3.60-3.68 (m, 1H), 3.98-4.36 (m, 4H), 4.45-4.55 (m, 2H), 6.82-7.04 (m, 8H), 7.06-7.17 (m, 3H), 7.18-7.28 (m, 3H), 7.29-7.44 (m, 4H), 12.01-12.27 (br s, 2H). MS (FAB) m/e 731 (M+H)⁺.

### Example 50

### (1α,2β,3β,4α)-1,2-Di[N-cyclohexyl-N-(4-phenoxybenzyl)aminocarbonyl]cyclobutane-3,4-dicarboxylic acid

Following the procedures described in Example 47A and substituting cyclohexylamine (2.5 g, 25.0 mmol) for cyclopropylmethylamine afforded 6.4 g (91%) of N-cyclohexyl-N-(4-phenoxybenzyl)amine as a colorless oil. ¹H NMR (CDCl₃, 300 MHz) δ 1.05-1.35 (m, 5H), 1.55-1.65 (m, 1H), 1.70-1.80 (m, 3H), 1.85-2.00 (m, 2H), 2.45-2.55 (m, 1H), 3.80 (s, 2H), 6.95-7.04 (m, 4H), 7.05 (tt, J=7.5, 1.5 Hz, 1H), 7.25-7.35 (m, 4H). MS (DCl/NH₃) m/e 282 (M+H)⁺.

Following the procedures described in Example 47B and using the amine prepared above (5.6 g, 20.0 mmol) provided the title compound as a wet foam after chromatography. The foam was dissolved in acetonitrile (15 mL), triturated with water, and lyophilized to provide 2.9 g (38%) of the title compound as a white powder. m.p. 133-135°C, ¹H NMR (DMSO-d₆, 300 MHz) δ 0.82-1.78 (br m, 22H), 3.48-3.69 (m, 2H), 3.64-3.73 (m, 1H), 3.84-3.94 (m, 1H), 4.10-4.64 (m, 4H), 6.80-7.05 (m, 8H), 7.07-7.18 (m, 3H), 7.19-7.44 (m, 7H), 12.15-12.29 (br s, 2H). MS (FAB) m/e 759 (M+H)⁺.

### Example 51

### (1α,2β,3β,4α)-1,2-Di[N-(cyclopentylmethyl)-N-(4-phenoxybenzyl)aminocarbonyl]cyclobutane-3,4-dicarboxylic acid

### Example 51A

### N-(Cyclopentylmethyl)amine

A cooled (0 °C) solution of cylopentanecarbonitrile (5.0 g, 53 mmol) in THF (175 mL) was treated with 1 M LAH in THF (53 mL). The solution was refluxed for 2 hours then cooled to 0 °C and quenched with Na₂SO₄·10H₂O. The suspension was diluted with THF and filtered through a pad of celite. Removal of the solvent *in vacuo* provided 4.9 g (95%) of the title compound as a pale green oil which was used without further purification. ¹H NMR (CDCI₃, 300 MHz) δ 1.10-2.10 (br m, 10H), 2.60 (d, J=7.5 Hz, 2H), 2.74-2.79 (m, 1H). MS (DCl/NH₃) m/e 100 (M+H)⁺.

### Example 51B

### (1α,2β,3β,4α)-1,2-Di[N-(cyclopentylmethyl)-N-(4-phenoxybenzyl)aminocarbonyl]cyclobutane-3,4-dicarboxylic acid

Following the procedures described in Example 47A and substituting the compound resulting from Example 51A (1.49 g, 15.1 mmol) for cyclopropylmethylamine afforded 3.4 g, 80% of N-(cyclopentylmethyl)-N-(4-phenoxybenzyl)amine as a colorless oil. ¹H NMR (CDCl₃, 300 MHz) δ 1.10.1.24 (m, 2H), 1.45-1.67 (m, 4H), 1.71-1.90 (m, 3H), 2.06 (p, J=7.5 Hz, 1H), 2.58 (d, J=7.5 Hz, 2H), 3.79 (s, 2H), 6.95-7.05 (m, 4H), 7.09 (tt, J=7.5, 1.5 Hz, 1H), 7.28-7.38 (m, 4H). MS (DCl) m/e 282 (M+H)⁺.

Following the procedures described in Example 47B and using the amine prepared above (5.6 g, 20 mmol) provided the title compound as a wet foam after chromatography. The foam was dissolved in acetonitrile (15 mL), triturated with water, and lyophilized to provide 2.8 g (37%) of the the title compound as a white powder. m.p. 98-99 °C. ¹H NMR (DMSO-d₆, 300 MHz) δ 1.00.1.22 (m, 4H), 1.34-1.70 (m, 16H), 2.00-2.18 (m, 2H), 2.75-3.00 (m, 1H), 3.13-3.70 (m, 2H), 3.66-4.06 (m, 1H), 4.22-4.40 (m, 2H), 4.60-4.78 (m, 2H), 6.85-7.04 (m, 8H), 7.08-7.29 (m, 6H), 7.32-7.44 (m, 4H), 12.17-12.34 (br s, 2H). MS (FAB) m/e 759 (M+H)⁺.

### Example 52

### (1α,2β,3β,4α)-1,2-Di[N-(cyclobutylmethyl)-N-(4-phenoxybenzyl)aminocarbonyl]cyclobutane-3,4-dicarboxylic acid

### Example 52A

### N-(4-phenoxybenzyl)cyclobutanecarboxamide

To a cooled (0 °C) solution of cyclobutanecarboxylic acid (0.26 g, 2.6 mmol), 4-phenoxybenzylamine hydrochloride (0.62 g, 2.6 mmol), HOBt, (0.71 g, 5.2 mmol) and triethylamine (1.1 mL, 7.83 mmol) in THF (9 mL) was added EDCI (0.55 g, 2.6 mmol). The mixture was stirred for 18 hours at room temperature. The solvent was removed, and the residue was suspended in ether and washed with 0.1 N HCI, 5% NaHCO₃ and brine. The ether layer was dried (MgSO₄), filtered through a plug of silica gel and concentrated *in vacuo* to provide 0.70 g (96%) of the title compound as a white solid. m.p. 69-71 °C. ¹H NMR (CDCl_{3,} 300 MHz) δ 1.80.2.06 (m, 2H), 2.10-2.14 (m, 2H), 2.15-2.39 (m, 2H), 3.02 (pd, J=7.5, 1.2 Hz, 1H), 4.41 (d, J=6 Hz, 2H), 5.55 (br s, 1H), 6.94-7.04 (m, 4H), 7.08-7.15 (tt, J=7.5, 1.5 Hz, 1H), 7.20-7.28 (m, 2H), 7.30-7.38 (m, 2H). MS (DCI/NH₃) m/e 282 (M+H)⁺.

### Example 52B

### N-(Cyclobutylmethyl)-N-(4-phenoxybenzyl)amine

A cooled (0 °C) solution of the compound resulting from Example 52A (0.60 g, 2.1 mmol) in THF (7 mL) was treated with 1 M LAH in THF (2.1 mL). The solution was refluxed for 2 hours then cooled to 0 °C and quenched with Na₂SO₄·H10H₂O. The suspension was diluted with THF and filtered through a pad of celite. Removal of the solvent provided 0.56 g (99%) of the title compound as a colorless oil. ¹H NMR (CDCl₃, 500 MHz) δ 1.15 (br s, 1H), 1.62-1.70 (m, 2H), 1.80-1.98 (m, 2H), 2.02-2.10 (m, 2H), 2.50 (p, J=7.5 Hz, 1H), 2.65 (d, J=7.5 Hz, 2H), 3.75 (s, 2H), 6.95-7.02 (m, 4H), 7.05-7.10 (tt, J=7.5,1.2 Hz, 1H), 7.25-7.35 (m, 4H). MS (DC/NH₃) m/e 268 (M+H)⁺.

### Example 52C

### (1α,2β,3β,4α)-1,2-Di[N-(cyclobutylmethyl)-N-(4-phenoxybenzyl)aminocarbonyl]cyclobutane-3.4-dicarboxylic acid

Following the procedures described in Example 47B and using the compound resulting from Example 52B (0.25 g, 0.94 mmol) provided the title compound as a wet foam after chromatography. The foam was dissolved in acetonitrile (2 mL), triturated with water, and lyophilized to provide 100 mg (29%) of the the title compound as a white powder. m.p. 94-95 °C. ¹H NMR (DMSO-d₆, 300 MHz) δ 1.55.2.00 (m, 12H), 2.90-3.01 (m, 1H), 3.04-3.15 (m, 1H), 3.35-3.46 (m, 3H), 3.51-3.68 (m, 3H), 3.82-3.97 (m, 1H), 4.00-4.14 (m, 1H), 4.18-4.35 (m, 2H), 4.60-4.83, (m, 2H), 6.89-7.05 (m, 8H), 7.09-7.17 (m, 2H), 7.18-7.28 (m, 4H), 7.34-7.44 (m, 4H), 12.00-12.27 (br s, 2H). MS (FAB) m/e 731 (M+H)⁺.

### Example 53

### (1α,2β,3β,4α)-1,2-Di[N-(4-fluorobenzyl)-N-(4-phenoxybenzyl)aminocarbonyl]cyclobutane-3,4-dicarboxylic acid

### Example 53A

### N-(4-Fluorobenzyl)-N-(4-phenoxybenzyl)amine

4-Phenoxybenzaldehyde (1.0 g, 5.0 mmol) and 4-fluorobenzylamine (631 mg, 5.0 mmol) were dissolved in methanol (17 mL) under nitrogen at room temperature. Sodium cyanoborohydride (317 mg, 5.0 mmol) was added, and stirring was continued for 48 hours. The solvent was evaporated and the residue was suspended in ether, washed with brine, and dried over Na₂SO₄. The ether was evaporated, and the crude product was chromatographed on silica gel eluting with 3% methanol-methylene chloride to provide 1.5 g (97%) of the title compound as a colorless oil. ¹H NMR (CDCl₃, 300 MHz) δ 1.55 (s, 1H), 3.76 (s, 2H), 3.78 (s, 2H), 6.95-7.15 (m, 7H), 7.25-7.40 (m, 6H). MS (DCl/NH₃) m/e 308 (M+H)⁺.

### Example 53B

### (1α,2β,3β,4α)-1,2-Di[N-(4-fluorobenzyl)-N-(4-phenoxybenzyl)aminocarbonyl]cyclobutane-3.4-dicarboxylic acid

A slurry of 1,2,3,4-cyclobutanetetracarboxylic dianhydride (450 mg, 2.3 mmol) in acetonitrile (4 mL) under nitrogen at room temperature was treated with a solution of the compound resulting from Example 53A (1.4 g, 4.6 mmol) in acetonitrile (6 mL). The resulting suspension was refluxed for 15 hours. The solvent was evaporated, and the residue was chromatographed on silica gel eluting with 98:1:1 chloroform-methanol-acetic acid to provide a wet foam. The foam was dissolved in acetonitrile (5 mL), triturated with water, and lyophilized to provide 890 mg (48%) of the title compound as a white powder. m.p. 97-99 °C. ¹H NMR (DMSO-d₆, 300 MHz) δ 3.66-3.74 (m, 2H), 3.95-4.24 (m, 2H), 4.62-4.73 (m, 4H), 4.80-4.88 (m, 4H), 6.90-7.28 (m, 18H), 7.34-7.42 (m, 8H), 12.62-12.75 (br s, 2H). MS (FAB) m/e 811 (M+H)⁺.

### Example 54

### (1α,2β,3β,4α)-1,2-Di[N-(4-phenoxybenzyl)-N-(3-methoxyphenethyl) aminocarbonyl]cyclobutane-3.4-dicarboxylic acid

### Example 54A

### N-(p-phenoxybenzyl)-N-(m-methoxyphenethyl)amine

A solution of 4-benzoxybenzaldehyde (2.5 g, 12.6 mmol), 3-methoxyphenethyl amine (1.9 g, 12.6 mmol), a catalytic amount of p-toluenesulfonic acid monohydrate in absolute ethanol (12 mL) was stirred at 80 °C for 1.5 hours. After cooling to room temperature, NaBH₄ (0.49 g, 13.0 mmol) was added in portions. The reaction mixture was stirred at 80 °C for 1 hour, then cooled to room temperature, and the ethanol was removed *in vacuo.* Water was added to the residue and the mixture was extracted with ethyl acetate. The combined extracts were washed with saturated NaCI, dried over MgSO₄, filtered, and solvent removed *in vacuo* to afford a colorless oil which was purified by silica gel chromatography eluting with 20% ethyl acetate in hexane saturated with NH₃ to afford the title compound (3.2 g, 76%) as a colorless oil. MS (DC/NH₃) m/e 334 (M+H)⁺. ¹H NMR (CDCl₃, 300 MHz) δ 2.82 (t, 2H), 2.92 (t, 2H), 3.77 (s, 2H), 3.80 (s, 3H), 6.80 (m, 3H), 6.98 (m, 3H), 6.08 (t, 1H), 7.22 (m, 3H), 7.32 (t, 2H).

### Example 54B

### (1α,2β,3β,4α)-1,2-Di[N-(4-phenoxybenzyl)-N-(3-methoxyphenethyl) aminocarbonyl]cyclobutane-3,4-dicarboxylic acid

A solution of the product from Example 54A (1.26 g, 3.8 mmol), 1,2,3,4-cyclobutanetetracarboxylic dianhydride (0.38 g, 1.95 mmol), triethylamine (0.053 mL, 3.8 mmol), and CH₃CN (14 mL) was stirred at room temperature for 3 hours. The solvent was removed *in vacuo,* and the residue was dissolved in ethyl acetate, washed successively with 1 N HCI and saturated NaCI, dried over MgSO₄, filtered, and evaporated to afford a white foam. The crude product (1.8 g) containing both isomers was purified by silica gel chromatography eluting with 97:2.5:0.5 CHCl₃-MeOH-HOAc. The slower running isomer was collected to afford 0.40 g (24%) of the desired product. ¹H NMR (300 MHz, CDCl₃) δ 2.71 (q, 4H), 3.21 (m, 2H), 3.40 (m, 3H), 3.70 (d,3H), 3.70 (m, 2H), 3.72 (d, 3H), 3.90 (m, 3H), 4.16 (m, 2H), 4.25 (m, 2H), 4.35 (m, 1H), 4.45 ( d, 2H), 4.55 (d, 1H), 6.68 (m, 5H), 6.85 (m, 3H), 6.92 (m, 5H), 7.05 (m, 8H), 7.25, (m, 5H). MS (FAB) m/e 863 (M+H)⁺.

### Example 55

### (1α,2β,3β,4α)-1,2-Di[N-(4-phenoxybenzyl)-N-(3,4-dimethoxyphenethyl) aminocarbonyl]cyclobutane-3,4-dicarboxylic acid

Using the procedures described in Example 54A, but substituting 3,4-dimethoxyphenethylamine for 3-methoxyphenethylamine, provided N-(4-phenoxybenzyl)-N-(3,4-dimethoxyphenethyl)amine. ¹H NMR (300 MHz, CDCl₃) δ 2 78 (t, 2H), 2.90 (t, 2H), 3.78 (s, 2H) 3.88 (s, 3H), 6.78 (m, 3H), 6.98 (t, 4 H), 7.09 (t, 1H), 7.26 (t, 2H), 7.32 (t, 2H). MS (DCl/NH₃) m/e 364 (M+H)⁺.

The amine prepared above was reacted by the procedures described in Example 54B to provide the title compound. ¹H NMR (300 MHz, CDCl₃) δ 2.70 (q, 4H), 3.10-3.50 (m, 4 H), 3.78 (s, 12H), 3.92 (m, 2H), 4.02-4.32 (m, 4H), 4.55 (q, 2H), 6.59-6.75 ( m, 6H), 6.85 (m, 3H), 6.94 (t, 4H), 7.09 (m, 6H), 7.28 (m, 5H). MS (FAB) m/e 923 (M+H)⁺.

### Example 56

### (1α,2β,3β,4α)-1,2-Di[N-(4-phenoxybenzyl)-N-phenethylaminocarbonyl] cyclobutane-3,4-dicarboxylic acid

Using the procedures described in Example 54A, but substituting phenethylamine for 3-methoxyphenethylamine, provided N-(4-phenoxybenzyl)-N-phenethylamine. ¹H NMR (300 MHz, CDCl₃) δ 2.85 (t, 2H), 2.93 (t, 2H), 3.80 (s, 2H), 6.98 (m, 4H), 7.10 (t, 1H), 7.30 (m, 9H). MS (DCI/NH₃) m/e 304 (M+H)⁺.

The amine prepared above was reacted by the procedures described in Example 54B to provide the title compound. ¹H NMR (300 MHz, CDCl₃) δ 2.78 (q, 4H), 3.19-3.30 (m, 1H), 3.30-3.50 (m, 2H), 3.60-3.80 (m, 2H), 3.90 (q, 2H), 4.11-4.29 (m, 2H), 4.34 ( t, 1H), 4.45 (m, 2H), 6 85 (m, 3H), 6.91 (m, 4H), 7.09 (m, 10H), 7.18 (m, 5H), 7.25 (m, 6H). MS (FAB) m/e 841 (M+K)⁺.

### Example 57

### (1α,2β,3β,4α)-1,2-Di[N-(4-phenoxybenzyl)-N-(3-phenyl-1-propyl)aminocarbonyl]cyclobutane-3,4-dicarboxylic acid

Using the procedures described in Example 54A, but substituting 3-phenyl-1-propyl amine for 3-methoxyphenethylamine, provided N-(4-phenoxybenzyl)-N-(3-phenyl-1-propyl)amine. ¹H NMR (300 MHz, CDCl₃) δ 1.85 (p, 2H), 2.70 (m, 4H), 3.75 (s, 2H), 7.00 (t, 3H), 7.10 ( t, 1H), 7.20 (m, 3H), 7.30 (m, 7H). MS (DC/NH₃) m/e 318 (M+H)⁺.

The amine prepared above was reacted by the procedures described in Example 54B to provide the title compound. ¹H NMR (300 MHz, CDCl₃) δ 1.78 (m, 4H), 2.50 (q, 4H), 3.10 (m, 2H), 3.12 (m, 1H), 3.35-3.58 (m, 1H), 3.80-3.92 (m, 2H), 4.06 (t, 1H), 4.10-4.20 (m, 2H), 4.39-4.59 (m, 3H), 6.82 (dd,3H), 6.94 (m, 6H), 7.12 (t, 2H), 7.28 (m, 5H). MS (FAB) m/e 831 (M+H)⁺.

### Example 58

### (1α,2β,3β,4α)-1,2-Di[N-(4-phenoxybenzyl)-N-(4-phenyl-1-butyl)aminocarbonyl]cyclobutane-3,4-dicarboxylic acid

Using the procedures described in Example 54A, but substituting N-(4-phenyl-1-butyl)amine for 3-methoxyphenethylamine, provided N-(4-phenoxybenzyl)-N-(4-phenyl-1-butyl)amine. ¹H NMR (300 MHz, CDCl₃): δ 1.50-1.72 (m, 4H), 2.65 (m, 4 H), 3.25 (s, 2H), 6.98 (t, 4H), 7.09 (t, 1H), 7.19 (m, 3H), 7.30 (m, 6H). MS (DCl/NH₃) m/e 332 (M+H)⁺.

The amine prepared above was reacted by the procedures described in Example 54B to provide the title compound. ¹H NMR (300 MHz, CDCl₃) δ 1.50 (br m, 8 H), 2.52 (br dt, 4H), 3.02-3.28 (m, 3H), 3.35-3.58 (m, 1H), 3.81-3.95 (m, 2H), 4.00-4.20 (m, 2H), 4.21-4.65 (m, 4H), 6.90 (m, 7H), 7.05 (m, 10H), 7.25 (m, 7H). MS (FAB) m/e 859 (M+H)⁺.

### Example 59

### (1α,2β,3β,4α)-1,2-Di[N-(4-phenoxybenzyl)-N-(methoxycarbonylmethyl)aminocarbonyl]cyclobutane-3,4-dicarboxylic acid

Following the procedures described in Examples 1A and 13, the title compound was prepared. ¹H NMR (CDCl₃, 500 MHz) δ 3.62-3.79 (m, 6H), 3.94-5.14 (m, 12H), 6.91-7.51 (m, 18H). MS m/e 739 (M+H)⁺.

### Example 60

### (1α,2β,3β,4α)-1,2-Di[N-(4-phenoxybenzyl)-N-(ethoxycarbonylethyl)aminocarbonyl]cyclobutane-3,4-dicarboxylic acid

Following the procedures described in Examples 1A and 13, the title compound was prepared. ¹H NMR (CDCl₃, 500 MHz) δ 1.25 (t, 6H, J = 7Hz), 2.56 (m, 2H), 3.73 (q, 4H, J =7Hz), 3.9-4.28 (m, 12H), 4.38 (m, 1H), 4.76 (m, 1H), 6.9-7.35 (m, 18H). MS m/e 795 (M+H)⁺.

### Example 61

### (1α,2β,3β,4α)-1,2-Di[N-propyl-N-(4-cyclohexyloxybenzyl)aminocarbonyl]cyclobutane-3,4-dicarboxylic acid

### Example 61A

### 4-(Cyclohex-2-enyloxy)benzaldehyde

4-(Cyclohex-2-enyloxy)benzoic acid (25 g, 115 mmol) was dissolved in 250 mL dry tetrahydrofuran and cooled to -10 °C under an atmosphere of dry nitrogen. N-Methylmorpholine (12.6 mL, 115 mmol) and isobutylchloroformate (15.9 mL, 115 mmol) were added. After 10 minutes at -10 °C the mixture was warmed to room temperature and filtered. The volume was reduced to 50 mL under reduced pressure before cooling to 0 °C and adding diisobutylaluminum hydride (1.5 M in toluene, 160 mL) over 10 minutes. After 30 minutes, the reaction was warmed to ambient temperature for 2 hours. The mixture was cooled in an ice bath, diluted with ether, quenched with methanol, and poured into cold Rochelle's salt solution. The crude product was extracted with ether, dried over Na₂SO₄, and chromatographed eluting with 20% ethyl acetate in hexane to give 4.24 g (18%) of 4-(cyclohex-2-enyloxy)benzyl alcohol.

The alcohol prepared above (4.24 g, 20.7 mmol) was dissolved in 250 mL methylene chloride under an atmosphere of dry nitrogen. Pyridinium chlorochormate (5.62 g, 26.1 mmol) was added over 5 minutes. After stirring at ambient temperature for 72 hours, the mixture was filtered through Celite and concentrated *in vacuo.* Flash silica gel chromatography eluting with 10% ethyl acetate in hexane afforded 3.25 g (77%) of the title compound.

### Example 61B

### (1α,2β,3β,4α)-1,2-Di[N-propyl-N-(4-cyclohexyloxybenzyl)aminocarbonyl]cyclobutane-3,4-dicarboxylic acid

N-propyl-N-(4-cyclohexyloxybenzyl)amine was prepared by the procedures described in Example 1A starting with the compound resulting from Example 61A.

The title compound was prepared using the amine prepared above and the procedures described in Example 13. ¹H NMR (CDCl₃, 500 MHz) δ 0.80 (m, 6H), 1.35 (m, 6H), 1.51 (m, 10H), 1.79 (m, 4H), 1.97 (m, 4H), 3.1 (m, 4H), 3.39 (m, 1H), 3.50 (m, 1H), 3.93-4.59 (m, 8H), 6.94 (m, 4H), 7.07-7.14 (m, 4H). MS m/e 691 (M+H)⁺.

### Example 62

### (1α,2β,3β,4α)-1-[N-Propyl-N-(4-phenoxybenzyl)aminocarbonyl]-2-[N-methyl-N-(homogeranyl)aminocarbonyl]cyclobutane-3,4-dicarboxylic acid

### Example 62A

### (1α,2β,3β,4α)-1-[N-Propyl-N-(4-phenoxybenzyl)aminocarbonyl]cyclobutane-2,3,4,tricarboxylic acid

To a solution of 1,2,3,4-cyclobutanetetracarboxylic dianhydride (1.0 g, 5.1 mmol) and triethylamine (0.7 mL, 5.1 mmol) in acetonitrile (50 mL) under an atmosphere of dry nitrogen was added N-propyl-4-phenoxybenzyl amine hydrochloride (1 equivalent). After stirring 18 hours, 1 M HCl was added and stirring was continued for 18 hours. The mixture was diluted with ethyl acetate which was subsequently washed with 1 M HCI and brine, dried over Na₂SO₄, and concentrated *in vacuo* to give 2.3 g of crude product.

A solution of crude triacid (1.1 g, 2.4 mmol) in methanol (25 mL) was treated with an excess of ethereal diazomethane. Evaporation and flash silica gel chromatography eluting with 50% ethyl acetate in hexane afforded the corresponding pure triester (0.8 g, 67%). The pure triester was dissolved in methanol (10 mL) and treated with 3 M NaOH (5 mL) at 40 °C for 48 hours. The reaction mixture was diluted with water and washed with ethyl acetate. The aqueous solution was acidified to pH 2 with concentrated HCI. The product was extracted into ethyl acetate which was then washed with brine, dried over Na₂SO₄, and concentrated under reduced pressure to give 0.73 g (99%) of the pure triacid.

### Example 62B

### (1α,2β,3β,4α)-1-[N-Propyl-N-(4-phenoxybenzyl)aminocarbonyl]-2-[N-methyl-N-(homogeranyl)aminocarbonyl]cyclobutane-3,4-dicarboxylic acid

To a solution of the compound resulting from Example 62A (0.43 g, 0.94 mmol) in dimethylformamide (1 mL) and methylene chloride (10 mL) at 0 °C, was added dicyclohexylcarbodiimide (0.19 g, 0.94 mmol). After 1 hour, N-methyl-N-(homogeranyl)amine (0.94 mmol) and triethylamine (0.39 mL, 2.8 mmol) were added. The reaction was allowed to warm to ambient temperature and stirred for 18 hours. Ethyl acetate was added to the reaction mixture which was then washed with 1 M HCI and brine, dried over Na₂SO₄, and concentrated under reduced pressure. The residue obtained was purified by flash silica gel chromatography eluting with 94:5:1 chloroform-methanol-acetic acid to give the title compound. ¹H NMR (CDCl₃, 300 MHz) δ 0.85 (m, 3H), 1.61 (m, 6H), 1.68 (m, 3H), 2.00 (m, 8H), 2.92 (m, 5H), 3.66 (m, 2H), 4.03 (m, 3H), 4.55 (m, 3H), 5.05 (m, 2H), 6.85-7.35 (m, 9H). MS m/e 619 (M+H)⁺.

### Example 63

### (1α,2β,3β,4α)-1-[N-Propyl-N-(4-phenoxybenzyl)aminocarbonyl]-2-[N-benzyl-N-(4-phenoxybenzyl)aminocarbonyl]cyclobutane-3,4-dicarboxylic acid

The title compound was prepared using the procedures described in Example 62. ¹H NMR (CDCl₃, 300 MHz) δ 0.88 (m, 3H), 1.35 (m, 2H), 3.62-4.61 (m, 12H), 6.89-7.35 (m, 23H). MS m/e 727 (M+H)⁺.

### Example 64

### (1α,2β,3β,4α)-1-[N-Propyl-N-(4-phenoxybenzyl)aminocarbonyl]-2-[N-(4-phenoxybenzyl)aminocarbonyl]cyclobutane-3,4-dicarboxylic acid

### Example 64A

### 4-Phenoxybenzyl alcohol

A solution of 4-phenoxybenzaldehyde (10.0 g, 50 mmol) and 10 mL dry THF was added dropwise to a 0 °C suspension of lithium aluminum hydride (2.1 g, 55.3 mmol) and 100 mL dry THF. The reaction mixture was stirred for 1 hour at 0 °C, then was quenched successively with 2.1 mL H₂O, 2.1 mL 10% NaOH, and 6.3 mL H₂O. The resultant slurry was stirred for 1.5 hours at room temperature, then was filtered through Celite and the filtrate was evaporated under reduced pressure to afford the title compound (9.9 g) as a white solid. ¹H NMR (300MHz, CDCl₃) δ 1.68 (t, 1H), 4.68 (d, 2H), 7.00 (m, 4H), 7.10 (t, 1H), 7.35 (m, 4H). MS (DC/NH₃) m/e 183 (M+1, -H₂O)⁺.

### Example 64B

### 4-Phenoxybenzyl phthalimide

To the compound resulting from Example 64A (5.0 g, 25 mmol) was added to a solution of lithium bromide (4.4 g, 51 mmol), trimethylsilyl chloride (8.2 mL, 64 mmol), and 51 mL acetonitrile. The reaction mixture was stirred at reflux for 2 hours, then was cooled to room temperature. Water (25 mL) was added, the acetonitrile was removed under reduced pressure, and the aqueous layer was extracted with ether. The combined organic extracts were washed with saturated aqueous sodium bicarbonate and brine, dried (MgSO₄), filtered, and solvent evaporated *in vacuo* to afford 5.35 g clear oil. The crude bromide (20.3 mmol) was stirred at room temperature for 24 hours with potassium phthalimide (4.2 g, 22.1 mmol) and 45 mL DMF. Water was added and the mixture was extracted with methylene chloride. The combined organic extracts were washed with water (2x), 1 N HCI and brine, dried (MgSO₄), filtered, and evaporated under reduced pressure to afford the title compound (7.9 g) as a white solid. ¹H NMR (300 MHz, CDCl₃) δ 4.83 (s, 2H), 6.95 (dd, 4H), 7.10 (t, 1H), 7.31 (t, 2H), 7.42 (d, 2H), 7.71 (m, 2H), 7.88 (m, 2H). MS (DCI/NH₃) m/e 347 (M+H+NH₃)⁺.

### Example 64C

### N-(4-Phenoxybenzyl)amine

The compound resulting from Example 64B (6.7 g, 20.4 mmol), hydrazine (2.1 mL, 35 wt. % in water) and 130 mL absolute ethanol were stirred at reflux for 4 hours. After cooling to room temperature, the solid present was filtered and air dried briefly. The solid was then partitioned between 1 N KOH and methylene chloride. The layers were separated and the aqueous layer was extracted 2 more times with methylene chloride. The combined organic layers were washed with water and brine, dried (MgSO₄), filtered, and evaporated *in vacuo* to afford the title compound (2.7 g) as a clear oil. ¹H NMR (300 MHz, CDCl₃) δ 1.48 (s, 2H), 3.87 (s, 2H), 7.00 (m, 3H), 7.10 (t, 1H), 7.30 (m, 5H). MS (DCl/NH₃) m/e 200 (M+H)⁺, 217 (M+H+NH₃)⁺.

### Example 64D

### (1α,2β,3β,4α)-1-[N-Propyl-N-(4-phenoxybenzyl)aminocarbonyl]-2-[N-(4-phenoxybenzyl)aminocarbonyl]cyclobutane-3,4-dicarboxylic acid

The compound resulting from Example 62A was reacted with the compound resulting from Example 64C by the procedures described in Example 62B to give the title compound. ¹H NMR (CDCl₃, 300 MHz) δ 0.88 (m, 3H), 1.35 (m, 2H), 3.5-4.3 (m, 11H), 6.83-7.33 (m, 18H). MS m/e 635 (M-H)⁻.

### Example 65

### (1α,2β,3β,4α)-1,2-Di-(4-phenoxybenzyloxycarbonyl)-3,4-dicarboxylic acid

### Example 65A

### 4-Phenoxybenzyl alcohol

A solution of 4-phenoxybenzaldehyde (5.0 g, 25 mmol) in dry tetrahydrofuran (10 mL) at 0 °C was treated with borane (1 M in tetrahydrofuran, 11.25 mL). After 45 minutes, the reaction was quenched with saturated NH₄CI, diluted with ethyl acetate, and washed with 1 M HCI, 5% NaHCO₃, and brine, and dried over Na₂SO₄, and concentrated under reduced pressure to give 4.7 g (92%) of 4-phenoxybenzyl alcohol.

### Example 65B

### (1α,2β,3β,4α)-1,2-Di-(4-phenoxybenzyloxycarbonyl)-3,4-dicarboxylic acid

To a solution of the compound resulting from Example 65A (1.0 g, 5.0 mmol) in dry tetrahydrofuran (100 mL) at -78°C under dry nitrogen was added n-butyl lithium (1.6 M in hexane, 3.1 mL). After 15 minutes, a suspension of 1,2,3,4-cyclobutanetetracarboxylic dianhydride (0.49 g, 2.5 mmol) in tetrahydrofuran (5 mL) was added. The reaction was allowed to warm slowly to ambient temperature and stirred for 18 hours before quenching with saturated NH₄CI. Solvents were removed under reduced pressure, and the residue was partitioned between ethyl acetate and 1 M HCI. The organic layer was washed with 1 M HCl and brine, dried over Na₂SO₄, and concentrated *in vacuo.* The residue obtained was purified by flash silica gel chromatography eluting with 94:5:1 chloroform-methanol-acetic acid to give 0.17 g (45%) of the title compound. ¹H NMR (CDCl₃, 300 MHz) δ 3.71 (m, 4H), 5.11 (s, 4H), 6.94-7.36 (m, 18H). MS m/e 595 (M-H)⁻.

### Example 66

### (1α,2β,3β,4α)-1,2-Di[N-propyl-N-(4-phenylaminophenyl)aminocarbonyl]cyclobutane-3,4-dicarboxylic acid

### Example 66A

### N-Phenyl-N'-Propionyl-1,4-phenylenediamine

A stirred mixture of 1.84 g (10.0 mmol, 1 eq.) of commercial N-phenyl-1,4-phenylenediamine (a black solid) and 1.18 g (14.0 mmol, 1.4 eq.) of NaHCO₃ in 40 mL of CH₂Cl₂ was cooled to 0 °C. To this suspension was added a solution of 0.96 mL (11.0 mmol, 1.1 eq.) of propionyl chloride in 10 mL of CH₂Cl₂ dropwise. The ice bath was removed and the mixture stirred for 1 hour and then poured into a seperatory funnel containing 50 mL of each CH₂Cl₂ and water. The organic phase was separated and extracted with 50 mL of saturated aqueous NaHCO₃, dried over MgSO₄, filtered and concentrated *in vacuo.* The residue was purified by flash column chromatography (100 g SiO₂,1:1 ethyl acetate-hexanes) to give 1.79 g (75%) of product. ¹H NMR (300 MHz, CDCl₃) δ 7.41 (d, 2H), 7.21-7.43 (m, 2H), 7.14 9 (bs, 1H), 6.96-7.07 (m, 4H) 6.90 (t, 1H), 5.66 (bs, 1H), 2.38 (q, 2H), 1.25 (t, 3H). MS(DCl) m/e 258 (M + NH₄)⁺, 241 (M + H)⁺.

### Example 66B

### N-Phenyl-N'-Propyl-4-phenylenediamine

A stirred solution of the compound resulting from Example 66A (1.5 g, 6.2 mmol, 1 eq.) in 10 mL of dry THF was cooled to 0 °C in an ice bath. To this solution was added 12.5 mL (12.5 mmol. 2 eq.) of a 1.0 M solution of LiAlH₄ in THF dropwise. The ice bath was removed and the mixture was stirred at ambient temperature for 24 hours. The heterogeneous mixture was cooled to 0 °C and quenched by the careful addition of 0.5 mL of water in 5 mL of THF followed by the addition of 0.5 mL of 15% aqueous THF and then an additional 1.5 mL of water. The mixture was then vigorously stirred for 5 minutes. Hexanes (25 mL) and Na₂SO₄ (2 g) were added and vigorous stirring continued for 20 minutes. The mixture was filtered through celite and the pad washed well with ethyl acetate and the filtrate concentrated. The residue was purified by flash column chromatography on SiO₂ (50 g, 20% ethyl acetate/hexanes) to give 1.26 g (89%) of a brown oil. ¹H NMR (300 MHz (CDCl₃) δ 7.18 (m (2H), 7.01 (m (2H), 6.82 (m (2H), 6.77 (m, 1H), 6.60 (m, 2H), 5.38 (bs, 1H), 3.53 (bs, 1H), 3.08 (t, 2H), 1.64 (hextet, 2H),1.02 (t, 3H). MS (DCI) m/e 244 (M + NH₄)⁺, 227 (M+H)⁺.

### Example 66C

### (1α,2β,3β,4α)-1,2-Di[N-propyl-N-(4-phenylaminophenyl)aminocarbonyl]cyclobutane-3,4-dicarboxylic acid

To a suspension of 1,2,3,4-cyclobutanetetracarboxlic anhydride (0.48 g, 2.47 mmol, 1 eq) in acetonitrile at 0 °C was added a solution of the compound resulting from Example 66B (1.23 g, 5.43 mmol, 2.2 eq.) in acetonitrile. The cooling bath was removed and the mixture stirred overnight. The resulting suspension was concentrated and partitioned between 3 N aqueous HCI and 3 portions of ethyl acetate. The combined organic phases were washed with brine, dried, filtered and concentrated *in vacuo.* The residue was purified by column chromatography on SiO₂ eluting with a CHCl₃-CH₃OH-HOAc solvent system to give 0.25 g (16%) of the 1,3-diacid and 0.77 g (48%) of the title compound. ¹H NMR (300 MHz (DMSO-d₆) δ 12.35 (bs, 2H), 8.34 (s, 2H), 7.25 (t , 4H), 7.09 (m, 12H), 6.86 (t, 2H), 3.57 (m, 2H), 3.26-3.40 (m, 4H), 3.09 (m, 2H), 1.36 (m, 4H), 0.74 (t, 6H). MS (FAB⁺) m/e 649 (MH). HRMS (FAB) calcd for C₃₈H₄₁N₄O₆ (M+H) 649.3026. Found: 649.3013. Anal calcd for C₃₈H₄₁N₄O₆: C, 70.35; H, 6.21; N, 8.63. Found: C, 69.05; H, 6.35; N, 8.05.

### Example 67

### (1α,2β,3β-4α)-1,2-Di[N-propyl-N-(4-phenylaminobenzyl)aminocarbonyl]cyclobutane-3,4-dicarboxylic acid

### Example 67A

### 4-Phenylamino-N-propylbenzamide

To a stirred solution of 1.06 g (5.0 mmol, 1 eq.) of 4-phenylaminobenzoic acid (Portnaya, B.S., Turitsyna, N.F., Bobkova, T.P., and Levkeov, I.I., *Zhur. Obshchei. Khim.,* 30, 2693 (1960)), n-propylamine (0.82 mL, 10.0 mmol, 2 eq.), and 0.061 g (0.5 mmol, 0.1 eq.) of DMAP in THF at 0°C was added 1.05 g (5.5 mmol, 1.1 eq.) of EDCI. The mixture was stirred overnight while the bath melted. The mixture was poured into 150 mL of ethyl acetate and washed with 50 mL each of water, 1 N aqueous HCI, water and saturated aqueous NaHCO₃. The ethyl acetate layer was then dried over Na₂SO₄, filtered and concentrated in *vacuo.* The residue was purified by flash column chromatography (80 g SiO₂, 1:1 ethyl acetate-hexanes) to give 0.51 g (40%) of the title compound. ¹H NMR (300 MHz, CDCl₃) δ 10.57 ( bs, 2H), 7.21-7.41 (m, 9H), 3.96 (s, 2H), 2.75 (m, 2H), 1.64 (hextet, 2H), 0.92 (t, 3H). MS (DCl) m/e 255 (M+H)⁺.

### Example 67B

### N-Propyl-N-(4-phenylaminobenzyl) amine

To a stirred solution of the compound resulting from Example 67A 0.50 g (1.96 mmol., 1 eq.) in 10 mL of dry THF at 0 °C was added 3.9 mL (3.9 mmol, 2 eq.) of a 1.0 M solution in THF of LiAlH₄. The ice bath was removed and stirring continued for 30 minutes. The solution was then heated at reflux for 20 hours and then cooled back to 0 °C. The mixture was carefully quenched by the addition of 0.15 mL of water, 0.15 mL of 15% aqueous NaOH, and 0.45 mL of water and vigorously stirred for 10 minutes. Ethyl ether (20 mL) and MgSO₄ (2 g) were added and stirring continued for an additional 15 minutes. The heterogeneous mixture was filtered through a pad of SiO₂ and the pad was washed with 100 mL of ether and then 100 mL of 5% CH₃OH-CHCl₃. The filtrate was concentrated to give 0.398 g (85%) of a yellow oil that was used without further purification. ¹H NMR (300 MHz, CDCl₃) δ 7.19-7.32 (m, 2H), 7.01-7.08 (m, 2H), 6.87-6.93 (m, 1H), 5.67 (bs, 1H), 3.72 (s, 2H), 2.61 (t, 2H), 1.53 (hextet, 2H), 1.39 (bs, 1H), 0.92 (t, 3H).

### Example 67C

### (1α,2β,3β,4α)-1,2-Di[N-propyl-N-(4-phenylaminobenzyl)aminocarbonyl]cyclobutane-3,4-dicarboxylic acid:

Following the procedures described in Example 66C, the compound resulting from Example 67B (0.378 g, 1.57 mmol, 2.2 eq.) and 1,2,3,4-cyclobutanetetracarboxylic dianhydride (0.140 g, 0.71 mmol, 1 eq.) were reacted to give 0.159 g (33%) of the title compound. ¹H NMR (300 MHz, CDCl₃) δ 7.14-7.30 (m, 8H), 6.83-7.10 (m, 12H), 3.84-4.85 (m, 8H), 2.94-3.56 (m, 4H), 1.37-1.62 (m, 4H), 0.72-0.88 (m, 6H. MS (FAB⁺) m/e 677 (M+H)⁺. HRMS calcd for C₄₀H₄₅N₄O₆ (MH) 677.3339. Found: 677.3331. Anal calcd for C₄₀H₄₅N₄O₆: C, 70.99; H, 6.55; N, 8.28. Found; C, 69.46; H, 6.67; N, 7.89.

### Example 68

### (1α,2β,3β,4α)-1,2-Di[N-propyl-N-(4-phenylthiobenzyl)aminocarbonyl]cyclobutane-3,4-dicarboxylic acid

### Example 68A

### N-Propyl-N-(4-phenylthiobenzyl)amine

To a stirred solution of 2.14 g (10 mmol, 1.0 eq.) of 4-phenylthiobenzaldehyde (Portnaya, B.S.,Turitsyna, N.F., Bobkova, T.P., and Levkeov, I.I., *Zhur. Obshchei. Khim.,* 30, 2693 (1960)) and 0.6 mL (10.0 mmol, 1.0 eq.) of acetic acid in 40 mL of CH₃OH at 0°C was added 1.6 mL (20.0 mmol, 2.0 eq.) of N-propylamine. After stirring 30 minutes at 0 °C, NaCNBH₃ (0.69 g, 11 mmol, 1.1 eq.) was added and the mixture was stirred for an additional 2 hours whereupon an additional 0.69 g of NaCNBH₃ was added. The reaction mixture was stirred for an additional 14 hours and then poured into 200 mL of saturated NaHCO₃ and extracted with 3x50 mL of ethyl acetate. The combined organic phases were washed with brine, dried (MgSO₄), filtered and concentrated *in vacuo.* Purification of the residue by column chromatography (150 g SiO₂, 10% CH₃OH-CHCl_{3,} trace HOAc) gave 1.59 g (62%) of the title compound as a light yellow oil. Spectral analysis indicated the presence of ~1 eq. of AcOH. ¹H NMR (300 MHz, CDCl₃) δ 7.54 (bs, ¹H (AcOH)), 7.21-7.37 (m, 9H), 3.83 (s, 2H), 2.64 (t, 2H), 1.98 (s, 3H (AcOH)), 1.60 (hextet, 2H), 0.92 (t, 3H). MS (DCl) m/e 258 (M+H)⁺.

### Example 68B

### (1α,2β,3β,4α)-1,2-Di[N-propyl-N-(4-phenylthiobenzyl)aminocarbonyl]cyclobutane-3,4-dicarboxylic acid

Following the procedure described in Example 66C, the compound resulting from Example 68A (0.566 g, 2.2 mmol, 2.2 eq.) and 0.196 g (1.0 mmol, 1.0 eq.) of 1,2,3,4-cyclobutanetetracarboxylic dianhydride were reacted to give 0.284 g (40%) of the title compound as an off white foam. ¹H NMR (300 MHz, CDCl₃) δ 7.02-7.37 (m, 18H), 2.92-4.68 (m, 12H), 1.5 (m. 4H), 0.61 (m, 6H). MS (FAB⁺) m/e 711 (M+H)⁺. Anal calcd for C₄₀H₄₂N₂O₆S₂: C, 67.58; H, 5.95; N, 3.94. Found: C, 65.78; H, 5.86; N, 3.62.

### Example 69

### (1α,2β,3β,4α)-1,2-Di[N-propyl-N-[4-phenoxymethylbenzyl]aminocarbonyl]cyclobutane-3,4-dicarboxylic acid

### Example 69A

### Methyl-4-phenoxymethylbenzoate

To a stirred mixture of 5.72 g (25.0 mmol, 1.0 eq.) of methyl 4-bromomethylbenzoate (Aldrich) and 3.80 g (27.5 mmol, 1.1 eq.) of K₂CO₃ in 10 mL of DMF at ambient temperature was added a solution of 2.59 g (27.5 mmol, 1.1 eq.) of phenol in 10 mL of DMF dropwise and the mixture stirred overnight. The reaction mixture was poured into 100 mL of water and extracted with 3 x 100 mL portions of 25% CH₂Cl₂-hexanes. The combined organic phases were extracted with 3 x 100 mL portions of water, dried (MgSO₄), filtered and concentrated *in vacuo.* The solid residue was recrystallized from ~50 mL of hexanes to give 5.31 g (88%) of the title compound as a white, crystalline solid. ¹H NMR (300MHz, CDCl₃) δ 8.05 (d, 2H), 7.51 (d, 2H), 7.26-7.33 (m, 2H), 6.95-7.02 (m, 3H), 5.13 (s, 2H), 3.92 (s, 3H). MS (DCl) m/e 260 (M+NH₄)⁺.

### Example 69B

### 4-Phenoxymethylbenzoic acid

To a suspension of 2.42 g (10 mmol, 1.0 eq.) of the compound resulting from Example 69A in 20 mL of CH₃OH at 0 °C was added a solution of 0.97 g (15.0 mmol, 1.5 eq.) of KOH (87%) in 10 mL of CH₃OH. The suspension was allowed to reach ambient temperature overnight whereupon 5 mL of water was added. After stirring was continued for 3 additional hours, the mixture was poured into 200 mL of water and extracted with 3 x 50 mL of ethyl ether. The aqueous phase was acidified with 3 N aqueous HCI and the resulting precipitate collected by filtration. Purification by recrystallization from acetone-CH₃OH gave 0.60 g (28%) of the title compound as a white crystalline solid. ¹H NMR (300 MHz, CDCl₃) δ 8.12 (d, 2H), 7.56 (d, 2H), 7.22-7.36 (m, 3H), 6.94-7.01 (m, 2H), 5.15 (s, 2H). MS (DCI) m/e 246 (M+NH₄)⁺.

### Example 69C

### N-Propyl-4-phenoxymethylbenzamide

To a stirred solution of 0.59 g (2.78 mmol, 1.0 eq.) of the compound resulting from Example 69B in 10 mL of THF at room temperature was added 0.495 g (3.05 mmol, 1.1 eq.) of 1,1'-carbonyldiimidazole. After the initial evolution of CO₂ was complete, the mixture was heated at reflux for 30 minutes and subsequently cooled to ambient temperature with a ice-water bath. The resulting nearly colorless solution was treated with 0.68 mL (8.33 mmol, 3.0 eq.) of N-propylamine and stirring was continued overnight. The reaction mixture was then partitioned between water (50 mL) and ethyl acetate (100 mL). The layers were separated and the aqueous phase was extracted with an additional 50 mL portion of ethyl acetate. The combined organic phases were then washed with 50 mL each of water, saturated NaHCO₃ and brine, dried (MgSO₄), filtered and concentrated *in vacuo.* The solid residue was purified by recrystallization form ethyl acetate to give 0.418 g (59%) of the title compound as a white solid. ¹H NMR (300 MHz, CDCl₃) δ 7.78 (m, 2H), 7.49 (d, 2H), 7.25-7.32 (m, 2H), 6.93-7.00 (m, 3H), 6.17 (bs, 1H), 5.11 (s, 2H), 3.42 (m, 2H), 1.62 (hextet, 2H), 0.98 (t, 3H). MS (DCI) m/e 287 (M+NH₄)⁺.

### Example 69D

### N-Propyl-N-(4-phenoxymethylbenzyl)amine hydrochloride

To a stirred solution of 0.400 g (1.58 mmol, 1.0 eq.) of the compound resulting from Example 69C in 2 mL of dry THF at 0 °C was added 3.16 mL (3.16 mmol, 2 eq.) of a 1.0 M solution of LiAlH₄ in THF dropwise. The ice bath was removed and the mixture heated at reflux for 18 hours. After cooling to 0 °C, the excess hydride was carefully quenched by the sequential addition of 0.12 mL of water in 1 mL of THF, 0.12 mL of 15 % aqueous NaOH and 0.36 mL of water. The resulting suspension was vigorously stirred for 10 minutes followed by the addition of ether (15 mL) and MgSO₄ (2 g) and the vigorous stirring continued for an additional 15 minutes. The reaction mixture was filtered through a pad of SiO₂ (pre-wetted with ether) and the pad was washed well with ethyl acetate. The filtrate was concentrated to dryness and the residue dissolved in THF and treated with a slight excess of aqueous HCl. The solution was concentrated to dryness again (using toluene to remove the excess water). The solid residue was purified by recrystallization from methanol-acetone to give 0.314 g (72%) of the title compound as a white crystalline solid. ¹H NMR (300 MHz, CD₃OD) δ 7.52 (q 4H), 7.25 (m, 2H), 6.89-7.00 (m, 3H), 5.13 (s, 2H), 4.20 (s, 2H), 3.01 (m, 2H), 1.72 (m, 2H), 1.02 (t, 3H).

### Example 69E

### (1α,2β,3β,4α)-1,2-Di[N-propyl-N-[4-phenoxymethylbenzyl]aminocarbonyl]cyclobutane-3,4-dicarboxylic acid

To a stirred suspension of 0.098 g (0.5 mmol, 1.0 eq.) of 1,2,3,4-cyclobutanetetracarboxylic acid dianhydride in 3 mL of acetonitrile at 0 °C was added 0.15 mL (1.05 mmol, 2. eq.) of Et₃N. After 15 minutes the compound resulting from Example 69D (0.276 g, 1.0 mmol, 2.0 eq.) was added and the mixture allowed to reach room temperature and stirring continued for 66 hours. The reaction mixture was poured into 20 mL of 4 N aqueous H₂SO₄ and extracted with 3 x 20 mL of ethyl acetate. The combined organic phases were then washed with water and brine, dried (MgSO₄), filtered and concentrated *in vacuo.* The residue was purified by column chromatography on SiO₂ (35 g) eluting with 94:5:1. CHCl₃-CH₃OH-acetic acid to give the 1,3-diacid (0.100 g, 28%) followed by the title compound (0.093 g, 26%) as a white foam. ¹H NMR (CDCl₃) δ 7.11-7.40 (m, 12H), 6.88-6.99 (m, 6H), 4.99 (m, 4H), 4.31-4.70 (m, 4H), 3.87-4.18 ( m, 4H), 2.59-3.63 (m, 4H), 1,49 (m, 4H), 0.82 (m, 6H). HRMS (FAB) calcd for C₄₂H₄₆N₂O₈: 707.3332. Found: 707.3323. Anal calcd for C₄₂H₄₆N₂O₈: C, 71.37; H, 6.56; N, 3.96. Found: C, 70.70; H, 6.56; N, 3.85.

### Example 70

### (1α,2β,3β,4α)-1,2-Di[N-propyl-N-(4-phenoxybenzyl)aminocarbonyl]-4-hydroxymethyl-cyclobutane-3-carboxylic acid

### Example 70A

### Methyl(1α,2β,3β,4α)-1,2-Di[N-propyl-N-[4-phenoxybenzyl]aminocarbonyl]-4-hydroxymethyl-cyclobutane-3-carboxylate

To a stirred solution of (1α,2β,3β,4α)-1,2-di[N-propyl-N-[4-phenoxybenzyl]aminocarbonyl]cyclobutane-3,4-dicarboxylic acid mono methyl ester, the compound resulting from Example 95, (0.562 g, 0.81 mmol, 1.0 eq.) in 12 mL of dry THF at -20 °C was added 0.098 mL (0.89 mmol, 1.1 eq.) of N-methylmorpholine followed by 0.115 mL (0.89 mmol, 1.1 eq.) of isobutylchloroformate. After stirring for 30 minutes, a cold (-20 °C) suspension of 0.184 g (4.86 mmol, 3 eq.) of NaBH₄ in 1 mL of CH₃OH was carefully added (a vigorous reaction occurs) and the mixture stirred for 30 minutes more. The reaction was quenched by the careful addition of 2 mL of 3 N aqueous HCI and the poured into 50 mL of cold 3 N aqueous HCI and extracted with 3 x 50 mL of ethyl acetate. The combined organic extracts were washed with 50 mL of saturated aqueous NaHCO₃, dried (MgSO₄), filtered and concentrated *in vacuo.* The residue was purified by flash column chromatography on SiO₂ (40 g, 1:1 ethyl acetate-hexanes) to give 0.507 g (92%) of the title compound as a colorless foam. ¹H NMR (CDCl₃) δ 6.84-7.35 (m, 18H, 2.68-5.02 (m, 18H), 1.41-1.72 (m, 4H), 0.73-0.97 (m, 6H). MS (DCl) m/e 679 (M+H)⁺.

### Example 70B

### (1α,2β,3β,4α)-1,2-Di[N-propyl-N-(4-phenoxybenzyl)aminocarbonyl]-4-hydroxymethyl-cyclobutane-3-carboxylic acid

To a stirred solution of the compound resulting from Example 70A (68 mg, 0.1 mmol, 1.0 eq.) in 1 mL of 3:1 THF-water at 0°C was added 8 mg (0.2 mmol, 2.0 eq.) of LiOH·H₂O and stirring was continued at 0 °C for 4 hours and at room temperature for 30 minutes. The reaction was then quenched by the addition of 2 mL of 3 N aqueous HCl and then concentrated to dryness. Purification of the residue by column chromatography on SiO₂ (9 g, 94:4:1 CHCl₃-CH₃OH-HOAc) gave the title compound 0.047 g (71%) as a white foam. ¹H NMR (DMSO d₆) δ 12.26 (bs, 1H), 7.38, m, 3H), 7.17-7.31 (m, 4H), 7.12 (m, 1H), 7.00 (m, 4H), 6.91 (m, 2H), 4.58-4.84 (m, 3H), 4.09-4.32 (m, 3H), 3.78-4.07 (m, 3H), 3.17-3.60 (m, 6H), 3.12 (m, 1H), 2.98 (m, 1H), 2.86 (m, 1H), 2.69 (m, 1H), 1.31-1.62 (m, 4H), 0.70-0.89 (m, 6H). MS (FAB⁺) m/e 665 (M+H), 687 (M+Na), 703 (M+K). HRMS calcd for C₄₀H₄₅N₂O₇ (MH) 665.3227. Found: 665.3217. Anal cacld for C₄₀H₄₅N₂O₇: C, 72.27; H, 6.67; N, 4.21. Found: C, 71.45; H, 6.79; N, 4.21.

### Example 71

### (1α,2β,3β,4α)-1,2-Di[N-propyl-N-[4-phenoxybenzyl]aminocarbonyl]-4-[(hydroxyimino)methyl]-cyclobutane-3-carboxylic acid

### Example 71A

### Methyl (1α,2β,3β,4α)-1,2-Di[N-propyl-N-(4-phenoxybenzyl)aminocarbonyl]-4-carboxaldehyde-cyclobutane-3-carboxylate

To a stirred solution of 0.339 g (0.5 mmol, 1.0 eq.) of the compound resulting from Example 70B in 5 mL of 10% acetonitrile-CH₂Cl₂ at room temperature was added 0.088 g (0.75 mmol. 1.5 eq.) of N-methylmorpholine-N-oxide followed by 0.50 g of powdered, activated 4Å molcular sieves. After stirring 15 minutes at room temperature, TPAP (0.009 g, 0.025 mmol, 0.05 eq) was added and the resulting black mixture was stirred for an additional 30 minutes. The reaction mixture was treated with ~1 g of celite and then diluted with 5 mL of ether. After 10 minutes further stirring, the mixture was filtered through a pad of SiO₂ (pre-wetted with ether). The pad was washed well with ether (~200 mL) and the filtrate concentrated. The green residue was purified by column chromatography on SiO₂ (25 g, 1:1 ethyl acetate-hexanes) to give 0.269 g (80%) of the title compound as a thick syrup. ¹H NMR (CDCl₃) δ 9.69-9.76 (m, 1H), 6.89-7.38 (m, 18H), 4.09-4.87 (m, 5H), 3.48-4.03 (m, 6H), 2.41-3.47 (m, 4H), 1.42-1.67) (m, 4H), 0.76-0.98 (m, 6H). MS (DCl) m/e 677 (M+H)⁺.

### Example 71B

### Methyl(1α,2β,3β,4α)-1,2-Di[N-propyl-N-(4-phenoxybenzyl)aminocarbonyl]-4-[(hydroxyimino)methyl]-cyclobutane-3-carboxylate

To a stirred solution of 0.054 g (0.78 mmol, 1.5 eq.) of NH₂OH·HCl in 1 mL of CH₃OH at room temperature was added 0.064 g (0.78 mmol, 1.5 eq.) of NaOAc. The resulting solution was treated with a solution of the compound resulting from Example 71A (355 mg, 0.52 mmol, 1.0 eq.) in 2 mL of CH₃OH and stirring was continued for 1 hour. The mixture was partitioned between ethyl acetate (20 mL) and water (20 mL) and the layers were separated. The aqueous layer was extracted with 2 additional 20 mL portions of ethyl acetate and the combined organic phases were dried (Na₂SO₄), filtered and concentrated *in vacuo.* The residue was purified by column chromatography on SiO₂ (30 g, 40% ethyl acetate-hexanes) to give 0.273 g (76%) of the title compound (as a mixture of *cis* and *trans* oximes) as a colorless syrup.

### Example 71C

### (1α,2β,3β,4α)-1,2-Di[N-propyl-N-(4-phenoxybenzyl)aminocarbonyl]-4-[(hydroxyimino)methyl]-cyclobutane-3-carboxylic acid

To a solution of 0.035 g (0.05 mmol, 1.0 eq.) of the compound resulting from Example 71B in 0.5 mL of THF at 0 °C was added a solution of 0.011 g (0.25 mmol, 5 eq.) of LiOH·H₂O in 0.2 mL of water. The resulting cloudy mixture was stirred for 3 hours and quenched by the addition of 1 mL of 0.5 M H₃PO₄. The mixture was extracted with 3 x 2 mL of ethyl acetate and the combined organic phases were washed with 5 mL of brine, dried (MgSO₄), filtered and concentrated *in vacuo.* The residue was purified by column chromatography on SiO₂ (10 g, 94:5:1 CHCl₃-CH₃OH-AcOH) to give a colorless oil. This mixture was dissolved in 2 mL of acetonitrile and water was added until cloudy. The mixture was then lyophilized to give 0.024 g (70%) of the title compound as a fluffy, white lyophilate. ¹H NMR (DMSO d₆) δ 12.46 (bs, 1H), 10.76-10 90 (m, 1H), 7.38 (m, 4H), 7.09-7.30 (m, 6H), 6.86-7.0 (m, 8H), 4.55-4.88 ( m, 2H), 3.87-4.29 ( m, 4H), 3.16-3.67 (m, 5H), 2.94 (m, 1H), 2,71 (m, 1H), 1.52-1.61 (m, 4H), 0.73-0.86 (m, 6H. MS (FAB⁺) m/e 678 (M+H)⁺. HRMS (FAB) calcd for C₄₀H₄₄N₃O₇ (MH) 678.3179. Found: 678.3190. Anal calc for C₄₀H₄₄N₃O₇: C, 70.88; H, 6.39; N, 6.20. Found: C, 70.80; H, 6.32; N, 6.03.

### Example 72

### (1α,2β,3β,4α)-1,2-Di[N-propyl-N-(4-phenoxybenzyl)aminocarbonyl]-4-tetrazolyl-cyclobutane-3-carboxylic acid

### Example 72A

### Methyl(1α,2β,3β,4α)-1,2-Di[N-propyl-N-(4-phenoxybenzyl)aminocarbonyl]-4-cyano-cyclobutane-3-carboxylate

To a stirred solution of 0.173 g (0.25 mmol, 1.0 eq.) of the compound resulting from Example 71C in 2 mL of dry acetonitrile at 0 °C was added 0.11 mL (0.75 mmol, 3.0 eq.) of Et₃N followed by the dropwise addition of 0.040 mL (0.28 mmol, 1.1 eq.) of trifluoroacetic anhydride. The cooling bath was removed and the mixture stirred for 3 hours and poured into 10 mL of saturated aqueous NaHCO₃. The aqueous mixture was extracted with 3 x 10 mL of ethyl acetate and the combined organic phases were dried (MgSO₄), filtered and concentrated *in vacuo.* Flash column chromatography of the residue on SiO₂ (40 g, 40% ethyl acetate-hexanes) gave 0.116 g (69%) of the title compound as a cloudy syrup. ¹H NMR (CDCl₃) δ 6.91-7.43 (m, 18H), 3.93-4.94 (m, 6H), 3.56-3.90 (m, 5H), 2.93-3.55 (m, 4H), 1.43-1.72 (m, 4H), 0.80-0.97 (m, 6H). MS (DCI) m/e 691 (M+18)⁺, 674 (M+H)⁺.

### Example 72B

### Methyl (1α,2β,3β,4α)-1,2-Di[N-propyl-N-(4-phenoxybenzyl)aminocarbonyl]-4-tetrazolyl-cyclobutane-3-carboxylate

A mixture of the compound resulting from Example 72A (0.116 g, 0.17 mmol, 1 eq.), NaN₃ (0.034 g, 0.52 mmol, 3.0 eq.) and Et₃N·HCl (0.072 g, 0.52 mmol, 3.0 eq.) in 1 mL of DMF were heated to 60 °C for 14 hours. The bath temperature was increased to 100 °C for 4 hours whereupon an additional 3.0 eq. each of NaN₃ ad Et₃N·HCl were added. After an additional 70 hours at 100 °C, the mixture was cooled to room temperature and partitioned between water (20 mL) and ethyl acetate (20 mL). The aqueous phase was extracted with 2 x 10 mL of ethyl acetate and the combined organic phases were washed with 3 x 10 mL of water and brine, dried (Na₂SO₄), filtered and concentrated *in vacuo* to give 0.115 g of the title compound which was used without purification. Analysis of this crude material showed by ¹H NMR showed that it contained ~2 eq. of DMF.

### Example 72C

### (1α,2β,3β,4α)-1,2-Di[N-propyl-N-(4-phenoxybenzyl)aminocarbonyl-4-tetrazolyl-cyclobutane-3-carboxylic acid

To a stirred solution of 0.030 g (0.042 mmol, 1.0 eq.) of the compound resulting from Example 72B in 0.75 mL of THF at 0 °C was added 0.25 mL of H₂O followed by 0.011 g (0.25 mmol, 6.0 eq.) of LiOH·H₂O. After stirring 2 hours at 0 °C and 2 hours at room temperature, the mixture was poured into 10 mL of 3 N aqueous HCI and extracted with 3 x 10 mL of ethyl acetate. The combined organic extracts were dried (MgSO₄), filtered and concentrated *in vacuo.* The residue was purified by column chromatography on SiO₂ (20 g, 94:5:1 CHCl₃-CH₃OH-AcOH) to give a thick oil. This oil was dissolved in acetonitrile and treated with water until turbid and then freeze-dried to give 0.021 g (70%) of the title compound as a fluffy, white lyophilate. ¹H NMR (DMSO d₆) δ 6.56-7.41 (m, 18H), 4.62-4.83 (m, 3H), 4.25-4.49 (m, 4H), 3.73- .17 (m, 4H), 3.06 (m, 1H), 2.76 (m, 1H), 2.41 (m, 1H), 1.50 (m, 4H), 0.44-0.92 m, 6H). MS (FAB⁺) m/e 703 (M+H)⁺. (FAB⁻) m/e 701 (MH)⁺. HRMS calcd for C₄₀H₄₃N₆O₆: 703.3244. Found: 703.3229. Anal calcd for C₄₀H₄₃N₆O₆: C, 68.36; H, 6.02; N, 11.96. Found: C, 65.94; H, 6.11; N, 11.14.

### Example 73

### (1α,2β,3β,4α)-1,2-Di[N-propyl-N-(4-phenoxybenzyl)aminocarbonyl-4-tetrazolylmethyl-cyclobutane-3-carboxylic acid

### Example 73A

### Methyl(1α,2β,3β,4α)-1,2-Di[N-propyl-N-(4-phenoxybenzyl)aminocarbonyl]-4-cyanomethyl-cyclobutane-3-carboxylate

To a solution of the compound resulting from Example 70A (0.260 g, 0.38 mmol, 1.0 eq.) in 4 mL of 1:1 CH₂Cl₂-2,6-lutidine at -10 °C was added 0.060 mL (0.76 mmol, 2.0 eq.) of methanesulfonyl chloride. The mixture was then placed in a refrigerator overnight. The resulting yellow solution was diluted with CH₂Cl₂ and extracted with 3 N aqueous HCI, dried (MgSO₄), filtered and concentrated *in vacuo* to give 0.284 g (99%) of the mesylate that was used directly. The mesylate (0.284 g, 0.38 mmol, 1.0 eq.) was dissolved in 1.5 mL of DMSO and treated with 0.073 g (1.12 mmol, 3.0 eq.) of KCN. This suspension was stirred vigorously ovemight at room temperature and then at 50-60 °C for 2 hours. This mixture was cooled to room temperature and poured into 25 mL of water and extracted with 3 x 10 mL of ethyl acetate. The combined organic phases were then extracted with 2 x 20 mL of water and 2 x 10 mL of brine, dried (Na₂SO₄), filtered and concentrated *in vacuo.* The residue was purified by column chromatography on SiO₂ (20 g, 40% ethyl acetate-hexanes) to give 0.178 g (68%) of the title compound as a thick oil. ¹H NMR (CDCl₃) δ 6.88-7.37 (m, 18H), 4.55-4.87 (m, 2H), 3.91-4.46, (m, 4H), 2.91-3.83 (m, 9H), 2.41-2.69 (m, 2H), 1.40-1.69 (m, 4H), 0.78-0.97 (m, 6H). MS (DCI) m/e 688 (M+H)⁺.

### Example 73B

### (1α,2β,3β,4α)-1,2-Di[N-propyl-N-(4-phenoxybenzyl)aminocarbonyl-4-tetrazolylmethyl-cyclobutane-3-carboxylic acid

To a solution of the compound resulting from Example 73A (0.170 g, 0.25 mmol, 1.0 eq.) in 1.5 mL of DMF was added 0.081 g (1.15 mmol, 5.0 eq.) of NaN₃ followed by 0.172 g (1.25 mmol, 5.0 eq.) of Et₃N·HCl. The resulting suspension was heated at 100 °C overnight whereupon an additional 5 eq. each of NaN₃ and Et₃N·HCl were added. After further heating at 100 °C for 24 hours, the mixture was cooled to room temperature and poured into 20 mL of dilute H₂SO₄ and extracted with 3 x 20 mL of ethyl acetate. The combined organic phases were extracted with 2 x 20 mL of water and 1 x 10 mL of brine, dried (MgSO₄), filtered and concentrated *in vacuo* to give 0.166 g (91%) of the tetrazole as a yellow syrup that was used directly. The crude tetrazole was dissolved in 1 mL of THF and cooled to 0 °C. To this solution was added a solution of 0.034 g (0.88 mmol, 4 eq.) of LiOH·H₂O in 0.3 mL of water. Methanol (20 drops) was added to obtain a homogeneous solution and the mixture stirred overnight while the bath was allowed to melt. The reaction mixture was poured into 20 mL of 4 N H₂SO₄ and extracted with 3 x 10 mL of ethyl acetate and the combined organic phases were washed with water and brine, dried (MgSO₄), filtered and concentrated *in vacuo.* The residue was purified by column chromatography on SiO₂ (30 g, 94:5:1 CHCl₃-CH₃OH-AcOH) to give an oil. The product was then lyophilized (CH₃CN/H₂O) to give 0.064 g (40%) of the title compound as a fluffy, off white solid. ¹H NMR (CDCl₃) δ 6.76-7.38 (m, 18H), 4.20-4.75, (m, 5H), 2.91-4.09 (m, 9H), 1.36-1.64 (m, 4H), 0.65-0.92 (m, 4H). MS (FAB⁺) m/e 717 (M+H)⁺, (FAB⁻) 715 (M-H)⁺. Anal calcd for C₄₁H₄₄N₆O₆: C, 68.70; H, 6.19; N, 11.72. Found: C, 66.74; H, 6.18; N, 11.21.

### Example 74

### (1α,2β,3β,4α)-1,2-Di[N-propyl-N-(4-phenoxybenzyl)aminocarbonyl]-4-(carboxycarbonylamino)cyclobutane-3-carboxylic acid

### Example 74A

### Methyl(1α,2β,3β,4α)-1,2-Di[N-propyl-N-(4-phenoxybenzyl)aminocarbonyl-4-(benzyloxycarbonyl)amino-cyclobutane-3-carboxylate

To a stirred solution of 0.346 g (0.5 mmol, 1.0 eq.) of the compound resulting from Example 95, (1α,2β,3β,4α)-1,2-di[N-propyl-N-(4-phenoxybenzyl)aminocarbonyl]cyclobutane-3,4-dicarboxylic acid mono methyl ester, in 5 mL of dry toluene at room temperature was added 0.077 mL (0.55 mmol, 1.1 eq.) of triethylamine followed by 0.13 mL (0.60 mmol, 1.2 eq.) of diphenylphosphorylazide. The yellow solution was heated at 65-70 °C for 3 hours. To this solution was added 0.52 mL (5 mmol, 10 eq.) of benzyl alcohol and the oil bath temperature was increased to 90 °C and stirring continued at this temperature overnight. After cooling to room temperature, the mixture was diluted with 50 mL of CH₂Cl₂ and extracted with 3 N aqueous HCl and saturated NaHCO₃, dried (MgSO₄), filtered, and concentrated *in vacuo.* The residue was purified by column chromatography on SiO₂ (40 g, 5-10% ethyl acetate-CH₂Cl₂) to give 0.235 g (59%) of the title compound as a thick syrup. ¹H NMR (CDCl₃) δ 6.81-7.39 (m, 23H), 5.62 (m, 1H), 4.94-5.18 (m, 3H), 4.05-4.87 (m, 5H), 2.86-4.05 (m, 9H), 1.34-1.65 (m, 4H), 0.76-0.94 (m, 6H). MS (DCI) m/e 798 (M)⁺.

### Example 74B

### Methyl(1α,2β,3β,4α)-1,2-Di[N-propyl-N-(4-phenoxybenzyl)aminocarbonyl]-4-amino-cyclobutane-3-carboxylate

A stirred mixture of 0.233 g (0.29 mmol) of the compound resulting from Example 74A and 0.050 g of 10% Pd/C in 5 mL of ethyl acetate were hydrogenated 8 hours. The mixture was filtered through celite and the celite pad washed well with ethyl acetate. The filtrate was concentrated to give 0.191 g of the title compound as a light yellow oil. ¹H NMR (CDCl₃) δ 6.89.7.38 (m, 18H), 4.46-4.90 (m, 2H), 4.24-4.42 (m, 3H), 3.78-4.07 (m, 2H), 3.50-3.73 (m, 3H), 2.91-3.41 (m, 5H), 1.39-1.68 (m, 4H), 1.39 (bs, 2H), 0.79-0.98 (m, 6H). MS (DCI) m/e 664 (M+H)⁺.

### Example 74C

### Methyl (1α,2β,3β,4α)-1-2-Di[N-propyl-N-(4-phenoxybenzyl)aminocarbonyl]-4-(ethoxylcarbonylcarbonyl)aminocyclobutane-3-carboxylate

To a solution of 0.033 g (0.05 mmol, 1.0 eq.) of the compound resulting from Example 74B in 1 mL of CH₂Cl₂ at -10 °C was added 0.012 mL (0.10 mmol, 2 eq.) of 2,6-lutidine followed by 0.009 mL (0.075 mmol, 1.5 eq.) of ethyl oxalyl chloride. After stirring for 30 minutes at this temperature, the mixture was diluted with 10 mL of CH₂Cl₂ and extracted with 10 mL of saturated NaHCO₃, dried (MgSO₄), filtered and concentrated *in vacuo.* The residue was purified by column chromatography on SiO₂ (15 g, 1:1 ethyl acetate-hexanes) to give 0.029 g (76%) of the title compound as a light yellow syrup. ¹H NMR (CDCl₃) δ 7.84-8.04 (m, 1H), 6.83-7.37 (m, 18H), 5.16-5.37 (m, 1H), 4.48-4.94 (m, 2H), 3.92-4.39 (m, 6H), 3.54-3.76 (m, 4H), 2.87-3.46 (m, 4H), 1.43-1.64 (m, 4H), 1.24-1.43 (m, 3H), 0.78-0.93 (m, 6H). MS (CCl) m/e 764 (M+H)⁺.

### Example 74D (1α,2β,3β,4α)-1,2-Di-N-propyl-N-(4-phenoxybenzyl)aminocarbonyl]-4-(carboxycarbonylamino)cyclobutane-3-carboxylic acid

To a stirred solution of 0.027 g (0.035 mmol, 1.0 eq.) of the compound resulting from Example 74C in 1 mL of THF at 0 °C was added 0.007 g (0.18 mmol, 5 eq.) of LiOH·H₂O in 0.3 mL of water. The cold bath was removed and the mixture was stirred for 2 hours and poured into 10 mL of 3 N aqueous HCI. The aqueous phase was extracted with 3 x 15 mL of ethyl acetate and the combined organic phases were extracted with brine, dried (MgSO₄), filtered and concentrated *in vacuo* to give an oil. This material was then lyophilized (CH₃CN/H₂O) to give 0.026 g (100%) of the title compound as a fluffy white solid. ¹H NMR (CDCl₃) δ 12.45 (bs, 2H), 6.81-7.40 (m, 18H), 4.58-5.00 (m, 3H), 3.57-4.31 (m, 9H), 2.58-3.01 (m, 3H), 1.32-1.56 (m, 4H), 0.65-0.84 (m, 6H). MS (FAB⁺) m/e 744 (M+Na), 722 (M+H)⁺; (FAB⁻) m/e 720 (M-H)⁺. HRMS calcd for C₄₁H₄₄N₃O₉ (MH): 722.3078. Found: 722.3081.

### Example 75

### (1α,2β,3β,4α)-1,2-Di[N-propyl-N-(4-phenoxybenzyl)aminocarbonyl]-4-(3-carboxypropionylamino)cyclobutane-3-carboxylic acid

### Example 75A

### Methyl(1α,2β,3β,4α)-1,2-Di[N-propyl-N-(4-phenoxybenzyl)aminocarbonyl]-4-(3-carboxypropionylamino)cyclobutane-3-carboxylate

To a stirred solution of 0.024 g (0.036 mmol, 1 eq.) of the compound resulting from Example 74B in 1 mL of dry CH₃CN at 0 °C was added 0.010 mL (0.072 mmol, 2 eq.) of Et₃N followed by 0.004 g (0.038 mmol, 1.1 eq) of succinic anhydride. The mixture was then stirred overnight (during which time the ice bath melted) and partitioned between 2 mL of 3 N aqueous HCI and 3 x 2 mL of ethyl acetate. The combined organic phases were washed with 2 mL each water and brine, dried (Na₂SO₄), filtered and concentrated to give 0.028 g (100%) of the title compound as a colorless syrup. ¹H NMR (CDCl₃) δ 6.88-7.39 (m, 18H), 5.06-5.29 (m, 1H), 3.92-4.88 (m, 7H), 3.41-3.73 (m, 5H), 2.84-3.32 (m, 4H), 2.04-2.74 (m, 6H), 1.39-1.65 (m, 4H), 1.47 (m, 1H), 0.78-0.97 (m, 6H). MS (DCI) m/e 764 (M+H)⁺.

### Example 75B

### (1α,2β,3β,4α)-1,2-Di[N-propyl-N-(4-phenoxybenzyl)aminocarbonyl]-4-(3-carboxypropionylamino)cyclobutane-3-carboxylic acid

To a solution of 0.026 g (0.034 mmol, 1 eq.) of the compound resulting from Example 75A in 1 mL of THF at 0 °C was added a solution of 7 mg (0.17 mmol, 5 eq) of LiOH·H₂O in 0.3 mL of water. The ice bath was removed and the mixture stirred for 6 hours and poured into 10 mL of 3 N aqueous HCI and extracted with 3 x 10 mL of ethyl acetate. The combined organic phases were washed with 10 mL each of water and brine, dried (MgSO₄), filtered and concentrated to give a nearly colorless syrup. This material was lyophilized (CH₃CN-H₂O) to give 0.021 g (84%) of the title compound as a fluffy, off white solid. ¹H NMR (CDCl₃) δ 6.87-7.37 (m, 18H), 5.02-5.25 (m, 1H), 4.13-4.74 (m, 5H), 3.51-4.09 (m, 5H), 2.92-3.35 (m, 3H), 2.23-2.79 (m, 4H), 1.40-1.62 (m, 4H), 1.27 (bs, 1H), 0.76-0.92 (m, 6H). MS (FAB⁺) m/e 750 (M+H)⁺. HRMS calcd for C₄₃H₄₈N₃O₉ (MH): 750.3391. Found: 750.3375.

### Example 76

### (1α,2β,3β,4α)-1,2-Di[N-propyl-N-(4-phenoxybenzyl)aminocarbonyl-4-(E-2-carboxyethenyl-cyclobutane-3-carboxylic acid

### Example 76A

### Methyl(1α,2β,3β,4α)-1,2-Di[N-propyl-N-(4-phenoxybenzyl)aminocarbonyl-4-(E-2-carbomethoxyethenylcyclobutane-3-carboxylate

To a stirred solution of 0.068 g (0.10 mmol, 1.0 eq.) of the compound resulting from Example 71A in 1 mL of CH₂Cl₂ at 0 °C was added 0.040 g (0.12 mmol, 1.2 eq.) of methyl triphenylphoranylideneacetate. The resulting solution was stirred for 3 hours and then applied directly to a column of SiO₂ (15 g) and eluted with 1:1 ethyl acetate-hexanes to give 0.062 g of the title compound as a thick syrup. Analysis of the ¹H NMR spectrum indicated a >95:5 ratio of olefin isomers. ¹H NMR (DMSO-d₆) δ 6.77-7.42 (m, 18H), 5.96-6.07 (m, 1H), 4.56-4.99 (m, 2H), 4.18-4.24 (m, 2H), 3.68-4.07 (m, 3H), 3.17-4.66 (m, 9H), 2.92-3.05 (m, 1H), 2.53-2.86 (m, 2H), 1.25-1.60 (m, 4H), 0.69-0.88 (m, 6H). MS (DCl) m/e 733 (M+H)⁺.

### Example 76B

### (1α,2β,3β,4α)-1,2-Di[N-propyl-N-(4-phenoxybenzyl)aminocarbonyl-4-(E-2-carboxyethenyl-cyclobutane-3-carboxylic acid

To a stirred solution of the compound resulting from Example 76A (25 mg, 0.034 mmol, 1.0 eq.) in 1 mL of THF at 0 °C was added 9 mg (0.204 mmol, 6 eq.) of LiOH·H₂O in 0.3 mL of water. After stirring for 1 hour at 0 °C and 2 hours at room temperature, the mixture was poured into 10 mL of 3 N aqueous HCI and extracted with 2 x 10 mL of ethyl acetate. The organic phases were then washed with brine, dried (MgSO₄), filtered and concentrated to give 0.025 g of product. This material was then lyophilized (CH₃CN-H₂O) to give 0.019 g (79%) of the title compound as a white lyophilate. ¹H NMR (DMSO-d₆) δ 6.80-7.41 (m, 18H), 6.86-6.95 (m, 1H), 4.10-5.02 (m, 5H), 3.16-4.08 (m, 9H), 2.44-2.98 (m, 3H), 1.30-1.44 (m, 4H), 0.66-0.91 (m, 6H). MS (FAB⁺) m/e 727 (M+Na), 705 (M+H)⁺.

### Example 77

### (1α,2β,3β,4α)-1,2-Di[N-propyl-N-(4-phenoxybenzyl)aminocarbonyl-4-(2-carboxyethyl)-cyclobutane-3-carboxylic acid

### Example 77A

### Methyl (1α,2β,3β,4α)-1,2-Di[N-propyl-N-(4-phenoxybenzyl)aminocarbonyl-4-(2-carboxyethyl)-cyclobutane-3-carboxylate

A stirred mixture of the compound resulting from Example 76A (32 mg, 0.044 mmol, 1.0 eq.), triethylsilane (0.011 mL, 0.066 mmol, 1.5 eq.) and Wilkinson's catalyst (4 mg, 0.004 mmol, 0.01 eq.) in 1 mL of dry toluene was heated to 80-90 °C. After 1 hour, additional triethylsilane (0.050 mL) and Wilkinson's (4 mg) were added and heating continued for an additional 2 hours. After the mixture had cooled to room temperature, 2 mL of CH₃OH was added and the mixture stirred for 15 minutes and filtered through a short plug of SiO₂. The filtrate was concentrated and purified by column chromatography on SiO₂ (10 g, 1:1 ethyl acetate-hexanes) to give 0.029 g (91%) of the title compound as a thick oil. ¹H NMR (CDCl₃) δ 6.88-7.41 (m, 18H), 4.48-5.04 (m, 3H), 3.93-4.38 (m, 4H), 3.49-3.75 (m, 6H), 2.76-3.33 (m, 5H), 2.12-2.35 (m, 2H), 1.41-1.88 (m, 6H), 0.77-1.02 (m, 6H). MS (DCl) m/e 735 (M)⁺.

### Example 77B

### (1α,2β,3β,4α)-1,2-Di[N-propyl-N-(4-phenoxybenzyl)aminocarbonyl-4-(2-carboxyethyl)-cyclobutane-3-carboxylic acid

To a stirred solution of the compound resulting from Example 77A (27 mg, 0.037 mmol, 1.0 eq.) in 1 mL of THF at 0 °C was added a solution of 8 mg (0.184 mmol, 5 eq.) of LiOH·H₂O in 0.3 mL of water. The cooling bath was removed and the mixture stirred for 6 hours and then poured into 10 mL of 3 N aqueous HCI. The aqueous phases was extracted with ethyl acetate (3 x 10 mL) and the combined organic phases were washed with saturated aqueous NaCI, dried (Na₂SO₄), filtered and concentrated. The residue was purified by column chromatography on SiO₂ (10 g, 94:5:1 CHCl₃-CH₃OH-AcOH) to give an oily residue. Lyophilization of this residue gave the title compound (0.019 g, 73%) as a fluffy, white lyophilate. ¹H NMR (CDCl₃) δ 6.84-7.37 (m, 18H), 4.55-4.98 (m, 2H), 4.13-4.48 (m, 2H), 3.45-3.96 (m, 2H), 2.81-3.37 (m, 6H), 2.25-2.43 (m, 2H), 1.39-1.82 (m, 6H), 0.74-0.94 (m, 6H). MS (FAB⁺) m/e 783 (M+2K-H)⁺, 745 (M+K)⁺, 707 (M+H)⁺.

### Example 78

### (1α,2β,3β,4α)-1,2-Di[N-propyl-N-(4-phenoxybenzyl)aminocarbonyl]-4-(1-carboxy-1-hydroxymethyl)cyclobutane-3-carboxylic acid

### Example 78A

### Methyl (1α,2β,3β,4α)-1,2-Di[N-propyl-N-(4-phenoxybenzyl)aminocarbonyl]-4-(1-acetoxy-1-(2-furanyl)-methyl)cyclobutane-3-carboxylate

To a solution of 0.029 mL (0.40 mmol, 4.0 eq.) of furan in 0.8 mL of dry THF at 0 °C was added 0.25 mL (0.4 mmol, 4.0 eq.) of a 1.6 M solution of n-BuLi in hexanes dropwise. The solution was briefly warmed to room temperature and then cooled to 0 °C. The yellow solution was then added *via* cannula to a suspension of 18 mg (0.2 mmol, 2.0 eq.) of CuCN in 0.6 mL of THF at -20 °C and this mixture stirred 5 minutes at 0 °C. To the resulting solution (yellow) was added a solution of 68 mg (0.1 mmol, 1.0 eq.) of the compound resulting from Example 71A in 0.5 mL of dry THF. The mixture was stirred for 1.5 hours at -20 - 0 °C and then quenched by the addition of 1 mL of 95:5 saturated aqueous NH₄CI-conc aqueous NH₄OH. The cooling bath was removed, 5 mL of ether was added, and the biphasic solution was stirred vigorously for 15 minutes. The mixture was then poured into 10 mL of the above NH₄Cl/NH₄OH solution and extracted with 2 x 10 mL of ethyl acetate. The combined organic phases were washed with the NH₄Cl/NH₄OH mixture (10 mL), dried (MgSO₄), filtered and concentrated. The residue was dissolved in 0.5 mL of CH₂Cl₂ and cooled to 0 °C. The mixture was then treated with excess 2,6-lutidine, DMAP (0.005 g) and acetic anhydride (0.017 mL). After stirring for 30 minutes at room temperature, the mixture was diluted with 25 mL of ethyl acetate and washed with water, 3 N aqueous HCI and saturated aqueous NaHCO₃ (10 mL each), dried (MgSO₄), filtered and concentrated. The residue was purified by column chromatography on SiO₂ (15 g, 40% ethyl acetate-hexanes) to give 0.031 g (39%) of the title compound as a colorless oil. ¹H NMR (CDCl₃) δ 6.89-7.38 (m, 18H), 5,93-6.42 (m, 3H), 4.92 (m, 1H), 3.92-4.68 (m, 5H), 3.37-3.81 (m, 5H), 2.75-3.37 (m, 3H), 1.71-2.29 (m, 3H), 1.20-1.67 (m, 4H), 0.73-0.98 (m, 6H). MS (DCI) m/e 787 (M+H)⁺.

### Example 78B

### (1α,2β,3β,4α)-1,2-Di[N-propyl-N-(4-phenoxybenzyl)aminocarbonyl]-4-(1-carboxy-1-hydroxymethyl)cyclobutane-3-carboxylic acid

To a vigorously stirred solution 1 mg (0.007 mmol, 0.2 eq.) of RuO₂·H₂O in 0.5 mL of 3:2:3 CH₃CN/CCl₄/H₂O at room temperature was added 62 mg (0.29 mmol, 8.0 eq.) of NalO₄. After 15 minutes, NaHCO₃ (150 mg) was added followed by the addition of the compound resulting from Example 78A (28 mg, 0.036 mmol, 1.0 eq.) in 0.3 mL CH₃CN. The yellow solution immediately turned black. Excess NalO₄ was added followed by 5 mL of ethyl acetate. The mixture was poured into water and acidified by the addition of 4 N aqueous H₂SO₄ and the phases were separated. The aqueous phase was extracted with 2 x 10 mL of ethyl acetate and the combined organic phases were washed with 3 x 10 mL of 10% aqueous NaHSO₃, dried (MgSO₄) filtered and concentrated to give 0.024 g (86%) of the title compound as a colorless syrup which was used directly.

To a solution of 0.024 g (0.031 mmol, 1.0 eq.) of the above acid-ester in 0.5 mL of THF at 0 °C was added a solution of 13 mg (0.31 mmol, 10 eq.) of LiOH·H₂O in 0.2 mL of water. The ice bath was removed and the mixture was stirred for 2 hours at room temperature and then poured into 10 mL of 3 N aqueous HCI. The aqueous phase was extracted with 3 x 10 mL of ethyl acetate and the combined organic phases were washed with brine, dried (Na₂SO₄), filtered and concentrated to give a light yellow oil. This oil was then lyophilized (CH₃CN-H₂O) to give 0.020 g (91%) of the title compound as an off white, fluffy lyophilate. ¹H NMR (CD₃OD) δ 6.86-7.37 (m, 18H), 4.69-4.78 (m, 1H), 4.23-4.47 (m, 3H), 2.84-3.67 (m, 12H), 1.46-1.71 (m, 4H), 0.81-0.97 (m, 6H). MS (FAB⁺) m/e 747 (M+K)⁺, 731 (M+Na)⁺, 709 (M+H)⁺, (FAB⁻) m/e 707 (M-H)⁺.

### Example 79

### (1α,2β,3β,4α)-1,2-Di[N-propyl-N-(4-phenoxybenzyl)aminocarbonyl]-3,4-di[(hydroxyimino)methyl]-cyclobutane

### Example 79A

### (1α,2α,3β,4α)-1,2-Di[N-propyl-N-(4-phenoxybenzyl)aminocarbonyl]-cyclobutane-3,4-di(O-benzyloxime)

A solution of the compound resulting from Example 64B (200 mg (0.29 mmol) in dry tetrahydrofuran (10 mL) was cooled to -15 °C under dry nitrogen. N-Methylmorpholine (60 mg, 0.59 mmol) and isobutylchloroformate (81 mg, 0.59 mmol) were added followed by a solution of O-benzylhydroxylamine hydrochloride (94 mg, 0.59 mmol) and N-methylmorpholine (60 mg, 0.59 mmol) in dimethylformamide (2 mL). After stirring at -15 °C for 15 minutes, the mixture was stirred at ambient temperature for 3 hours. The reaction mixture was concentrated under reduced pressure, dissolved in ethyl acetate, washed with 1 M HCI, 5% NaHCO₃, and brine, dried over Na₂SO₄, and concentrated *in vacuo.* The residue obtained was purified by flash silica gel chromatography eluting with 5% methanol in chloroform to give 100 mg (38%) of the benzyl protected oximes.

### Example 79B

### (1α,2β,3β,4α)-1,2-Di[N-propyl-N-(4-phenoxybenzyl)aminocarbonyl]-3,4-di[(hydroxyimino)methyl]-cyclobutane

A solution of the compound resulting from Example 79A (50 mg (56 µmol) in methanol (15 mL) with 10% palladium on carbon (5 mg) was stirred under an atmosphere of hydrogen for 5 hours. Filtration through Celite and evaporation provided 20.5 mg (51%) of the title compound. ¹H NMR (CDCl₃, 300 MHz) δ 0.88 (m (6H) (1.45 (m (4H) (2.95 (m (4H) (3.62 (m (4H) (4.18 (m (4H) (6.85-7.37 (m (18H). MS m/e 709 (M+H)⁺.

### Example 80

### (1α,2β,3β,4α)-1,2-Di[N-methyl-N-(4-phenoxybenzyl)aminocarbonyl]cyclobutane-3,4-dicarboxylic acid dimethyl ester

To the dicarboxylic acid resulting from Example 13 (1.00 g, 1.60 mmol) dissolved in 20 mL of anhydrous methanol was added 5 drops of concentrated sulfuric acid. The clear mixture was stirred at ambient temperature overnight. The solvents were removed under reduced pressure to give a residue which was dissolved in ethyl acetate, washed with sodium bicarbonate solution, water and saturated sodium chloride solution, dried, filtered and concentrated *in vacuo.* The residue obtained was chromatographed on silica gel eluting with 4:1 ethyl acetate-hexane to give the title compound in 70% yield. ¹H NMR (DMSO-d₆, 300 MHz) δ 2.89 (s (6H) (3/59 (s (6H) (3.76 (d (J = 9.6Hz (2H) (4.14 (d (2H) (4.40 (d (2H) (4.56 (d (2H) (6.95-7.40 (m (18H). MS m/e 651 (M+H)⁺.

### Example 81

### (1α,2β,3β,4α)-1,2-Di[N-propyl-N-(3-phenoxybenzyl)aminocarbonyl]-cyclobutane-3,4-dicarboxylic acid

### Example 81A

### N-Propyl-N-(3-phenoxybenzyl)amine

3-Phenoxybenzaldehyde (2.00 mL, 2.29 g, 11.6 mmol), n-propylamine (0.95 mL, 0.68g, 11.5mmol), and *p*-toluenesulfonic acid monohydrate (15 mg, 0.08 mmol) were dissolved in absolute ethanol (15 mL), then heated to 80 °C in a sealed tube for 2.5 hours. The reaction was cooled to room temperature, transfered to a round-bottom flask, then sodium borohydride (440 mg, 11.6 mmol) was added, followed by heating under reflux for 2.5 hours. The reaction was concentrated, the residue was partitioned between water and EtOAc, and the EtOAc layer was washed with water and brine. After drying with Na₂SO₄, filtration, and concentration, the residue was purified by chromatography on silica gel using 4:6 followed by 3:7 hexane-EtOAc to give 1.59 g (57%) light yellow oil. ¹H NMR (CDCl₃) δ 7.30 (m, 3H), 7.08 (m, 2H), 7.00 (m, 3H), 6.88 (dd, 1H), 3.77 (s, 2H), 2.60 (t, 2H), 1.53 (m, 2H), 0.92 (t, 3H). MS (DCI/NH₃) m/e 242 (M+H)⁺.

### Example 81B

### (1α,2β,3β,4α)-1,2-Di[N-propyl-N-(3-phenoxybenzyl)aminocarbonyl]cyclobutane-3.4-dicarboxylic acid

To 1,2,3,4-cyclobutanetetracarboxylic dianhydride (313 mg, 1.60 mmol) slurried in CH₃CN (4.0 mL) was added the compound resulting from Example 81A (803 mg, 3.30 mmol). The reaction was stirred at room temperature under N₂ overnight, then diluted with EtOAc, washed with 2 x 1 M H₃PO₄ and brine, dried over Na₂SO₄ and concentrated *in vacuo.* Chromatography of the residue obtained using 98.5:1.5:0.5 followed by 97:3:1 CHCl₃-MeOH-CH₃CO₂H gave a glass which was dissolved in CH₃CN (10 mL). Water (10 mL) was added to the CH₃CN solution, then the solution was frozen and lyophilized to give 390 mg (36%) of the title compound as a white solid. ¹H NMR (DMSO-d₆) δ 7.39, 7.28, 7.13, 7.00, 6.93, 6.85 (all m, total 18H), 4.60, 4.45, 4.30 (all m, total 4H), 3.90, 3.60 (both m, total 4H), 3.45, 3.25, 3.00, 2.80 (all m, total 4H), 1.40 (m, 4H), 0.78 (m, 6H). MS (FAB⁺) m/e 679 (M+H)⁺. Anal. calcd for C₄₀H₄₂N₂O₈ · 0.25H₂O: C, 70.32; H, 6.27; N, 4.10. Found: C, 70.20; H, 6.51; N, 4.04.

### Example 82

### (1α,2β,3β,4α)-1,2-Di[N-propyl-N-(5-phenoxyfurfuryl)aminocarbonyl)cyclobutane-3,4-dicarboxylic acid

### Example 82A

### 5-Phenoxy-2-furaldehyde

Phenol (3.80 g, 40.4 mmol) in DMSO (25 mL) was added dropwise to a suspension of NaH (60%, 1.63 g, 40.4 mmol) in DMSO (25 mL) over a period of 15 minutes. After another 15 minutes, a solution of 5-nitro-2-furaldehyde (4.85 g, 34.4 mmol) in DMSO (20 mL) was added. The reaction was stirred at room temperature for 4.5 hours, then the reaction was partitioned between ice-water, brine and Et₂O. The aqueous layer was extracted with Et₂O, and the combined Et₂O extracts were washed with 7% KOH, dried over Na₂SO₄ and concentrated *in vacuo* to afford a brown oil. Vacuum distillation (2.8 mm Hg, 137-8 °C) gave 3.3 g (51%) yellow oil. ¹H NMR (CDCl₃) δ 9.42 (s, 1H), 7.42 (m, 2H), 7.28 (m, 1H), 7.20 (m, 3H), 5.55 (d, 1H). MS (DC/NH₃) m/e 189 (M+H)⁺, 206 (M+H+NH₃)⁺.

### Example 82B

### (1α,2β,3β,4α)-1,2-Di[N-propyl-N-(5-phenoxyfurfuryl)aminocarbonyl}cyclobutane-3,4-dicarboxylic acid

Using the compound resulting from Example 82A, N-propyl-N-(5-phenoxyfurfuryl)amine was prepared by the method of Example 81A. ¹H NMR (CDCl₃) δ 7.32 (m, 2H), 7.10, (m, 1H), 7.04 (m, 2H), 6.12 (d, 1H), 5.51 (d, 1H), 3.70 (s, 2H), 2.59 (t, 2H), 1.54 (m, 2H), 0.92 (t, 3H). MS (DC/NH₃) (M+H)⁺ 232.

Using the amine prepared above, the title compound was prepared using the method of Example 81B. ¹H NMR (DMSO-d₆) δ 7.38 (m, 4H), 7.15 (m, 2H), 7.07 (m, 2H), 7.02 (m, 2H), 6.34, 6.32, 6.29, 6.25 (all d, total 2H), 5.70, 5.67 (d, m, total 2H), 4.52, 4.35, 4.17 (all m, total 4H), 3.90, 3.65, 3.60, 3.55 (all m, total 4H), 3.40-3.20, 3.00 (envelope, m, total 4H), 1.40 (m, 4H), 0.75 (m, 6H). MS (FAB-) m/e 657 (M-H)⁻. Anal cald for C₃₆H₃₈N₂O₁₀ · 0.5 H₂O: C, 64.76; H, 5.89; N, 4.20. Found: C, 64.80; H, 5.79; N, 4.02.

### Example 83

### (1α,2β,3β,4α)-1,2-Di[N-propyl-N-(5-phenoxythien-2-ylmethyl)aminocarbonyl]cyclobutane-3,4-dicarboxylic acid

### Example 83A

### N-Propyl-N-(5-phenoxythiophene-2-methyl)amine

Using 5-nitrothiophene-2-carboxaldehyde, 5-phenoxythiophene-2-carboxaldehyde was prepared by the method of Example 82A, except chromatography using 9:1 hexane-EtOAc was used for purification. ¹H NMR (CDCl₃) δ 9.72 (s, 1H), 7.54 (d, 1H), 7.42 (m, 2H), 7.25 (m, 1H), 7.20 (m, 2H), 6.51 (d, 1H). MS (DCl/NH₃) m/e 205 (M+H)⁺, 222 (M+H+NH₃)⁺.

### Example 83B

### (1α,2β,3β,4α)-1,2-Di[N-propyl-N-(5-phenoxythien-2-ylmethyl)aminocarbonyl]cyclobutane-3,4-dicarboxylic acid

Using the compound resulting from Example 83A, N-propyl-N-(5-phenoxythiophene-2-methyl)amine was prepared by the method of Example 81A. ¹H NMR (CDCl₃) δ 7.32 (m, 2H), 7.10, (m, 3H), 6.52 (dt, 1H), 6.38 (d, 1H), 3.88 (d, 2H), 2.63 (t, 2H), 1.54 (m, 2H), 0.92 (t, 3H); MS (DC/NH₃) (M+H)⁺ 248.

Using the amine prepared above, the title compound was prepared by the method of Example 81B. ¹H NMR (DMSO-d₆) δ 7.37 (m, 4H), 7.13, 7.05 (both m, total 6H), 6.98, 6.94 (both m, total 2H), 6.52, 6.46, 6.45, 6.40 (all d, total 2H), 4.65, 4.40 (both m, total 4H), 3.95-3.50 (envelope, total 4H), 3.43, 3.20, 3.03, 2.88 (all m, total 4H), 1.43 (m, total 4H), 0.80, 0.70 (both m, total 6H). MS (FAB⁻) m/e 689 (M-H)⁻. Anal cald for C₃₆H₃₈N₂O₈S₂ · 0.25 H₂O: C, 62.19; H, 5.58; N, 4.03. Found: C, 62.08; H, 5.51; N, 3.92.

### Example 84

### (1α,2β,3β,4α)-1,2-Di[N-propyl-N-(4-(furan-2-yloxybenzyl)aminocarbonyl]cyclobutane-3,4-dicarboxylic acid

### Example 84A

### 5-(4-Ethoxycarbonylphenoxy)-2-furaldehyde

Using ethyl-4-hydroxybenzoate, the title compound was prepared by the method of Example 82A, except chromatography using 3:1 hexane-EtOAc was used for purification. ¹H NMR (CDCl₃) δ 9.45 (s, 1H), 8.10 (m, 2H), 7.25 (d, 1H), 7.19 (m, 2H), 5.75 (d, 1H), 4.40 (q, 2H), 1.41 (t, 3H). MS (DCI/NH₃) m/e 261 (M+H)⁺, 278 (M+H+NH₃)⁺.

### Example 84B

### 5-(4-Ethoxycarbonylphenoxy)-2-furoic acid

A solution of potassium dihydrogen phosphate (10.8 g, 79.0 mmol) and sodium chlorite (80%, 12.0 g, 106 mmol) in water (95 mL) was added to a solution of the compound resulting from Example 84A (3.00 g, 11.5 mmol) in t-butanol (240 mL) and 2-methyl-2-butene (57 mL). The two-phase reaction mixture was mechanically stirred at room temperature for 2 hours, then the aqueous layer was removed, and the organic layer concentrated. The aqueous layer and the organic residue were combined, the pH was adjusted to 2 with 1.1 N NaHSO₄, then extracted with Et₂O. The Et₂O layer was washed with 5% sodium bisulfite, then extracted with saturated NaHCO₃. The saturated NaHCO₃ layer was washed with 3 x Et₂O, then the pH was adjusted to 1 with 1.1 N NaHSO₄, and extracted with 3 x Et₂O. After drying over Na₂SO₄ and concentrating under reduced pressure 1.10 g (34%) of the title compound as a light yellow solid was obtained. ¹H NMR (CD₃OD) δ 8.08 (m, 2H), 7.27 (d, 1H), 7.21 (m, 2H), 5.90 (d, 1H), 4.36 (q, 2H), 1.39 (t, 3H). MS (DC/NH₃) m/e 294 (M+H+NH₃)⁺.

### Example 84C

### Ethyl 4-(furan-2-yloxy)benzoate

To the compound resulting from Example 84B (1.05 g, 3.80 mmol) slurried in quinoline (1.7 mL) was added copper powder (70 mg). The reaction was heated to 200 °C for 1 hour, cooled to room temperature, and partioned between Et₂O and 1 M H₃PO₄. The Et₂O layer was washed with 3 x 1 M H₃PO₄, 3 x saturated NaHCO₃ and brine, dried over Na₂SO₄ and concentrated *in vacuo.* Chromatography of the residue using 2% EtOAc in hexanes gave 550 mg of the title compound as a yellow oil (62%). ¹H NMR (CDCl₃) δ 8.04 (m, 2H), 7.10 (dd, 1H), 7.03 (m, 2H), 6.40 (dd, 1H), 5.70 (dd, 1H), 4.36 (q, 2H), 1.39 (t, 3H). MS (DCI/NH₃) m/e 233 (M+H)⁺, 250 (M+H+NH₃)⁺.

### Example 84D

### 4-(Furan-2-yloxy)benzyl alcohol

A solution of the compound resulting from Example 84C (535 mg, 2.30 mmol) in THF (4 mL) was added to a solution of LAH in THF (4 mL of 1.0 M LAH). The reaction mixture was stirred at room temperature for 1hour and then cooled to 5 °C. Water (0.13 mL), 15% NaOH (0.13 mL), and water (0.35 mL) were added sequentially, and the mixture was stirred for 15 minutes. After the addition of Et₂O and MgSO₄, the mixture was filtered through a small plug of silica gel and the filtrate concentrated under reduced pressure to afford 435 mg (100%) of the title compound as a yellow oil. ¹H NMR (CDCl₃) δ 7.35 (m, 2H), 7.05 (dd, 1H), 7.03 (m, 2H), 6.44 (dd, 1H), 5.60 (dd, 1H), 4.66 (d, 2H), 1.63 (t, 1H); MS (DCI/NH₃) m/e 191 (M+H)⁺.

### Example 84E

### 4-(Furan-2-yloxy)benzaldehyde

To the compound resulting from Example 84D (430 mg, 2.30 mmol) dissolved in 9:1 CH₂Cl₂-CH₃CN (22 mL) was added N-morpholine-N-oxide (400 mg, 3.40 mmol) and powdered activated molcular sieves (2.10 g). After stirring for 10 minutes, tetrapropylammonium perruthenate (40 mg, 0.11 mmol) was added, the reaction was stirred at room temperature for 30 minutes, and then celite and Et₂O were added. The mixture was filtered through a small plug of silica gel, and the filtrate concentrated to give 350 mg (81%) of the title compound as a brown oil. ¹H NMR (CDCl₃) δ 9.95 (s, 1H), 7.88 (m, 2H), 7.12 (m, 3H), 6.42 (dd, 1H), 5.76 (dd, 1H). MS (DCI/NH₃) m/e 189 (M+H)⁺, 206 (M+H+NH₃)⁺.

### Example 84F

### N-Propyl-N-4-(furan-2-yloxy)benzylamine

Using the compound resulting from Example 84E, the title compound was prepared by the method of Example 81A. ¹H NMR (CDCl₃) δ 7.28 (m, 2H), 7.05 (dd, 1H), 6.99 (m, 2H), 6.33 (dd, 1H), 5.56 (dd, 1H), 3.77 (s, 2H), 2.60 (t, 2H), 1.55 (m, 2H), 0.93 (t, 3H). MS (DC/NH₃) m/e 232 (M+H)⁺.

### Example 84G

### (1α,2β,3β,4α)-1,2-Di[N-propyl-N-(4-(furan-2-yloxybenzyl)aminocarbonyl]cyclobutane-3,4,dicarbozylic acid

Using the compound resulting from Example 84F, the title compound was prepared by the method of Example 81B. ¹H NMR (DMSO-d₆) δ 7.32, 7.25 (both m, total 6H), 7.02, 6.95 (both m, total 4H), 6.48 (m, 2H), 5.78 (m, 2H), 4.70, 4.25 (both m, total 4H), 3.90, 3.60 (both m, total 4H), 3.55-3.15, 2.93, 2.78 (envelope, m, m, total 4H), 1.48 (m, 4H), 0.80 (m, 6H). MS (FAB⁻) m/e 657 (M-H)⁻. Anal cald for C₃₆H₃₈N₂O₁₀ · 0.5 H₂O: C, 64.76; H, 5.89; N, 4.20. Found: C, 64.82; H, 5.81; N, 3.99.

### Example 85

### (1α,2β,3β,4α)-1,2-Di[N-propyl-N-(4-(thiazol-2-yloxybenzyl)aminocarbonyl]cyclobutane-3,4-dicarboxylic acid

Using ethyl-4-hydroxybenzoate and 2-bromothiazole, ethyl 4-(thiazol-2-yloxy)benzoate was prepared by the method of Example 82A, except the reaction was heated to 120 °C overnight, and chromatography using 9:1 hexane-EtOAc was used for the purification. ¹H NMR (CDCl₃) δ 8.10 (m, 2H), 7.35 (m, 2H), 7.28 (d, 1H), 6.90 (d, 1H), 4.38 (q, 2H), 1.39 (t, 3H). MS (DCl/NH₃) (M+H)⁺ 250 and (M+H+NH₃)⁺ 267.

Using the ester prepared above and the procedures described in Example 84D provided 4-(thiazol-2-yloxy)benzyl alcohol. ¹H NMR (CDCl₃) δ 7.43 (m, 2H), 7.28 (m, 2H), 7.23 (d, 1H), 6.82 (d, 1H), 4.70 (d, 2H), 1.88 (t, 1H). MS (DCI/NH₃) (M+H)⁺ 208.

Using the alcohol prepared above and the procedures described in Example 84E, except chromatography using 4:1 hexane-EtOAc was used for purification, provided 4-(thiazol-2-yloxy)benzaldehyde. ¹H NMR (CDCl₃) δ 10.00 (s, 1H), 7.95 (m, 2H), 7.46 (m, 2H), 7.30 (d, 1H), 6.95 (d, 1H). MS (DCl/NH₃) (M+H)⁺ 206 and (M+H+NH₃)⁺ 223.

Using the aldehyde prepared above and the procedures described in Example 81A, except 3-5% MeOH in CHCl₃ was used for the chromatography, provided N-propyl-N-(4-(thiazoly-2-yloxy)benzyl)amine. ¹H NMR (CDCl₃) δ 7.39 (m, 2H), 7.23 (d, 1H), 7.23 (m, 2H), 6.80 (d, 1H), 5.56 (dd, 1H), 3.80 (s, 2H), 2.63 (t, 2H), 1.55 (m, 2H), 0.96 (t, 3H). MS (DCI/NH₃) (M+H)⁺ 249.

Using the amine prepared above, the title compound was prepared by the method of Example 81B, except additional purification by preparative HPLC was required (Rainin Dynamax-60A C18 column, using a gradient of 20-100% CH₃CN vs 0.1% TFA in water). ¹H NMR (DMSO-d₆) δ 7.40-7.20 (envelope, 12H), 4.75, 4.30 (both m, total 4H), 4.00, 3.89, 3.65 (all m, total 4H), 3.55-3.20, 2.96, 2.78 (envelope, m, m, total 4H), 1.50 (m, 4H), 0.80 (m, 6H). MS (FAB⁺) m/e 693 (M+H)⁺. Anal cald for C₃₄H₃₆N₄O₈S₂ · 0.33 TFA: C, 56.99; H, 5.01; N, 7.67. Found: C, 57.02; H, 5.11; N, 7.64.

### Example 86

### (1α,2β,3β,4α)-1,2-Di[N-propyl-N-(4-(pyrrol-1-ylmethyl)benzyl)aminocarbonyl]cyclobutane-3,4-dicarboxylic acid

### Example 86A

### N-(4-(Pyrrol-1-ylmethyl)benzyl)amine

Using pyrrole and 4-(bromomethyl)benzonitrile and the method of Example 83A, except using DMF as the solvent, provided N-(4-cyanobenzyl)pyrrole. ¹H NMR (CDCl₃) δ 7.62 (m, 2H), 7.14 (m, 2H), 6.69 (m, 2H), 6.24 (m, 2H), 5.15 (s, 2H). MS (DCI/NH₃) (M+H)⁺ 183 and (M+H+NH₃)⁺ 200.

Using the nitrile prepared above and the method of Example 84D, except after 1 hour at room temperature the reaction was heated under reflux for 75 minutes, provided the title compound. ¹H NMR (CDCl₃) δ 7.28 (m, 2H), 7.10 (m, 2H), 6.69 (m, 2H), 6.19 (m, 2H), 5.05 (s, 2H) 3.85 (s, 2H). MS (DC/NH₃) (M+H)⁺ 187 and (M+H+NH₃)⁺ 204.

### Example 86B

### N-[4-(Pyrrol-1-ylmethyl)benzyl]propylamide

To the compound resulting from Example 86A (1.00 g, 5.38 mmol) and triethylamine (0.60 g, 0.82 mL, 5.93 mmol) in CH2C12 (8 mL) cooled to 0 °C was added propionyl chloride (0.50 g, 0.47 mL, 5.41 mmol) in CH₂Cl₂ (5.5 mL) dropwise. The bath was removed and the reaction mixture stirred for 10 minutes, then diluted with EtOAc. The resulting solution was washed with 3 x 1 M H₃PO₄, 3 x saturated NaHCO₃ and brine, dried over Na₂SO₄ and concentrated under reduced pressure to afford 1.25 g (96%) white solid. ¹H NMR (CDCl₃) δ 7.24 (d, 2H), 7.08 (d, 2H), 6.69 (m, 2H), 6.19 (m, 2H), 5.75 (br s, 1H), 5.05 (s, 2H) 4.40 (d, 2H), 2.23 (q, 2H), 1.18 (t, 3H). MS (DCl/NH₃) (M+H)⁺ 243 and (M+H+NH₃)⁺ 260.

### Example 86C

### N-Propyl-N-(4-(pyrrol-1-ylmethyl)benzyl)amine

A solution of the compound resulting from Example 86B (1.20 g, 4.96 mmol) in THF (8 mL) was added to a solution of LAH in THF (9.9 mL of 1.0 M LAH), heated under reflux 3 hours, then cooled to 5 °C Then water (0.50 mL), 15% NaOH (0.50 mL), and water (1.50 mL) were added and the mixture stirred for 15 minutes. After the addition of Et₂O and MgSO₄, the mixture was filtered through a small plug of silica gel, which was rinsed with CH₂Cl₂. The filtrate was concentrated under reduced pressue and purified by chromatography using 97:3 CHCl₃-MeOH to give a yellow oil (880 mg, 78%). ¹H NMR (CDCl₃) δ 7.28 (d, 2H), 7.08 (d, 2H), 6.69 (m, 2H), 6.19 (m, 2H), 5.05 (s, 2H) 3.77 (s, 2H), 2.59 (t, 2H), 1.55 (m, 2H), 0.93 (t, 3H). MS (DCl/NH₃) (M+H)⁺ 229.

### Example 86D (1α,2β,3β,4α)-1,2-Di[N-propyl-N-(4,(pyrrol-1-ylmethyl)benzyl)aminocarbonyl]cyclobutane-3,4-dicarboxylic acid

Using the compound resulting from Example 86C, the title compound was prepared by the method of Example 81B. ¹H NMR (DMSO-d₆) δ 7.20-7.00 (envelope, 8H), 6.80 (m, 4H), 6.02 (m, 4H) 5.78 (m, 2H), 5.10, 5.08 (both s, total 4H), 4.70, 4.25 (both m, total 4H), 3.92, 3.80, 3.60 (all m, total 4H), 3.55-3.10, 2.90, 2.75 (envelope, m, m, total 4H), 1.45 (m, 4H), 0.78 (m, 6H). MS (FAB⁺) m/e 653 (M+H)⁺. Anal cald for C₃₈H₄₄N₄O₆ · 0.25 H₂O: C, 69.44; H, 6.82; N, 8.52. Found: C, 69.47; H, 6.70; N, 8.35.

### Example 87

### (1α,2β,3β,4α)-1,2-Di[N-propyl-N-(3-methyl-4-phenoxybenzyl)aminocarbonyl]cyclobutane-3,4-dicarboxylic acid

### Example 87A

### 3-Methyl-4-phenoxybenzaldehyde

*m*-Cresol (2.58 g, 24.0 mmol) in DMSO (5 mL) was added dropwise to a suspension of NaH (60%, 1.63 g, 40.4 mmol) in DMSO (10 mL) over a period of 15 minutes. After another 15 minutes, a solution of *p*-fluorobenzaldehyde (2.54 g, 20.5 mmol) in DMSO (5 mL) was added. The reaction was heated at 125 °C for 3 hours, then cooled to room temperature and partitioned between 1 N HCI and Et₂O. The Et₂O layer was washed with 15% NaOH and brine, dried over Na₂SO₄. Vacuum distillation (3.4 mm Hg, 154-8 °C) gave 2.35 g oil which was purified by chromatography using 9:1 hexane-Et₂O to afford 1.98 g (46%) of a colorless oil. ¹H NMR (CDCl₃) δ 9.92 (s, 1H), 7.85 (m, 2H), 7.30 (m, 1H), 7.05 (m, 3H), 6.90 (m, 2H), 2.38 (s, 3H). MS (DCl/NH₃) (M+H)⁺ 213 and (M+H+NH₃)⁺ 230.

### Example 87B

### (1α,2β,3β,4α)-1,2-Di{N-propyl-[4-(3'-methyl)phenoxybenzyl]aminocarbonyl}cyclobutane-3,4-dicarboxylic acid

Using the compound resulting from Example 87A and the procedures described in Example 81A provided N-propyl-N-(3-methyl-4-phenoxybenzyl)amine. ¹H NMR (CDCl₃) δ 7.28 (m, 2H), 7.30 (m, 1H), 6.95 (m, 2H), 6.90 (m, 1H), 6.80 (m, 2H), 3.77 (s, 2H), 2.33 (s. 3H), 2.62 (t, 2H), 1.55 (m, 2H), 0.93 (t, 3H). MS (DCl/NH₃) (M+H)⁺ 256.

Using the amine prepared above and the procedures described in Example 81B provided the title compound. ¹H NMR (DMSO-d₆) δ 7.30-7.10 (envelope, 6H), 7.00-6.75 (envelope, 10H), 4.70, 4.25 (both m, total 4H), 3.95, 3.63 (both m, total 4H), 3.55-3.15, 2.95, 2.80 (envelope, m, m, total 4H), 2.27 (m, 6H), 1.50 (m, 4H), 0.80 (m, 6H). MS (FAB+) (M+H)⁺ 707. Anal cald for C₄₂H₄₆N₂O₈: C, 71.37; H, 6.56; N, 3.96. Found: C, 71.12; H, 6.49; N, 3.82.

### Example 88

### (1α,2β,3β,4α)-1,2-Di[N-propyl-N-(4-naphth-2-yloxybenzyl)aminocarbonyl]cyclobutane-3,4-dicarboxylic acid

Using 2-naphthol and the method of Example 87A, except the vacuum distillation was omitted, afforded 4-(naphth-2-yloxy)benzaldehyde. ¹H NMR (CDCl₃) δ 9.94 (s, 1H), 7.90 (m, 4H), 7.78 (m, 1H), 7.50 (m, 3H), 7.28 (dd, 1H), 7.12 (m, 2H). MS (DCl/NH₃) (M+H)⁺ 249 and (M+H+NH₃)⁺ 266.

Using the aldehyde prepared above and the procedures described in Example 81A afforded N-propyl-N-(4-(naphth-2-yloxybenzyl)amine. ¹H NMR (CDCl₃) δ 7.82 (m, 2H), 7.70 (m, 1H), 7.42 (m, 2H), 7.32 (m, 1H), 7.27 (m, 3H), 7.04 (m, 2H), 3.79 (s, 2H), 2.62 (t, 2H), 1.55 (m, 2H), 0.93 (t, 3H). MS (DCl/NH₃) (M+H)⁺ 292.

Using the amine prepared above and the procedures described in Example 81B afforded the title compound. ¹H NMR (DMSO-d₆) δ 7.90 (m, 4H), 7.78 (m, 2H), 7.44 (m, 6H), 7.25 (m, 6H), 7.00 (m, 4H), 4.70, 4.30 (both m, total 4H), 3.95, 3.63 (both m, total 4H), 3.55-3.15, 3.00, 2.80 (envelope, m, m, total 4H), 1.50 (m, 4H), 0.80 (m, 6H). MS (FAB+) (M+H)⁺ 779. Anal cald for C₄₈H₄₆N₂O₈: C, 74.02; H, 5.95; N, 3.60. Found: C, 73.70; H, 6.10; N, 3.54.

### Example 89

### (1α,2β,3α,4α)-1,2-Di[N-propyl-N-(4-(3-methyl-1-phenoxy)benzyl)aminocarbonyl]cyclobutane-3,4-dicarboxylic acid

### Example 89A

### 4-(3-Methyl-1-phenoxy)benzonitrile

Using phenol and 2-fluoro-5-nitrotoluene and the procedures described in Example 82A, except the reaction was heated to 60 °C overnight and vacuum distilled at 4.5 mm Hg, 174-5 °C, provided 4-(3-methyl-1-phenoxy)nitrobenzene. ¹H NMR (CDCl₃) δ 8.16 (dd, 1H), 8.00 (dd, 1H), 7.42 (m, 2H), 7.23 (m, 1H), 7.05 (m, 2H), 6.78 (d, 1H),2.42 (s, 3H). MS (DC/NH₃) (M+H)⁺ 247.

The nitro compound prepared above was reduced under H₂ using 10% Pd/C catalyst in MeOH to give 4-(3-methyl-1-phenoxy)aniline. ¹H NMR (CDCl₃) δ 7.27 (m, 2H), 6.97 (m, 1H), 6.83 (m, 2H), 6.80 (d, 1H), 6.60 (d, 1H), 6.52 (m, 1H), 3.53 (br s, 2H), 2.11 (s, 3H). MS (DC/NH₃) (M+H)⁺ 200 and (M+H+NH₃)⁺ 217.

The amine prepared above (2.75 g, 13.8 mmol) was added to 2 N HCl (20 mL), then cooled to 5 °C, giving a thick purple slurry. A solution of sodium nitrite (0.84 g, 14.2 mmol) in water (2 mL) was added dropwise, keeping the reaction temperature ~5°C. Addition of another small portion of sodium nitrite to the reaction resulted in a positive HONO test with a Kl-starch strip, so the pH was adjusted to 7-8 using solid Na₂CO₃. This solution was added in portions to a vigorously stirred mixture of toluene (10 mL) and a solution of sodium cyanide (1.65 g, 33.6 mmol) and copper(I) cyanide (1.45 g, 16.2 mmol) in water (15 mL), keeping the reaction temperature ~5°C. The very thick, brown reaction mixture was diluted with more toluene, stirred at 5 °C for 15 minutes, then at room temperature for 2 hours. The reaction mixture was added to EtOAc and 2 N HCI; the organic layer was dried with Na₂SO₄ and concentrated in *vacuo.* The residue obtained was chromatographed eluting with 95:5 hexane-Et₂O to give the title compound as an orange-red oil (500 mg, 17%). ¹H NMR (CDCl₃) δ 7.55 (d, 1H), 7.40 (m, 3H), 7.20 (m, 1H), 7.00 (m, 2H), 6.79 (d, 1H), 2.35 (s, 3H). MS (DCl/NH₃) (M+H+NH₃)⁺ 227.

### Example 89B

### 4-(3-Methyl-1-phenoxy)benzaldehyde

To the compound resulting from Example 89A (490 mg, 2.34 mmol) dissolved in toluene (12 mL) and cooled to 0 °C was added 5 mL of 1.5 M DIBAL in toluene. The reaction mixture was stirred at 0-10 °C for 2.5 hours, then EtOAc and 1 N HCI were added. The organic layer was washed with water and brine, dried over Na₂SO₄ and concentrated under reduced pressure. Chromatography of the residue using 95:5 hexane-EtOAc gave the title compound as an orange oil (330 mg, 66%). ¹H NMR (CDCl₃) δ 9.90 (s, 1H), 7.80 (d, 1H), 7.63 (dd, 1H), 7.40 (m, 2H), 7.19 (m, 1H), 7.03 (m, 2H), 6.85 (d, 1H), 2.40 (s, 3H). MS (DC/NH₃) (M+H)⁺ 213 and (M+H+NH₃)⁺ 230.

### Example 89C

### (1α,2β,3β,4α)-1-2-Di{N-propyl-[3-methyl-4-phenoxybenzyl]aminocarbonyl}cyclobutane-3,4-dicarboxylic acid

Using the compound resulting from Example 89B and the procedures described in Example 81A afforded N-propyl-N-(4-(3-methyl-1-phenoxy)benzyl)amine. ¹H NMR (CDCl₃) δ 7.30 (m, 2H), 7.22 (d, 1H), 7.10 (dd, 1H), 7.03 (m, 1H), 6.88 (m, 3H), 3.76 (s, 2H), 2.64 (t, 2H), 2.22 (s, 3H), 1.58 (m, 2H), 0.95 (t, 3H). MS (DCl/NH₃) (M+H)⁺ 256.

Using the amine prepared above and the procedures described in Example 81B, the title compound was prepared. ¹H NMR (DMSO-d₆) δ 7.35 (m, 4H), 7.19 (m, 2H), 7.07 (m, 4H), 6.84 (m, 6H), 4.65, 4.30 (both m, total 4H), 3.95, 3.63 (both m, total 4H), 3.55-3.10, 3.00, 2.80 (envelope, m, m, total 4H), 2.18, 2.15, 2.13, 2.10 (all s, total 3H), 1.50 (m, 4H), 0.80 (m, 6H). MS (FAB⁺) (M+H)⁺ 707. Anal cald for C₄₂H₄₆N₂O₈: C, 71.37; H, 6.56; N, 3.96. Found: C, 70:96; H, 6.39; N, 3.84.

### Example 90

### (1α,2β,3β,4α)-1,2-Di[N-propyl-N-(4-(4-methyl-1-phenoxy)benzyl)aminocarbonyl]cyclobutane-3,4-dicarboxylic acid

Using *p*-cresol and the procedures described in Example 87A, except the vacuum distillation was omitted, afforded 4-(4-methyl-1-phenoxy)benzaldehyde. ¹H NMR (CDCl₃) δ 9.92 (s, 1H), 7.82 (m, 2H), 7.21 (m, 2H), 7.04 (m, 2H), 6.98 (m, 2H), 2.38 (s, 3H). MS (DCI/NH₃) (M+H)⁺ 213 and (M+H+NH₃)⁺ 230.

Using the aldehyde prepared above and the procedures described in Example 81A afforded N-propyl-N-(4-(4-methyl-1-phenoxy)benzyl)amine. ¹H NMR (CDCl₃) δ 7.28 (m, 2H), 7.13 (m, 2H), 6.95 (m, 2H), 6.90 (m, 2H), 3.77 (s, 2H), 2.62 (t, 2H), 2.32 (s, 3H), 1.55 (m, 2H), 0.93 (t, 3H). MS (DCI/NH₃) (M+H)⁺ 256.

Using the amine prepared above and the procedures described in Example 81B afforded the title compound. ¹H NMR (DMSO-d₆) δ 7.20 (m, 8H), 6.96-6.82 (envelope, 8H), 4.70, 4.25 (both m, total 4H), 3.90, 3.60 (both m, total 4H), 3.55-3.15, 2.95, 2.78 (envelope, m, m, total 4H), 2.27 (m, 6H), 1.50 (m, 4H), 0.80 (m, 6H). MS (FAB⁺) (M+H)⁺ 707. Anal cald for C₄₂H₄₆N₂O₈ · 0.25 H₂O: C, 70.92; H, 6.59; N, 3.94. Found: C, 70.80; H, 6.69; N, 3.83.

### Example 91

### (1α,2β,3β,4α)-1,2-Di[N-propyl-N-(4-naphth-1-yloxybenzyl)aminocarbonyl]cyclobutane-3,4-dicarboxylic acid:

Using 1-naphthol and the procedures described in Example 87A, except the vacuum distillation was omitted, afforded 4-(naphth-1-yloxy)benzaldehyde. ¹H NMR (CDCl₃) δ 9.93 (s, 1H), 8.00 (dd, 1H), 7.93 (d, 1H), 7.85 (m, 2H), 7.76 (d, 1H), 7.55 (m, 1H), 7.49 (m, 2H), 7.17 (dd, 1H), 7.08 (m, 2H). MS (DC/NH₃) (M+H)⁺ 249 and (M+H+NH₃)⁺ 266.

Using the aldehyde prepared above and the procedures described in Example 81A afforded N-propyl-N-(4-(naphth-1-yloxy)benzyl)amine. ¹H NMR (CDCl₃) δ 8.21 (m, 1H), 7.87 (m, 1H), 7.60 (d, 1H) 7.50 (m, 2H), 7.37 (dd, 1H), 7.30 (m, 2H), 7.02 (m, 2H), 6.93 (dd, 1H), 3.79 (s, 2H), 2.62 (t, 2H), 1.55 (m, 2H), 0.93 (t, 3H). MS (DCl/NH₃) (M+H)⁺ 292.

Using the amine prepared above and the procedures described in Example 81B, the title compound was prepared. ¹H NMR (DMSO-d₆) δ 8.05 (m, 2H), 7.97 (m, 2H), 7.73 (m, 2H), 7.60-7.40 (envelope, 6H), 7.25, 7.18 (both m, total 4H), 6.95 (m, 6H), 4.70, 4.27 (both m, total 4H), 3.90, 3.63 (both m, total 4H), 3.55-3.15, 2.95, 2.78 (envelope, m, m, total 4H), 1.50 (m, 4H), 0.80 (m, 6H). MS (FAB⁺) (M+H)⁺ 779. Anal cal'd for C₄₈H₄₆N₂O₈ · 0.25 H₂O, 73.59 C, 5.98 H, 3.58 N. Found: C, 73.46; H, 5.94; N, 3.48.

### Example 92

### (1α,2β,3β,4α)-1,2-Di[N-propyl-N-(4-phenoxybenzyl)aminocarbonyl]cyclobutane-3-[N-(5-tetrazolyl)]carboxamide-4-carboxylic acid

### Example 92A

### Methyl(1α,2β,3β,4α)-1,2-Di[N-propyl-N-(4-phenoxybenzyl)aminocarbonyl]cyclobutane-3-[N-(5-tetrazolyl)]carboxamide-4-carboxylate

To the compound resulting from Example 95 (118 mg, 0.17 mmol) dissolved in THF (0.8 mL) was added carbonyl diimidazole (28 mg, 0.17 mmol). The reaction was heated under reflux for 1.5 hours, and then anhydrous 5-aminotetrazole (22 mg, 0.26 mmol) was added, and the reflux was continued for another 2.5 hours. After cooling to room temperature, ice and 3 N HCI were added, followed by extraction with EtOAc. The combined organic extracts were washed with brine, dried over Na₂SO₄, and concentrated under reduced pressure. The residue obtained was purified by chromatography eluting with 98.75:1.25:0.5 CHCl₃-MeOH-CH₃CO₂H to afford 50 mg (38%) of the title compound as a glass. MS (FAB+) m/e 760 (M+H)⁺.

### Example 92B

### (1α,2β,3β,4a)-1,2-Di[N-propyl-N-(4-phenoxybenzyl)aminocarbonyl]cyclobutane-3-[N-(5-tetrazolyl)]carboxamide-4-carboxylic acid

To the compound resulting from Example 92A (47 mg, 0.062 mmol) dissolved in MeOH (1.2 mL) and cooled to 0 °C was added a solution of lithium hydroxide monohydrate (11 mg, 0.26 mmol) in water (0.3 mL). The reaction was stirred at 0.10 °C under N₂ for 1.5 hours, then warmed at room temperature for 3 hours. The reaction mixture was partitioned between EtOAc and 1 M H₃PO₄, and the organic phase was washed with brine, dried over Na₂SO₄, and concentrated under reduced pressure. The residue obtained was purified by chromatography eluting with 97.5:2.5:0.5 CHCl₃-MeOH-CH₃CO₂H to afford a glass that was treated as in Example 81B to give 20 mg (44%) of the title compound as a white solid. ¹H NMR (DMSO-d₆) δ 7.39, 7.28, 7.13, 7.00 (all m, total 18H), 6.63 (m, 1H), 4.75, 4.33 (both m, total 4H), 4.00, 3.75 (both m, total 4H), 3.60-2.60 (envelope, total 4H), 1.50, 1.15 (both m, total 4H), 0.83, 0.55 (both m, total 6H). MS(FAB+) (M+H)⁺ 746. Anal cald for C₄₁H₄₃N₇O₇ · 1.0 H₂O: C, 64.47; H, 5.94; N, 12.84. Found: C, 64.59; H, 6.00; N, 12.60.

### Example 93

### (1α,2β,3β,4α)-1,2-Di[N-propyl-N-(4-phenoxybenzyl)aminocarbonyl]cyclobutane-3-(N-(5-tetrazolyl)aminocarbonylamino)-4-carboxylic acid

### Example 93A

### Methyl (1α,2β,3β,4α)-1,2-Di[N-propyl-N-(4-phenoxybenzyl)aminocarbonyl]cyclobutane-3-(N-(5-tetrazolyl)aminocarbonylamino)-4-carboxylate

To the compound resulting from Example 95 (212 mg, 0.30 mmol) dissolved in toluene (3 mL) was added triethylamine (0.42 mL, 31 mg, 0.30 mmol) and diphenylphosphorylazide (0.72 mL, 92 mg, 0.33 mmol). After stirring at 65 °C under N₂ for 2.25 hours, anhydrous 5-aminotetrazole (255 mg, 3.00 mmol) was added and stirring was continued at 90 °C under N₂ overnight. The reaction was diluted with EtOAc, washed with 1 M H₃PO₄ and brine, then dried over Na₂SO₄. After filtration and evaporation, the residue was purified by chromatography using 98.75:1.25:0.5 CHCl₃-MeOH-CH₃CO₂H to afford 50 mg (21%) of the title compound as a glass. MS (FAB+) (M+H)⁺ 775.

### Example 93B

### (1α,2β,3β,4α)-1,2-Di[N-propyl-N-(4-phenoxybenzyl)aminocarbonyl]cyclobutane-3-(N-(5-tetrazolyl)aminocarbonylamino)-4-carboxylic acid

Using the compound resulting from Example 93A and the procedures described in Example 82B, except the reaction was run at room temperature overnight and the chromatography used 97:3:1 CHCl₃-MeOH-CH₃CO₂H, afforded the title compound. ¹H NMR (DMSO-d₆) was consistent with expected structure. MS (FAB+) (M+H)⁺ 761. Anal cald for C₄₁H₄₄N₈O₇ · 1.25 H₂O: C, 62.87; H, 5.98; N, 14.30. Found: C, 62.61; H, 5.80; N, 14.06.

### Example 94

### (1α,2β,3β,4α)-1,2-Di[N-propyl-N-(4-phenoxybenzyl)aminocarbonyl]cyclobutane-3,4-dicarboxylic acid Dimethyl Ester

The resultant acid from Example 17 (268 mg, 0.395 mmol) in dichloromethane (5 mL) was treated with an excess of an ether solution of diazomethane. Evaporation of the solvent afforded 283 mg (100%) of the title compound as an oil. ¹H NMR (CDCl₃, 300 MHz) δ 7.40-6.87 (m, 18H), 4.83, 4.81, 4.64, 4.57, 4.34, 4.22 (6 d, total 4H), 4.20-4.08, 3.97-3.81 (2 m, total 4H), 3.72, 3.69, 3.53, 3.52 (4 s, total 6H), 3.63-3.43, 3.35-3.17, 3.17-3.01, 3.01-2.86 (4 m, total 4H), 1.68-1.48 (m, 4H), 0.97-0.78 (m, 6H).

### Example 95

### (1α,2β,3β,4α)-1,2-Di[N-propyl-N-(4-phenoxybenzyl)aminocarbonyl]-3-(methoxycarbonyl)cyclobutane-4-carboxylic Acid

The resultant acid from Example 17 (10.97 g, 16.16 mmol) in dichloromethane was treated with an excess of an ether solution of diazomethane. The solvent was evaporated, and the residue was dissolved in a mixture of tetrahydrofuran (135 mL) and methanol (20 mL) and cooled to -10 °C. A cold solution of LiOH monohydrate (680 mg, 16.2 mmol) in water (45 mL) was added, and the reaction was stirred at -10 to 0 °C for 3 hours and then was placed in a -20 °C freezer overnight. The reaction was quenched with 2 M HCI and concentrated. The residue was dissolved in ethyl acetate, washed with brine, dried over Na₂SO₄ and evaporated. Chromatography of the residue on silica gel with 2-4% methanol in chloroform afforded 5.56 g (50%) of the title compound as a foam. ¹H NMR (CDCl₃, 300 MHz) δ 7.40-6.83 (m, 18H), 4.88-3.80 (envelope, 8H), 3.71, 3.68, 3.52, 3.51 (4 s, total 6H), 1.65-1.38 (m, 4H), 0.94-0.75 (m, 6H). Anal calcd for C₄₁H₄₄N₂O₈ · 0.5 H₂O: C, 70.17; H, 6.46; N, 3.99. Found: C, 69.82, H, 6.28; N, 4.01.

### Example 96

### (1α,2β,3α,4α)-1,2-Di[N-propyl-N-(4-phenoxybenzyl)aminocarbonyl]cyclobutane-3,4-dicarboxylic Acid

### Example 96A

### (1α,2β,3α,4α)-1,2-Di[N-propyl-N-(4-phenoxybenzyl)aminocarbonyl]-3-(methoxycarbonyl)cyclobutane-4-carboxylic Acid

To diisopropylamine (36 µL, 0.26 mmol) in tetrahydrofuran (1 mL) at -78°C was added n-BuLi (110 µL, 0.237 mmol, 2.15 M in hexane). After 10 minutes the resultant compound from Example 95 (70.1 mg, 0.101 mmol) in tetrahydrofuran (1 mL) was added. After 20 minutes at -78 °C, acetic acid (60 µL) in tetrahydrofuran (1 mL) was added, the solvent was evaporated, and the residue was partitioned between chloroform and a 2:1 mixture of brine and 2 M HCI. The mixture was extracted with chloroform which was dried over Na₂SO₄ and evaporated to afford 71.2 mg of a mixture of the title compound and the starting monoester. ¹H NMR δ (CDCl₃, 300 MHz) 3.655, 3.650, 3.595, 3.590 (title compound) and 3.71, 3.68, 3.52, 3.51 (starting monoacid) (8 s, total 6H).

### Example 96B

### (1α,2β,3α,4α)-1,2-Di[N-propyl-N-(4-phenoxybenzyl)aminocarbonyl]cyclobutane-3,4-dicarboxylic Acid

To the resultant compound mixture from Example 96A (64.3 mg, 0.093 mmol) in tetrahydrofuran (1.5 mL) at 0 °C was added LiOH monohydrate (14 mg, 0.33 mmol) in water (0.5 mL). After 2 hours at 0 °C and 1 hour at ambient temperature the reaction was quenched with a 1:1 mixture of brine and 2 M HCI. The mixture was extracted with chloroform which was dried over Na₂SO₄ and evaporated to afford 59.4 mg of a mixture of the title compound and the resultant compound from Example 17. Separation by reverse-phase HPLC (60% CH₃CN/40% H₂O/0.1% trifluoroacetic acid mobile eluent) afforded 23.9 mg of the title compound. ¹H NMR δ (D₆-DMSO, 300 MHz) 7.40-7.30, 7.20-7.06, 7.02-6.91, 6.91-6.82 (4 m, total 18H), 5.15-4.98, 4.78-4.53, 4.45-4.30, 4.27-4.08 (4 m, total 4H).

### Example 97

### (1α,2β,3α,4β)-1,2-Di[N-propyl-N-(4-phenoxybenzyl)aminocarbonyl]cyclobutane-3,4-dicarboxylic Acid

Sodium methoxide in methanol (2 mL of 27.4 mg Na in 10 mL methanol) was added to the resultant compound from Example 94 (70.3 mg, 0.099 mmol), and the mixture was heated at reflux for 18 hours. The mixture was diluted with ethyl acetate, washed with 2 M HCI and brine, and then was dried over Na₂SO₄ and evaporated to afford 60.9 mg (87%) of an oil. This material was dissolved in methanol (2 mL) and treated with 1 M NaOH (0.5 mL). After 5 hours at ambient temperature, the solvent was evaporated, and the residue was partitioned between chloroform and a 2:1 mixture of brine and 2 M HCI. The mixture was extracted with chloroform which was dried over Na₂SO₄ and evaporated. Recrystallization of the residue from a mixture of ethyl acetate and hexane afforded 42.3 mg (63%) of the title compound as a white solid. ¹H NMR (D₆-DMSO, 150 °C, 300 MHz) δ 7.40-6.90 (envelope, 18H), 4 55 (d, 2H), 4.44 (d, 2H), 3.76 (d, 2H), 3.36 (d, 2H), 3.21 (t, 4H), 1.53-1.40 (m, 4H), 0.80 (t, 6H). Anal calcd for C₄₀H₄₂N₂O₈: C, 70.78; H, 6.24; N, 4.13. Found: C, 70.66; H, 6.20; N, 4.07.

### Example 98

### (1α,2β,3β,4α)-1,2-Di[N-propyl-N-(4-phenoxybenzyl)aminocarbonyl]-3-[(hydroxyamino)carbonyl]cyclobutane-4-carboxylic acid

### Example 98A

### (1α,2β,3β,4α)-3-(Benzyloxyamino)carbonyl-1,2-di[N-propyl-N-(4-phenoxybenzyl)aminocarbonyl]-4-(methoxycarbonyl)cyclobutane

To the resultant compound from Example 95 (72.5 mg, 0.105 mmol) in dichloromethane (2 mL) at -10 °C was added 4-methylmorpholine (28 µL, 0.26 mmol) followed by isobutylchloroformate (15 µL, 0.11 mmol). After 3 minutes, (O-benzyl)hydroxylamine hydrochloride (20 mg, 0.13 mmol) was added. The reaction was stirred at -10 to 0°C for 15 minutes and then at ambient temperature for 2 hours. The mixture was diluted with ethyl acetate and washed sequentially with 2 M HCI, saturated NaHCO₃ solution and brine, dried over Na₂SO₄ and evaporated. Chromatography of the residue on silica gel with 50% ethyl acetate in hexane afforded 67.1 mg (80%) of the title compound. ¹H NMR (CDCl₃, 300 MHz) δ 7.45-6.85 (m, 23H), 5.43-4.70 (m, 2H), 3.67, 3.62, 3.53, 3.51 (4 s, total 3H).

### Example 98B

### (1α,2α,3β,4α)-1,2-Di[N-propyl-N-(4-phenoxybenzyl)aminocarbonyl]-3-(hydroxyamino)carbonyl-4-(methoxycarbonyl)cyclobutane

The resultant compound from Example 98A (65.0 mg, 0.081 mmol) and 10% Pd/C (51 mg) in ethyl acetate (2 mL) were stirred under a hydrogen atmosphere for 4 hours. The mixture was filtered and evaporated to afford 51.7 mg (90%) of the title compound as a foam. ¹H NMR (CDCl₃, 300 MHz) δ 7.40-6.77 (m, 19H), 4.85-4.22 (m, 4H), 4.22-3.70 (m, 4H), 3.69, 3.66, 3.55, 3.53 (4 s, total 3H), 3.60-2.90 (envelope, 4H), 1.65-1.30 (m, 4H), 0.93-0.75 (m, 6H).

### Example 98C

### (1α,2β,3β,4α)-1,2-Di[N-propyl-N-(4-phenoxybenzyl)aminocarbonyl]-3-[(hydroxyamino)carbonyl]cyclobutane-4-carboxylic Acid

To the resultant compound mixture from Example 98B (48.9 mg, 0.069 mmol) in tetrahydrofuran (1.5 mL) at 0 °C was added LiOH monohydrate (11.0 mg, 0.26 mmol) in water (0.5 mL). After 1 hour at 0 °C the reaction was quenched with 2 M HCl and concentrated. The residue was dissolved in ethyl acetate, washed with a 1:1 mixture of brine and 2 M HCI and then brine, dried over Na₂SO₄ and evaporated. The residue was dissolved in dichloromethane which was made turbid with hexane and evaporated to afford 45.8 mg (96%) of the title product as a foam. ¹H NMR (CDCl₃, 300 MHz) δ 7.45-6.75 (m, 19H), 4.85-2.65 (envelope, 12H), 1.60-1.25 (m, 4H), 0.90-0.65 (m, 6H). Anal calcd for C₄₀H₄₃N₃O₈: C, 69.25; H, 6.25; N, 6.06. Found: C, 69.25; H, 6.47; N, 5.79.

### Example 99

### (1α,2β,3β,4α)-3-(Amino)carbonyl-1,2-Di[N-propyl-N-(4-phenoxybenzyl)aminocarbonyl]cyclobutane-4-carboxylic acid

### Example 99A

### (1α,2β,3β,4α)-3-(Amino)carbonyl-1,2-di[N-propyl-N-(4-phenoxybenzyl)aminncarbonyl]-4-(methoxycarbonyl)cyclobutane

Using the procedure of Example 98A but replacing (O-benzyl)hydroxylamine hydrochloride with concentrated aqueous NH₄OH gave, after chromatography on silica gel with 80% ethyl acetate in hexane, the title compound in 89% yield as a foam. ¹H NMR (CDCl₃, 300 MHz) δ 7.25-6.72 (m, 18H), 6.32-6.05, 5.29-5.08 (2 br, total 2H), 4.80, 4.75, 4.65, 4.58, 4.45, 4.38, 4.35-4.25 (6 d, 1 m, total 4H), 4.16-3.92 (m, 4H), 3.75 3.73, 3.62 (3 s, total 3H), 3.75-2.90 (envelope, total 4H), 1.70-1.35 (m, 4H), 0.95-0.75 (m, 6H).

### Example 99B

### (1α,2β,3β,4α)-3-(Amino)carbonyl-1,2-di[N-propyl-N-(4-phenoxybenzyl)aminocarbonyl]cyclobutane-4-carboxylic Acid

Using the procedure of Example 98C with the resultant compound from Example 99A afforded the title compound in 100% yield as a foam. ¹H NMR (CDCl₃, 300 MHz) 6 7.40-6.85 (m, 18H), 6.50-6.20, 5.60-5.40 (2 br, total 2H), 4.82-4.15 (envelope, total 4H), 4.15-3.90 (m, 4H), 3.75-2.90 (envelope, total 4H), 1.70-1.35 (m, 4H), 0.95-0.72 (m, 6H). Anal calcd for C₄₀H₄₃N₃O₇ · 0.4 hexane: C, 71.52; H, 6.89; N, 5.89. Found: C, 71.26; H, 6.91; N, 5.57.

### Example 100

### (1α,2β,3β,4α)-1,2-Di[N-propyl-N-(4-phenoxybenzyl)aminocarbonyl]-3-(trifluoromethanesulfonylamino)cyclobutane-4-carboxylic acid

### Example 100A

### (1α,2β,3β,4α)-3-(tert-Butyloxycarbonylamino)-1,2-di[N-propyl-N-(4-phenoxybenzyl)aminocarbonyl]-4-(methoxycarbonyl)cyclobutane

The resultant compound from Example 95 (147.5 mg, 0.213 mmol) in toluene (2 mL) was treated with triethylamine (30 µL, 0.21 mmol) and diphenylphosphoryl azide (50 µL, 0.23 mmol) and then was heated to 65-70 °C for 2 hours. *tert*-Butanol (200 µL, 2.1 mmol) was added, and the reaction was stirred at 90 °C for 90 hours. The mixture was diluted with ethyl acetate, washed sequentially with 2 M HCI, saturated NaHCO₃ solution and brine, dried over Na₂SO₄ and evaporated. Chromatography of the residue on silica gel with 25-30% ethyl acetate in hexane afforded 64.0 mg (39%) of the title compound as a foam. ¹H NMR (CDCl₃, 300 MHz) δ 7.47-6.87 (m, 18H), 5.40-2.90 (envelope, total 13H), 3.71, 3.69, 3.56 (3 s, total 3H), 1.63-1.30 (m, 4H), 1.54, 1.53, 1.45 (3 s, total 9H), 0.95-0.77 (m, 6H).

### Example 100B

### (1α,2β,3β,4α)-1,2-Di[N-propyl-N-(4-phenoxybenzyl)aminocarbonyl]-4-methoxycarbonyl-3-[(trifluoromethanesulfonyl)amino]cyclobutane

The resultant compound from Example 100A (62.0 mg, 0.081 mmol) was stirred for 1 hour in 4 M HCl in dioxane and evaporated. The residue was dissolved in dichloromethane, treated with triethylamine (25 µL) and applied directly to the top of a silica gel column which was eluted with ethyl acetate to afford 44.5 mg (83%) of the primary amine as a foam. To this compound (44.5 mg, 0.067 mmol) in dichloromethane (2 mL) at -78 °C was added triethylamine (10 µL, 0.071 mmol) and trifluoromethanesulfonic anhydride (12 µL, 0.071 mmol). After 1 hour, the reaction was warmed to ambient temperature and applied directly to the top of a silica gel column which was eluted with 25% ethyl acetate in hexane to afford 39.0 mg (73%) of the title compound as a foam. ¹H NMR (CDCl₃, 300 MHz) δ 7.45-6.85 (m, 18H), 5.00-2.87 (envelope, total 12H), 3.75, 3.72, 3.56, 3.54 (4 s, total 3H), 1.70-1.37 (m, 4H), 0.95-0.75 (m, 6H).

### Example 100C

### (1α,2β,3β,4α)-1,2-Di[N-propyl-N-(4-phenoxybenzyl)aminocarbonyl]-3-[(trifluoromethanesulfonyl)amino]cyclobutane-4-carboxylic Acid

Using the hydrolysis procedure of Example 97 with the resultant compound from Example 100B afforded the title compound in 100% yield as a glass. ¹H NMR (CDCl₃, 300 MHz) δ 7.40-6.80 (m, 18H), 5.02-2.90 (envelope, total 12H), 1.70-1.35 (m, 4H), 0.95-0.75 (m, 6H). Anal calcd for C₄₀H₄₂N₃O₈F₃S · 0.25 H₂O: C, 61.10; H, 5.45; N, 5.34. Found: C, 60.99; H, 5.13; N, 5.13.

### Example 101

### (1α,2β,3β,4α)-4-(Carboxy)methyl)-1,2-di[N-propyl-N-(4-phenoxybenzyl)aminocarbonyl]cyclobutane-3-carboxylic acid

### Example 101A

### (1α,2β,3β,4α)-1,2-Di[N-propyl-N-(4-phenoxybenzyl)aminocarbonyl]-3-diazoacetyl-4-(methoxycarbonyl)cyclobutane

To the resultant compound from Example 95 (259 mg, 0.374 mmol) in dichloromethane (3 mL) at -10 °C was added 4-methylmorpholine (50 µL, 0.46 mmol) followed by isobutylchloroformate (54 µL, 0.41 mmol). After 3 minutes, an excess of an ether solution of diazomethane was added. The reaction was warmed from -10 to 10 °C over 3 hours. The mixture was washed with saturated NaHCO₃ solution and brine, dried over Na₂SO₄ and evaporated. Chromatography of the residue on silica gel with 40% ethyl acetate in hexane afforded 213 mg (80%) of the title compound as a pale yellow foam. ¹H NMR (CDCl₃, 300 MHz) δ 7.39-6.87 (m, 18H), 5.47, 5.43, 5.33, 5.29 (4 s, total 1H), 4.98-2.90 (envelope, total 12H), 3.72, 3.71, 3.562, 3.560 (4 s, total 3H), 1.67-1.40 (m, 4H), 0.95-0.76 (m, 6H).

### Example 101B

### (1α,2β,3β,4α)-1,2-Di[N-propyl-N-(4-phenoxybenzyl)aminocarbonyl]-3-methoxycarbonyl-4-(methoxycarbonylmethyl)cyclobutane

The resultant compound from Example 101A (210 mg, 0.293 mmol) in methanol (2 mL) was treated with a solution of silver benzoate in triethylamine (50 µL of 50 mg AgOBn in 1.0 mL triethylamine). After 1 hour, the solvent was evaporated, and the residue was chromatographed on silica gel with 25% ethyl acetate in hexane to afford 174 mg (82%) of the title compound as an oil. ¹H NMR (CDCl₃, 300 MHz) δ 7.40-6.87 (m, 18H), 4.98-4.05 (envelope, total 8H), 3.71, 3.68, 3.65, 3.64, 3.542, 3.540, 3.52, 3.51 (8 s, total 6H), 3.60-2.75 (envelope, total 4H), 2.62-2.38 (m, 2H), 1.69-1.39 (m, 4H), 0.97-0.78 (m, 6H).

### Example 101C

### (1α,2β,3β,4α)-4-(Carboxy)methyl)1,2-di[N-propyl-N-(4-phenoxybenzyl)aminocarbonyl]cyclobutane-3-carboxylic Acid

Using the procedure of Example 98C with the resultant compound from Example 101B afforded the title compound in 100% yield as a foam. ¹H NMR (CDCl₃, 300 MHz) δ 7.47-6.82 (m, 18H), 4.88-2.78 (envelope, total 12H), 2.68-2.25 (m, 2H), 1.65-1.35 (m, 4H), 0.95-0.72 (m, 6H). Anal calcd for C₄₁H₄₄N₂O₈: C, 71.08; H, 6.40; N, 4.04. Found: C, 71.31; H, 6.55; N, 3.73.

### Example 102

### (1α,2β,3β,4α)-3,4-Bis(diazoacetyl)-1,2-di[N-propyl-N-(4-phenoxybenzyl)aminocarbonyl]cyclobutane

To the resultant compound from Example 17 (202 mg, 0.298 mmol) in dichloromethane (2 mL) at -10 °C was added 4-methylmorpholine (75 µL, 0.68 mmol) followed by isobutylchloroformate (85 µL, 0.65 mmol). After 4 minutes, an excess of an ether solution of diazomethane was added. The reaction was warmed from -10 to 10 °C over 3 hours. The mixture was washed with saturated NaHCO₃ solution and brine, dried over Na₂SO₄ and evaporated. Chromatography of the residue on silica gel with 50% ethyl acetate in hexane afforded 130 mg (60%) of the title compound as a yellow foam. ¹H NMR (CDCl₃, 300 MHz) δ 7.38-6.88 (m, 18H), 5.28, 5.23, 5.15, 5.12 (4 s, total 2H), 4.98-3.84 (envelope, total 8H), 3.65-2.97 (envelope, total 4H), 1.68-1.45 (m, 4H), 0.97-0.79 (m, 6H).

### Example 103

### (1α,2β,3β,4α)-1,2-Di[-N-propyl-N-(4-phenoxybenzyl)aminocarbonyl]cyclobutane-3,4-diacetic acid

### Example 103A

### (1α,2β,3β,4α)-1,2-di[N-propyl-N-(4-phenoxybenzyl)aminocarbonyl]-cyclobutane-3,4-diacetic acid dimethylester

The resultant compound from Example 102 (120 mg, 0.165 mmol) in methanol (2 mL) was treated with a solution of silver benzoate in triethylamine (50 µL of 50 mg AgOBn in 1.0 mL triethylamine). After 2 hours, the solvent was evaporated, and the residue was chromatographed on silica gel with 25% ethyl acetate in hexane to afford 90.6 mg (75%) of the title compound as an oil. ¹H NMR (CDCl₃, 300 MHz) δ 7.40-6.87 (m, 18H), 4.97-4.87, 4.73-4.62, 4.25-4.05 (3 m, total 8H), 3.66, 3.64, 3.55 (3 s, total 6H), 3.73-3.54, 3.32-3.15, 3.08-2.95, 2.85-2.35 (4 m, total 8H), 1.72-1.42 (m, 4H), 0.98-0.78 (m, 6H).

### Example 103B

### (1α,2β,3β,4α)-1,2-Di[-N-propyl-N-(4-phenoxybenzyl)aminocarbonyl]cyclobutane-3-4-diacetic acid

To the resultant compound from Example 103A (87.8 mg, 0.119 mmol) in methanol (1.5 mL) and tetrahydrofuran was added 1 M NaOH (0.5 mL). After 15 hours the reaction was quenched with 2 M HCl and concentrated. The residue was dissolved in ethyl acetate and washed with a 1:1 mixture of brine and 2 M HCI and then brine, dried over Na₂SO₄ and evaporated to afford 88 mg (100%) of the final product as a foam. ¹H NMR (CDCl₃, 300 MHz) δ 7.37-6.90 (m, 18H), 4.97-4.89, 4.62-4.54, 4.35-4.23, 4.20-4.10, 3.68-3.58, 3.21-3.04, 2.96-2.51, 2.32-2.22, 2.19-2.08 (multiplets, total 16H), 1.76.1.46 (m, 4H), 0.98-0.82 (m, 6H). Anal calcd for C₄₂H₄₆N₂O₈: C, 71.37; H, 6.56; N, 3.96. Found: C, 71.09; H, 6.83; N, 3.70.

### Examples 104A and 104B

### (1α,2β,3β,4α)-1,2-Di[N-propyl-N-(4-phenoxybenzyl)aminocarbonyl]cyclobutane-3,4-dicarboxylic acid Mono-Norleucine Amide

The resultant compound from Example 95 (100 mg, 0.144 mmol), *d,l*-Norleucine-OMe hydrochloride (35.0 mg, 0.193 mmol), 1-hydroxybenzotriazole (53.0 mg, 0.392 mmol) and 4-methylmorpholine (35 µL, 0.32 mmol) in dimethylformamide (1.5 mL) at -10 °C were treated with 1-(3-dimethylaminopropyl)-3-ethylcarbodiimide hydrochloride (40 mg, 0.21 mmol). After 2 hours the cooling bath was removed, and the reaction was stirred at ambient temperature for 14 hours. The mixture was poured into saturated NaHCO₃ solution and extracted into ethyl acetate which was washed with water and brine, dried over Na₂SO₄ and evaporated. Chromatography of the residue on silica gel with 30-35% ethyl acetate in hexane afforded two mono-esters, diastereomeric at the leucine α-carbon, in 89% total yield. Each was hydrolyzed according to the procedure of Example 98C to afford the title compounds. Anal calcd for 104A C₄₆H₅₃N₃O₉: C, 69.76; H, 6.75; N, 5.31. Found: C, 69.73; H, 6.69; N, 5.10. Anal calcd for 104B C₄₆H₅₃N₃O₉ · 0.3 H₂O: C, 69.29; H, 6.76; N, 5.27. Found: C, 68.90; H, 6.85; N, 5.04.

### Example 105-113

The following compounds were prepared according to the method of Example 104.

### Example 105A

### (1α,2β,3β,4α)-1,2-Di[N-propyl-N-(4-phenoxybenzyl)aminocarbonyl]cyclobutane-3-phenoxycarbonyl-4-carboxylic acid

Anal calcd for C₄₉H₅₁N₃O₉: C, 71.26; H, 6.22; N, 5.09. Found: C, 70.99; H, 6.32; N, 4.86.

### Example 105B

### (1α,2β,3β,4α)-1,2-Di[N-propyl-N-(4-phenoxybenzyl)aminocarbonyl]cyclobutane-3-phenoxycarbonyl-4-carboxylic acid

Anal calcd for C₄₉H₅₁N₃O₉ · 0.5 H₂O: C, 70.49; H, 6.28; N, 5.03. Found: C, 70.33; H, 6.33; N, 4.85.

### Example 106

### (1α,2β,3β,4α)-1,2-Di[N-propyl-N-(4-phenoxybenzyl)aminocarbonyl]cyclobutane-3-4-dicarboxylic acid mono-Glycine amide

Anal calcd for C₄₂H₄₅N₃O₉ · 0.25 H₂O: C, 68.14; H, 6.19; N, 5.66. Found: C, 68.28; H, 6.16; N, 5.28.

### Example 107

### (1α,2β,3β,4α)-1,2-Di[N-propyl-N-(4-phenoxybenzyl)aminocarbonyl]cyclobutane-3,4-dicarboxylic acid mono-d,l-Proline amide

### Example 108

### (1α,2β,3β,4α)-1,2-Di[N-propyl-N-(4-phenoxybenzyl)aminocarbonyl]cyclobutane-3,4-dicarboxylic acid mono-Sarcosine amide

Anal calcd for C₄₃H₄₇N₃O₉ · 0.25 H₂O: C, 68.47; H, 6.35; N, 5.57. Found: C, 68.27; H, 6.19; N, 5.39.

### Example 109

### (1α,2β,3β,4α)-1,2-Di[N-propyl-N-(4-phenoxybenzyl)aminocarbonyl]cyclobutane-3,4-dicarboxylic acid mono-d,l-Aspartic acid amide

Anal calcd for C₄₄H₄₇N₃O₁₁ · 0.5 H₂O: C, 65.82; H, 6.03; N, 5.23. Found: C, 65.66; H, 6.00; N, 4.94.

### Example 110A

### (1α,2β,3β,4α)-1,2-Di[N-propyl-N-(4-phenoxybenzyl)aminocarbonyl]cyclobutane-3.4-dicarboxylic acid mono-Serine amide

Anal calcd for C₄₃H₄₇N₃O₁₀ · 0.75 H₂O: C, 66.27; H, 6.27; N, 5.39. Found: C, 66.00; H, 6.20; N, 5.13.

### Example 110B

### (1α,2β,3β,4α)-1,2-Di[N-propyl-N-(4-phenoxybenzyl)aminocarbonyl]cyclobutane-3,4-dicarboxylic acid mono-Serine amide

Anal calcd for C₄₃H₄₇N₃O₁₀ · 0.75 H₂O: C, 66.27; H, 6.27; N, 5.39. Found: C, 66.25; H, 6.36; N, 5.16.

### Example 111

### (1α,2β,3β,4α)-1,2-Di[N-propyl-N-(4-phenoxybenzyl)aminocarbonyl]cyclobutane-3,4-dicarboxylic acid mono-β-Alanine amide

Anal calcd for C₄₃H₄₇N₃O₉: C, 68.88; H, 6.32; N, 5.60. Found: C, 68.49; H, 6.66; N, 5.47.

### Example 112

### (1α,2β,3β,4α)-1,2-Di[N-propyl-N-(4-phenoxybenzyl)aminocarbonyl]cyclobutane-3,4-dicarboxylic acid mono-d-Norleucine amide

The title compound was obtained as a single enentiomer. Anal calcd for C₅₂H₆₄N₄O₁₀ · H₂O: C, 67.65; H, 7.20; N, 6.07. Found: C, 67.60; H, 7.00; N, 5.93.

### Example 113

### (1α,2β,3β,4α)-1,2-Di[N-propyl-N-(4-phenoxybenzyl)aminocarbonyl]cyclobutane-3,4-dicarboxylic acid mono-l-Norleucine amide

The title compound was obtained as a single enentiomer. Anal calcd for C₅₂H₆₄N₄O₁₀:

### Example 114

### (1α,2β,3β,4α)-1,2-Di[N-(4-pyridyl)methyl-N-(phenoxybenzyl)aminocarbonyl]cyclobutane-3,4-dicarboxylic acid

To a slurry of 1,2,3,4-cyclobutanetetracarboxylic dianhydride (0.3 g, 1.5 mmol) in CH₃CN (5 mL) was added N-pyridylmethyl-N-phenoxybenzylamine (0.93 g, 3.21 mmol), prepared by the procedures described in Example 11, in CH₃CN (10 ml). The slurry was stirred for 5 minutes at 20 °C resulting in a homogeneous solution. The solution was stirred 20 hours at 20 °C, then concentrated *in vacuo* to a white foam. The foam was dissolved in 100 mL of ethyl acetate and washed successively with 50 mL 1 N H₃PO₄ and 10% NaCI, then dried over anhydrous sodium sulfate, filtered and solvent removed *in vacuo* to afford 1.0 g of a white foamy solid. The crude product containing both isomers was purified by silica gel chromatography eluting with 94:5:1 CHCl₃-MeOH-HOAc. The slower moving product was isolated and characterized as the title compound. ¹H NMR (DMSO-d₆, 500 MHz) δ 8.51-8.61 (m, 4H), 7.38-6.87 (m, 24H), 4.65-4.76 (m, 2H), 4.52-4.41(m, 2H), 4.39-4.13 (m, 8H), 3.95-4.05 (m, 2H).

### Example 115

### (1α,2α,3β,4β)-1,2-Di[N-propyl-N-(phenoxybenzyl)aminocarbonyl]cyclobutane-3,4-dicarboxylic acid

### Example 115A

### (1α,2α,3β,4β)-1,2-Di-(2-furyl)cyclobutane-3,4-dicarboxylic acid and (1α,2α,3α,4α)-1,2-Di-(2-furyl)cyclobutane-3,4-dicarboxylic acid

Furan-2-acrylic acid (2 g, 0.014 mol), freshly recrystallized from ethanol/water, was dissolved in 60 mL CH₃CN. Benzophenone (0.001 mol) was added and the resulting clear solution was bubbled with nitrogen for 30 minutes and during irradiation for 7 hours with a high pressure 450 W Hg lamp. The mixture was cooled to room temperature, and the solvent was evaporated *in vacuo* leaving a clear yellow oil. The oil was dissolved in 40 mL ethyl ether and washed twice with 50 mL portions of saturated sodium bicarbonate solution. The combined bicarbonate washes were re-acidified to pH 2 and extracted with two 50 mL portions ethyl acetate. The combined organic extracts were washed with 50 mL 10% NaCI solution, dried over anhydrous sodium sulfate, filtered and solvent removed *in vacuo* leaving 1.5 g yellow oil. The resulting crude yellow oil was dissolved in CHCl₃ and purified by silica gel chromatography eluting with 1% MeOH in CHCl₃. Two products were isolated and characterized by NMR spectroscopy. The faster moving product obtained in 30% yield was found to be (1α,2α,3β,4β)-1,2-di-(2-furyl)cyclobutane-3,4-dicarboxylic acid. NMR (CDCl₃, 300 MHz) δ 7.35 (m, 1H), 6.38 (m, 1H), 6.17 (m, 1H), 3.82 (m, 1H, J=10 Hz), 3.58 (m, 1H, J=10 Hz). MS (DCl/NH₄) m/e 276. The slower moving product, obtained in 15% yield eluted from the column with 8% MeOH in CHCl₃. This product was characterized as (1α,2α,3α,4α)-1,2-di-(2-furyl)cyclobutane-3,4-dicarboxylic acid. NMR (CDCl₃, 300 MHz) δ 7.48 (m, 1H), 7.20 (m, 1H), 5.94 (m, 1H), 4.24 (m, 1H, J=7 Hz), 3.93 (m, 1H, J=7 Hz). MS (DCl/NH₄) m/e 276.

### Example 115B

### (1α,2α,3β,4β)-1,2-Di[N-propyl-N-(phenoxybenzyl)aminocarbonyl]cyclobutane-3,4-dicarboxylic acid

The (1α,2α,3β,4β)-compound resulting from Example 115A (2 g, 7.2 mmol) was dissolved in 20 mL of DMF and cooled to 0-5 °C in an ice bath. To this solution was added 4.2 g (15 mmol) of N-propyl-N-4-phenoxybenzylamine hydrochloride in 5 mL DMF, and the salt was neutralized with 2.1 mL (15 mmol) triethylamine. The mixture was stirred 5 minutes then 3.8 g BOP-CI (15 mmol) was added followed by an additional 2.1 mL of triethylamine. The mixture was stirred 2 hours at 0-5 °C then warmed to room temperature and stirred another 20 hours at room temperature. When the reaction was deemed complete by TLC analysis, the mixture was diluted with 200 mL ethyl acetate and washed with 2 x 50 mL 1 N H₃PO₄, 2 x 50 mL satdurated NaHCO₃, and 2 x 50 mL 10% NaCI. The organic layer was dried over anhydrous Na₂SO₄, filtered and concentrated *in vacuo* leaving 2.8 g (54%) of crude product. The crude product was purified by silica gel chromatography eluting with 3:1 hexane-ethyl acetate yielding (1α,2α,3β,4β)-1,2-di[N-propyl-N-(phenoxybenzyl)aminocarbonyl]-3,4-di(2-furyl)cyclobutane (1.5 g) as a light yellow oil. ¹NMR (CDCl₃, 300 MHz) δ 7.37-6.86 (m, 18H), 6.29-6.06 (m, 6H), 4.50-4.61 (m, 4H), 3.81-4.24 (m, 4H), 2.88-3.39 (m , 4H), 1.28-1.73 (m, 4H), 0.71-0.87 (m, 6H). MS (FAB⁺): 723.

The furan groups were converted to carboxylic acids using the procedure described by Danishefsky et al., J. Amer. Chem. Soc., 110 (12), 3929 - 3940 (1988). The crude product was purified by silica gel chromatography eluting with 94:5:1 CHCl₃-MeOH-HOAc to give the title compound (12 mg, 13%). ¹H NMR (CDCl₃, 500 MHz) δ 0.80-0.95 (m, 6H), 1.25-1.70 (m, 4H), 3.08-3.40 (m, 4H), 3.50-3.65 (m, 2H), 3.88-4.05 (m, 2H), 4.20-4.27 (dd, 2H), 4.40-4.65 (m, 2H), 6.87-7.39 (m, 18H). MS (FAB⁺) m/e 679, (FAB⁻) m/e 677.

### Example 116

### (1α,2α,3α,4α)-1,2-Di[N-propyl-N-(phenoxybenzyl)aminocarbonyl]cyclobutane-3,4-dicarboxylic acid

The (1α,2α,3α,4α)-1,2-di-(2-furyl)cyclobutane-3,4-dicarboxylic acid prepared in Example 115A was coupled with N-propyl-N-4-phenoxybenzylamine and the furan groups converted to carboxylic acids by the procedures described in Example 115B. The crude product was purified by silica gel chromatography eluting with 94:5:1 CHCl₃-MeOH-HOAc to give the title compound (10 mg, 7%). ¹H NMR (DMSO-d₆, 500 MHz) δ 0.67-0.80 (m, 6H), 1.24-1.45 (m, 4H), 2.90-2.93 (m, 2H), 3.11-3.14 (m, 2H), 3.50-3.59 (m, 2H), 3.74-3.83 (m, 2H), 4.20-4.26 (m, 2H), 4.58-4.63 (m, 2H), 6.84-7.38 (m, 18H). MS (FAB⁺) m/e 679, (FAB⁻) m/e 677.

### Example 117

### (1α,2α,3α,4β)-1,2-Di[N-propyl-N-(4-phenoxybenzyl)aminocarbonyl]cyclobutane-3,4-dicarboxylic acid

The compound resulting from Example 116 was converted to the mono-ester by the procedure described in Example 95. Equilibration of the mono-ester was carried out by the procedures described in Example 96A substituting LDA for n-BuLi at -78 °C to afford 8 mg (10%) of the title compound. ¹H NMR (DMSO-d₆, 500 MHz) δ 0.67-0.80 (m, 6H), 1.24-1.45 (m, 4H),2.90-2.93 (m, 2H), 3.11-3.14 (m, 2H), 3.50-3.59 (m, 2H), 3.83-3.94 (m, 2H), 4.20-4.26 (m, 2H), 4.58-4.63 (m, 2H), 6.84-7.38 (m, 18H). MS (FAB⁺) m/e 679, (FAB⁻) m/e 677.

### Example 118

### (1α,2β,3β,4α)-1,2-Di[N-propyl-N-(4-benzoylbenzyl)aminocarbonyl]cyclobutane-3,4-carboxylic acid

### Example 118A

### N-Propyl-N-(4-benzoylbenzyl)amine

A solution of α-bromo-*p*-benzoyl toluene (2.75 g, 0.01 mol) in benzene (100 mL) was added dropwise over 30-40 minutes to a stirred solution of n-propylamine (10 mL, 0.12 mol) in benzene (50 mL) under a nitrogen atmosphere. After stirring for 4-5 hours at ambient temperature, most of the solvent was removed under reduced pressure. The residue obtained was triturated with ice-water and ether, and basified with dilute aqueous sodium hydroxide. The ether layer was separated, and the aqueous layer was extracted with additional ether. The combined ether extracts were washed with saturated sodium chloride, dried over sodium sulfate, filtered and concentrated *in vacuo* to give 2.5 g of a yellow liquid. The liquid was taken up in ether, filtered from undissolved impurities, and acidified with HCl in isopropanol. The solid obtained was filtered and recrystallized from methanol-ethyl acetate to give 1.9 g (58%) of the title compound as colorless crystalline flakes. m.p. 193.5-194.5 °C. ¹H NMR (CDCl₃, 300 MHz) δ 0.98 (t, 3H), 1.9 (q, 2H), 2.8 (s, 2H), 4.15 (s, 2H), 7.4-7.8 (m, 9H), 10.09 (s, 2H). MS (DCl/NH₃) m/e 254 (M+H)⁺.

### Example 118B

### (1α,2β,3β,4α)-1,2-Di[N-propyl-N-(4-benzoylbenzyl)aminocarbonyl]cyclobutane-3,4-carboxylic acid

A suspension of the compound resulting from Example 118A (1.45 g, 5 mmol) in acetonitrile (20 mL) containing triethylamine (0.7 mL) was added all at once to a stirred suspension of 1,2,3,4-cyclobutanetetracarboxylic dianhydride in acetonitrile (20 mL) at 0 °C under nitrogen. The mixture was stirred, and the temperature was slowly allowed to rise to ambient temperature. Stirring was continued for 60 hours, and then the clear reaction mixture was poured onto ice-water containing HCI and extracted with ether. The combined ether extracts were washed with ice-water and saturated aqueous sodium chloride solution, dried over sodium sulfate, filtered and concentrated under reduced pressure to give 1.2 g of a mixture of 1,2- and 1,3-isomers as a solid foam. The foam was purified by chromatography on silica gel eluting with 97:2.5:0.5 chloroform-methanol-acetic acid to give 0.22 g (13%) of the title compound. m.p. 100-107 °C. ¹H NMR (DMSO-d₆, 500 MHz) δ 0.8 (m, 6H), 1.5 (m, 4H), 2.85 (m, 1H), 2.35 (s, 4H), 2.7 (m, 2H), 3.1 (m, 1H), 3.8 (d, 1H), 4.0 (d, 1H), 4.4 (m, 2H), 4.9 (m, 2H), 7.3-7.7 (m, 18H). MS (DC/NH₃) m/e 703 (M+H)⁺

### Example 119

### (1α,2β,3β,4α)-1,2-Di[N-propyl-N-(4-(3,4-methylenedioxyphenoxy)benzyl)aminocarbonyl]cyclobutane-3,4-carboxylic acid

### Example 119A

### 4-(3,4-Methylenedioxyphenoxy)benzaldehyde

A solution of 3,4-methylenedioxy phenol (7.0 g, 50.7 mmol) in anhydrous DMSO was added dropwise over 30 minutes to a stirred suspension of 60% oil dispersion NaH (2.02 g, 50 mmol) in anhydrous DMSO (40 mL) under nitrogen. Following the addition, a solution of p-fluorobenzaldehyde (6.2 g, 0.05 mol) in DMSO (10 mL) was added, and the temperature was slowly raised to 150 °C. The temperature was maintained between 130-160 °C for 1 hour and then allowed to cool to ambient temperature. After 12 hours at ambient temperature, the reaction mixture was poured onto crushed ice-water and made barely acidic with concentrated HCl and extracted with ether. The combined ether extracts were washed successively with cold dilute aqueous sodium hydroxide solution, cold dilute HCI solution, cold water and saturated aqueous sodium chloride solution, dried over sodium sulfate, filtered and evaporated to give a red liquid which solidified. The solid was triturated with boiling n-pentane, filtered and washed with boiling pentane to give 8.2 g (67%) of the title compound. m.p. 67-70 °C. ¹H NMR (CDCl₃, 300 MHz) δ 6.0 (s, 2H), 6.6 (m, 2H), 6.8 (d, 1H), 7.04 (m, 2H), 7.84 (m, 2H), 9.92 (s, 1H). MS (DCI/NH3) m/e 243 (M+H)⁺.

### Example 119B

### N-Propyl-N-[4-(3,4-methylenedioxyphenoxy)benzyl]amine hydrochloride

A mixture of the compound resulting from Example 119A (6.0 g, 24 mmol) and 0.6 g of 10% Pd/C and n-propylamine (9.3 mL) in ethanol (200 mL) was shaken, first for 24 hours at ambient temperature, and then for 48 hours with hydrogen at atmospheric pressure. The reaction mixture was filtered and the filtrate evaporated. The resulting residue was taken up in ether, converted to the hydrochloride salt, which was then decolorized with norite and recrystallized from methanol-ethyl acetate to give 6.2 g (88%) of the title compound as a colorless crystalline solid. m.p. 185-186 °C. ¹H NMR (CDCl₃, 300 MHz) δ 0.95 (t, 3H), 1.86 (q, 2H), 2.75 (t, 2H), 3.98 (s, 2H), 5.98 (s, 2H), 6.45 (q, 1H), 6.52 (d, 1H), 6.75 (d, 1H), 6.95 (m, 1H), 7.26 (s, 1H), 7.5 (d, 2H), 9.85 (s, 2H). MS (DCI/NH₃) m/e 286 (M+H)⁺.

### Example 119C

### (1α,2β,3β,4α)-1,2-Di[N-propyl-N-(4-(3,4-methylenedioxyphenoxy)benzyl)aminocarbonyl]cyclobutane-3,4-carboxylic acid

To a suspension of 1,2,3,4-cyclobutanetetracarboxylic dianhydride (0.98 g, 5 mmol) in acetonitrile (10 mL) was added 1.6 mL (11 mmol) of triethylamine at 0-5 °C. After several minutes, a suspension of the compound resulting from Example 119B (3.23 g, 10 mmol) in acetonitrile (30 mL) and triethylamine (1.6 mL, 11 mmol) was added to the above stirred mixture. The reaction mixture was stirred for 24 hours at ambient temperature and then poured into cold dilute HCI. The acidic solution was extracted with ether, and the combined ether extracts were washed with saturated sodium chloride solution, dried over sodium sulfate and evaporated. The residue obtained was taken up in methylene chloride, filtered and evaporated to give 2.6 g of a mixture of the 1,2- and 1,3-isomers as a soild foam. The foam was chromatographed on silica gel eluting with 97:2.5:0.5 chloroform-methanol-acetic acid to give 0.8 g (21%) of the title compound. m.p. 95-110 °C. ¹H NMR (DMSO-d₆, 500 MHz) δ 0.8 (m, 6H), 1.5 (m, 4H), 2.5 (s, 1H), 2.8 (m, 1H), 3.3 (s, 8H), 3.6 (m, 1H), 3.9 (m, 1H), 4.3 (m, 1H), 4.7 (m, 1H), 5.6 (s, 4H), 6.5-7.2 (m, 14H). MS (DCI/NH3) m/e 267 (M+H)⁺.

### Example 120

### (1α,2β,3β,4α)-1,2-Di[N-methyl-N-(4-phenoxybenzyl)aminocarbonylamino]cyclobutane-3,4-dicarboxylic acid

A mixture of 1,2-di[methoxycarbonyl]cyclobutane-3,4-dicarboxylic acid (400 mg, 1.54 mmol), prepared by the procedure described in Angew. Chem. International Ed. vol 8: 208 (1969), diphenylphosphoryl azide (DPPA) (936 mg, 3.4 mmol) and triethylamine (344 mg, 3.4 mmol) in 20 mL of toluene was heated at 80 °C for 3 hours. The mixture was allowed to cool to ambient temperature, and then the compound resulting from Example 1 (700 mg, 3.4 mmol) was added. After stirring at ambient temperature for 16 hours, the mixture was taken up in ethyl acetate and washed with dilute hydrochloric acid, water, saturated sodium bicarbonate solution and saturated sodium chloride solution, dried and filtered. The filtrate was evaporated under reduced pressure to an oily residue which was chromatographed on silica gel eluting with 4:1 ethyl acetate-hexane to give 100 mg (19.2%) of the diester.

To the diester (40 mg, 0.058 mmol) dissolved in 30 mL of methanol was added KOH (33 mg, 0.58 mmol) in 1 mL of water. The clear solution was stirred at ambient temperature for 3 hours and left standing overnight. The methanol was removed under reduced pressure, and the aqueous solution was acidified with sodium hydrogen sulfate. The precipitate formed was filtered, washed with water and dried to give 27 mg of the title compounds m.p. 192-195 °C (dec). ¹H NMR δ (DMSO-d₆, 300 MHz) δ 2.72 (s, 6H), 4.30-4.50 (dd, 4H), 6.90-7.40 (m, 18H).

### Example 121

### (1α,2β,3β,4α)-1,2-Di[N,N-dibenzylaminocarbonyl]cyclobutane-3,4-dicarboxylic acid

To a slurry of 1,2,3,4-cyclobutanetetracarboxylic dianhydride (2.7 g, 13.8 mmol) in THF (30 mL) was added N,N-dibenzylamine (5.72 g, 28.9 mmol) in THF (10 mL). The slurry was stirred for 5 minutes at 20 °C, whereupon, a homogeneous solution resulted. The resulting solution was stirred for 20 hours at 20 °C, then concentrated *in vacuo* to a white foam. The foam was dissolved in 100 mL of ethyl acetate and washed successively with 50 mL 1N H₃PO₄ and 10% NaCI, then dried over anhydrous sodium sulfate, filtered and concentrated *in vacuo* to afford 4.4 g of a white foamy solid. The crude product (2.1 g) containing both isomers was purified by silica gel chromatography eluting with 94:5:1 CHCl₃-MeOH-HOAc. The slower moving product was isolated in 22% yield and characterized as the title compound. ¹H NMR δ (CDCl₃, 500 MHz) δ 3.98-4.00 (d, 2H), 4.22-4.31 (m, 6H), 4.46-4.49 (d, 2H), 4.73-4.76 (d, 2H), 7.11-7.31 (m, 20H). MS (FAB) m/e 591 (M+H)⁺.

### Example 122

### (1α,2β,3β,4α)-1,2-Di[N-benzyl-N-(4-chlorobenzyl)aminocarbonyl]cyclobutane-3,4-dicarboxylic acid

### Example 122A

### N-Benzyl-N-(4-chlorobenzyl)amine Hydrochloride

To 4-chlorobenzaldehyde (1.5 g, 10.7 mmol) dissolved in 40 mL CH₂Cl₂ was added benzylamine (1.15g, 10.7 mmol) followed by 1 mL of 6 N HCl. The resulting cloudy solution was stirred overnight at room temperature and then concentrated *in vacuo* to give a white solid. The solid was immediately dissolved in 50 mL of MeOH and the pH of the solution was adjusted to 6 with glacial acetic acid. To this solution was added NaCNBH₃ (0.67 g, 10.7 mol), and the resulting clear solution was stirred three hours at room temperature. The mixture was acidified to pH 2 with 6 M HCI whereupon a thick white slurry developed. The solid was stirred for 30 minutes and filtered, yielding the title compound as a white solid. ¹H NMR δ (DMSO-d₆, 300 MHz) δ 4.1-4.2 (broad multiplet, 4H), 7.4-7.6 (m, 9H). MS (DCI/NH₃) m/e 232 (M+H)⁺

### Example 122B

### (1α,2β,3β,4α)-1,2-Di[N-benzyl-N-(4-chlorobenzyl)aminocarbonyl]cyclobutane-3,4-dicarboxylic acid

To a slurry of 1,2,3,4-cyclobutanetetracarboxylic dianhydride (0.5 g, 2.5 mmol) in DMF (6 mL) was added the compound resulting from Example 122A (1.36 g, 5.1 mmol) in DMF (3 mL) containing Et₃N (0.71 mL, 5.1 mmol). The slurry was stirred for 5 minutes at 20 °C, whereupon, a homogeneous solution resulted. The resulting solution was stirred 20 hours at 20 °C. The solution was diluted with 100 mL of ethyl acetate and washed successively with 50 mL 1 N H₃PO₄ and 10% NaCI, then dried over anhydrous sodium sulfate, filtered and concentrated *in vacuo* to afford 1.1 g of a white foamy solid. The crude product containing both isomers was purified by silica gel chromatography eluting with 94:5:1 CHCl₃-MeOH-HOAC. The slower moving product was isolated in 21% yield and characterized as the title compound. ¹H NMR δ (DMSO-d₆, 300 MHz) δ 3.68-3.71 (m, 2H), 3.92-4.06 (m, 4H), 4.13-4.21 (m, 2H), 4.65-4.72 (m, 2H), 4.82-4.92 (m, 2H), 7.14-7.42 (m, 18H), 12.55 (bs, 2H). MS (FAB) m/e 659 (M+H)⁺.

### Example 123

### Alternate Preparation of (1α,2β,3β,4a)-1,2-Di[N-propyl-N-(4-phenoxybenzyl)aminocarbonyl]cyclobutane-3,4-dicarboxylic acid

### Example 123A

### (1α,2β,3β,4α)-1,2-Di(benzyloxycarbonyl)cyclobutane-3,4-dicarboxylic acid

To a solution of 1,2,3,4-cyclobutanetetracarboxylic anhydride (21 g, 107.1 mmol) in acetonitrile (530 mL) at -7.5 °C was added benzyl alcohol (70 mL, 680 mmol) all at once. Triethylamine (30 mL, 210 mmol) was added dropwise over 3-5 minutes and the temperature rose to 2.8 °C. Dimethylaminopyridine (1.3 g, 10.6 mmol) was added and the temperature returned to -5°C. The reaction mixture was stirred for 18 hours at which time the internal temperature was 9.2 °C and then concentrated *in vacuo.* The residue was dissolved in ethyl acetate (1 L) and washed with 2 M HCl (2 x 375 mL). The product was then extracted into saturated NaHCO₃ solution (2 x 375 mL). The aqueous solution was allowed to stand at ambient temperature and then was cooled in a refrigerator overnight. The solid was collected and washed with cold saturated NaHCO₃ solution (200 mL) and then was dissolved in water (500 mL), acidified with 2 M HCl (375 mL) and extracted into ethyl acetate (2 x 375 mL). The combined organic extracts were washed with brine, dried over sodium sulfate and concentrated *in vacuo* to afford the title compound (25.65 g, 58%) as a white solid. m.p. 164.5-165.5 °C.

### Example 123B

### Benzyl (1α,2β,3β,4α)-1,2-Di[N-propyl-N-(4-phenoxybenzyl)aminocarbonyl]cyclobutane-3,4-dicarboxylate

To the compound resulting from Example 123A (66 mg, 0.15 mmol) slurried in methylene chloride (0.5 mL) was added oxalyl chloride (28 µL, 0.32 mmol) followed by 1 drop DMF. To this solution was added N-propyl-N-(4-phenoxybenzyl)amine (81 mg, 0.34 mmol) and Hunig's base (115 µL, 0.66 mmol) in methylene chloride (0.2 mL). The reaction mixture was stirred at ambient temperature for 1 hour and then diluted with ethyl acetate. The solution was washed 3 times with 1 M H₃PO₄, 3 times with saturated NaHCO₃ solution and brine, dried over sodium sulfate and concentrated under reduced pressure. The residue obtained was dissolved in methylene chloride and applied to the top of a silica gel plug which was eluted with 7:3 hexane-ethyl acetate to give the title compound (114 mg, 88%) as an oil.

### Example 123C

### (1α,2β,3β,4α)-1,2-Di[N-propyl-N-(4-phenoxybenzyl)aminocarbonyl]cyclobutane-3,4-dicarboxylic acid

The compound resulting from Example 123B (100 mg, 0.12 mmol) was dissolved in 1.5 mL 1:1 methanol-ethyl acetate, treated with 9 mg of 10% Pd/C and then hydrogenolyzed under a H2 balloon for 1.5 hours. The catalyst was removed by filtration through Celite and the filtrate concentrated *in vacuo* to afford the title compound. ¹H NMR (DMSO-d₆, 300 MHz) δ 0.80 (m, 6H), 1.46 (m, 4H), 2.70-3.00 (m, 4H), 3.60 (m, 2H), 3.92 (m, 2H), 4.27 (m, 2H), 4.70 (dd, 2H), 6.87-7.43 (m, 18H). MS (FAB) m/e 679 (M+H)⁺, 701 (M+Na)⁺.

### Example 124

### (1α,2β,3β,4α)-1,2-Di[(4-phenoxybenzyl)carbonylamino]cyclobutane-3,4-dicarboxylic acid

### Example 124A

### Methyl 1,2-diaminocyclobutane-3,4-dicarboxylate

A mixture of 1,2-di[methoxycarbonyl]cyclobutane-3,4-dicarboxylic acid (500 mg, 1.92 mmol), prepared by the procedure described in Angew. Chem. International Ed. vol 8: 208 (1969), in 20 mL of toluene was treated with 407 mg (4.03 mmol) of triethylamine and 1.11 g (4.03 mmol) of diphenylphosphoryl azide. The mixture was warmed to 80 °C under a nitrogen atmosphere for 3 hours. Volatiles were removed under reduced pressure to afford a viscous oil. The oil was dissolved in 20 mL of 2-methyl-2-propanol and stirred for 18 hours. Volatiles were removed under reduced pressure to afford an oil. Purification by flash column chromatography on silica gel eluting with 2:1 hexane-ethyl acetate afforded methyl 1,2-di(Boc-amino)cyclobutane-3,4-dicarboxylate as a white powder (123 mg, 16%. ¹H NMR (CDCl₃) δ 1.43 (s, 18H), 3.45-3.52 (m, 1H), 3.75 (s, 6H), 4.52-4.63 (m, 1H), 5.55-5.65 (m, 1H).

A solution of 70 mg (0.17 mmol) of protected diamine prepared above in 3 mL of trifluoroacetic acid was stirred for 3 hours. A 50 mL portion of ethyl ether was added, causing the product to precipitate as a white solid. The solid was collected by filtration and dried, affording 56 mg of the title compound which was used without further purification.

### Example 124B

### Methyl (1α,2β,3β,4α)-1,2-Di[(4-phenoxybenzyl)carbonylamino]cyclobutane-3,4-dicarboxylate

A mixture of the compound resulting from Example 124A (56 mg, 0.13 mmol), 4-phenoxyphenylacetic acid (61 mg, 0.27 mmol), 1-(3-dimethylaminopropyl)-3-ethylcarbodiimide · HCl (54 mg, 0.28 mmol), 1-hydroxybenzotriazole hydrate (38 mg, 0.28 mmol), and 59 mg (0.59 mmol) of triethylamine in 5 mL of dry tetrahydrofuran was stirred under an atmosphere of nitrogen for 3 days. A 50 mL portion of water was added, and the mixture was extracted with three 50 mL portions of ethyl acetate. The combined extracts were dried (MgSO₄), and all volatiles were removed under reduced pressure to afford an oil. Purification of the oil by flash column chromatography on silica gel eluting with 2:1 hexane-ethyl acetate afforded 60 mg (75%) of the title compound as a clear colorless oil that solidified on standing. MS (FAB) m/e 623 (M+H)⁺.

### Example 124C

### (1α,2β,3β,4α)-1,2-Di[(4-phenoxybenzyl)carbonylamino]cyclobutane-3,4-dicarboxylic acid

A mixture of 60 mg (0.097 mmol) of the compound resulting from Example 124B in 3 mL of methanol was treated with 0.5 mL of a 50% aqueous solution of sodium hydroxide. After 3 hours the mixture was treated with concentrated HCI until the mixture was acidic. All volatiles were removed under reduced pressure to afford a solid. The solid was washed with water and dried, affording 25 mg (42%) of the title compound as a white powder. MS (FAB) m/e 595 (M+H)⁺.

### Example 125

### (1α,2β,3β,4α)-1,2-Di[(N-propyl-N-(4-phenoxybenzyl)carbonylamino]cyclobutane-3,4-dicarboxylic acid

The compound resulting from Example 124A is reacted with propionyl chloride in the presence of triethylamine to give methyl 1,2-di(propionylamino)cyclobutane-3,4-dicarboxylate. Reduction of the diamide with borane-tetrahydrofuran complex at 0° C affords methyl 1,2-di(n-propylamino)cyclobutane-3,4-dicarboxylate. Treatment of this secondary amine with the acid chloride of 4-phenoxyphenylacetic acid in the presence of triethylamine yields methyl1,2-di[(N-propyl-N-(4-phenoxybenzyl)carbonylamino]cyclobutane-3,4-dicarboxylate. Hydrolysis of the methyl esters with aqueous sodium hydroxide affords the title compound.

### Example 126

### (1α,2β,3β,4α)-1,2-Di-[N-propyl-N-(4-phenoxybenzyl)aminocarbonyl]-3,4-bis(tetrazolylmethyl)cyclobutane

### Example 126A

### (1α,2β,3β,4α)-1,2-Di-(benzyloxycarbonyl)-3,4-bis(hydroxymethyl)cyclobutane

To the compound resulting from Example 123A (2.06 g, 5 mmol) dissolved in THF (3 mL) at -10 °C was added BH₃ (1.0 M in THF, 11 mL, 11 mmol) at such a rate so as to maintain the internal temperature below 0 °C. The mixture was then stirred for 5 hours during which time the bath melted. The colorless solution was quenched by the careful addition of 1:1 acetic acid-water (0.3 mL) and poured into 50 mL of saturated NaHCO₃ and extracted with 2 x 75 mL portions of ethyl acetate. The combined organic phases were extracted with water (50 mL) and brine (50 mL), dried over MgSO₄, filtered and concentrated. The residue was purified by column chromatography on SiO₂ (120 g) using ethyl acetate as the eluent to give 1.42 g (74%) as a white solid. ¹H NMR (300 MHz, CDCl₃) δ 7.24.7.48 (m, 12H), 5.16 (m, 4H), 3.72 (m, 4H), 3.61 (m, 2H), 2.67 (m, 2H), 2.19 (t, 2H). MS (DCI) m/e 402 (4%); 294 (6%); 203 (52%); 186 (100%); 143 (22%); 126 (50%).

### Example 126B

### (1α,2β,3β,4α)-1,2-Di-(benzyloxycarbonyl)-3,4-bis(methanesulfonyloxymethyl)cyclobutane

To a solution of the compound resulting from Example 126A (577 mg, 1.5 mmol) in 10 mL of CH₂Cl₂ at -10 °C was added triethylamine (0.63 mL, 4.5 mmol), followed by the dropwise addition of methanesulfonyl chloride (0.29 mL, 3.75 mmol). The solution was warmed to 0 °C and stirred for 1.5 hours. The mixture was diluted with 40 mL of CH₂Cl₂ and washed with 25 mL each water and dilute aqueous HCI. The organic fraction was dried over MgSO₄, filtered and concentrated to give 767 mg (95%) of the desired compound as an off white solid. This material was used without further purification. ¹H NMR (300 MHz, CDCl₃) δ 7.38 (s, 12H), 5.15 (AB quartet, 4H), 4.32 (m, 4H), 3.77 (d, 2H), 2.95 (m, 2H), 2.88 (s, 6H). MS (DCI) m/e 558 (28%); 372 (35%); 204 (40%); 186 (100%).

### Example 126C

### (1α,2β,3β,4α)-1,2-Di-(benzyloxycarbonyl)-3,4-bis(cyanomethyl)cyclobutane

A mixture of the compound resulting from Example 126B (270 mg, 0.5 mmol) and KCN (195 mg, 3 mmol) in 5 mL of DMSO was heated with stirring at 65 °C for 3 hours. The mixture was cooled to room temperature and partitioned between 50 mL of water and 2 x 50 mL of ethyl acetate. The combined organic fractions were washed with 2 x 25 mL of water and 25 mL of brine, dried over MgSO₄, filtered and concentrated. The residue was purified by column chromatography on SiO₂ (20 g) eluting with 1:1 ethyl acetate-hexanes to give 182 mg (76%) of the title compound as a thick oil. ¹H NMR (300 MHz, CDCI3) δ 7.26-7.42 (m, 12H), 5.17 (s, 4H), 3.18 (m, 2H), 2.57-2.78 (m, 6H). MS (DCI) m/e 420 (100%); 393 (10%); 344 (8%); 286 (22%); 221 (25%); 219 (100%); 125 (23%).

### Example 126D

### (1α,2β,3β,4α)-1,2-Di[N-propyl-N-(4-phenoxybenzyl)aminocarbonyl]-3,4-bis(cyanomethyl)cyclobutane

A solution of the compound resulting from Example 126C (175 mg, 0.43 mmol) in 3 mL of 10% CH₂Cl₂-ethyl acetate was treated with 100 mg of 10% Pd/C and stirred under a balloon of H₂ for 3 hours. The black mixture was filtered through celite, the celite pad was washed well with methanol and the resulting filtrate was concentrated. The residue was suspended in CH₂Cl₂ (4 mL) and treated with oxalyl chloride (0.092 mL, 1.05 mmol) and 1 drop of DMF. The mixture was stirred for 1 hour and then treated with 2 mL of acetonitrile (to try to dissolve the unreacted acid). After stirring for 1 hour more, the mixture was concentrated. The residue was concentrated twice with 2 mL of toluene (to remove HCl and unreacted oxalyl chloride) and then dissolved in 2 mL of CH₂Cl₂. In a separate flask was added the amine from Example 3 (302 mg, 1.26 mmol), CH₂Cl₂ (5 mL) and saturated aqueous NaHCO₃ (5 mL). This mixture was cooled to 0 °C and then treated with the acid chloride solution dropwise. The mixture was stirred for 1 hour and then poured into a separatory funnel. The layers were separated and the organic phase was washed with dilute aqueous HCI, dried over MgSO₄, filtered and concentrated. The residue was purified by column chromatography on SiO₂ (30 g) eluting with 1:1 ethyl acetate-hexanes to give 149 mg (53%) of the title compound as a colorless oil. ¹H NMR (300 MHz, CDCl₃) δ 6.87-7.40 (m, 18H), 4.18-4.75 (m, 4H), 2.11-3.63 (m, 10H), 2.38 (m, 2H), 1.42-1.62 (m, 4H), 0.78-0.97 (m, 6H). MS (DCl) m/e 686 (8%); 669 (4%); 240 (100%).

### Example 126E

### (1α,2β,3β,4α)-1,2- Di-[N-propyl-N-(4-phenoxybenzyl)aminocarbonyl]-3,4-bis(tetrazolylmethyl)cyclobutane

To a solution of the above dinitrile (136 mg, 0.20 mmol) in 2 mL of DMF was added NaN₃ (198 mg, 3.04 mmol) and triethylamine hydrochloride (418 mg, 304 mmol). The mixture was then heated to 100-110 °C for 20 hours and cooled to room temperature. The yellow mixture was partitioned between dilute aqueous HCl (25 mL) and ethyl acetate (3 x 20 mL). The combined organic fractions were washed with water (3 x 10 mL) and brine (10 mL), dried over MgSO₄, filtered and concentrated. The resulting off white solid was recrystallized from ethyl acetate to give 111 mg (in two crops) of the title compound as a white solid. ¹H NMR (300 MHz, CDCl₃) δ 6.83-7.34 (m, 18H), 4.30-4.77 (m, 4H), 2.79-3.76 (m, 12H), 1.34-1.63 (m, 4H), 0.75-0.90 (m, 6H). MS (FAB⁺) m/e 755 (75%); 240 (18%); 183 (100%). Anal calcd for C₄₂H₄₆N₁₀O₄: C, 66.83; H, 6.14; N, 18.55. Found: C, 66.54; H, 6.05; N, 18.94.

### Example 127

### (1α,2β,3β,4α)-1,2-Di-[N-propyl-N-(4-phenoxybenzyl)aminocarbonylmethyl]-cyclobutane-3,4-dicarboxylic acid

### Example 127A

### (1α,2β,3β,4α)-1,2-Di-[benzyloxycarbonyl]-cyclobutane-3,4-diacetic acid

To (1α,2β,3β,4α)-1,2-Di-[benzyloxycarbonyl]-3,4-bis(diazoacetyl)cyclobutane (50 mg, 0.109 mmol), the compound resulting from Example 128A, in THF (2 mL) and water (1 mL) was added silver benzoate in triethylamine (35 µL of a 50 mg/1 mL solution). After stirring at room temperature for 2 hours, the reaction mixture was poured into a saturated sodium bicarbonate solution. The mixture was washed two time with ethyl acetate, acidified with 2 M HCI, and extracted into ethyl acetate. The combined organic extracts were washed with brine, dried over sodium sulfate and concentrated *in vacuo* to afford 28 mg (58%) of the title compound as an off white powder.

### Example 127B

### (1α,2β,3β,4α)-1,2-Di-[N-propyl-N-(4-phenoxybenzyl)aminocarbonylmethyl]-cyclobutane-3,4-dicarboxylic acid dibenzyl ester

To the compound resulting from Example 127A (45 mg, 0.'02 mmol) in methylene chloride (1 mL) was added oxalyl chloride (19 µL, 0.23 mmol) and DMF (0.5 µL). After stirring at room temperature approximately 3 hours, the reaction mixture was cooled to 0 °C and treated with N-propyl-N-(4-phenoxybenzyl)amine hydrochloride (51 mg, 0.229 mmol) and 1 mL of saturated sodium bicarbonate solution. The cooling bath was removed, and the reaction mixture was stirred 14 hours at room temperature and diluted with methylene chloride. The solution was washed with saturated sodium bicarbonate solution, dried over sodium sulfate and concentrated *in vacuo* to afford 85 mg of crude material. Purification by chromatography on silica gel eluting with 30% ethyl acetate in hexane afforded 72.5 mg (80%) of the title compound as a colorless oil.

### Example 127C

### (1α,2β,3β,4α)-1,2-Di-[N-propyl-N-(4-phenoxybenzyl)aminocarbonylmethyl]-cyclobutane-3,4-dicarboxylic acid

The compound resulting from Example 127B (69.4 mg, 0.078 mmol) and 10% palladium on carbon (70 mg) in 1:1 methanol-ethyl acetate (2 mL) were stirred under hydrogen for 2 hours. The catalyst was removed by filtration and the filtrate concentrated *in vacuo* to afford the title compound (51.6 mg, 93%) as a slightly gray powder. ¹H NMR (CDCl₃, 300 MHz) δ 6.87-7.40 (m, 18H), 4.39-4.68 (m, 3H), 3.89 (m, 2H), 3.07-3.52 (m, 3H), 2.20-2.90 (m, 5H), 1.55 (m, 6H), 1.27 (m, 1H), 0.88 (m, 6H).

### Example 128

### (1α,2β,3β,4α)-1,2-Di(N-benzyl-N-[(4-phenoxy)benzyl]aminocarbonyl)cyclobutane-3,4-diacetic acid

### Example 128A

### (1α,2β,3β,4α)-1,2-Di(benzyloxycarbonyl)-3,4-bis(diazoacetyl)cyclobutane

The compound resulting from Example 123A was converted to the bis(acid chloride) by the method described in the first sentence of Example 123B. A solution of the bis(acid chloride) (25.6 g, 62.0 mmol) in CH₂Cl₂ (250 mL) at 0 °C was treated with ~850 mL of an ether solution of diazomethane, prepared by adding 1-methyl-3-nitro-1-nitrosoguanidine (63.0 g, 428 mmol) to ether (1 L) over 40% aqueous KOH (220 mL), previously cooled to 5 °C. The resultant slurry was periodically shaken while maintaining the temperature at 5 °C for 30 minutes, then nitrogen was vigorously bubbled through the slurry for 40 minutes. The solids were filtered and recrystallized from EtOAc/hexane, to give the title compound as a light yellow solid (13.0 g). The mother liquor from this recrystallization was combined with the initial filtrate, concentrated under reduced pressure, and the residue was purified by chromatography eluting with 6:4 hexane-EtOAc to give an additional 4.2 g, for a total of 17.2 g (60%). ¹H NMR (CDCl₃) 6 7.35 (m, 10H), 5.18 (s, 2H), 5.12 (dd, 4H), 3.86 (d, 2H), 3.70 (br d, 2H). MS (FAB⁺) m/e 461 (M+H)⁺.

### Example 128B

### Alternative Preparation of (1α,2β,3β,4α)-1.2-Di(benzyloxycarbonyl)-3,4-bis(diazoacetyl)cyclobutane

The bis(acid chloride) described in Example 128A in CH₃CN (0.4 M) was added to a 0 °C 1:2 mixture of trimethylsilyldiazomethane (2 M in hexane) in CH₃CN, (4.4 moles TMSCHN₂ per mole bis(acid chloride)). The reaction was stirred at 0-17 °C over 4 hours then concentrated. The residue was partitioned between saturated aqueous NaHCO₃ and EtOAc, and the EtOAc layer was washed with brine and dried over Na₂SO₄, and concentrated *in vacuo.* The crude solids were recrystallized by dissolving in hot EtOAc (80-100 mg/mL), then adding five volumes of hexane to the hot solution and allowing the mixture to cool at room temperature overnight.

### Example 128C

### (1α,2β,3β,4α)-1,2-Di(benzyloxycarbonyl)cyclobutane-3,4-diacetic acid dimethyl ester

Using the compound resulting from Example 128A or B, the title compound was prepared by the method of Example 103A. ¹H NMR (CDCl₃) δ 7.35 (m, 10H), 5.10 (dd, 4H), 3.70 (br d, 2H), 3.55 (s, 6H), 2.92 (m, 2H), 2.49 (m, 4H). MS (DCl/NH₃) m/k 469 (M+H)⁺, 486 (M+H+NH₃)⁺.

### Example 128D

### (1α,2β,3β,4α)-1,2-Di(carboxy)cyclobutane-3,4-diacetic acid dimethyl ester

Using the compound resulting from Example 128C, the title compound was prepared by the method of Example 123C. ¹H NMR (CD₃OD) δ 3.65 (s, 6H), 3.51 (m, 2H), 2.82 (m, 2H), 2.57 (m, 4H). MS (FAB⁺) m/e 289 (M+H)⁺.

### Example 128E

### (1α,2β,3β,4α)-1,2-Di{N-benzyl-N-[(4-phenoxy)benzyl]aminocarbonyl)cyclobutane-3,4-diacetic acid dimethyl ester

Using the compound resulting from Example 128D and the amine described in Example 12, the title compound was prepared by the method of Example 123B. ¹H NMR (CDCl₃) δ 7-40-6.90 (envelope, 28H), 5.14 (dd, 2H), 4.65 (m, 2H), 4.32 (m, 2H), 4.13 (dd, 2H), 3.80 (m, 2H) 3.56-3.51 (4 s, total 6H), 2.75-2.40 (envelope, 6H). MS (DCl/NH₃) m/e 831 (M+H)⁺.

### Example 128F

### (1α,2β,3β,4α)-1,2-Di{N-benzyl-N-[(4-phenoxy)benzyl]aminocarbonyl)cyclobutane-3,4-diacetic acid

Using the compound resulting from Example 128E, the title compound was prepared by the method of Example 103B. ¹H NMR (DMSO-d₆) δ 7.42-7.10 (envelop, 20H), 6.95 (m, 6H), 6.90 (d, 2H), 4.80, 4.70 (d, dd, total 4H), 4.20 (m, 2H), 4.05 (m, 4H), 2.67 (br m, 2H), 2.40 (br m, 4H). MS (FAB⁺) m/e 803 (M+H)⁺ and 801 (FAB-) (M-H)⁻. Anal cald for C₅₀H₄₆N₂O₈ · 0.5 H₂O: C, 73.97; H, 5.83; N, 3.45. Found: C, 73.66; H, 5.75; N, 3.20.

### Example 129

### (-)-(1α,2β,3β,4α)-1,2-Di[N-cyclopropylmethyl-N-(4-phenoxybenzyl)aminocarbonyl]cyclobutane-3,4-dicarboxylic acid

### Example 129A

### (-)-(1α,2β,3β,4α)-1,2-Di[benzyloxycarbonyl]cyclobutane-3,4-dicarboxylic acid

To the compound resulting from Example 123A (1.0 g, 2.4 mmol) dissoved in absolute EtOH (45 mL) was added a solution of (-)-norephedrine (0.74 g, 4.9 mmol) in absolute EtOH (5 mL). The solution allowed to sit at room temperature overnight. The resultant crystals were collected and recrystallized twice from hot absolute EtOH (4.5 mg/mL), then partitioned between 1 M H₃PO₄ and Et₂O. The Et₂O layer was washed with brine, dried over Na₂SO₄, filtered and concentrated to give the title compound. [α]_{D} = +17.3° (c = 0.92, MeOH).

### Example 129B

### (-)-(1α,2β,3β,4α)-1,2-Di[N-cyclopropylmethyl-N-(4-phenoxybenzyl)aminocarbonyl]cyclobutane-3,4-dicarboxylic acid

The compound resulting from Example 129A and the amine resulting from Example 47A were reacted by the methods of Examples 123B and 123C to give the title compound which was obtained by lyophilization. ¹H NMR (DMSO-d₆), δ 0.01-1.25 (m, 4H), 0.27-0.52 (m, 4H), 0.80-0.96 (m, 2H), 2.68-2.92 (m, 2H), 3.20-3.55 (m, 2H), 3.56-3.68 (m, 2H), 3.89-4.98 (m, 1H), 4.00-4.10 (m, 1H), 4.28-4.58 (m, 2H), 4.65-4.89 (m, 2H), 6.85-7.04 (m, 8H), 7.08-7.30 (m, 6H), 7.33-7.43 (m, 4H). MS (FAB⁻) m/e 701 (M-H)⁻. Anal calcd for C₄₂H₄₂N₂O₈ · 0.5 H₂O: C, 70.87; H, 6.09; N, 3.94. Found: C, 70.68; H, 5.91; N, 3.85. [α]_{D} = -80.6° (c=0.625, MeOH).

### Example 130

### (1α,2β,3β,4α)-1,2-Di[N-propyl-N-(4-phenoxybenzyl)aminothiocarbonyl]cyclobutane-3,4-dicarboxylic acid

### Example 130A

### (1α,2β,3β,4α)-1,2-Di[N-propyl-N-(4-phenoxybenzyl)aminothiocarbonyl]cyclobutane-3,4-dicarboxylic acid dibenzyl ester

A solution of the compound resulting from Example 123B (500 mg, 0.6 mmol) in benzene (4 mL) was treated with Lawesson's reagent (235 mg, 0.6 mmol). The resulting suspension was refluxed for 96 hours. The solvent was evaporated, and the residue was dissolved in ethyl acetate (20 mL), washed with 1 M Na₃PO₄ (20 mL), and dried over MgSO₄. The solvent was evaporated, and the residue was dissolved in methylene chloride, treated with silica gel, and concentrated to dryness. The silica gel was poured onto a prepacked column and eluted with 10% ethyl acetate/hexane to provide 205 mg (39%) of the title compound as a colorless oil. ¹H NMR (CDCl₃, 300 MHz) δ 0.65-0.9 (m, 6H), 1.4-1.6 (m, 1H), 1.65-1.75 (m, 1H), 3.39-3.52 (m, 2H), 3.53-3.8 (m, 2H), 3.84-3.90 (m, 2H) 4.5 (dd, J=16.5, 4.5Hz, 1H), 4.78-4.99 (m, 2H), 5.0-5.3 (m, 9H), 6.85-7.14 (m, 10H), 7.15-7.4 (m, 18H). MS (DCI) m/e 891 (M+H)⁺

### Example 130B

### (1α,2β,3β,4α)-1,2-Di[N-propyl-N-(4-phenoxybenzyl)aminothiocarbonyl]cyclobutane-3,4-dicarboxylic acid

The compound resulting from Example 129A (71 mg, 0.08 mmol) in 3:1 THF/H₂O (2 mL) at 0 °C was treated with 1 M LiOH (800 µL). The solution was stirred at 0 °C for 8 hours then at room temperature for 18 hours. The solution was acidified with 1 N HCI (1 mL) and concentrated to dryness. The residue was chromatographed eluting with 98:1:1 CHCl₃-MeOH-HOAC to provide 10 mg (18%) of the title compound as a colorless oil which was dissolved in CH₃CN and H₂O and lyophilized to provide a white solid. m.p. 92-93 °C. MS (DCI) m/e 710 (M+H)⁺.

### Example 131

### (1α,2β,3α,4α)-Di[N-(S)-α-(cyclopropylmethyl)benzyl-N-(4-phenoxybenzyl)-aminocarbonyl]cyclobutane-3,4-dicarboxylic acid

### Example 131A

### (4S,5R)-3-(2-Phenyl)acetyl-4-methyl-5-phenyl-1,3-oxazolidin-2-one

A mechanically stirred solution of phenylacetic acid at -78 °C is treated with one part pivaloyl chloride then one part triethylamine. The mixture is stirred at -78°C for 15 minutes, at 0 °C for 45 minutes, then recooled to -78 °C. In a separate flask, one part 2.5 M nBuLi is added to a solution of (4S,5R)-(-)-4-methyl-5-phenyl-2- oxazolidinone in THF at -78 °C. The solution is stirred for 15 minutes then transferred to the flask containing pivalic anhydride via cannula. The mixture is stirred for 15 minutes at -78°C and 9 hours at room temperature. The mixture is quenched with 2 M KHSO₄ and, after evaporation of the THF, is extracted 3 times with ethyl acetate. The combined ethyl acetate extracts are dried over MgSO₄, filtered and concentrated to provide the crude title compound which is purified by column chromatography eluting with ethyl acetate-hexane.

### Example 131B

### (4S,5R,2'S)-3-(2-Propenyl-2-phenyl)acetyl-4-methyl-5-phenyl-1,3-oxazolidin-2-one

A solution of one equivalent of the compound resulting from Example 131A in THF at -78°C is transferred via cannula to a solution of one equivalent 1 M sodium bis(trimethylsilyl)amide in THF at -78 °C. The cold enolate solution is stirred for 15 minutes at -78°C then treated via cannula with 2 parts allyl iodide in THF at -78 °C. The solution is stirred for 6 hours at -78°C then warmed to 0 °C and quenched with saturated ammonium chloride. The THF is removed, and the water layer is extracted 2 times with ethyl acetate. The combined washings are dried over MgSO₄, filtered and concentrated to provide the crude title compound which is purified by column chromatography eluting with ethyl acetate-hexane.

### Example 131C

### (4S,5R,2'S)-3-[2-(Cyclopropylmethyl)-2-phenyl]acetyl-4-methyl-5-phenyl-1,3-oxazolidin-2-one

One equivalent of the compound resulting from Example 131B in CH₂Cl₂ at -23 °C is treated with 5 equivalents of 1 M diethyl zinc and 10 equivalents of diiodomethane. The solution is stirred at -23 °C to 0 °C over 12 hours, quenched with saturated NH4CI, and extracted with ether. The organic layer is washed with 10% NaHCO₃ and brine, dried over MgSO₄, filtered, concentrated and chromatogtaphed eluting with ethyl acetate-hexane to provide the title compound.

### Example 131D

### (S)-α-(Cyclopropylmethyl)phenylacetic acid

To a solution of one equivalent of the compound resulting from Example 131C in 3:1 THF-H₂O at 0 °C is added 8 equivalents of 30% H₂O₂ in H₂O followed by 2 equivalents of 1 M LiOH. After stirring 2 hours at 0 °C, theTHF is removed, and the aqueous solution is extracted 3 times with CH₂Cl₂. The aqueous solution is acidified to pH 1 with 1 N HCI and extracted three times with ethyl acetate. The combined organic extracts are dried over MgSO₄, filtered and concentrated to provide the title compound.

### Example 131E

### N-t-Butyloxycarbonyl-(S)-α-(cyclopropylmethyl)benzylamine

A solution of one equivalent of the compound resulting from Example 131D in toluene is treated with bne equivalent of diphenylphosphoryl azide and warmed at 70 °C for 2 hours. The toluene is removed, and the residue is treated with 50 equivalents of 2-methyl-2-propanol and refluxed for 24 hours. The solvent is removed, and the residue is dissloved in ethyl acetate and washed with brine. The organic layer is dried over MgSO₄, filtered, concentrated, and chromatographed eluting with ethyl acetate-hexane to provide the title compound.

### Example 131F

### (S)-N-α-(Cyclopropylmethyl)benzylamine

One equivalent of the compound resulting from Example 131E in CH₂Cl₂ at 0 °C is treated with 10 equivalents of trifluoroacetic acid. The solution is stirred at room temperature for 18 hours, and all volatiles are removed *in vacuo.* The residue is dissolved in methanol and treated with 10 parts of Amberlite® IRA-400(OH) ion exchange resin. After 3 hours, the solution is filtered and concentrated to provide the title compound.

### Example 131G

### (1α,2β,3β,4α)-Di[N-(S)-α-(cyclopropylmethyl)benzyl-N-(4-phenoxybenzyl)-aminocarbonyl]cyclobutane-3,4-dicarboxylic acid

The compound resulting from Example 131F is reacted with 4-phenoxybenzaldehyde by the procedures described in Example 11. The resulting N-(S)-α-(cyclopropylmethyl)benzyl-N-(4-phenoxybenzyl)amine is reacted with 1,2,3,4-cyclobutanetetracarboxylic dianhydride by the procedures described in Example 15 to afford the title compound.

### Example 132

### (1α,2β,3β,4α)-Di[N-(R)-α-(cyclopropylmethyl)benzyl-N-(4-phenoxybenzyl)-aminocarbonyl]cyclobutane-3,4-dicarboxylic acid

The title compound is prepared by the procedures described in Example 131 substituting (4R,5S)-(+)-4-methyl-5-phenyl-2-oxazolidinone for (4S,5R)-(-)-4-methyl-5-'phenyl-2-oxazolidinone in Example 131A.

### Example 133

### (1α,2β,3β,4α)-Di[N-propyl-N-(S)-α-(cyclopropylmethyl)-(4-phenoxybenzyl)-aminocarbonyl]cyclohutane-3,4-dicarboxylic acid

### Example 133A

### (45,5R)-3-(2-(4-(Phenoxyphenyl)acetyl)-4-methyl-5-phenyl-1,3-oxazolidin-2-one

A mechanically stirred solution of 4-phenoxyphenylacetic acid at -78°C is treated with one equivalent pivaloyl chloride then one equivalent triethylamine. The mixture is stirred at -78°C for 15 minutes, at 0 °C for 45 minutes, then recooled to -78 °C. In a separate flask, one part 2.5 M nBuLi is added to a solution of (4S,5R)-(-)-4-methyl-5-phenyl-2- oxazolidinone in THF at -78 °C. The solution is stirred for 15 minutes then transferred to the flask containing pivalic anhydride via cannula. The mixture is stirred for 15 minutes at -78°C and 9 hours at room temperature. The mixture is quenched with 2 M KHSO₄ and, after evaporation of the THF, is extracted 3 times with ethyl acetate. The combined ethyl acetate extracts are dried over MgSO₄, filtered and concentrated to provide the crude title compound which is purified by column chromatography eluting with ethyl acetate-hexane.

### Example 133B

### (4S,5R,2'S)-3-[2-(2-Propenyl)-2-(4-phenoxyphenyl)]acetyl-4-methyl-5-phenyl-1,3-oxazolidin-2-one

A solution of one equivalent of the compound resulting from Example 133A in THF at -78 °C is transferred via cannula to a solution of one equivalent of 1 M sodium bis(trimethylsilyl)amide in THF at -78 °C. The cold enolate solution is stirred for 15 minutes at -78 °C then treated via cannula with 2 equivalents of allyl iodide in THF at -78 °C. The solution is stirred for 6 hours at -78 °C then warmed to 0 °C and quenched with saturated ammonium chloride. The THF is removed, and the water layer is extracted 2 times with ethyl acetate. The combined washings are dried over MgSO₄, filtered and concentrated to provide the crude title compound which is purified by column chromatography eluting with ethyl acetate-hexane.

### Example 133C

### (4S,5R,2'S)-3-[2-(Cyclopropylmethyl)-2-(4-phenoxyphenyl)]acetyl-4-methyl-5-phenyl-1,3-oxazolidin-2-one

One equivalent of the compound resulting from Example 133B in CH₂Cl₂ at -23 °C is treated with 5 equivalents of 1 M diethyl zinc and 10 equivalents of diiodomethane. The solution is stirred at -23 °C to 0 °C over 12 hours, quenched with saturated NH₄CI, and extracted with ether. The organic layer is washed with 10% NaHCO₃ and brine, dried over MgSO₄, filtered, concentrated and chromatogtaphed with ethyl acetate-hexane to provide the title compound.

### Example 133D

### (S)-α-(Cyclopropylmethyl)-4-phenoxyphenylacetic acid

To a solution of one equivalent of the compound resulting from Example 133C in 3:1 THF-H₂O at 0 °C is added 8 equivalents of 30% H₂O₂ in H₂O followed by 2 equivalents of 1 M LiOH. After stirring 2 hours at 0 °C, theTHF is removed, and the aqueous solution is extracted 3 times with CH₂Cl_{2,} The aqueous solution is acidified to pH 1 with 1 N HCI and extracted three times with ethyl acetate. The combined organic extracts are dried over MgSO₄, filtered and concentrated to provide the title compound.

### Example 133E

### N-t-Butyloxycarbonyl-(S)-α-(cyclopropylmethyl)-4-phenoxybenzylamine

A solution of one equivalent of the compound resulting from Example 133D in toluene is treated with one equivalent of diphenylphosphoryl azide and warmed at 70 °C for 2 hours. The toluene is removed, and the residue is treated with 50 equivalents of 2-methyl-2-propanol and refluxed for 24 hours. The solvent is removed, and the residue is dissloved in ethyl acetate and washed with brine. The organic layer is dried over MgSO₄, filtered, concentrated, and chromatographed eluting with ethyl acetate-hexane to provide the title compound.

### Example 133F

### (S)-N-α-(Cyclopropylmethyl)-4-phenoxybenzylamine

One equivalent of the compound resulting from Example 133E in CH₂Cl₂ at 0 °C is treated with 10 equivalents of trifluoroacetic acid. The solution is stirred at room temperature for 18 hours, and all volatiles are removed *in vacuo.* The residue is dissolved in methanol and treated with 10 parts Amberlite® IRA-400(OH) ion exchange resin. After 3 hours, the solution is filtered and concentrated to provide the title compound.

### Example 133G

### (1α,2β,3β,4α)-Di[N-propyl-N-(S)-α-(cyclopropylmethyl)-(4-phenoxybenzyl)-aminocarbonyl]cyclobutane-3,4-dicarboxylic acid

The compound resulting from Example 131F is reacted with propionaldehyde in analogy to the procedures described in Examples 1 and 2. The resulting N-propyl-N-(S)-α-(cyclopropylmethyl)-(4-phenoxybenzyl)-amine is reacted with 1,2,3,4-cyclobutanetetracarboxylic dianhydride by the procedures described in Example 13 to afford the title compound.

### Example 134

### (1α,2β,3β,4α)-Di[N-propyl-N-(R)-α-(cyclopropylmethyl)-(4-phenoxybenzyl)-aminocarbonyl]cyclobutane-3,4-dicarboxylic acid

The title compound is prepared by the procedures described in Example 133 substituting (4R,5S)-(+)-4-methyl-5-phenyl-2-oxazolidinone is substituted for (4S,5,R)-(-)-4-methy-5-phenyl-2-oxazolidinone in Example 133A.

### Example 135

### Alternate Preparation of (1α,2β,3β,4α)-Di[N-propyl-N-(4-phenoxybenzyl)carbonylamino]cyclobutane-3,4-dicarboxylic acid

A mixture of 507 mg (1.23 mmol) of the compound resulting from Example 123A in 10 mL of toluene was reacted with 5 mL of oxalyl chloride. The mixture was heated to reflux for 0.5 hour. All volatiles were removed under reduced pressure affording a pale yellow oil. The oil was dissolved in 5 mL of acetone and reacted with 168 mg (2.58 mmol) of sodium azide dissolved in 1 mL of water. After 2 hours the mixture was filtered and the volatiles were removed under reduced pressure while keeping the flask cool. The oil was dissolved in 30 mL of toluene and the solution was heated at 70 °C for 2 hours. Half of the solvent was removed under reduced pressure and 1.0 mL of 2-methyl-2-propanol and 20 mg of copper (I) chloride was added. The mixture was heated at 70 °C for 6 hours. All volatiles were removed under reduced pressure to afford a white solid. Purification by flash column chromatography on silica gel eluting with 2:1 hexane-ethyl acetate afforded 250 mg (36.7%) of white powder.

A 200 mg (0.361 mmol) sample of this powder was dissolved in 4 mL of dry DMF and was reacted sequentially with 127 mg (0.758 mmol) of allyl iodide and 30 mg (0.76 mmol) of a 60% dispersion of sodium hydride in mineral oil. After 18 hours water was added and the mixture was extracted with ethyl acetate. All volatiles were removed under reduced pressure affording an oil. Purification by flash column chromatography on silica gel eluting with 2:1 hexane-ethyl acetate afforded 110 mg (48%) of a clear oil. This oil was dissolved in 10 mL of methylene chloride and cooled to 0 °C under nitrogen, whereupon 1 mL of trifluoroacetic acid was added. After 2 hours all volatiles were removed under reduced pressure, affording 75.5 mg of crude product. This product was dissolved in 10 mL of dry tetrahydrofuran and was treated with 87.3 mg (0.382 mmol) of 4-phenoxyphenylacetic acid, 80.8 mg (0.421 mmol) of 1-(3-dimethylaminopropyl)-3-ethylcarbodiimide hydrochloride, 56.8 mg (0.421 mmol) of 1-hydroxybenzotriazole hydrate, and 128 mg (1.26 mmol) of triethylamine. After 3 days 50 mL of ethyl acetate was added and the mixture was washed with brine and dilute hydrochloric acid. All volatiles are removed under reduced pressure affording an oil. The oil was dissolved in 20 mL of ethyl acetate with 40 mg of 10% palladium on charcoal and was stirred under an atmosphere of hydrogen for two days. The mixture was filtered and purified by flash column chromatography on silica gel eluting with 40:1:1 ethyl acetate-formic acid-water to yield 6 mg of the title compound. MS (FAB) m/e 678.

### Examples 136-167

The following compounds can be prepared according to the methods described in the previous examples, particularly Examples 131, 132, 133 and 134.

| Ex. No. | Name |
|---|---|
| 136 | (1α,2β,3β,4α)-Di[N-(R)-α-ethylbenzyl-N-(4-phenoxyphenyl)aminocarbonyl]-cyclobutane-3,4-dicarboxylic acid |
| 137 | (1α,2β,3β,4α)-Di[N-(S)-α-ethylbenzyl-N-(4-phenoxyphenyl)aminocarbonyl]-cyclobutane-3,4-dicarboxylic acid |
| 138 | (1α,2β,3β,4α)-Di[N-(R)-α-propyl-(4-phenoxybenzyl)aminocarbonyl]cyclobutane- 3,4-dicarboxylic acid |
| 139 | (1α,2β,3β,4α)-Di[N-(S)-α-propyl-(4-phenoxybenzyl)aminocarbonyl]cyclobutane-3,4-dicarboxylic acid |
| 140 | (1α,2β,3β,4α)-Di[N-propyl-N-(R)-α-propyl-(4-phenoxybenzyl)aminocarbonyl]-cyclobutane-3,4-dicarboxylic acid |
| 141 | (1α,2β,3β,4α)-Di[N-propyl-N-(S)-α-propyl-(4-phenoxybenzyl)aminocarbonyl]-cyclobutane-3,4-dicarboxylic acid |
| 142 | (1α,2β,3β,4α)-Di[N-benzyl-N-(R)-α-propyl-(4-phenoxybenzyl)aminocarbonyl]-cyclobutane-3,4-dicarboxylic acid |
| 143 | (1α,2β,3β,4α)-Di[N-benzyl-N-(S)-α-propyl-(4-phenoxybenzyl)aminocarbonyl]-cyclobutane-3,4-dicarboxylic acid |
| 144 | (1α,2β,3β,4α)-Di[N-(cyclopropylmethyl)-N-(R)-α-propyl-(4-phenoxybenzyl)-aminocarbonyl]cyclobutane-3,4-dicarboxylic acid |
| 145 | (1α,2β,3β,4α)-Di(N-(cyclopropylmethyl)-N-(S)-α-propyl-(4-phenoxybenzyl)-aminocarbonyl]cyclobutane-3,4-dicarboxylic acid |
| 146 | (1α,2β,3β,4α)-Di[N-(R)-α-ethyl-(4-phenoxybenzyl)aminocarbonyl]cyclobutane-3,4-dicarboxylic acid |
| 147 | (1α,2β,3β,4α)-Di[N-(S)-α-ethyl-(4-phenoxybenzyl)aminocarbonyl]cyclobutane-3,4-dicarboxylic acid |
| 148 | (1α,2β,3β,4α)-Di[N-propyl-N-(R)-α-ethyl-N-(4-phenoxybenzyl)aminocarbonyl]-cyclobutane-3,4-dicarboxylic acid |
| 149 | (1α,2β,3β,4α)-Di[N-propyl-N-(S)-α-ethyl-N-(4-phenoxybenzyl)aminocarbonyl]-cyclobutane-3,4-dicarboxylic acid |
| 150 | (1α,2β,3β,4α)-Di[N-benzyl-N-(R)-α-ethyl-N-(4-phenoxybenzyl)aminocarbonyl]-cyclobutane-3,4-dicarboxylic acid |
| 151 | (1α,2β,3β,4α)-Di[N-benzyl-N-(S)-α-ethyl-N-(4-phenoxybenzyl)aminocarbonyl]-cyclobutane-3,4-dicarboxylic acid |
| 152 | (1α,2β,3β,4α)-Di[N-(cyclopropylmethyl)-N-(R)-α-ethyl-(4-phenoxybenzyl)- aminocarbonyl]cyclobutane-3,4-dicarboxylic acid |
| 153 | (1α,2β,3β,4α)-Di[N-(cyclopropylmethyl)-N-(S)-α-ethyl-(4-phenoxybenzyl)-aminocarbonyl]cyclobutane-3,4-dicarboxylic acid |
| 154 | (1α,2β,3β,4α)-Di[N-(R)-α-methyl-(4-phenoxybenzyl)aminocarbonyl]cyclobutane-3,4-dicarboxylic acid |
| 155 | (1α,2β,3β,4α)-Di[N-(S)-α-methyl-(4-phenoxybenzyl)aminocarbonyl]cyclobutane-3,4-dicarboxylic acid |
| 156 | (1α,2β,3β,4α)-Di[N-propyl-N-(R)-α-methyl-(4-phenoxybenzyl)aminocarbonyl]-cyclobutane-3,4-dicarboxylic acid |
| 157 | (1α,2β,3β,4α)-Di[N-propyl-N-(S)-α-methyl-(4-phenoxybenzyl)aminocarbonyl]-cyclobutane-3,4-dicarboxyl acid |
| 158 | (1α,2β,3β,4α)-Di[N-benzyl-N-(R)-α-methyl-(4-phenoxybenzy)aminocarbonyl]-cyclobutane-3,4-dicarboxylic acid |
| 159 | (1α,2β,3β,4α)-Di[N-benzyl-N-(S)-α-methyl-(4-phenoxybenzyl)aminocarbonyl]-cyclobutane-3,4-dicarboxylic acid |
| 160 | (1α,2β,3β,4α)-Di[N-(cyclopropylmethyl)-N-(R)-α-methyl-(4-phenoxybenzyl)-aminocarbonyl]cyclobutane-3,4-dicarboxylic acid |
| 161 | (1α,2β,3β,4α)--Di[N-(cyclopropylmethyl)-N-(S)-α-methyl-(4-phenoxybenzyl)-aminocarbonyl]cyclobutane-3,4-dicarboxylic acid |
| 162 | (1α,2β,2β,4α)-Di[N-(R)-α-(cyclopropylmethyl)-(4-phenoxybenzyl)amino-carbonyl]cyclobutane-3,4-dicarboxylic acid |
| 163 | (1α,2β,3β,4α)-Di[N-(S)-α-(cyclopropylmethyl)-(4-phenoxybenzyl)amino-carbonyl]cyclobutane-3,4-dicarboxylic acid |
| 164 | (1α,2β,3β,4α)-Di[N-benzyl-N-(R)-α-(cyclopropylmethyl)-(4-phenoxybenzyl)-aminocarbonyl]cyclobutane-3,4-dicarboxylic acid |
| 165 | (1α,2β,3β,4α)-Di[N-benzyl-N-(S)-α-(cyclopropylmethyl)-(4-phenoxybenzyl)-aminocarbonyl]cyclobutane-3,4-dicarboxylic acid |
| 166 | (1α,2β,3β,4α)-Di[N-(cyclopropylmethyl)-N-(R)-α-(cyclopropylmethyl)-(4-phenoxybenzyl)aminocarbonyl]cyclobutane-3,4-dicarboxylic acid |
| 167 | (1α,2β,3β,4α)-Di[N-(cyclopropylmethyl)-N-(S)-α-(cyclopropylmethyl)-(4-phenoxybenzyl)aminocarbonyl]cyclobutane-3,4-dicarboxylic acid |

### Inhibition of Squalene Synthetase

In vitro inhibition of squalene synthetase may be measured by the following procedure.

Rat liver microsomal squalene synthetase activity was measured using farnesyl pyrophosphate as a substrate and quantitating squalene synthesis using labelled farnesyl pyrophosphate and counting the squalene formed.

Rat liver microsome, the source of enzyme, was prepared according to the method of Gillies, P. J., et al., Exp. Molc. Pathol. 44: 329-339 (1986), a modification of the procedure of Erickson, S. K., and Cooper, A. D., Metabolism, 29: 991-996 (1980). Approximately 30 µg of microsomal protein was incubated for 10 minutes at 37 °C with 11 µmol of ³H-famesyl pyrophosphate, 49 mCi/mmol, and at least three concentrations of test compound in the presence of squalene (2 µΛ) (Mg⁺⁺, KF, reduced B-nicotinamide adenine dinucleotide phosphate, dithiothreitol, and K₂PO₄, PH 7.35, in a total volume of 200 µΛ. Oxygen was excluded from the closed incubation tube by degassing with nitrogen. The reaction was terminated by the addition of ethanolic KOH and after degassing with N₂, the microsomal membranes were solubilized by heating at 60 °C for 30 minutes. The squalene was extracted into hexane, and the squalene was separated from all other radioactive molecules by passage over an activated alumina column. The solution was collected in scintillation vials, evaporated to dryness, liquid scintillation fluid was added, and the radioactivity was determined in a liquid scintillation counter. The per cent inhibition at a dose of 10 µM (or 1 µM) compared to controls with no test compound was determined. The % inhibition values for the compounds of the invention are shown in Table 1. The data show that compounds of the invention are inhibitors of squalene synthetase.

**Table 1**

| In vitro Inhibition of Squalene Synthetase | | | |
|---|---|---|---|
| Ex. No. | % Inhibition at 10 µM | Ex. No. | % Inhibition at 10 µM |
| 13 | 54 | 14 | 31 |
| 15 | 99 | 16 | 40* |
| 17 | 88* | 18 | 55 |
| 19 | 86 | 20 | 50 |
| 21 | 52 | 22 | 20 |
| 23 | 65* | 24 | 77* |
| 25 | 84 | 26 | 89* |
| 27 | 79 | 28 | 91 |
| 29 | 72* | 30 | 67 |
| 31 | 37* | 34 | 89 |
| 35 | 59* | 36 | 63 |
| 37B | 91 | 38H | 51 |
| 39C | 59 | 40B | 82 |
| 41B | 72* | 42 | 81* |
| 43 | 47* | 44 | 56* |
| 45 | 50* | 47B | 87 |
| 48 | 79* | 53B | 63* |
| 54B | 74 | 55 | 78 |
| 56 | 86 | 57 | 64 |
| 58 | 63 | 59 | 50 |
| 60 | 65* | 61B | 43 |
| 62B | 83 | 63 | 90 |
| 64D | 45 | 65B | 58 |
| 66C | 33 | 67C | 87 |
| 68B | 84* | 69E | 56* |
| 70B | 80 | 71C | 77 |
| 72C | 51* | 77B | 51* |
| 74D | 55* | 79B | 83 |
| 76B | 57* | 82B | 67 |
| 78B | 82* | 84G | 89 |
| 81B | 73 | 86D | 86 |
| 83B | 85 | 88 | 86 |
| 85 | 56* | 90 | 53 |
| 87B | 72 | 92B | 80* |
| 89C | 88 | 96B | 76 |
| 91 | 62 | 98C | 84 |
| 95 | 78 | 100C | 84 |
| 97 | 72 | 102 | 58 |
| 99B | 80 | 104A | 70* |
| 101C | 86* | 105A | 48* |
| 103B | 90 | 106 | 92 |
| 104B | 71* | 108 | 59* |
| 105B | 57* | 110A | 73* |
| 107 | 75* | 111 | 49* |
| 109 | 78* | 113 | 31* |
| 110B | 69* | 116 | 79 |
| 112 | 21* | 118B | 51 |
| 115B | 75 | 120 | 54 |
| 117 | 53* | 122B | 57 |
| 119C | 45* | 127C | 68* |
| 121 | 44 | 128F | 80* |
| 73B | 75* | 129B | 92* |
| 75B | 73* | 130B | 48* |

| | | | |
|---|---|---|---|
| * % Inhibition at 1 µM | | | |

### In vivo Inhibition of Cholesterol Synthesis

Monkeys, Cynomolgus, 2 males and 2 females per group, Controls: 3.7-6.1 Kg body weight, Dosed animals: 4.8-5.6 Kg body weight) were fasted overnight and bled in the morning. Plasma samples were prepared and analyzed for total cholesterol, HDL-cholesterol and triglycerides. The treated animals were dosed with the product of Example 17, 20 mg/kg, po, while control monkeys were dosed with vehicle containing 0.2% Methocel (hydroxypropylmethyl cellulose) in water. Dosing was continued daily for a total of 5 days. Prior to the last dose the animals were fasted overnight and bled after dosing but before feeding. Plasma samples (anticoagulated with EDTA) were prepared and analyzed as before. One-sample t-tests were calculated to test for significant change within a group across the experiment. Total plasma cholesterol was lowered an average of 15.5% in the monkeys that received the product of Example 17.

### Inhibition of Protein Farnesyltransferase

In vitro inhibition of protein farnesyltransferase can be measured by the following procedure. (Procedures for determination of the inhibition of farnesylation of the oncogene protein Ras are described by Goldstein, et al., J. Biol. Chem., 266:15575-15578 (1991) and by Singh in United States patent No. 5245061.)

Rat brain protein farnesyltransferase activity is measured using the biotin-lamin substrate (which is known to undergo farnesylation in a manner analogous to Ras protein) and radioactive farnesyl diphosphate provided by Amersham Life Science in their commercial scintillation proximity assay kit for the determination of farnesyltransferase. The enzyme is purified according to Reiss, Y., et al., Cell, 62: 81-88 (1990), utilizing steps one through three. The specific activity of the enzyme used is approximately 10 nmol substrate farnesylated/mg enzyme/hour. The percent inhibition of the farnesylation caused by the compounds of the invention (at 1 x 10⁻⁵ M) compared to an uninhibited control sample was evaluated in the same Amersham test system.

The ability of the compounds of the invention to treat carcinoma can be demonstrated according to the methods of Mazerska Z., Woynarowska B., Stefanska B., Borowski S., Drugs Exptl. Clin. Res. 13(6), 345-351 (1987); Bissery, MC, Guenard F, Guerritte-Voegelein F, Lavelle F., Cancer Res. 51, 4845-4852 (1991); and Rygaard J, and Povlsen C., Acta Pathol. Microbiol. Scand. 77, 758 (1969).

The ability of the compounds of the invention to treat fungal infections can be demonstrated according to the method described by S. Shadomy and M.A. Pfaller. 1991. Laboratory Studies with Antifungal Agents: Susceptibility Tests and Quantitation in Body Fluids, pp. 1173-1183. In A.Balows, W.J.Hausler,Jr., K.L.Herrmann, H.lsenberg and H.J.Shadomy, Eds. Manual of Clinical Microbiology, 5th Ed. American Society for Microbiology, Washington, D.C. The antifungal activity of squalene synthetase inhibitors has been reported by a number of researchers including Dufresne et al. Tetrahedron 48/47 10221-10226 (1992) and Dawson, M.J., et al. J. Antibiot. (Tokyo) 45: 639-647 (1992).

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxy-ethanesulfonate, lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, p-toluenesulfonate and undecanoate. Also, the basic nitrogen-containing groups can be quaternized with such agents as loweralkyl halides (such as methyl, ethyl, propyl, and butyl chloride, bromides, and iodides), dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Basic addition salts can be prepared *in situ* during the final isolation and purification of the compounds of formula (I), or separately by reacting the carboxylic acid function with a suitable base such as the hydroxide, carbonate or bicarbonate of a pharmaceutically acceptable metal cation or with ammonia, or an organic primary, secondary or tertiary amine. Pharmaceutically acceptable salts include, but are not limited to, cations based on the alkali and alkaline earth metals, such as sodium, lithium, potassium, calcium, magnesium, aluminum salts, as well as nontoxic ammonium, quaternary ammonium, and amine cations, including, but not limited to ammonium, tetramethylammonium, tetraethylammonium, methylamine, dimethylamine, trimethylamine, triethylamine, ethylamine. Other representative organic amines useful for the formation of base addition salts include diethylamine, ethylenediamine, ethanolamine, diethanolamine, piperazine.

The compounds of the invention are useful (in humans and other mammals) for treating atherosclerosis to inhibit progression of the disease or hyperlipidemia to inhibit the development of atherosclerosis or treating fungal infections.

The compounds of the invention are useful (in humans and other mammals) for inhibiting protein farnesyltransferase and the farnesylation of Ras. These inhibitors of protein farnesyltransferase are also useful as antitiumor agents for treating cancer in humans and other mammals. Examples of the kinds of cancers which may be treated with the compounds of the invention include, but are not limited to, lung carcinoma, colorectal carcinoma, exocrine pancreatic carcinoma and myeloid leukemias.

For inhibition of squalene synthetase, the total daily dose administered to a host in single or divided doses may be in amounts, for example, from 0.001 to 1000 mg/kg body weight daily and more preferred from 1.0 to 30 mg/kg body weight daily. Dosage unit compositions may contain such amounts of submultiples thereof to make up the daily dose.

For use as a chemotherapeutic agent, the total daily dose administered to a host in single or divided doses may be in amounts, for example, from 0.01 to 500 mg/kg body weight daily, preferably in amounts from 0.1 to 20 mg/kg body weight daily and more preferably in amounts from 0.5 to 10 mg/kg body weight daily. Dosage unit compositions may contain such amounts of submultiples thereof to make up the daily dose.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

The compounds of the present invention may be administered orally, parenterally, sublingually, by inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or iontophoresis devices. The term parenteral as used herein includes subcutaneous injections, intravenous, intramuscular, intrasternal injection, or infusion techniques.

Injectable preparations, for example, sterile injectable aqueous or oleagenous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-propanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose, lactose, or starch. Such dosage forms may also comprise, as is normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

The compounds of the present invention can also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically aceptable and metabolizable lipid capable of forming liposomes can be used. The present compositions in liposome form can contain, in addition to a compound of the present invention, stabilizers, preservatives, excipients. The preferred lipids are the phospholipids and phosphatidyl cholines (lecithins), both natural and synthetic.

Methods to form liposomes are known in the art. See, for example, Prescott, Ed., Methods in Cell Biology, Volume XIV, Academic Press, New York, N.Y. (1976), p. 33 et seq.

While the compounds of the invention can be administered as the sole active pharmaceutical agent, they can also be used in combination with one or more other cardiovascular agents independently selected from HMG CoA reductase inhibitors, antihyperlipoproteinemic agents and serum cholesterol lowering agents.

Representative HMG CoA reductase inhibitors include lovastatin, pravastatin, velostatin, simvastatin.

Representative antihyperlipoproteinemic agents include probucol and the like.

Representative serum cholesterol lowering agents include Lopid®, gemfibrozil), bile acid sequestrants such as cholestyramine, colestipol, polidexide, DEAE-Sephadex, clofibrate, nicotinic acid and its derivatives, neomycin, p-aminosalicylic acid, bezafibrate.

The above compounds to be employed in combination with the squalene synthetase inhibitor of the invention will be used in therapeutic amounts as indicated in the Physicians' Desk Reference (PDR) 47th Edition (1993), or such therapeutically useful amounts as would be known to one of ordinary skill in the art.

The compounds of the invention and the other cardiovascular agent can be administered at the recommended maximum clinical dosage or at lower doses. Dosage levels of the active compounds in the compositions of the invention may be varied so as to obtain a desired therapeutic response depending on the route of administration, severity of the disease and the response of the patient. The combination can be administered as separate compositions or as a single dosage form containing both agents.

When administered as a combination, the therapeutic agents can be formulated as separate compositions which are given at the same time or different times, or the therapeutic agents can be given as a single composition.

The foregoing is merely illustrative of the invention and is not intended to limit the invention to the disclosed compounds.

## Claims

1. A compound of the formula: 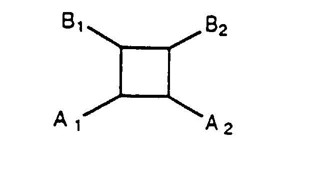 wherein
A₁ and A₂ are independently selected from
(1) -X-C(O)-G or -X-C(S)-G wherein at each occurrence X is independently selected from (a) a covalent bond, (b) -CH₂-, (c) -O-, (d) -S- and (e) -N(Rₐ)- wherein Rₐ is hydrogen, loweralkyl, cycloalkyl or cycloalkylalkyl and at each occurrence G is independently selected from -R₂, -N(R₁)(R₂), -OR₂ and -SR₂ wherein at each occurrence R₁ is independently selected from (a) hydrogen, (b) loweralkyl, (c) alkenyl, (d) alkynyl, (e) cycloalkyl, (f) cycloalkylalkyl, (g) alkoxycarbonylalkyl, (h) alkoxyalkyl, (i) thioalkoxyalkyl, (j) haloalkyl, (k) aryl, (I) heterocyclic, (m) arylalkyl, (n) aryl-substituted cycloalkylalkyl, (o) (heterocyclic)alkyl, (p) heterocyclic-substituted cycloalkylalkyl and (q) aryl, heterocyclic, arylalkyl, aryl-substituted cycloalkylalkyl, (heterocyclic)alkyl or heterocyclic-substituted cycloalkylalkyl wherein the aryl group, the aryl part of the arylalkyl group, the aryl part of the aryl-substituted cycloalkylalkyl group, the heterocyclic group, the heterocyclic part of the (heterocyclic)alkyl group or the heterocyclic part of the heterocyclic-substituted cycloalkylalkyl group is substituted with -Y-R₃ wherein at each occurrence Y is independently selected from (i) a covalent bond, (ii) -C(O)-, (iii) -CH₂-, (iv) -O-, (v) -S(O)ₘ- wherein m is 0, 1 or 2, (vi) -N(R_{b})- wherein R_{b} is hydrogen or loweralkyl, (vii) -CH₂O-, (viii) -CH₂S(O)ₘ- wherein m is 0, 1 or 2 and (ix) -CH₂N(R_{b})- wherein R_{b} is hydrogen or I oweralkyl and at each occurrence R₃ is independently selected from (i) aryl, (ii) arylalkyl, (iii) cycloalkyl, (iv) cycloalkylalkyl, (v) heterocyclic and (vi) (heterocyclic)alkyl and at each occurrence R₂ is independently selected from (i) alkenyl, (ii) alkynyl, (iii) aryl, (iv) arylalkyl, (v) arylalkenyl, (vi) heterocyclic, (vii) (heterocyclic)alkyl and (vi) aryl, heterocyclic, arylalkyl or (heterocyclic)alkyl wherein the aryl group, the aryl part of the arylalkyl group, the heterocyclic group or the heterocyclic part of the (heterocyclic)alkyl group is substituted with -Z-R₄ wherein at each occurrence Z is independently selected from (i) a covalent bond, (ii) -C(O)-, (iii) -CH₂-, (iv) -O-, (v) -S(O)ₚ- wherein p is 0, 1 or 2, (vi) -N(R_{c})- wherein R_{c} is hydrogen or loweralkyl, (vii) -CH₂O-, (viii) -CH₂S(O)ₚ-. wherein p is 0, 1 or 2 and (ix) -CH₂N(R_{c})- wherein R_{c} is hydrogen or loweralkyl and at each occurrence R₄ is independently selected from (i) aryl, (ii) arylalkyl, (iii) cycloalkyl, (iv) cycloalkylalkyl, (v) heterocyclic and (vi) (heterocyclic)alkyl,
wherein, when R₃ or R₄ is a heterocyclic, (heterocyclic)alkyl, aryl or arylalkyl group, the heterocyclic group or the heterocyclic part of the (heterocyclic)alkyl group may be optionally substituted with one or two substituents independently selected from hydroxy, halo, oxo, loweralkylimino, amino, alkylamino, dialkylamino, alkoxy, alkoxyalkoxy, haloalkyl, cycloalkyl, aryl, arylalkyl, -COOH,-SO₃H and loweralkyl, and a nitrogen-containing heterocycle may be N-protected, and the aryl group or the aryl part of the arylalkyl group may be optionally substituted with one, two or three substituents independently selected from loweralkyl, haloalkyl, alkoxy, thioalkoxy, amino, alkylamino, dialkylamino, hydroxy, halo, mercapto, nitro, carboxaldehyde, carboxy, alkoxycarbonyl and carboxamide, or the aryl group or the aryl part of the arylalkyl group is tetrafluorophenyl or pentafluorophenyl, and
(2) -(CH₂)_{q}-N(R₁)R₂) wherein q is 0, 1 or 2 and at each occurrence R₁ and R₂ are independently defined as above; and
B₁ and B₂ are independently selected from
(1) -CH₂OH,
(2) -CH=NOH,
(3) -W-R₅ wherein at each occurrence W is independently selected from (a) a covalent bond, (b) alkylene, (c) alkenylene, (d) -C(O)NH- and (e) -NHC(O)NH- and R₅ is independently selected from
(a) 5-tretrazolyl,
(b) 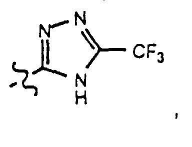
(c) 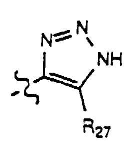 wherein R₂₇ is -ON, -NO₂, or -CO₂R₂₈ wherein R₂₈ is hydrogen, aryl or loweralkyl,
wherein the aryl group may be optionally substituted with one, two or three substituents independently selected from loweralkyl, haloalkyl, alkoxy, thioalkoxy, amino, alkylamino, dialkylamino, hydroxy, halo, mercapto, nitro, carboxaldehyde, carboxy, alkoxycarbonyl and carboxamide, or the aryl group may be tetrafluorophenyl or pentafluorophenyl,
(d) 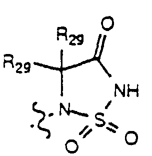 wherein at each occurrence R₂₉ is selected from hydrogen and loweralkyl,
(e) 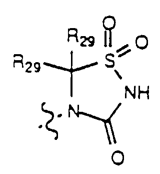 wherein R₂₉ is as defined above,
(f) 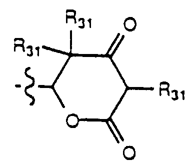 wherein at each occurrence R₃₁ is selected from hydrogen, loweralkyl, alkenyl, alkoxyalkyl and benzyl,
(g) 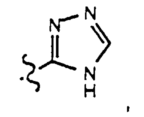
(h) 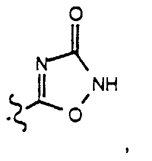
(i) 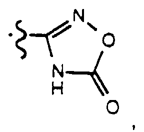
(j) 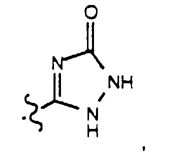
(k) 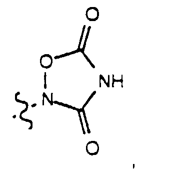
(l) 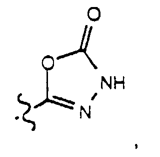
(m) 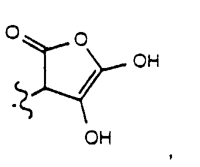 and
(n) 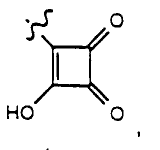
(4) -Q-C(O)R₆ wherein at each occurrence Q is independently selected from (a) a covalent bond, (b) alkylene, (c) alkenylene, (d) -CH(OH)- and (e) -NHC(O)(CH₂)ᵣ- wherein r is 0 to 4 and at each occurrence R₆ is independently selected from (a) -OR₇ wherein R₇ is hydrogen or a carboxy-protecting group, (b) -NH₂, (c) -NHOH, (d) -NHSO₂CF₃ (e) an alpha-amino acid or a beta-amino acid which is bonded via the alpha- or beta-amino group and (f) a di-, tri- or tetra-peptide which is bonded via the amino terminal amino group,
(5) -CH₂-N(OH)-C(O)-R₂₅ wherein R₂₅ is hydrogen, methyl or trifluoromethyl, and
(6) -C(O)-NH-S(O)₂-R₂₆ wherein R₂₆ is aryl, heterocyclic, arylalkyl, (heterocyclic)alkyl, C₃-C₇-cycloalkyl, C₁-C₈-alkyl or perfluoro-C₁-C₄-alkyl,
wherein the heterocyclic group or the heterocyclic part of the (heterocyclic)alkyl group may be optionally substituted with one or two substituents independently selected from hydroxy, halo, oxo, loweralkylimino, amino, alkylamino, dialkylamino, alkoxy, alkoxyalkoxy, haloalkyl, cycloalkyl, aryl, arylalkyl, -COOH, -SO₃H and loweralkyl, and a nitrogen-containing heterocycle may be N-protected, and the aryl group or the aryl part of the arylalkyl group may be optionally substituted with one, two or three substituents independently selected from loweralkyl, haloalkyl, alkoxy, thioalkoxy, amino, alkylamino, dialkylamino, hydroxy, halo, mercapto, nitro, carboxaldehyde, carboxy, alkoxycarbonyl and carboxamide, or the aryl group or the aryl part of the arylalkyl group is tetrafluorophenyl or pentafluorophenyl;
or a pharmaceutically acceptable salt thereof.

2. A compound as defined by Claim 1 wherein
A₁ and A₂ are independently selected from -C(O)-G wherein G is defined as above and
B₁ and B₂ are independently selected from
(a) -W-R₅ wherein W is a covalent bond or alkylene and R₅ is 5-tetrazolyl and
(b) -Q-C(O)-R₆ wherein at each occurrence Q is independently selected from a covalent bond and alkylene and at each occurrence R₆ is independently selected from
(1) -OR₇ wherein R₇ is hydrogen or a carboxy-protecting group,
(2) an alpha-amino acid or a beta-amino acid which is bonded via the alpha- or beta-amino group and
(3) a di-, tri- or tetra-peptide which is bonded via the amino terminal amino group.

3. A compound as defined by Claim 1 wherein
A₁ and A₂ are independently selected from -C(O)-G wherein G is -N(R₁)(R₂) wherein R₁ and R₂ are defined as above and
B₁ and B₂ are independently selected from
(a) -W-R₅ wherein W is a covalent bond or alkylene and R₅ is 5-tetrazolyl and
(b) -Q-C(O)-R₆ wherein at each occurrence Q is independently selected from a covalent bond and alkylene and at each occurrence R₆ is independently selected from
(1) -OR₇ wherein R₇ is hydrogen or a carboxy-protecting group,
(2) an alpha-amino acid or a beta-amino acid which is bonded via the alpha- or beta-amino group and
(3) a di-, tri- or tetra-peptide which is bonded via the amino terminal amino group.

4. A compound as defined by Claim 1 wherein
A₁ and A₂ are independently selected from -C(O)-G wherein G is -N(R₁)(R₂) wherein at each occurrence R₁ is independently selected from (a) hydrogen, (b) loweralkyl, (c) cycloalkyl, (d) cycloalkylalkyl, (e) alkoxyalkyl, (f) thioalkoxyalkyl, (g) aryl, (h) heterocyclic, (i) arylalkyl, (j) (heterocyclic)alkyl, (k) aryl-substituted cycloalkylalkyl, (I) heterocyclic-substituted cycloalkylalkyl and (m) aryl, heterocyclic, arylalkyl, (heterocyclic)alkyl, aryl-substituted cycloalkylalkyl or heterocyclic-substituted cycloalkylalkyl wherein the aryl group, the aryl part of the arylalkyl group, the aryl part of the aryl-substituted cycloalkylalkyl group, the heterocyclic group, the heterocyclic part of the (heterocyclic)alkyl group or the heterocyclic part of the heterocyclic-substituted cycloalkylalkyl group is substituted with -Y-R₃ wherein at each occurrence Y is independently selected from (i) -O-, (ii) -S(O)ₘ- wherein m is 0, 1 or 2 and (iii) -N(R_{b})- wherein R_{b} is hydrogen or loweralkyl and at each occurrence R₃ is independently selected from (i) aryl, (ii) arylalkyl, (iii) cycloalkyl, (iv) cycloalkylalkyl, (v) heterocyclic and (vi) (heterocyclic)alkyl and at each occurrence R₂ is independently selected from aryl, heterocyclic, arylalkyl and (heterocyclic)alkyl wherein the aryl group, the aryl part of the arylalkyl group, the heterocyclic group or the heterocyclic part of the (heterocyclic)alkyl group is substituted with -Z-R₄ wherein at each occurrence Z is independently selected from (i) -O-, (ii) -S(O)ₚ- wherein p is 0, 1 or 2 and (iii) -N(R_{c})- wherein R_{c} is hydrogen or loweralkyl and at each occurrence R₄ is independently selected from (i) aryl, (ii) arylalkyl, (iii) cycloalkyl, (iv) cycloalkylalkyl, (v) heterocyclic and (vi) (heterocyclic)alkyl, wherein, when R₃ or R₄ is a heterocyclic, (heterocyclic)alkyl, aryl or arylalkyl group, the heterocyclic group or the heterocyclic part of the (heterocyclic)alkyl group may be optionally substituted with one or two substituents independently selected from hydroxy, halo, oxo, loweralkylimino, amino, alkylamino, dialkylamino, alkoxy, alkoxyalkoxy, haloalkyl, cycloalkyl, aryl, arylalkyl, -COOH,-SO₃H and loweralkyl, and a nitrogen-containing heterocycle may be N-protected, and the aryl group or the aryl part of the arylalkyl group may be optionally substituted with one, two or three substituents independently selected from loweralkyl, haloalkyl, alkoxy, thioalkoxy, amino, alkylamino, dialkylamino, hydroxy, halo, mercapto, nitro, carboxaldehyde, carboxy, alkoxycarbonyl and carboxamide, or the aryl group or the aryl part of the arylalkyl group is tetrafluorophenyl or pentafluorophenyl, and
B₁ and B₂ are independently selected from
(a) -W-R₅ wherein W is a covalent bond or alkylene and R₅ is 5-tetrazolyl and
(b) -Q-C(O)-R₆ wherein at each occurrence Q is independently selected from a covalent bond and alkylene and at each occurrence R₆ is independently selected from
(1) -OR₇ wherein R₇ is hydrogen or a carboxy-protecting group,
(2) an alpha-amino acid or a beta-amino acid which is bonded via the alpha- or beta-amino group and
(3) a di-, tri- or tetra-peptide which is bonded via the amino terminal amino group.

5. A compound as defined by Claim 1 wherein
A₁ and A₂ are independently selected from -C(O)-G wherein G is -N(R₁)(R₂) wherein at each occurrence R₁ is independently selected from (a) loweralkyl, (b) cycloalkyl and (c) cycloalkylalkyl and at each occurrence R₂ is independently selected from aryl and arylalkyl wherein the aryl group or the aryl part of the arylalkyl group is substituted with -Z-R₄ wherein at each occurrence Z is independently selected from (i) -O- and (ii) -S- and at each occurrence R₄ is independently selected from (i) aryl, (ii) arylalkyl, (iii) cycloalkyl, (iv) cycloalkylalkyl, (v) heterocyclic and (vi) (heterocyclic)alkyl, wherein the heterocyclic group or the heterocyclic part of the (heterocyclic)alkyl group may be optionally substituted with one or two substituents independently selected from hydroxy, halo, oxo, loweralkylimino, amino, alkylamino, dialkylamino, alkoxy, alkoxyalkoxy, haloalkyl, cycloalkyl, aryl, arylalkyl, -COOH,-SO₃H and loweralkyl, and a nitrogen-containing heterocycle may be N-protected, and the aryl group or the aryl part of the arylalkyl group may be optionally substituted with one, two or three substituents independently selected from loweralkyl, haloalkyl, alkoxy, thioalkoxy, amino, alkylamino, dialkylamino, hydroxy, halo, mercapto, nitro, carboxaldehyde, carboxy, alkoxycarbonyl and carboxamide, or the aryl group or the aryl part of the arylalkyl group is tetrafluorophenyl or pentafluorophenyl, and
B₁ and B₂ are independently selected from -W-R₅ and -Q-C(O)-R₆ wherein at each occurrence Q and W are independently selected from a covalent bond and alkylene, R₆ is -OR₇ wherein R₇ is hydrogen or a carboxy-protecting group and R₅ is 5-tetrazolyl.

6. A compound as defined by Claim 1 wherein
A₁ and A₂ are independently selected from -C(O)-G wherein G is -N(R₁)(R₂) wherein at each occurrence R₁ is independently selected from
(a) loweralkyl, (b) cycloalkyl and (c) cycloalkylalkyl and at each occurrence R₂ is independently selected from phenyl and benzyl wherein the phenyl group or the phenyl ring of the benzyl group is substituted with -Z-R₄ wherein at each occurrence Z is independently selected from (i) -O- and (ii) -S- and at each occurrence R₄ is independently selected from (i) aryl, (ii) arylalkyl, (iii) heterocyclic and (iv) (heterocyclic)alkyl, wherein the heterocyclic group or the heterocyclic part of the (heterocyclic)alkyl group may be optionally substituted with one or two substituents independently selected from hydroxy, halo, oxo, loweralkylimino, amino, alkylamino, dialkylamino, alkoxy, alkoxyalkoxy, haloalkyl, cycloalkyl, aryl, arylalkyl, -COOH,-SO₃H and loweralkyl, and a nitrogen-containing heterocycle may be N-protected, and the aryl group or the aryl part of the arylalkyl group may be optionally substituted with one, two or three substituents independently selected from loweralkyl, haloalkyl, alkoxy, thioalkoxy, amino, alkylamino, dialkylamino, hydroxy, halo, mercapto, nitro, carboxaldehyde, carboxy, alkoxycarbonyl and carboxamide, or the aryl group or the aryl part of the arylalkyl group is tetrafluorophenyl or pentafluorophenyl, and
B₁ and B₂ are independently selected from -W-R₅ and -Q-C(O)-R₆ wherein at each occurrence Q and W are independently selected from a covalent bond and alkylene, R₆ is -OR₇ wherein R₇ is hydrogen or a carboxy-protecting group and R₅ is 5-tetrazolyl.

7. A compound as defined by Claim 1 wherein
A₁ and A₂ are independently selected from -C(O)-G wherein G is -N(R₁)(R₂) wherein at each occurrence R₁ is independently selected from (a) loweralkyl, (b) cycloalkyl and (c) cycloalkylalkyl and R₂ is benzyl wherein the phenyl ring of the benzyl group is substituted with -Z-R₄ wherein at each occurrence Z is independently selected from (i) -O- and (ii) -S- and R₄ is aryl and
B₁ and B₂ are independently selected from -W-R₅ and -Q-C(O)-R₆ wherein at each occurrence Q and W are independently selected from a covalent bond and alkylene, R₆ is -OR₇ wherein R₇ is hydrogen or a carboxy-protecting group and R₅ is 5-tetrazolyl.

8. A compound as defined by Claim 1 wherein
A₁ and A₂ are independently selected from -C(O)-G wherein G is -N(R₁)(R₂) wherein at each occurrence R₁ is (a) loweralkyl, (b) cycloalkyl and (c) cycloalkylalkyl and R₂ is benzyl wherein the phenyl ring of the benzyl group is substituted with -Z-R₄ wherein at each occurrence Z is independently selected from (i) -O- and (ii) -S- and R₄ is heterocyclic, wherein the heterocyclic group may be optionally substituted with one or two substituents independently selected from hydroxy, halo, oxo, loweralkylimino, amino, alkylamino, dialkylamino, alkoxy, alkoxyalkoxy, haloalkyl, cycloalkyl, aryl, arylalkyl, -COOH, -SO₃H and loweralkyl, and a nitrogen-containing heterocycle may be N-protected, and the aryl group or the aryl part of the arylalkyl group may be optionally substituted with one, two or three substituents independently selected from loweralkyl, haloalkyl, alkoxy, thioalkoxy, amino, alkylamino, dialkylamino, hydroxy, halo, mercapto, nitro, carboxaldehyde, carboxy, alkoxycarbonyl and carboxamide, or the aryl group or the aryl part of the arylalkyl group is tetrafluorophenyl or pentafluorophenyl, and
B₁ and B₂ are independently selected from -W-R₅ and -Q-C(O)-R₆ wherein at each occurrence Q and W are independently selected from a covalent bond and alkylene, R₆ is -OR₇ wherein R₇ is hydrogen or a carboxy-protecting group and R₅ is 5-tetrazolyl.

9. The compound as defined by Claim 1 of the formula: 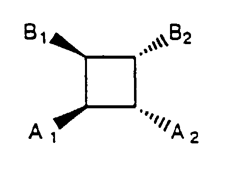
wherein A₁ and A₂ are independently selected from
(1) -X-C(O)-G or -X-C(S)-G wherein at each occurrence X is independently selected from (a) a covalent bond, (b) -CH₂-, (c) -O-, (d) -S- and (e) -N(Rₐ)- wherein Rₐ is hydrogen, loweralkyl, cycloalkyl or cycloalkylalkyl and at each occurrence G is independently selected from -R₂, -N(R₁)(R₂), -OR₂ and -SR₂ wherein at each occurrence R₁ is independently selected from (a) hydrogen, (b) loweralkyl, (c) alkenyl, (d) alkynyl, (e) cycloalkyl, (f) cycloalkylalkyl, (g) alkoxycarbonylalkyl, (h) alkoxyalkyl, (i) thioalkoxyalkyl, (j) haloalkyl, (k) aryl, (I) heterocyclic, (m) arylalkyl, (n) aryl-substituted cycloalkylalkyl, (o) (heterocyclic)alkyl, (p) heterocyclic-substituted cycloalkylalkyl and (q) aryl, heterocyclic, arylalkyl, aryl-substituted cycloalkylalkyl, (heterocyclic)alkyl or heterocyclic-substituted cycloalkylalkyl wherein the aryl group, the aryl part of the arylalkyl group, the aryl part of the aryl-substituted cycloalkylalkyl group, the heterocyclic group, the heterocyclic part of the (heterocyclic)alkyl group or the heterocyclic part of the heterocyclic-substituted cycloalkylalkyl group is substituted with -Y-R₃ wherein at each occurrence Y is independently selected from (i) a covalent bond, (ii) -C(O)-, (iii) -CH₂-, (iv) -O-, (v) -S(O)ₘ- wherein m is 0, 1 or 2, (vi) -N(R_{b})- wherein R_{b} is hydrogen or loweralkyl, (vii) -CH₂O-, (viii) -CH₂S(O)ₘ- wherein m is 0, 1 or 2 and (ix) -CH₂N(R_{b})- wherein R_{b} is hydrogen or loweralkyl and at each occurrence R₃ is independently selected from (i) aryl, (ii) arylalkyl, (iii) cycloalkyl, (iv) cycloalkylalkyl,
(v) heterocyclic and (vi) (heterocyclic)alkyl and at each occurrence R₂ is independently selected from (i) alkenyl, (ii) alkynyl, (iii) aryl, (iv) arylalkyl, (v) arylalkenyl, (vi) heterocyclic, (vii) (heterocyclic)alkyl and (vi) aryl, heterocyclic, arylalkyl or (heterocyclic)alkyl wherein the aryl group, the aryl part of the arylalkyl group, the heterocyclic group or the heterocyclic part of the (heterocyclic)alkyl group is substituted with -Z-R₄ wherein at each occurrence Z is independently selected from (i) a covalent bond, (ii) -C(O)-, (iii) -CH₂-, (iv) -O-, (v) -S(O)ₚ- wherein p is 0, 1 or 2, (vi) -N(R_{c})- wherein R_{c} is hydrogen or loweralkyl, (vii) -CH₂O-, (viii) -CH₂S(O)ₚ-. wherein p is 0, 1 or 2 and (ix) -CH₂N(R_{c})- wherein R_{c} is hydrogen or loweralkyl and at each occurrence R₄ is independently selected from (i) aryl, (ii) arylalkyl, (iii) cycloalkyl, (iv) cycloalkylalkyl, (v) heterocyclic and (vi) (heterocyclic)alkyl, wherein, when R₃ or R₄ is a heterocyclic, (heterocyclic)alkyl, aryl or arylalkyl group, the heterocyclic group or the heterocyclic part of the (heterocyclic)alkyl group may be optionally substituted with one or two substituents independently selected from hydroxy, halo, oxo, loweralkylimino, amino, alkylamino, dialkylamino, alkoxy, alkoxyalkoxy, haloalkyl, cycloalkyl, aryl, arylalkyl, -COOH, -SO₃H and loweralkyl, and a nitrogen-containing heterocycle may be N-protected, and the aryl group or the aryl part of the arylalkyl group may be optionally substituted with one, two or three substituents independently selected from loweralkyl, haloalkyl, alkoxy, thioalkoxy, amino, alkylamino, dialkylamino, hydroxy, halo, mercapto, nitro, carboxaldehyde, carboxy, alkoxycarbonyl and carboxamide, or the aryl group or the aryl part of the arylalkyl group is tetrafluorophenyl or pentafluorophenyl, and
(2) -(CH₂)_{q}-N(R₁)(R₂) wherein q is 0, 1 or 2 and at each occurrence R₁ and R₂ are independently defined as above; and
B₁ and B₂ are independently selected from
(1) -CH₂OH,
(2) -CH=NOH,
(3) -W-R₅ wherein at each occurrence W is independently selected from (a) a covalent bond, (b) alkylene, (c) alkenylene, (d) -C(O)NH- and (e) -NHC(O)NH- and R₅ is independently selected from
(a) 5-tetrazolyl
(b) 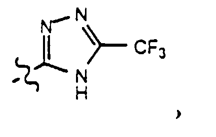
(c) 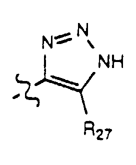 wherein R₂₇ is -CN, -NO₂, or -CO₂R₂₈ wherein R₂₈ is hydrogen, aryl or loweralkyl,
wherein the aryl group may be optionally substituted with one, two or three substituents independently selected from loweralkyl, haloalkyl, alkoxy, thioalkoxy, amino, alkylamino, dialkylamino, hydroxy, halo, mercapto, nitro, carboxaldehyde, carboxy, alkoxycarbonyl and carboxamide, or the aryl group may be tetrafluorophenyl or pentafluorophenyl
(d) 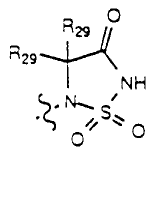 wherein at each occurrence R₂₉ is selected from hydrogen and loweralkyl,
(e) 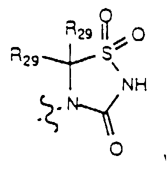 wherein R₂₉ is as defined above,
(f) 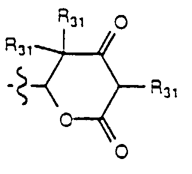 wherein at each occurrence R₃₁ is selected from hydrogen, loweralkyl, alkenyl, alkoxyalkyl and benzyl,
(g) 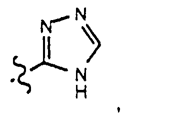
(h) 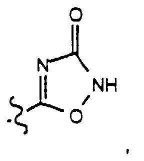
(i) 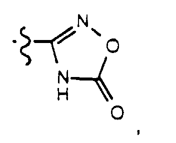
(j) 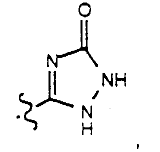
(k) 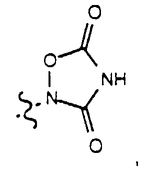
(l) 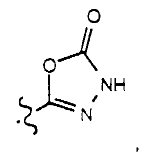
(m) 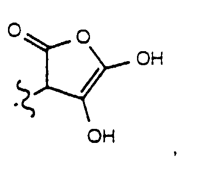 and
(n) 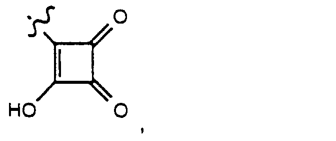
(4) -Q-C(O)R₆ wherein at each occurrence Q is independently selected from (a) a covalent bond, (b) alkylene, (c) alkenylene, (d) -CH(OH)- and (e) -NHC(O)(CH₂)ᵣ- wherein r is 0 to 4 and at each occurrence R₆ is independently selected from (a) -OR₇ wherein R₇ is hydrogen or a carboxy-protecting group, (b) -NH₂, (c) -NHOH, (d) -NHSO₂CF₃ (e) an alpha-amino acid or a beta-amino acid which is bonded via the alpha- or beta-amino group and (f) a di-, tri- or tetra-peptide which is bonded via the amino terminal amino group,
(5) -CH₂-N(OH)-C(O)-R₂₅ wherein R₂₅ is hydrogen, methyl or trifluoromethyl, and
(6) -C(O)-NH-S(O)2-R₂₆ wherein R₂₆ is aryl, heterocyclic, arylalkyl, (heterocyclic)alkyl, C₃-C₇-cycloalkyl, C₁-C₈-alkyl or perfluoro-C₁-C₄-alkyl,wherein the heterocyclic group or the heterocyclic part of the (heterocyclic)alkyl group may be optionally substituted with one or two substituents independently selected from hydroxy, halo, oxo, loweralkylimino, amino, alkylamino, dialkylamino, alkoxy, alkoxyalkoxy, haloalkyl, cycloalkyl, aryl, arylalkyl, -COOH,-SO₃H and loweralkyl, and a nitrogen-containing heterocycle may be N-protected, and the aryl group or the aryl part of the arylalkyl group may be optionally substituted with one, two or three substituents independently selected from loweralkyl, haloalkyl, alkoxy, thioalkoxy, amino, alkylamino, dialkylamino, hydroxy, halo, mercapto, nitro, carboxaldehyde, carboxy, alkoxycarbonyl and carboxamide, or the aryl group or the aryl part of the arylalkyl group is tetrafluorophenyl or pentafluorophenyl;
or a pharmaceutically acceptable salt thereof.

10. A compound as defined by Claim g wherein
A₁ and A₂ are independently selected from -C(O)-G wherein G is defined as above and
B₁ and B₂ are independently selected from
(a) -W-R₅ wherein W is a covalent bond or alkylene and R₅ is 5-tetrazolyl and
(b) -Q-C(O)-R₆ wherein at each occurrence Q is independently selected from a covalent bond and alkylene and at each occurrence R₆ is independently selected from
(1) -OR₇ wherein R₇ is hydrogen or a carboxy-protecting group,
(2) an alpha-amino acid or a beta-amino acid which is bonded via the alpha- or beta-amino group and
(3) a di-, tri- or tetra-peptide which is bonded via the amino terminal amino group.

11. A compound as defined by Claim 9 wherein
A₁ and A₂ are independently selected from -C(O)-G wherein G is -N(R₁)(R₂) wherein R₁ and R₂ are defined as above and
B₁ and B₂ are independently selected
(a) -W-R₅ wherein W is a covalent bond or alkylene and R₅ is 5-tetrazolyl and
(b) -Q-C(O)-R₆ wherein at each occurrence Q is independently selected from a covalent bond and alkylene and at each occurrence R₆ is independently selected from
(1) -OR₇ wherein R₇ is hydrogen or a carboxy-protecting group,
(2) an alpha-amino acid or a beta-amino acid which is bonded via the alpha- or beta-amino group and
(3) a di-, tri- or tetra-peptide which is bonded via the amino terminal amino group.

12. A compound as defined by Claim 9 wherein
A₁ and A₂ are independently selected from -C(O)-G wherein G is -N(R₁)(R₂) wherein at each occurrence R₁ is independently selected from (a) hydrogen, (b) loweralkyl, (c) cycloalkyl, (d) cycloalkylalkyl, (e) alkoxyalkyl, (f) thioalkoxyalkyl, (9) aryl, (h) heterocyclic, (i) arylalkyl, (j) (heterocyclic)alkyl, (k) aryl-substituted cycloalkylalkyl, (I) heterocyclic-substituted cycloalkylalkyl and (m) aryl, heterocyclic, arylalkyl, (heterocyclic)alkyl, aryl-substituted cycloalkylalkyl or heterocyclic-substituted cycloalkylalkyl wherein the aryl group, the aryl part of the arylalkyl group, the aryl part of the aryl-substituted cycloalkylalkyl group, the heterocyclic group, the heterocyclic part of the (heterocyclic)alkyl group or the heterocyclic part of the heterocyclic-substituted cycloalkylalkyl group is substituted with -Y-R₃ wherein at each occurrence Y is independently selected from (i) -O-, (ii) -S(O)ₘ- wherein m is 0, 1 or 2 and (iii) -N(R_{b})- wherein R_{b} is hydrogen or loweralkyl and at each occurrence R₃ is independently selected from (i) aryl, (ii) arylalkyl, (iii) cycloalkyl, (iv) cycloalkylalkyl, (v) heterocyclic and (vi) (heterocyclic)alkyl and at each occurrence R₂ is independently selected from aryl, heterocyclic, arylalkyl and (heterocyclic)alkyl wherein the aryl group, the aryl part of the arylalkyl group, the heterocyclic group or the heterocyclic part of the (heterocyclic)alkyl group is substituted with -Z-R₄ wherein at each occurrence Z is independently selected from (i) -O-, (ii) -S(O)ₚ- wherein p is 0, 1 or 2 and (iii) -N(R_{c})- wherein R_{c} is hydrogen or loweralkyl and at each occurrence R₄ is independently selected from (i) aryl, (ii) arylalkyl, (iii) cycloalkyl, (iv) cycloalkylalkyl, (v) heterocyclic and (vi) (heterocyclic)alkyl, wherein, when R₃ or R₄ is a heterocyclic, (heterocyclic)alkyl, aryl or arylalkyl group, the heterocyclic group or the heterocyclic part of the (heterocyclic)alkyl group may be optionally substituted with one or two substituents independently selected from hydroxy, halo, oxo, loweralkylimino, amino, alkylamino, dialkylamino, alkoxy, alkoxyalkoxy, haloalkyl, cycloalkyl, aryl, arylalkyl, -COOH, -SO₃H and loweralkyl, and a nitrogen-containing heterocycle may be N-protected, and the aryl group or the aryl part of the arylalkyl group may be optionally substituted with one, two or three substituents independently selected from loweralkyl, haloalkyl, alkoxy, thioalkoxy, amino, alkylamino, dialkylamino, hydroxy, halo, mercapto, nitro, carboxaldehyde, carboxy, alkoxycarbonyl and carboxamide, or the aryl group or the aryl part of the arylalkyl group is tetrafluorophenyl or pentafluorophenyl, and
B₁ and B₂ are independently selected from
(a) -W-R₅ wherein W is a covalent bond or alkylene and R₅ is 5-tetrazolyl and
(b) -Q-C(O)-R₆ wherein at each occurrence Q is independently selected from a covalent bond and alkylene and at each occurrence R₆ is independently selected from
(1) -OR₇ wherein R₇ is hydrogen or a carboxy-protecting group,
(2) an alpha-amino acid or a beta-amino acid which is bonded via the alpha- or beta-amino group and
(3) a di-, tri- or tetra-peptide which is bonded via the amino terminal amino group.

13. A compound as defined by Claim 9 wherein
A₁ and A₂ are independently selected from -C(O)-G wherein G is -N(R₁)(R₂) wherein at each occurrence R₁ is independently selected from (a) loweralkyl, (b) cycloalkyl and (c) cycloalkylalkyl and at each occurrence R₂ is independently selected from aryl and arylalkyl wherein the aryl group or the aryl part of arylalkyl is substituted with -Z-R₄ wherein at each occurrence Z is independently selected from (i) -O- and (ii) -S- and at each occurrence R₄ is independently selected from (i) aryl, (ii) arylalkyl, (iii) cycloalkyl, (iv) cycloalkylalkyl, (v) heterocyclic and (vi) (heterocyclic)alkyl,
wherein the heterocyclic group or the heterocyclic part of the (heterocyclic)alkyl group may be optionally substituted with one or two substituents independently selected from hydroxy, halo, oxo, loweralkylimino, amino, alkylamino, dialkylamino, alkoxy, alkoxyalkoxy, haloalkyl, cycloalkyl, aryl, arylalkyl, -COOH, -SO₃H and loweralkyl, and a nitrogen-containing heterocycle may be N-protected, and the aryl group or the aryl part of the arylalkyl group may be optionally substituted with one, two or three substituents independently selected from loweralkyl, haloalkyl, alkoxy, thioalkoxy, amino, alkylamino, dialkylamino, hydroxy, halo, mercapto, nitro, carboxaldehyde, carboxy, alkoxycarbonyl and carboxamide, or the aryl group or the aryl part of the arylalkyl group is tetrafluorophenyl or pentafluorophenyl, and
B₁ and B₂ are independently selected from -W-R₅ and -Q-C(O)-R₆ wherein at each occurrence Q and W are independently selected from a covalent bond and alkylene, R₆ is -OR₇ wherein R₇ is hydrogen or a carboxy-protecting group and R₅ is 5-tetrazolyl.

14. A compound as defined by Claim 9 wherein
A₁ and A₂ are independently selected from -C(O)-G wherein G is -N(R₁)(R₂) wherein at each occurrence R₁ is independently selected from (a) loweralkyl, (b) cycloalkyl and (c) cycloalkylalkyl and at each occurrence R₂ is independently selected from phenyl and benzyl wherein the phenyl group or the phenyl ring of the benzyl group is substituted with -Z-R₄ wherein at each occurrence Z is independently selected from (i) -O- and (ii) -S- and at each occurrence R₄ is independently selected from (i) aryl, (ii) arylalkyl, (iii) heterocyclic and (iv) (heterocyclic)alkyl,
wherein the heterocyclic group or the heterocyclic part of the (heterocyclic)alkyl group may be optionally substituted with one or two substituents independently selected from hydroxy, halo, oxo, loweralkylimino, amino, alkylamino, dialkylamino, alkoxy, alkoxyalkoxy, haloalkyl, cycloalkyl, aryl, arylalkyl, -COOH, -SO₃H and loweralkyl, and a nitrogen-containing heterocycle may be N-protected, and the aryl group or the aryl part of the arylalkyl group may be optionally substituted with one, two or three substituents independently selected from loweralkyl, haloalkyl, alkoxy, thioalkoxy, amino, alkylamino, dialkylamino, hydroxy, halo, mercapto, nitro, carboxaldehyde, carboxy, alkoxycarbonyl and carboxamide, or the aryl group or the aryl part of the arylalkyl group is tetrafluorophenyl or pentafluorophenyl, and
B₁ and B₂ are independently selected from -W-R₅ and -Q-C(O)-R₆ wherein at each occurrence Q and W are independently selected from a covalent bond and alkylene, R₆ is -OR₇ wherein R₇ is hydrogen or a carboxy-protecting group and R₅ is 5-tetrazolyl.

15. A compound as defined by Claim 9 wherein
A₁ and A₂ are independently selected from -C(O)-G wherein G is -N(R₁)(R₂) wherein at each occurrence R₁ is independently selected from (a) loweralkyl, (b) cycloalkyl and (c) cycloalkylalkyl and R₂ is benzyl wherein the phenyl ring of the benzyl group is substituted with -Z-R₄ wherein at each occurrence Z is independently selected from (i) -O- and (ii) -Sand R₄ is aryl and
B₁ and B₂ are independently selected from -W-R₅ and -Q-C(O)-R₆ wherein at each occurrence Q and W are independently selected from a covalent bond and alkylene, R₆ is -OR₇ wherein R₇ is hydrogen or a carboxy-protecting group and R₅ is 5-tetrazolyl.

16. A compound as defined by Claim 9 wherein
A₁ and A₂ are independently selected from -C(O)-G wherein G is -N(R₁)(R₂) wherein at each occurrence R₁ is (a) loweralkyl, (b) cycloalkyl and (c) cycloalkylalkyl and R₂ is benzyl wherein the phenyl ring of the benzyl group is substituted with -Z-R₄ wherein at each occurrence Z is independently selected from (i) -O- and (ii) -S- and R₄ is heterocyclic, wherein the heterocyclic group may be optionally substituted with one or two substituents independently selected from hydroxy, halo, oxo, loweralkylimino, amino, alkylamino, dialkylamino, alkoxy, alkoxyalkoxy, haloalkyl, cycloalkyl, aryl, arylalkyl, -COOH, -SO₃H and loweralkyl, and a nitrogen-containing heterocycle may be N-protected, and the aryl group or the aryl part of the arylalkyl group may be optionally substituted with one, two or three substituents independently selected from loweralkyl, haloalkyl, alkoxy, thioalkoxy, amino, alkylamino, dialkylamino, hydroxy, halo, mercapto, nitro, carboxaldehyde, carboxy, alkoxycarbonyl and carboxamide, or the aryl group or the aryl part of the arylalkyl group is tetrafluorophenyl or pentafluorophenyl, and
B₁ and B₂ are independently selected from -W-R₅ and -Q-C(O)-R₆ wherein at each occurrence Q and W are independently selected from a covalent bond and alkylene, R₆ is -OR₇ wherein R₇ is hydrogen or a carboxy-protecting group and R₅ is 5-tetrazolyl.

17. A compound selected from the group consisting of
(1α,2β,3β,4α)-1,2-Di(N-benzyl-N-(4-phenoxybenzyl)aminocarbonyl)cyclobutane-3,4-dicarboxylic acid;
(1α,2β,3β,4α)-1,2-Di(N-propyl-N-(4-phenoxybenzyl)aminocarbonyl)cyclobutane-3,4-dicarboxylic acid;
(-)-(1α,2β,3β,4α)-1,2-Di(N-propyl-N-(4-phenoxybenzyl)aminocarbonyl)cyclobutane-3,4-dicarboxylic acid;
(1α,2β,3β,4α)-1,2-Di(N-(2-ethylthioethyl)-N-(4-phenoxybenzyl)aminocarbonyl)cyclobutane-3,4-dicarboxylic acid;
(1α,2β,3β,4α)-1,2-Di(N-(cyclopropylmethyl)-N-(4-phenoxybenzyl)aminocarbonyl)cyclobutane-3,4-dicarboxylic acid;
(1α,2β,3β,4α)-1-(N-Propyl-N-(4-phenoxybenzyl)aminocarbonyl)2-(N-benzyl-N-(4-phenoxybenzyl)aminocarbonyl)cyclobutane-3,4-dicarboxylic acid;
(1α,2β,3β,4α)-1,2-Di(N-propyl-N-(4-phenylthiobenzyl)aminocarbonyl)cyclobutane-3,4-dicarboxylic acid;
(1α,2β,3β,4α)-1,2-Di(N-propyl-N-(4-phenoxybenzyl)aminocarbonyl 4-tetrazolylmethyl-cyclobutane-3-carboxylic acid;
(1α,2β,3β,4α)-1,2-Di(N-propyl-N-(4-phenoxybenzyl)aminocarbonyl)-4-(3-carboxypropionylamino)cyclobutane-3-carboxylic acid;
(1α,2β,3β,4α)-1,2-Di(N-propyl-N-(4-phenoxybenzyl)aminocarbonyl)-4-(1-carboxy-1-hydroxymethyl)cyclobutane-3-carboxylic acid;
(1α,2β,3β,4α)-1,2-Di(N-propyl-N-(4-phenoxybenzyl)aminocarbonyl)cyclobutane-3-(N-(5-tetrazolyl))carboxamide-4-carboxylic acid;
(1α,2β,3β,4α)-4-(Carboxymethyl)-1,2-di(N-propyl-N-(4-phenoxybenzyl)aminocarbonyl)cyclobutane-3-carboxylic acid; and
(1α,2β,3β,4α)-1,2-Di(-N-propyl-N-(4-phenoxybenzyl)aminocarbonyl)cyclobutane-3,4-diacetic acid;
or a pharmaceutically acceptable salt thereof.

18. A pharmaceutical composition for inhibiting squalene synthetase comprising a therapeutically effective amount of a compound according to Claim 1 and a pharmaceutically acceptable carrier.

19. A pharmaceutical composition for inhibiting squalene synthetase comprising a therapeutically effective amount of a compound according to Claim 9 and a pharmaceutically acceptable carrier.

20. Use of a compound according to Claim 1 for manufacturing a medicament for inhibiting cholesterol biosynthesis in a human or lower mammal.

21. Use of a compound according to Claim 9 for manufacturing a medicament for inhibiting cholesterol biosynthesis in a human or lower mammal.

22. Use of a compound as defined by Claim 1 for manufacturing a medicament for treating hyperlipidaemia or atherosclerosis in a mammal.

23. Use of a compound as defined by Claim 9 for manufacturing a medicament for treating hyperlipidaemia or atherosclerosis in a mammal.

24. Use of a compound as defined by Claim 1 for manufacturing a medicament for treating a fungal infection in a mammal.

25. Use of a compound as defined by Claim 9 for manufacturing a medicament for treating a fungal infection in a mammal.

26. A compound as defined by Claim 1 for use as a therapeutic agent.

## Patentansprüche

1. Verbindung nach folgender Formel: 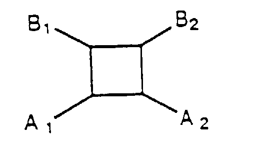 worin
A₁ und A₂ unabhängig aus folgendem gewählt sind:
(1) -X-C(O)-G oder -X-C(S)G, worin bei jedem Vorkommen X unabhängig aus folgendem gewählt ist: (a) einer kovalenten Bindung, (b) -CH₂-, (c) -O-, (d) -S- und (e)-N(Rₐ)-, worin Rₐ gleich Wasserstoff, Niederalkyl, Cycloalkyl oder Cycloalkylalkyl ist, und wobei bei jedem Vorkommen G unabhängig aus -R₂, -N(R₁) (R₂), -OR₂ und -SR₂ gewählt ist, wobei bei jedem Vorkommen R₁ unabhängig aus folgendem gewählt ist: (a) Wasserstoff, (b) Niederalkyl, (c) Alkenyl, (d) Alkinyl, (e) Cycloalkyl, (f) Cycloalkylalkyl, (g) Alkoxycarbonylalkyl, (h) Alkoxyalkyl, (i) Thioalkoxyalkyl, (j) Haloalkyl, (k) Aryl, (l) Heterocyclus, (m) Arylalkyl, (n) Aryl-substituiertem Cycloalkylalkyl, (o) (Heterocyclus)alkyl, (p) Heterocyclus-substituiertem Cycloalkylalkyl und (q) Aryl, Heterocyclus, Arylalkyl, Aryl-substituiertem Cycloalkylalkyl, (Heterocyclus)alkyl oder Heterocyclus-substituiertem Cycloalkylalkyl, worin die Arylgruppe, der Arylteil der Aryl alkylgruppe , der Arylteil der Aryl-substituierten Cycloalkylalkylgruppe, die heterocyclische Gruppe, der heterocyclische Teil der (Heterocyclus)alkylgruppe oder der heterocyclische Teil der Heterocyclus-substituierten Cycloalkylalkylgruppe mit -Y-R₃ substituiert ist, wobei bei jedem Vorkommen Y unabhängig aus (i) einer kovalenten Bindung, (ii) -C(O)-, (iii) -CH₂-, (iv) -O-, (v) -S(O)ₘ-, worin m gleich 0,1 oder 2 ist, (vi) -N(R_{b})-, worin R_{b} gleich Wasserstoff oder Niederalkyl ist, (vii) -CH₂O-, (viii) -CH₂S(O)m-, worin m gleich 0,1 oder 2 ist und (ix) -CH₂N(R_{b})- gewählt ist, worin R_{b} gleich Wasserstoff oder Niederalkyl ist, und wobei R₃ bei jedem Vorkommen unabhängig aus folgendem gewählt ist: (i) Aryl, (ii) Arylalkyl, (iii) Cycloalkyl, (iv) Cycloalkylalkyl, (v) Heterocyclus und (vi) (Heterocyclus)alkyl und wobei R₂ bei jedem Vorkommen unabhängig aus folgendem gewählt ist: (i) Alkenyl, (ii) Alkinyl, (iii) Aryl, (iv) Arylalkyl, (v) Arylalkenyl, (vi) Heterocyclus, (vii) (Heterocyclus)alkyl und (vi) Aryl, Heterocyclus, Arylalkyl oder (Heterocyclus)alkyl, worin die Arylgruppe, der Arylteil der Arylalkylgruppe, die heterocyclische Gruppe oder der heterocyclische Teil der (Heterocyclus)alkylgruppe mit -Z-R₄ subsituiert ist, wobei Z bei jedem Vorkommen unabhängig aus folgendem gewählt ist: (i) einer kovalenten Bindung, (ii) -C(O)-, (iii) -CH₂-, (iv) -O-, (v) -S(O)ₚ-, worin p gleich 0,1 oder 2 ist, (vi) -N(R_{c})-, worin R_{c} gleich Wasserstoff oder Niederalkyl ist, (vii) -CH₂O-, (viii) -CH₂S(O)ₚ-, worin p gleich 0,1 oder 2 ist und (ix) -CH₂N(R_{c})-, worin R_{c} gleich Wasserstoff oder Niederalkyl ist, und wobei R₄ bei jedem Vorkommen unabhängig aus folgendem gewählt ist, (i) Aryl, (ii) Arylalkyl, (iii) Cycloalkyl, (iv) Cycloalkylalkyl, (v) Heterocyclus und (vi) (Heterocyclus)alkyl,
worin, wenn R₃ oder R₄ ein Heterocyclus, (Heterocyclus)alkyl, Aryl oder eine Arylalkylgruppe ist, die heterocyclische Gruppe oder der heterocyclische Teil der (Heterocyclus)alkylgruppe wahlweise mit einem oder zwei Substituenten substituiert sein kann, der/die unabhängig aus Hydroxy, Halo, Oxo, Niederalkylimino, Amino, Alkylamino, Dialkylamino, Alkoxy, Alkoxyalkoxy, Haloalkyl, Cycloalkyl, Aryl, Arylalkyl, -COOH, -SO₃H und Niederalkyl gewählt ist/sind, und wobei ein stickstoffhaltiger Heterocyclus N-geschützt sein kann, und wobei die Arylgruppe oder der Arylteil der Arylalkylgruppe wahlweise mit ein, zwei oder drei Substituenten substituiert sein kann, der/die aus Niederalkyl, Haloalkyl, Alkoxy, Thioalkyoxy, Amino, Alkylamino, Dialkylamino, Hydroxy, Halo, Mercapto, Nitro, Carboxyaldehyd, Carboxy, Alkoxycarbonyl und Carboxamid gewählt ist/sind, oder wobei die Arylgruppe oder der Arylteil der Arylalkylgruppe gleich Tetrafluorphenyl oder Pentafluorphenyl ist, und
(2) -(CH₂)_{q}-N(R₁) (R₂), worin q gleich 0,1 oder 2 ist, und wobei R₁ und R₂ bei jedem Vorkommen unabhängig die oben definierte Bedeutung haben; und wobei B₁ und B₂ unabhängig aus folgendem gewählt sind:
(1) -CH₂OH,
(2) -CH=NOH,
(3) -W-R₅, wobei W bei jedem Vorkommen unabhängig aus folgendem gewählt ist: (a) einer kovalenten Bindung, (b) Alkylen, (c) Alkenylen, (d) -C(O)NH- und (e) -NHC(O)NH-, und wobei R₅ unabhängig aus folgendem gewählt ist:
(a) 5-Tetrazolyl,
(b) 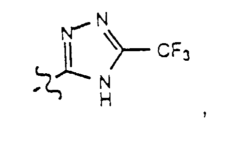
(c) 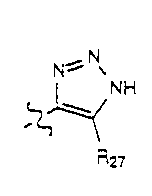 worin R₂₇ gleich -CN, -NO₂ oder -CO₂R₂₈ ist, wobei R₂₈ gleich Wasserstoff, Aryl oder Niederalkyl ist, worin die Arylgruppe wahlweise mit einem, zwei oder drei Substituenten substituiert sein kann, der/die unabhängig aus Niederalkyl, Haloalkyl, Alkoxy, Thioalkoxy, Amino, Alkylamino, Dialkylamino, Hydroxy, Halo, Mercapto, Nitro, Carboxaldehyd, Carboxy, Alkoxycarbonyl und Carboxamid gewählt ist/sind, oder worin die Arylgruppe gleich Tetrafluorphenyl oder Pentafluorphenyl sein kann,
(d) 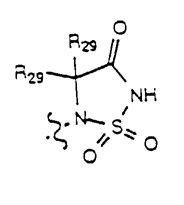 worin R₂₉ bei jedem Vorkommen aus Wasserstoff und Niederalkyl gewählt ist
(e) 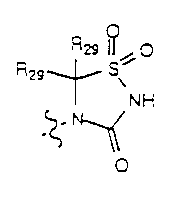 worin R₂₉ die oben definierte Bedeutung aufweist,
(f) 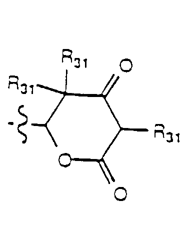 worin R₃₁ bei jedem Vorkommen aus Wasserstoff, Niederalkyl, Alkenyl, Alkoxyalkyl und Benzyl gewählt ist,
(g) 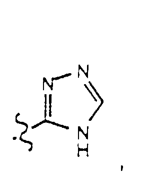
(h) 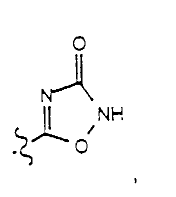
(i) 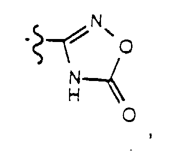
(j) 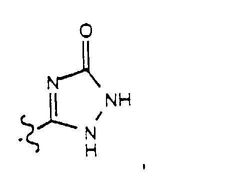
(k) 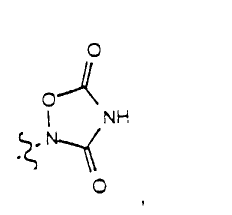
(l) 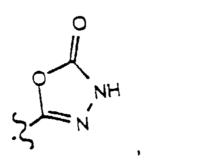
(m) 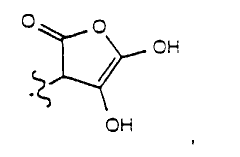 und
(n) 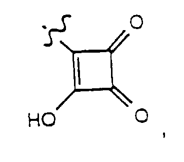
(4) -Q-C(O)R₆, wobei Q bei jedem Vorkommen unabhängig aus folgendem gewählt ist: (a) einer kovalenten Bindung, (b) Alkylen, (c) Alkenylen, (d) -CH(OH)- und (e) -NHC(O) (CH₂)ᵣ-, worin r gleich 0 bis 4 ist, und worin R₆ bei jedem Vorkommen unabhängig aus folgendem gewählt ist: (a) -OR₇, worin R₇ gleich Wasserstoff oder einer Carboxyschutzgruppe ist, (b) -NH₂, (c) -NHOH, (d) -NHSO₂CF₃ (e) einer alpha-Aminosäure oder einer beta-Aminosäure, die über die alpha- oder beta-Aminogruppe gebunden ist, und (f) einem Di-, Tri-oder Tetrapeptid, das über die aminoterminale Aminogruppe gebunden ist,
(5) -CH₂-N(OH)-C(O)-R₂₅, worin R₂₅ gleich Wasserstoff, Methyl oder Trifluormethyl ist, und
(6) -C(O)-NH-S(O)₂-R₂₆, worin R₂₆ gleich Aryl, Heterocyclus, Arylalkyl, (Heterocyclus)alkyl, C₃-C₇-Cycloalkyl, C₁-C₈-Alkyl oder Perfluor-C₁-C₄-Alkyl ist, worin die heterocyclische Gruppe oder der heterocyclische Teil der (Heterocyclus)alkylgruppe wahlweise mit einem oder zwei Substituenten substituiert sein kann, der/die unabhängig aus Hydroxy, Halo, Oxo, Niederalkylimino, Amino, Alkylamino, Dialkylamino, Alkoxy, Alkoxyalkoxy, Haloalkyl, Cycloalkyl, Aryl, Arylalkyl, -COOH, -SO₃H und Niederalkyl gewählt ist/sind, und worin ein stickstoffhaltiger Heterocyclus N-geschützt sein kann, und worin die Arylgruppe oder der Arylteil der Arylalkylgruppe wahlweise mit ein, zwei oder drei Substituenten substituiert sein kann, die unabhängig aus Niederalkyl, Haloalkyl, Alkoxy, Thioalkoxy, Amino, Alkylamino, Dialkylamino, Hydroxy, Halo, Mercapto, Nitro, Carboxaldehyd, Carboxy, Alkoxycarbonyl und Carboxamid gewählt sind, oder wobei die Arylgruppe oder der Arylteil der Arylalkylgruppe gleich Tetrafluorphenyl oder Pentafluorphenyl ist;
oder ein pharmazeutisch verträgliches Salz davon.

2. Verbindung nach Anspruch 1, worin A₁ und A₂ unabhängig aus -C(O)-G gewählt sind, wobei G die oben definierte Bedeutung aufweist, und wobei B₁ und B₂ unabhängig aus folgendem gewählt sind:
(a) -W-R₅, wobei W eine kovalente Bindung oder Alkylen ist, und wobei R₅ gleich 5-Tetrazolyl ist, und (b) -Q-C(O)-R₆, wobei Q bei jedem Auftreten unabhängig aus einer kovalenten Bindung und Alkylen gewählt ist, und wobei R₆ bei jedem Auftreten unabhängig aus folgendem gewählt ist:
(1) -OR₇, worin R₇ gleich Wasserstoff oder eine Carboxyschutzgruppe ist;
(2) einer alpha-Aminosäure oder einer beta-Aminosäure, die über die alpha- oder beta-Aminogruppe gebunden ist, und
(3) einem Di-, Tri- oder Tetrapeptid, das über die aminoterminale Aminogruppe gebunden ist.

3. Verbindung nach Anspruch 1, worin A₁ und A₂ unabhängig aus -C(O)-G gewählt sind, worin G gleich -N(R₁) (R₂)ist, worin R₁ und R₂ die oben definierte Bedeutung haben, und worin B₁ und B₂ unabhängig aus folgendem gewählt sind:
(a) -W-R₅, worin W eine kovalente Bindung oder Alkylen ist, und worin R₅ gleich 5-Tetrazolyl ist, und (b) -Q-C(O)-R₆, wobei Q bei jedem Vorkommen unabhängig aus einer kovalenten Bindung und Alkylen gewählt ist, und wobei R₆ bei jedem Vorkommen unabhängig aus folgendem gewählt ist:
(1) -OR₇, worin R₇ gleich Wasserstoff oder einer Carboxyschutzgruppe ist
(2) einer alpha-Aminosäure oder einer beta-Aminosäure, die über die alpha- oder beta-Aminogruppe gebunden ist, und
(3) ein Di-, Tri- oder Tetrapeptid, das über die aminoterminale Aminogruppe gebunden ist.

4. Verbindung nach Anspruch 1, worin A₁ und A₂ unabhängig aus -C(O)-G gewählt sind, worin G gleich -N(R₁) (R₂) ist,
wobei R₁ bei jedem Vorkommen unabhängig aus folgendem gewählt ist: (a) Wasserstoff, (b) Niederalkyl, (c) Cycloalkyl, (d) Cycloalkylalkyl, (e) Alkoxyalkyl, (f) Thioalkoxyalkyl, (g) Aryl, (h) Heterocyclus, (i) Arylalkyl, (j) (Heterocyclus)alkyl, (k) Aryl-substituiertes Cycloalkylalkyl, (1) Heterocyclus-substituiertem Cycloalkylalkyl und (m) Aryl, Heterocyclus, Arylalkyl, (Heterocyclus)alkyl, Aryl-substituiertem Cycloalkylalkyl oder Heterocyclus-substituiertem Cycloalkylalkyl, worin die Arylgruppe, der Arylteil der Arylalkylgruppe, der Arylteil der Aryl-substituierten Cycloalkylalkylgruppe, die heterocyclische Gruppe, der heterocyclische Teil der (Heterocyclus)alkylgruppe oder der heterocyclische Teil der Heterocyclus-substituierten Cycloalkylalkylgruppe mit -Y-R₃ substituiert ist, worin Y bei jedem Vorkommen unabhängig aus folgendem gewähltist: (i) -O-, (ii) -S(O)ₘ-, worin m gleich 0, 1 oder 2 ist, und (iii) -N(R_{b})-, worin R_{b} gleich Wasserstoff oder Niederalkyl ist, und wobei R₃ bei jedem Auftreten unabhängig aus folgendem gewählt ist: (i) Aryl, (ii) Arylalkyl, (iii) Cycloalkyl, (iv) Cycloalkylalkyl, (v) Heterocyclus und (vi) (Heterocyclus)alkyl und wobei R₂ bei jedem Vorkommen unabhängig aus Aryl, Heterocyclus, Arylalkyl und (Heterocyclus)alkyl gewählt ist, worin die Arylgruppe, der Arylteil der Arylalkylgruppe, die heterocyclische Gruppe oder der heterocyclische Teil der (Heterocyclus)alkylgruppe mit -Z-R₄ substituiert ist, worin Z bei jedem Vorkommen unabhängig aus folgendem gewählt ist: (i) -O-, (ii) -S(O)ₚ-, worin p gleich 0, 1 oder 2 ist und (iii) -N(R_{c})-, worin R_{c} gleich Wasserstoff oder Niederalkyl ist, und worin R₄ bei jedem Vorkommen unabhängig aus folgendem gewählt ist: (i) Aryl, (ii) Arylalkyl, (iii) Cycloalkyl, (iv) Cycloalkylalkyl, (v) Heterocyclus und (vi) (Heterocyclus)alkyl, worin, wenn R₃ oder R₄ gleich einem Heterocyclus, (Heterocyclus)alkyl, Aryl oder gleich einer Arylalkylgruppe ist, die heterocyclische Gruppe oder der heterocyclische Teil der (Heterocyclus)alkylgruppe wahlweise mit einem oder zwei Substituenten substituiert sein kann, der/die unabhängig aus Hydroxy, Halo, Oxo, Niederalkylimino, Amino, Alkylamino, Dialkylamino, Alkoxy, Alkoxyalkoxy, Haloalkyl, Cycloalkyl, Aryl, Arylalkyl, -COOH,
-SO₃H und Niederalkyl gewählt ist/sind, und worin ein stickstoffhaltiger Heterocyclus N-geschützt sein kann, und worin die Arylgruppe oder der Arylteil der Arylalkylgruppe wahlweise mit einem, zwei oder drei Substituenten substituiert sein kann, der/die unabhängig aus Niederalkyl, Haloalkyl, Alkoxy, Thioalkoxy, Amino, Alkylamino, Dialkylamino, Hydroxy, Halo, Mercapto, Nitro, Carboxaldehyd, Carboxy, Alkoxycarbonyl und Carboxamid gewählt ist/sind, oder worin die Arylgruppe oder der Arylteil der Arylalkylgruppe gleich Tetrafluorphenyl oder Pentafluorphenyl ist, und worin B₁ und B₂ unabhängig aus folgendem gewählt sind: (a) -W-R₅, worin W eine kovalente Bindung oder Alkylen ist, und worin R₅ gleich 5-Tetrazolyl ist, und (b) -Q-C(O)-R₆, wobei Q bei jedem Vorkommen unabhängig aus einer kovalenten Bindung und Alkylen gewählt ist, und wobei R₆ bei jedem Vorkommen unabhängig aus folgendem gewählt ist:
(1) -OR₇, worin R₇ gleich Wasserstoff oder eine Carboxyschutzgruppe ist,
(2) eine alpha-Aminosäure oder eine beta-Aminosäure, die über die alpha- oder beta-Aminogruppe gebunden ist, und
(3) ein Di-, Tri- oder Tetrapeptid, das über die aminoterminale Aminogruppe gebunden ist.

5. Verbindung nach Anspruch 1, worin A₁ und A₂ unabhängig aus -C(O)-G gewählt sind, worin G gleich -N(R₁) (R₂) ist,
wobei R₁ bei jedem Vorkommen unabhängig aus folgendem gewählt ist: (a) Niederalkyl, (b) Cycloalkyl und (c) Cycloalkylalkyl, und
wobei R₂ bei jedem Vorkommen unabhängig aus Aryl und Arylalkyl gewählt ist, worin die Arylgruppe oder der Arylteil der Arylalkylgruppe mit -Z-R₄ substituiert ist, wobei Z bei jedem Vorkommen unabhängig aus folgendem gewählt ist: (i) -O- und (ii) -S-, und wobei R₄ bei jedem Vorkommen unabhängig aus folgendem gewählt ist: (i) Aryl, (ii) Arylalkyl, (iii) Cycloalkyl, (iv) Cycloalkylalkyl, (v) Heterocyclus und (vi) (Heterocyclus)alkyl, worin die heterocyclische Gruppe oder der heterocyclische Teil der (Heterocyclus)alkylgruppe wahlweise mit einem oder zwei Substituenten substituiert sein kann, die unabhängig aus Hydroxy, Halo, Oxo, Niederalkylimino, Amino, Alkylamino, Dialkylamino, Alkoxy, Alkoxyalkoxy, Haloalkyl, Cycloalkyl, Aryl, Arylalkyl, -COOH, -SO₃H und Niederalkyl gewählt sind, und wobei ein stickstoffhaltiger Heterocyclus N-geschützt sein kann, und wobei die Arylgruppe oder der Arylteil der Arylalkylgruppe wahlweise mit einem, zwei oder drei Substituenten substituiert sein kann, der/die unabhängig aus Niederalkyl, Haloalkyl, Alkoxy, Thioalkoxy, Amino, Alkylamino, Dialkylamino, Hydroxy, Halo, Mercapto, Nitro, Carboxaldehyd, Carboxy, Alkoxycarbonyl und Carboxamid gewählt ist/sind, oder wobei die Arylgruppe oder der Arylteil der Arylalkylgruppe gleich Tetrafluorphenyl oder Pentafluorphenyl ist,
und worin B₁ und B₂ unabhängig aus
-W-R₅ und -Q-C(O)-R₆ gewählt sind, worin Q und W bei jedem Vorkommen unabhängig aus einer kovalenten Bindung und Alkylen gewählt sind, wobei R6 gleich -OR₇ ist, wobei R₇ gleich Wasserstoff oder eine Carboxyschutzgruppe ist, und worin R₅ gleich 5-Tetrazolyl ist.

6. Verbindung nach Anspruch 1, worin A₁ und A₂ unabhängig aus -C(O)-G gewählt sind, worin G gleich -N(R₁)(R₂) ist, wobei R₁ bei jedem Vorkommen unabhängig aus folgendem gewählt ist:
(a) Niederalkyl, (b) Cycloalkyl und (c) Cycloalkylalkyl, und wobei R₂ bei jedem Auftreten unabhängig aus Phenyl und Benzyl gewählt ist, worin die Phenylgruppe oder der Phenylring der Benzylgruppe mit -Z-R₄ substituiert ist, wobei Z bei jedem Vorkommen unabhängig aus folgendem gewählt ist:
(i) -O- und (ii) -S-,
und wobei R₄ bei jedem Vorkommen unabhängig aus folgendem gewählt ist:
(i) Aryl, (ii) Arylalkyl, (iii) Heterocyclus und (iv) (Heterocyclus)alkyl, wobei die heterocyclische Gruppe oder der heterocyclische Teil der (Heterocyclus)alkylgruppe wahlweise mit einem oder zwei Substituenten substituiert sein kann, der/die unabhängig aus Hydroxy, Halo, Oxo, Niederalklimino, Amino, Alkylamino, Dialkylamino, Alkoxy, Alkoxyalkoxy, Haloalkyl, Cycloalkyl, Aryl, Arylalkyl, -COOH, -SO₃H und Niederalkyl gewählt sind, und wobei ein stickstoffhaltiger Heterocyclus N-geschützt sein kann, und wobei die Arylgruppe oder der Arylteil der Arylalkylgruppe wahlweise mit ein, zwei oder drei Substituenten substituiert sein kann, der/die aus Niederalkyl, Haloalkyl, Alkoxy, Thioalkoxy, Amino, Alkylamino, Dialkylamino, Hydroxy, Halo, Mercapto, Nitro, Carboxaldehyd, Carboxy, Alkoxycarbonyl und Carboxamid gewählt ist/sind, oder wobei die Arylgruppe oder der Arylteil der Arylalkylgruppe gleich Tetrafluorphenyl oder Pentafluorphenyl ist, und worin
B₁ und B₂ unabhängig aus -W-R₅ und -Q-C(O)-R₆ gewählt sind, wobei Q und W bei jedem Vorkommen unabhängig aus einer kovalenten Bindung und Alkylen gewählt sind, worin R₆ gleich-OR₇ ist, worin R₇ gleich Wasserstoff oder eine Carboxyschutzgruppe ist, und worin R₅ gleich 5-Tetrazolyl ist.

7. Verbindung nach Anspruch 1, worin A₁ und A₂ unabhängigaus -C(O)-G gewählt sind, worin G gleich -N(R₁) (R₂) ist, wobei R₁ bei jedem Vorkommen unabhängig aus folgendem gewählt ist:
(a) Niederalkyl, (b) Cycloalkyl und (c) Cycloalkylalkyl, und wobei R₂ gleich Benzyl ist, wobei der Phenylring der Benzylgruppe mit -Z-R₄ substituiert ist,
wobei Z bei jedem Vorkommen unabhängig aus (i) -O- und (ii) -S- gewählt ist, und wobei R₄ gleich Aryl ist, und wobei
B₁ und B₂ unabhängig aus -W-R₅ und-Q-C(O)-R₆ gewählt sind, wobei Q und W bei jedem Vorkommen unabhängig aus einer kovalenten Bindung und Alkylen gewählt sind, wobei R₆ gleich -OR₇ ist, wobei R₇ gleich Wasserstoff oder eine Carboxyschutzgruppe ist, und wobei R₅ gleich 5-Tetrazolyl ist.

8. Verbindung nach Anspruch 1, worin A₁ und A₂ unabhängig aus -C(O)-G gewählt sind, wobei G gleich -N(R₁) (R₂) ist, worin R₁ bei jedem Vorkommen gleich (a) Niederalkyl, (b) Cycloalkyl und (c) Cycloalkylalkyl ist, und wobei R₂ gleich Benzyl ist, wobei der Phenylring der Benzylgruppe mit -Z-R₄ substituiert ist, wobei Z bei jedem Vorkommen unabhängig aus (i) -O- und (ii) -S- gewählt ist, und wobei R₄ ein Heterocyclus ist, worin die heterocyclische Gruppe wahlweise mit einem oder zwei Substituenten substituiert sein kann, der/die unabhängig aus Hydroxy, Halo, Oxo, Niederalkylimino, Amino, Alkylamino, Dialkylamino, Alkoxy, Alkoxyalkoxy, Haloalkyl, Cycloalkyl, Aryl, Arylalkyl, -COOH, -SO₃H und Niederalkyl gewählt ist/sind, und worin ein stickstoffhaltiger Heterocyclus N-geschützt sein kann, und worin die Arylgruppe oder der Arylteil der Arylalkylgruppe wahlweise mit einem, zwei oder drei Substituenten substituiert sein kann, der/die unabhängig aus Niederalkyl, Haloalkyl, Alkoxy, Thioalkoxy, Amino, Alkylamino, Dialkylamino, Hydroxy, Halo, Mercapto, Nitro, Carboxaldehyd, Carboxy, Alkoxycarbonyl und Carboxamid gewählt ist/sind, oder worin die Arylgruppe oder der Arylteil der Arylalkylgruppe gleich Tetrafluorphenyl oder Pentafluorphenyl ist,
und worin B₁ und B₂ unabhängig aus
-W-R₅ und -Q-C(O)-R₆ gewählt sind, wobei Q und W bei jedem Vorkommen unabhängig aus einer kovalenten Bindung und Alkylen gewählt sind, worin R₆ gleich -OR₇ ist, worin R₇ gleich Wasserstoff oder eine Carboxyschutzgruppe ist, und worin R₅ gleich 5-Tetrazolyl ist.

9. Verbindung nach Anspruch 1 mit folgender Formel: 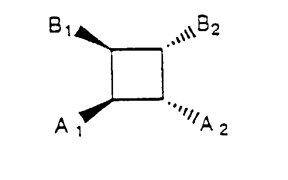 worin
A₁ und A₂ unabhängig aus folgendem gewählt sind:
(1) -X-C(O)-G oder-X-C(S)-G, wobei x bei jedem Vorkommen unabhängig aus folgendem gewählt ist: (a) einer kovalenten Bindung, (b)-CH₂-, (c) -O-, (d)-S- und (e) -N(Rₐ)-, worin Rₐ gleich Wasserstoff, Niederalkyl, Cycloalkyl oder Cycloalkylalkyl ist, und wobei G bei jedem Vorkommen unabhängig aus -R₂, -N(R₁)(R₂), -OR₂ und -SR₂ gewählt ist, wobei R₁ bei jedem Auftreten unabhängig aus folgendem gewählt ist: (a) Wasserstoff, (b) Niederalkyl, (c) Alkenyl, (d) Alkinyl, (e) Cycloalkyl, (f) Cycloalkylalkyl, (g) Alkoxycarbonylalkyl, (h) Alkoxyalkyl, (i) Thioalkoxyalkyl, (j) Haloalkyl, (k) Aryl, (1) Heterocyclus, (m) Arylalkyl, (n) Aryl-substituiertes Cycloalkylalkyl, (o) (Heterocyclus)alkyl, (p) Heterocyclus-substituiertes Cycloalkylalkyl und (q) Aryl, Heterocyclus, Arylalkyl, Aryl-substituiertes Cycloalkylalkyl, (Heterocyclus)alkyl oder Heterocyclus-substituiertes Cycloalkylalkyl, worin die Arylgruppe, der Arylteil der Arylalkylgruppe, der Arylteil der Aryl-substituierten Cycloalkylalkylgruppe, die heterocyclische Gruppe, der heterocyclische Teil der (Heterocyclus)alkylgruppe oder der heterocyclische Teil der Heterocyclus-substituierten Cycloalkylalkylgruppe mit -Y-R₃ substituiert ist, wobei Y bei jedem Vorkommen unabhängig aus folgendem gewählt ist: (i) einer kovalenten Bindung (ii) -C(O)-, (iii) -CH₂-, (iv) -O-, (v) -S(O)ₘ, worin m gleich 0, 1 oder 2 ist (vi) -N(R_{b})-, worin R_{b} gleich Wasserstoff oder Niederalkyl ist, (vii) -CH₂O-, (viii) -CH₂S(O)ₘ-, worin m gleich 0, 1 oder 2 ist und (ix) -CH₂N(R_{b})-, worin R_{b} gleich Wasserstoff oder Niederalkyl ist, und wobei R₃ bei jedem Auftreten unabhängig aus folgendem gewählt ist: (i) Aryl, (ii) Arylalkyl, (iii) Cycloalkyl, (iv) Cycloalkylalkyl, (v) Heterocyclus und (vi) (Heterocyclus)alkyl, und wobei R₂ bei jedem Vorkommen unabhängig aus folgendem gewählt ist: (i) Alkenyl, (ii) Alkinyl, (iii) Aryl, (iv) Arylalkyl, (v) Arylalkenyl, (vi) Heterocyclus, (vii) (Heterocyclus)alkyl, und (vi) Aryl, Heterocyclus, Arylalkyl oder (Heterocyclus)alkyl, wobei die Arylgruppe, der Arylteil der Arylalkylgruppe, die heterocyclische Gruppe oder der heterocyclische Teil der (Heterocyclus)alkylgruppe mit -Z-R₄ substituiert ist, worin Z bei jedem Vorkommen unabhängig aus folgendem gewählt ist: (i) einer kovalenten Bindung (ii) -C(O)-, (iii) -CH₂-, (iv) -O-, (v)-S(O)ₚ-, worin p gleich 0, 1 oder 2 ist, (vi) -N(R_{c})-, worin R_{c} gleich Wasserstoff oder Niederalkyl ist, (vii) -CH₂O-, (viii) -CH₂S(O)p-, worin p gleich 0, 1 oder 2 ist, und (ix) -CH₂N(R_{c})- worin R_{c} gleich Wasserstoff oder Niederalkyl ist, und wobei R₄ bei jedem Vorkommen unabhängig aus folgendem gewählt ist: (i) Aryl, (ii) Arylalkyl, (iii) Cycloalkyl, (iv) Cycloalkylalkyl, (v) Heterocyclus und (vi) (Heterocyclus)alkyl, worin, wenn R₃ oder R₄ gleich einem Heterocyclus, gleich (Heterocyclus)alkyl, gleich Aryl oder gleich einer Arylalkylgruppe ist, die heterocyclische Gruppe oder der heterocyclische Teil der (Heterocyclus)Alkylgruppe wahlweise mit einem oder zwei Substituenten substituiert sein kann, der/die unabhängig aus Hydroxy, Halo, Oxo, Niederalkylimino, Amino, Alkylamino, Dialkylamino, Alkoxy, Alkoxyalkoxy, Haloalkyl, Cycloalkyl, Aryl, Arylalkyl, -COOH, -SO₃H und Niederalkyl gewählt ist/sind, und worin ein stickstoffhaltiger Heterocyclus N-geschützt sein kann, und worin die Arylgruppe oder der Arylteil der Arylalkylgruppe wahlweise mit einem, zwei oder drei Substituenten substituiert sein kann, der/die unabhängig aus Niederalkyl, Haloalkyl, Alkoxy, Thioalkoxy, Amino, Alkylamino, Dialkylamino, Hydroxy, Halo, Mercapto, Nitro, Carboxaldehyd, Carboxy, Alkoxycarbonyl und Carboxamid gewählt ist/sind, oder worin die Arylgruppe oder der Arylteil der Arylalkylgruppe gleich Tetrafluorphenyl oder Pentafluorphenyl ist, und
(2) -(CH₂)_{q}-N(R₁)(R₂), worin q gleich 0, 1 oder 2 ist, und wobei R₁ und R₂ bei jedem Vorkommen unabhängig die oben definierte Bedeutung haben;
und worin B₁ und B₂ unabhängig aus folgendem gewählt sind:
(1) -CH₂OH,
(2) -CH=NOH,
(3) -W-R₅, wobei W bei jedem Vorkommen unabhängig aus folgendem gewählt ist: (a) einer kovalenten Bindung, (b) Alkylen, (c) Alkenylen, (d) -C(O)NH- und (e) -NHC(O)NH-, und wobei R₅ unabhängig aus folgendem gewählt ist:
(a) 5-Tetrazolyl,
(b) 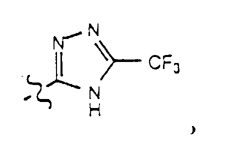
(c) 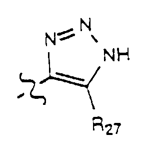 worin R₂₇ gleich -CN, -NO₂ oder -CO₂R₂₈ ist, worin R₂₈ gleich Wasserstoff, Aryl oder Niederalkyl ist, worin die Arylgruppe wahlweise mit einem, zwei oder drei Substituenten substituiert sein kann, die unabhängig aus Niederalkyl, Haloalkyl, Alkoxy, Thioalkoxy, Amino, Alkylamino, Dialkylamino, Hydroxy, Halo, Mercapto, Nitro, Carboxaldehyd, Carboxy, Alkoxycarbonyl und Carboxamid gewählt sind, oder worin die Arylgruppe gleich Tetrafluorphenyl oder Pentafluorphenyl sein kann,
(d) 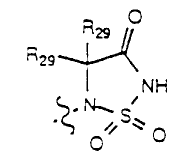 worin R₂₉ bei jedem Vorkommen aus Wasserstoff und Niederalkyl gewählt ist,
(e) 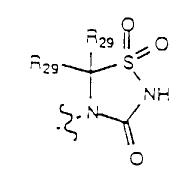 worin R₂₉ die oben definierte Bedeutung hat,
(f) 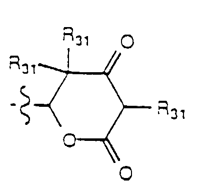 worin R₃₁ bei jedem Vorkommen aus Wasserstoff, Niederalkyl, Alkenyl, Alkoxyalkyl und Benzyl gewählt ist,
(g) 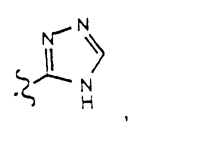
(h) 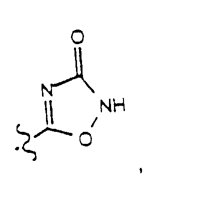
(i) 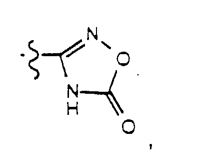
(j) 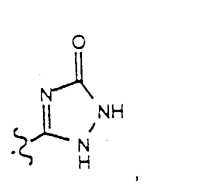
(k) 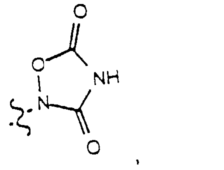
(l) 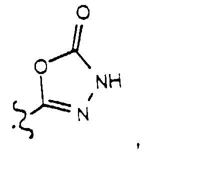
(m) 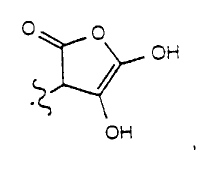 und
(n) 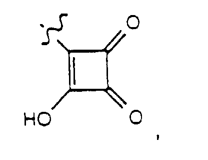
(4) -Q-C(O)R_{6,} worin Q bei jedem Vorkommen unabhängig aus folgendem gewählt ist: (a) einer kovalenten Bindung, (b) Alkylen, (c) Alkenylen, (d) -CH(OH)- und (e) -NHC(O)(CH₂)ᵣ-, worin r gleich 0 bis 4 ist, und worin R₆ bei jedem Vorkommen unabhängig aus folgendem gewählt ist: (a) -OR₇, worin R₇ gleich Wasserstoff oder einer Carboxyschutzgruppe ist, (b) -NH₂, (c) -NHOH, (d) -NHSO₂CF₃, (e) einer alpha-Aminosäure oder einer beta-Aminosäure, die über die alpha- oder beta-Aminogruppe gebunden ist, und (f) einem Di-, Tri- oder Tetrapeptid, das über die aminoterminale Aminogruppe gebunden ist,
(5) -CH₂-N(OH)-C(O)-R₂₅, worin R₂₅ gleich Wasserstoff, Methyl oder Trifluormethyl ist, und
(6) -C(O)-NH-S(O)₂-R₂₆, worin R₂₆ gleich Aryl, Heterocyclus, Arylalkyl, (Heterocyclus)alkyl, C₃-C₇-Cycloalkyl, C₁-C₈-Alkyl oder Perfluor-C₁-C₄-alkyl ist, worin die heterocyclische Gruppe oder der heterocyclische Teil der (Heterocyclus)alkylgruppe wahlweise mit einem oder zwei Substituenten substituiert sein kann, der/die die unabhängig aus Hydroxy, Halo, Oxo, Niederalkylimino, Amino, Alkylamino, Dialkylamino, Alkoxy, Alkoxyalkoxy, Haloalkyl, Cycloalkyl, Aryl, Arylalkyl, -COOH, -SO₃H und Niederalkyl gewählt ist/sind, und worin ein stickstoffhaltiger Heterocyclus N-geschützt sein kann, und wobei die Arylgruppe oder der Arylteil der Arylalkylgruppe wahlweise mit einem, zwei oder drei Substituenten substituiert sein kann, der/die unabhängig aus Niederalkyl, Haloalkyl, Alkoxy, Thioalkoxy, Amino, Alkylamino, Dialkylamino, Hydroxy, Halo, Mercapto, Nitro, Carboxaldehyd, Carboxy, Alkoxycarbonyl und Carboxamid gewählt ist/sind, oder worin die Arylgruppe oder der Arylteil der Arylalkylgruppe gleich Tetrafluorphenyl oder Pentafluorphenyl ist;
oder ein pharmazeutisch verträgliches Salz davon.

10. Verbindung nach Anspruch 9, worin A₁ und A₂ unabhängig aus -C(O)-G gewählt sind, worin G die oben angegebene Bedeutung hat, und
worin B₁ und B₂ unabhängig aus folgendem gewählt sind:
(a) -W-R₅, worin W eine kovalente Bindung oder Alkylen ist, und worin R₅ gleich 5-Tetrazolyl ist, und
(b) -Q-C(O)-R₆, wobei Q bei jedem Vorkommen unabhängig aus einer kovalenten Bindung und Alkylen gewählt ist, und wobei R₆ bei jedem Auftreten unabhängig aus folgendem gewählt ist:
(1) -OR₇, worin R₇ gleich Wasserstoff oder einer Carboxyschutzgruppe ist,
(2) einer alpha-Aminosäure oder einer beta-Aminosäure, die über die alpha- oder beta-Aminogruppe gebunden ist, und
(3) ein Di-, Tri- oder Tetrapeptid, das über die aminoterminale Aminogruppe gebunden ist.

11. Verbindung nach Anspruch 9, worin A₁ und A₂ unabhängig aus -C(O)-G gewählt sind, worin G gleich -N(R₁)(R₂) ist, worin R₁ und R₂ die oben definierte Bedeutung haben, und worin B₁ und B₂ unabhängig aus folgendem gewählt sind:
(a) -W-R₅, worin W eine kovalente Bindung oder Alkylen ist, und worin R₅ gleich 5-Tetrazolyl ist, und (b) -Q-C(O)-R₆, worin Q bei jedem Vorkommen unabhängig aus einer kovalenten Bindung und Alkylen gewählt ist, und wobei R₆ bei jedem Auftreten unabhängig aus folgendem gewählt ist:
(1) -OR₇, worin R₇ gleich Wasserstoff oder einer Carboxyschutzgruppe ist,
(2) einer alpha-Aminosäure oder einer beta-Aminosäure, die über die alpha- oder die beta-Aminogruppe gebunden ist, und
(3) einem Di-, Tri- oder Tetrapeptid, das über die aminoterminale Aminogruppe gebunden ist.

12. Verbindung nach Anspruch 9, worin A₁ und A₂ unabhängig aus -C(O)-G gewählt sind, worin G gleich -N(R₁)(R₂) ist, wobei R₁ bei jedem Auftreten unabhängig aus folgendem gewählt ist:
(a) Wasserstoff, (b) Niederalkyl, (c) Cycloalkyl, (d) Cycloalkylalkyl, (e) Alkoxyalkyl, (f) Thioalkoxyalkyl, (g) Aryl, (h) Heterocyclus, (i) Arylalkyl, (j) (Heterocyclus)alkyl, (k) Aryl-substituiertes Cycloalkylalkyl, (1) Heterocyclus-substituiertes Cycloalkylalkyl und (m) Aryl, Heterocyclus, Arylalkyl, (Heterocyclus)alkyl, Aryl-substituiertes Cycloalkylalkyl oder Heterocyclus-substituiertes Cycloalkylalkyl, worin die Arylgruppe, der Arylteil der Aryl alkyl gruppe , der Arylteil der Aryl-substituierten Alkylgruppe, die heterocyclische Gruppe, der heterocyclische Teil der (Heterocyclus)alkylgruppe oder der heterocyclische Teil der Heterocyclus-substituierten Cycloalkylalkylgruppe mit -Y-R₃ substituiert ist, wobei Y bei jedem Vorkommen unabhängig aus (i)-O-, (ii) -S(O)m-, worin m gleich 0, 1 oder 2 ist, und aus (iii) -N(R_{b})-, worin R_{b} gleich Wasserstoff oder Niederalkyl ist, gewählt ist, und wobei R₃ bei jedem Auftreten unabhängig aus folgendem gewählt ist: (i) Aryl, (ii) Arylalkyl, (iii) Cycloalkyl, (iv) Cycloalkylalkyl, (v) Heterocyclus und (vi) (Heterocyclus)alkyl, und wobei R₂ bei jedem Auftreten unabhängig aus Aryl, Heterocyclus, Arylalkyl und (Heterocyclus)alkyl gewählt ist, wobei die Arylgruppe, der Arylteil der Arylalkylgruppe, die heterocyclische Gruppe oder der heterocyclische Teil der (Heterocyclus)alkylgruppe mit -Z-R₄ substituiert ist,
wobei Z bei jedem Vorkommen unabhängig aus folgendem gewählt ist: (i) -O-, (ii) -S(O)ₚ-, worin p gleich 0, 1 oder 2 ist und (iii) -N(R_{c})-, worin R_{c} gleich Wasserstoff oder Niederalkyl ist, und wobei R₄ bei jedem Vorkommen unabhängig aus folgendem gewählt ist: (i) Aryl, (ii) Arylalkyl, (iii) Cycloalkyl, (iv) Cycloalkylalkyl, (v) Heterocyclus und (vi) (Heterocyclus)alkyl, worin, wenn R₃ oder R₄ ein Heterocyclus, (Heterocyclus)alkyl, Aryl oder eine Arylalkylgruppe ist, die heterocyclische Gruppe oder der heterocyclische Teil der (Heterocyclus)alkylgruppe wahlweise mit einem oder zwei Substituenten substituiert sein kann, der/die unabhängig aus Hydroxy, Halo, Oxo, Niederalkylimino, Amino, Alkylamino, Dialkylamino, Alkoxy, Alkoxyalkoxy, Haloalkyl, Cycloalky, Aryl, Arylalkyl, -COOH, -SO₃H und Niederalkyl gewählt ist/sind, und wobei ein stickstoffhaltiger Heterocyclus N-geschützt sein kann, und wobei die Arylgruppe oder der Arylteil der Arylalkylgruppe wahlweise mit einem, zwei oder drei Substituenten substituiert sein kann, der/die unabhängig aus Niederalkyl, Haloalkyl, Alkoxy, Thioalkoxy, Amino, Alkylamino, Dialkylamino, Hydroxy, Halo, Mercapto, Nitro, Carboxaldehyd, Carboxy, Alkoxycarbonyl und Carboxamid gewählt ist/sind, oder worin die Arylgruppe oder der Arylteil der Arylalkylgruppe gleich Tetrafluorphenyl oder Pentafluorphenyl ist, und wobei
B₁ und B₂ unabhängig aus folgendem gewählt sind:
(a) -W-R₅, worin W eine kovalente Bindung oder Alkylen ist, und wobei R₅ gleich 5-Tetrazolyl ist, und
(b) -Q-C(O)-R₆, worin Q bei jedem Vorkommen unabhängig aus einer kovalenten Bindung und Alkylen gewählt ist, und wobei R₆ bei jedem Vorkommen unabhängig aus folgendem gewählt ist:
(1) -OR₇, worin R₇ gleich Wasserstoff oder einer Carboxyschutzgruppe ist,
(2) einer alpha-Aminosäure oder einer beta-Aminosäure, die über die alpha- oder beta-Aminogruppe gebunden ist und
(3) einem Di-, Tri- oder Tetrapeptid, das über die aminoterminale Aminogruppe gebunden ist.

13. Verbindung nach Anspruch 9, worin
A₁ und A₂ unabhängig aus -C(O)-G gewählt sind, worin G gleich-N(R₁) (R₂) ist, worin R₁ bei jedem Vorkommen unabhängig aus folgendem gewählt ist:
(a) Niederalkyl, (b) Cycloalkyl und (c) Cycloalkylalkyl, und worin R₂ bei jedem Vorkommen unabhängig aus Aryl und Arylalkyl gewählt ist, wobei die Arylgruppe oder der Arylteil des Arylalkyls mit -Z-R₄ substituiert ist, wobei Z bei jedem Vorkommen unabhängig aus (i) -O- und (ii) -S- gewählt ist, und wobei R₄ bei jedem Vorkommen unabhängig aus folgendem gewählt ist: (i) Aryl, (ii) Arylalkyl, (iii) Cycloalkyl, (iv) Cycloalkylalkyl, (v) Heterocyclus und (vi) (Heterocyclus)alkyl, wobei die heterocyclische Gruppe oder der heterocyclische Teil der (Heterocyclus)alkylgruppe wahlweise mit einem oder zwei Substituenten substituiert sein kann, der/die unabhängig aus Hydroxy, Halo, Oxo, Niederalkylimino, Amino, Alkylamino, Dialkylamino, Alkoxy, Alkoxyalkoxy, Haloalkyl, Cycloalkyl, Aryl, Arylakyl, -COOH, -SO₃H und Niederalkyl gewählt ist/sind, und worin ein stickstoffhaltiger Heterocyclus N-geschützt sein kann, und worin die Arylgruppe oder der Arylteil der Arylalkylgruppe wahlweise mit einem, zwei oder drei Substituenten substituiert sein kann, der/die unabhängig aus Niederalkyl, Haloalkyl, Alkoxy, Thioalkoxy, Amino, Alkylamino, Dialkylamino, Hydroxy, Halo, Mercapto, Nitro, Carboxaldehyd, Carboxy, Alkoxycarbonyl und Carboxamid gewählt ist/sind, oder wobei die Arylgruppe oder der Arylteil der Arylalkylgruppe gleich Tetrafluorphenyl oder Pentafluorphenyl ist, und wobei B₁ und B₂ unabhängig aus -W-R₅ und -Q-C(O)-R₆ gewählt sind, wobei Q und W bei jedem Vorkommen unabhängig aus einer kovalenten Bindung und Alkylen gewählt sind, worin R₆ gleich -OR₇ ist, worin R₇ gleich Wasserstoff oder eine Carboxyschutzgruppe ist, und worin R₅ gleich 5-Tetrazolyl ist.

14. Verbindung nach Anspruch 9, worin
A₁ und A₂ unabhängig aus -C(O)-G gewählt sind, worin G gleich -N(R₁)(R₂) ist, worin R₁ bei jedem Vorkommen unabhängig aus folgendem gewählt ist:
(a) Niederalkyl, (b) Cycloalkyl und (c) Cycloalkylalkyl, und wobei R₂ bei jedem Vorkommen unabhängig aus Phenyl und Benzyl gewählt ist, worin die Phenylgruppe oder der Phenylring der Benzylgruppe mit -Z-R₄ substituiert ist,
wobei Z bei jedem Vorkommen unabhängig aus (i) -O- und (ii) -S- gewählt ist, und wobei R₄ bei jedem Vorkommen unabhängig aus (i) Aryl, (ii) Arylalkyl, (iii) Heterocyclus und (iv) (Heterocyclus)alkyl gewählt ist, worin die heterocyclische Gruppe oder der heterocyclische Teil der (Heterocyclus)alkylgruppe wahlweise mit einem oder zwei Substituenten substituiert sein kann, der/die unabhängig aus Hydroxy, Halo, Oxo, Niederalkylimino, Amino, Alkylamino, Dialkylamino, Alkoxy, Alkoxyalkoxy, Haloalkyl, Cycloalkyl, Aryl, Arylalkoxy, -COOH, -SO₃H und Niederalkyl gewählt ist/sind, und worin ein stickstoffhaltiger Heterocyclus N-geschützt sein kann, und worin die Arylgruppe oder der Arylteil der Arylalkylgruppe wahlweise mit einem, zwei oder drei Substituenten substituiert sein kann, die unabhängig aus Niederalkyl, Haloalkyl, Alkoxy, Thioalkoxy, Amino, Alkylamino, Dialkylamino, Hydroxy, Halo, Mercapto, Nitro, Carboxaldehyd, Carboxy, Alkoxycarbonyl und Carboxamid gewählt ist/sind, oder worin die Arylgruppe oder der Arylteil der Arylalkylgruppe gleich Tetrafluorphenyl oder Pentafluorphenyl ist, und
worin B₁ und B₂ unabhängig aus -W-R₅ und -Q-C(O)-R₆ gewählt sind, wobei Q und W bei jedem Vorkommen unabhängig aus einer kovalenten Bindung und Alkylen gewählt sind, worin R₆ gleich -OR₇ ist, worin R₇ gleich Wasserstoff oder eine Carboxyschutzgruppe ist, und worin R₅ gleich 5-Tetrazolyl ist.

15. Verbindung nach Anspruch 9,
worin A₁ und A₂ unabhängig aus -C(O)-G gewählt sind, worin G gleich -N(R₁) (R₂) ist,
worin R₁ bei jedem Vorkommen unabhängig aus (a) Niederalkyl, (b) Cycloalkyl und (c) Cycloalkylalkyl gewählt ist, und worin R₂ gleich Benzyl ist, worin der Phenylring der Benzylgruppe mit -Z-R₄ substituiert ist, worin Z bei jedem Vorkommen unabhängig aus (i) -O- und (ii) -S- gewählt ist, und worin R₄ gleich Aryl ist, und worin
B₁ und B₂ unabhängig aus -W-R₅ und -Q-C(O)-R₆ gewählt sind,
wobei Q und W bei jedem Vorkommen unabhängig aus einer kovalenten Bindung und Alkylen gewählt sind, worin R₆ gleich -OR₇ ist, worin R₇ gleich Wasserstoff oder einer Carboxyschutzgruppe ist, und worin R₅ gleich 5-Tetrazolyl ist.

16. Verbindung nach Anspruch 9, worin A₁ und A₂ unabhängig aus -C(O)-G gewählt sind, worin G gleich-N(R₁) (R₂) ist, worin R₁ bei jedem Vorkommen gleich (a) Niederalkyl, (b) Cycloalkyl, (c) Cycloalkylalkyl ist, und worin R₂ gleich Benzyl ist, worin der Phenylring der Benzylgruppe mit -Z-R₄ substituiert ist, worin Z bei jedem Vorkommen unabhängig aus (i) -O- und (ii) -S- gewählt ist, und worin R₄ gleich einem Heterocyclus ist, worin die heterocyclische Gruppe wahlweise mit einem oder zwei Substituenten substituiert sein kann, der/die unabhängig aus Hydroxy, Halo, Oxo, Niederalkylimino, Amino, Alkylamino, Dialkylamino, Alkoxy, Alkoxyalkoxy, Haloalkyl, Cyloalkyl, Aryl, Arylalkyl, -COOH, -SO₃H und Niederalkyl gewählt ist/sind, und worin ein stickstoffhaltiger Heterocyclus N-geschützt sein kann, und worin die Arylgruppe oder der Arylteil der Arylalkylgruppe wahlweise mit einem, zwei oder drei Substituenten substituiert sein kann, der/die unabhängig aus Niederalkyl, Haloalkyl, Alkoxy, Thioalkoxy, Amino, Alkylamino, Dialkylamino, Hydroxy, Halo, Mercapto, Nitro, Carboxaldehyd, Carboxy, Alkoxycarbonyl und Carboxamid gewählt ist/sind, oder worin die Arylgruppe oder der Arylteil der Arylalkylgruppe gleich Tetrafluorphenyl oder Pentafluorphenyl ist, und worin B₁ und B₂ unabhängig aus -W-R₅ und-Q-C(O)-R₆ gewählt sind, wobei Q und W bei jedem Vorkommen unabhängig aus einer kovalenten Bindung und Alkylen gewählt sind, wobei R₆ gleich -OR₇ ist, worin R₇ gleich Wasserstoff oder einer Carboxyschutzgruppe ist, und wobei R₅ gleich 5-Tetrazolyl ist.

17. Eine Verbindung, die aus der Gruppe gewählt ist, die aus folgendem besteht:
(1α,2β,3β,4α) -1,2-Di(N-Benzyl-N-(4-phenoxybenzyl)aminocarbonyl)cyclobutan-3,4-dicarbonsäure;
(1α,2β,3β,4α)-1,2-Di(N-Propyl-N-(4-phenoxybenzyl)aminocarbonyl)cyclobutan-3,4-dicarbonsäure;
(-)-(1α,2β,3β,4α)-1,2-Di(N-Propyl-N-(4-phenoxybenzyl)aminocarbonyl)cyclobutan-3,4-dicarbonsäure;
(1α,2β,3β,4α)-1,2-Di(N-(2-Ethylthioethyl)-N-(4-phenoxybenzyl)aminocarbonyl)cyclobutan-3,4-dicarbonsäure;
(1α,2β,3β,4α)-1,2-Di(N-(Cyclopropylmethyl)-N-(4phenoxybenzyl)aminocarbonyl)cyclobutan-3,4-dicarbonsäure;
(1α,2β,3β,4α)-1-(N-Propyl-N-(4-phenoxybenzyl)aminocarbonyl)-2-(N-benzyl-N-(4-phenoxybenzyl)aminocarbonyl)-cyclobutan-3,4-dicarbonsäure;
(1α,2β,3β,4α)-1,2-Di(N-Propyl-N-(4-phenylthiobenzyl)aminocarbonyl)cyclobutan-3,4-dicarbonsäure;
(1α,2β,3β,4α)-1,2-Di(N-Propyl-N-(4-phenoxybenzyl)-aminocarbonyl)-4-tetrazolylmethyl-cyclobutan-3-carbonsäure;
(1α,2β,3β,4α)-1,2-Di(N-Propyl-N-(4-phenoxybenzyl)-aminocarbonyl)-4-(3-carboxypropionylamino)cyclobutan-3-carbonsäure;
(1α,2β,3β,4α)-1,2-Di(N-Propyl-N-(4-phenoxybenzyl)-aminocarbonyl)-4-(1-carboxy-1-hydroxymethyl)cyclobutan-3-carbonsäure;
(1α,2β,3β,4α)-1,2-Di(N-Propyl-N-(4-phenoxybenzyl)aminocarbonyl)cyclobutan-3-(N-(5-tetrazolyl))carboxamid-4-carbonsäure;
(1α,2β,3β,4α)-4-(Carboxymethyl)-1,2-di(N-propyl-N-(4-phenoxybenzyl)aminocarbonyl)cyclobutan-3-carbonsäure; und
(1α,2β,3β,4α)-1,2-Di(-N-Propyl-N-(4-phenoxybenzyl)aminocarbonyl)cyclobutan-3,4-diessigsäure;
oder ein pharmazeutisch verträgliches Salz davon.

18. Pharmazeutische Zusammensetzung zur Inhibition von Squalensynthetase, die eine therapeutisch wirksame Menge einer Verbindung nach Anspruch 1 und einen pharmazeutisch verträglichen Träger umfaßt.

19. Pharmazeutische Zusammensetzung zur Inhibition der Squalensynthetase, die eine therapeutisch wirksame Menge einer Verbindung nach Anspruch 9 und einen pharmazeutisch verträglichen Träger umfaßt.

20. Verwendung einer Verbindung nach Anspruch 1 zur Herstellung eines Medikamentes zur Inhibition der Cholesterinbiosynthese bei einem Menschen oder Säuger.

21. Verwendung einer Verbindung nach Anspruch 9 zur Herstellung eines Medikamentes zur Inhibition der Cholersterinbiosynthese bei einem Menschen oder Säuger.

22. Verwendung einer Verbindung nach Anspruch 1 zur Herstellung eines Medikamentes zur Behandlung von Hyperlipidaemie oder Atherosklerose bei einem Säuger.

23. Verwendung eines Verbindung nach Anspruch 9 zur Herstellung eines Medikamentes zur Behandlung von Hyperlipidaemie oder Atherosklerose bei einem Säuger.

24. Verwendung einer Verbindung nach Anspruch 1 zur Herstellung eines Medikamentes zur Behandlung einer Pilzinfektion bei einem Säuger.

25. Verwendung einer Verbindung nach Anspruch 9 zur Herstellung eines Medikamentes zur Behandlung einer Pilzinfektion bei einem Säuger.

26. Verbindung nach Anspruch 1 zur Verwendung als therapeutischer Wirkstoff.

## Revendications

1. Composé de formule : 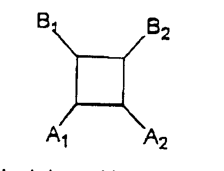 dans laquelle A₁ et A₂ sont choisis indépendamment parmi
(1) -X-C(O)-G ou -X-C(S)-G dans lesquels à chaque occurrence X est choisi indépendamment parmi (a) une liaison covalente, (b) -CH₂-, (c) -O-, (d) -S- et (e) -N(Rₐ)- dans lequel Rₐ est l'hydrogène, un groupe alkyle inférieur, cycloalkyle ou cycloalkylalkyle et chaque occurrence G est choisi indépendamment parmi -R₂, -N(R₁)(R₂), -OR₂ et -SR₂ dans lesquels à chaque occurrence R₁ est choisi indépendamment parmi (a) l'hydrogène, un groupe (b) alkyle inférieur, (c) alcényle, (d) alcynyle, (e) cycloalkyle, (f) cycloalkylalkyle, (g) alcoxycarbonylalkyle, (h) alcoxyalkyle, (i) thioalcoxyalkyle, (j) haloalkyle, (k) aryle, (I) hétérocyclique, (m) arylalkyle ,(n) cycloalkylalkyle substitué par un aryle, (o) alkylhétérocyclique, (p) cycloalkylalkyle substitué par un hétérocycle et (q) un groupe aryle, hétérocyclique, arylalkyle, cycloalkylalkyle substitué par un aryle, alkylhétérocyclique ou cycloalkylalkyle substitué par un hétérocycle dans lequel le groupe aryle, la partie aryle du groupe arylalkyle, la partie aryle du groupe cycloalkylalkyle substitué par un aryle, le groupe hétérocyclique, la partie hétérocyclique du groupe alkylhétérocyclique ou la partie hétérocyclique du groupe cycloalkylalkyle substitué par un hétérocycle est substituée par -Y-R₃ dans lequel à chaque occurrence Y est choisi indépendamment parmi (i) une liaison covalente, (ii) -C(O)-, (iii) -CH₂-, (iv) -O-, (v) -S(O)ₘ- dans lequel m vaut 0, 1 ou 2, (vi) -N(R_{b})- dans lequel R_{b} est l'hydrogène ou un alkyle inférieur, (vii) -CH₂O-, (viii) -CH₂S(O)ₘ- dans lequel m vaut 0, 1 ou 2 et (ix) -CH₂N(R_{b})- dans lequel R_{b} est l'hydrogène ou un alkyle inférieur et à chaque occurrence R₃ est choisi indépendamment parmi un groupe (i) aryle, (ii) arylalkyle, (iii) cycloalkyle, (iv) cycloalkylalkyle, (v) hétérocyclique et (vi) alkylhétérocyclique et à chaque occurrence R₂ est choisi indépendamment parmi un groupe (i) alcényle, (ii) alcynyle, (iii) aryle, (iv) arylalkyle, (v) arylalcényle, (vi) hétérocyclique, (vii) alkylhétérocyclique et (vi) aryle, hétérocyclique, arylalkyle ou alkylhétérocyclique dans lequel le groupe aryle, la partie aryle du groupe arylalkyle, le groupe hétérocyclique ou la partie hétérocyclique du groupe alkylhétérocyclique est substitué par -Z-R₄ dans lequel à chaque occurrence Z est choisi indépendamment parmi (i) une liaison covalente, (ii) -C(O)-, (iii) -CH₂-, (iv) -O-, (v) -S(O)ₚ- dans lequel p vaut 0, 1 ou 2, (vi) -N(R_{c})- dans lequel R_{c} est l'hydrogène ou un alkyle inférieur, (vii) -CH₂O-, (viii) -CH₂S(O)ₚ- dans lequel p vaut 0, 1 ou 2 et (ix) -CH₂N(R_{c})- dans lequel R_{c} est l'hydrogène ou un alkyle inférieur et à chaque occurrence R₄ est choisi indépendamment parmi un groupe (i) aryle, (ii) arylalkyle, (iii) cycloalkyle, (iv) cycloalkylalkyle, (v) hétérocyclique et (vi) alkylhétérocyclique,
formule dans laquelle R₃ ou R₄ est un groupe hétérocyclique, alkylhétérocyclique, aryle ou arylalkyle, le groupe hétérocyclique ou la partie hétérocyclique du groupe alkylhétérocyclique peut être éventuellement substitué par un ou deux substituants choisis indépendamment parmi un groupe hydroxy, halo, oxo, alkylimino inférieur, amino, alkylamino, dialkylamino, alcoxy, alcoxyalcoxy, haloalkyle, cycloalkyle, aryle, arylalkyle, -COOH, -SO₃H et un alkyle inférieur, et un hétérocycle contenant de l'azote pouvant être protégé sur N, et le groupe aryle ou la partie aryle du groupe arylalkyle peut être éventuellement substitué par un, deux ou trois substituants choisis indépendamment parmi un groupe alkyle inférieur, haloalkyle, alcoxy, thioalcoxy, amino, alkylamino, dialkylamino, hydroxy, halo, mercapto, nitro, carboxaldéhyde, carboxy, alcoxycarbonyle et carboxamide, ou le groupe aryle ou la partie aryle du groupe arylalkyle est un groupe tétrafluorophényle ou pentafluorophényle, et
(2) -(CH₂)_{q}-N(R₁)(R₂) dans lequel q vaut 0, 1 ou 2 et à chaque occurrence R₁ et R₂ sont définis indépendamment comme ci-dessus ; et
B₁ et B₂ sont choisis indépendamment parmi
(1) -CH₂OH,
(2) -CH = NOH,
(3) -W-R₅ dans lequel à chaque occurrence W est choisi indépendamment parmi (a) une liaison covalente, un groupe (b) alkyléne, (c) alcénylène, (d) -C(O)NH- et (e) -NHC(O)NH- et R₅ est choisi indépendamment parmi
(a) un groupe 5-tétrazolyle,
(b) 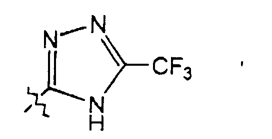
(c) 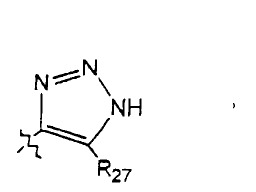 dans laquelle R₂₇ est -CN, -NO₂ ou -CO₂R₂₈ dans lequel R₂₈ est l'hydrogène, un groupe aryle ou alkyle inférieur, dans lequel le groupe aryle peut éventuellement être substitué par un, deux ou trois substituants choisis indépendamment parmi un groupe alkyle inférieur, haloalkyle, alcoxy, thioalcoxy, amino, alkylamino, dialkylamino, hydroxy, halo, mercapto, nitro, carboxaldéhyde, carboxy, alcoxycarbonyle et carboxamide, ou le groupe aryle peut être le groupe tétrafluorophényle ou pentafluorophényle,
(d) 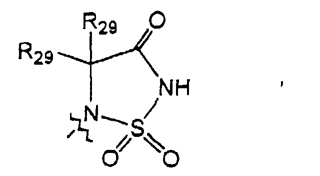 formule dans laquelle à chaque occurrence R₂₉ est choisi parmi l'hydrogène et un alkyle inférieur,
(e) 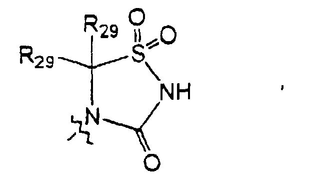 formule dans laquelle R₂₉ est tel que défini ci-dessus,
(f) 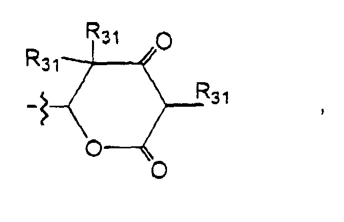 formule dans laquelle R₃₁ est à chaque occurrence choisi parmi l'hydrogène, un alkyle inférieur, alcényle, alcoxyalkyle et benzyle,
(g) 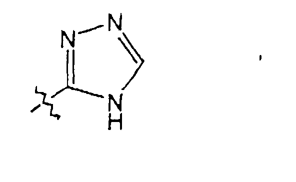
(h) 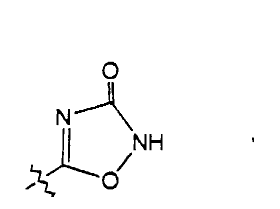
(i) 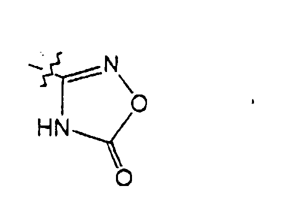
(j) 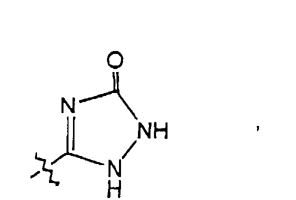
(k) 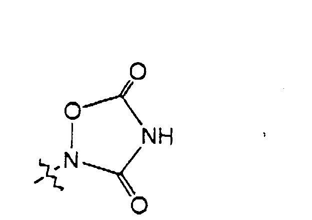
(l) 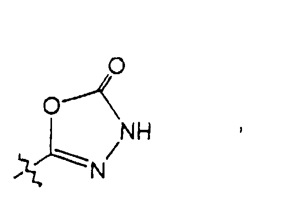
(m) 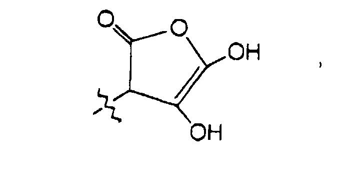 et
(n) 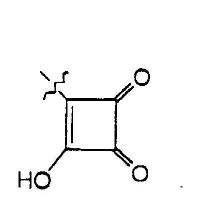
(4) -Q-C(O)R₆ dans laquelle à chaque occurrence Q est choisi indépendamment parmi (a) une liaison covalente, un groupe (b) alkylène, (c) alcénylène, (d) -CH(OH)- et (e) -NHC(O)(CH₂)ᵣ- dans lequel r vaut de 0 à 4 et à chaque occurrence R₆ est choisi indépendamment parmi (a) -OR₇ dans lequel R₇ est l'hydrogène ou un groupe protecteur du groupe carboxy, (b) -NH₂, (c) -NHOH, (d) -NHSO₂CF₃, (e) un acide alpha-aminé ou un acide bêta-aminé qui est lié via le groupe alpha- ou béta-amino et (f) un di-, tri- ou tétra-peptide qui est lié via le groupe amino terminal amino,
(5) -CH₂-N(OH)-C(O)-R₂₅ dans lequel R₂₅ est l'hydrogène, un groupe méthyle ou trifluorométhyle, et
(6) -C(O)-NH-S(O)₂-R₂₆ dans lequel R₂₆ est un groupe aryle, hétérocyclique, arylakyle, alkylhétérocyclique, cycloalkyle en C₃-C₇, alkyle en C₁-C₈ ou perfluoroalkyle en C₁-C₄,
dans lequel le groupe hétérocyclique ou la partie hétérocyclique du groupe alkylhétérocyclique peut éventuellement être substitué par un ou deux substituants choisis indépendamment parmi un groupe hydroxy, halo, oxo, alkylimino inférieur, amino, alkylamino, dialkylamino, alcoxy, alcoxyalcoxy, haloalkyle, cycloalkyle, aryle, arylalkyle, -COOH, -SO₃H et alkyle inférieur, et un hétérocycle contenant de l'azote pouvant avoir le N protégé, et le groupe aryle ou la partie aryle du groupe arylalkyle pouvant éventuellement être substitué par un, deux ou trois substituants choisis indépendamment parmi un groupe alkyle inférieur, haloalkyle, alcoxy, thioalcoxy, amino, alkylamino, dialkylamino, hydroxy, halo, mercapto, nitro, carboxaldéhyde, carboxy, alcoxycarbonyle et carboxamide, ou le groupe aryle ou la partie aryle du groupe arylalkyle est un groupe tétrafluorophényle ou pentafluorophényle;
ou son sel acceptable pharmaceutiquement.

2. Composé tel que défini dans la revendication 1, caractérisé en ce que A₁ et A₂ sont choisis indépendamment parmi -C(O)-G dans lequel G est défini comme ci-dessus et
B₁ et B₂ sont choisis indépendamment parmi
(a) -W-R₅ dans lequel W est une liaison covalente ou un groupe alkylène et R₅ est le groupe tétrazolyle et
(b) -Q-C(O)-R₆ dans lequel à chaque occurrence Q est choisi indépendamment parmi une liaison covalente et un groupe alkylène et à chaque occurrence R₆ est choisi indépendamment parmi
(1) -OR₇ dans lequel R₇ est l'hydrogène ou un groupe protecteur du groupe carboxy,
(2) un acide alpha-aminé ou un acide béta-aminé qui est lié via le groupe alpha- ou béta-amino et
(3) un di-, tri- ou tétra-peptide qui est lié via le groupe amino terminal amino.

3. Composé tel que défini dans la revendication 1, caractérisé en ce que A₁ et A₂ sont choisis indépendamment parmi -C(O)-G dans lequel G est -N(R₁)(R₂) dans lequel R₁ et R₂ sont définis comme ci-dessus et B₁ et B₂ sont choisis indépendamment parmi
(a) -W-R₅ dans lequel W est une liaison covalente ou un groupe alkylène et R₆ est le groupe 5-tétrazolyle et
(b) -Q-C(O)-R₆ dans lequel à chaque occurrence Q est choisi indépendamment parmi une liaison covalente et un groupe alkylène et à chaque occurrence R₆ est choisi indépendamment parmi
(1) -OR₇ dans lequel R₇ est l'hydrogène ou un groupe protecteur du groupe carboxy,
(2) un acide alpha-aminé ou un acide bêta-aminé qui est lié via le groupe alpha-amino ou béta-amino et
(3) un di-, tri- ou tétra-peptide qui est lié via le groupe amino terminal amino.

4. Composé tel que défini dans la revendication 1, caractérisé en ce que A₁ et A₂ sont indépendamment choisis parmi -C(O)-G dans lequel G est -N(R₁)(R₂) dans lequel à chaque occurrence R₁ est choisi indépendamment parmi (a) l'hydrogène, un groupe (b) alkyle inférieur (c) cycloalkyle, (d) cycloalkylalkyle, (e) alcoxyalkyle, (f) thioalcoxyalkyle, (g) aryle, (h) hétérocyclique, (i) arylalkyle, (j) alkylhétérocyclique, (k) cycloalkylalyle substitué par un groupe aryle, (I) cycloalkylalkyle substitué par un groupe hétérocyclique et (m) un groupe aryle, hétérocyclique, arylalkyle, alkylhétérocyclique, cycloalkylalkyle substitué par un groupe aryle ou cycloalkylalkyle substitué par un groupe hétérocyclique dans lequel le groupe aryle, la partie aryle du groupe arylalkyle, la partie aryle du groupe cycloalkylalkyle substitué par un groupe aryle, le groupe hétérocyclique, la partie hétérocyclique du groupe alkylhétérocyclique ou la partie hétérocyclique du groupe cycloalkylalkyle substitué par un groupe hétérocyclique est substituée par -Y-R₃ dans lequel à chaque occurrence Y est choisi indépendamment parmi (i) -O-, (ii) -S(O)ₘ- dans lequel m vaut 0, 1 ou 2 et (iii) -N(R_{b})- dans lequel R_{b} est l'hydrogène ou un groupe alkyle inférieur et à chaque occurrence R₃ est choisi indépendamment parmi un groupe (i) aryle, (ii) arylalkyle, (iii) cycloalkyle, (iv) cycloalkylalkyle, (v) hétérocyclique et (vi) alkylhétérocyclique et à chaque occurrence R₂ est choisi indépendamment parmi un groupe aryle, hétérocyclique, arylalkyle et alkylhétérocyclique dans lequel le groupe aryle, la partie aryle du groupe arylalkyle, le groupe hétérocyclique ou la partie hétérocyclique du groupe alkylhétérocyclique est substitué par -Z- R₄ dans lequel à chaque occurrence Z est choisi indépendamment parmi (i) -O-, (ii) -S(O)ₚ- dans lequel p vaut 0, 1 ou 2 et (iii) -N(R_{c})- dans lequel R_{c} est l'hydrogène ou un groupe alkyle inférieur et à chaque occurrence R₄ est choisi indépendamment parmi un groupe (i) aryle, (ii) arylalkyle, (iii) cycloalkyle, (iv) cycloalkylalkyle, (v) hétérocyclique, et (vi) alkylhétérocyclique, dans lequel, quand R₃ ou R₄ est un groupe hétérocyclique, alkylhétérocyclique, aryle ou arylalkyle, le groupe hétérocyclique ou la partie hétérocyclique du groupe alkylhétérocyclique peut être éventuellement substitué par un ou deux substituants choisis indépendamment parmi un groupe hydroxy, halo, oxo, alkylimino inférieur, amino, alkylamino, dialkylamino, alcoxy, alcoxyalcoxy, haloalkyle, cycloalkyle, aryle, arylalkyle, -COOH, -SO₃H et alkyle inférieur, et un hétérocycle contenant de l'azote peut être protégé pour N, et le groupe aryle ou la partie aryle du groupe arylalkyle peut être éventuellement substitué par un, deux ou trois substituants choisis indépendamment parmi un groupe alkyle inférieur, haloalkyle, alcoxy, thioalcoxy, amino, alkylamino, dialkylamino, hydroxy, halo, mercapto, nitro, carboxaldéhyde, carboxy, alcoxycarbonyle et carboxamide, ou le groupe aryle ou la partie aryle du groupe arylalkyle est le groupe tétrafluorophényle ou pentafluorophényle, et
B₁ et B₂ sont choisis indépendamment parmi
(a) -W-R₅ dans lequel W est une liaison covalente ou un groupe alkylène et R₅ est le groupe 5-tétrazolyle et
(b) -Q-C(O)-R₆ dans lequel à chaque occurrence Q est choisi indépendamment parmi une liaison covalente et un groupe alkylène et à chaque occurrence R₆ est choisi indépendamment parmi
(1) -OR₇ dans lequel R₇ est l'hydrogène ou un groupe protecteur du groupe carboxy,
(2) un acide alpha-aminé ou un acide béta-aminé qui est lié via le groupe alpha-amino ou béta-amino et
(3) un di-, tri- ou tétra-peptide qui est lié via le groupe amino terminal amino.

5. Composé tel que défini dans la revendication 1, caractérisé en ce que A₁ et A₂ sont choisis indépendamment parmi -C(O)-G dans lequel G est -N(R₁)(R₂) dans lequel à chaque occurrence R₁ est choisi indépendamment parmi (a) un groupe alkyle inférieur (b) cycloalkyle, et (c) cycloalkylalkyle et à chaque occurrence R₂ est choisi indépendamment parmi un groupe aryle et arylalkyle dans lequel le groupe aryle ou la partie aryle du groupe arylalkyle est substitué par -Z-R₄ dans lequel à chaque occurrence Z est choisi indépendamment parmi (i) -O-, (ii) -S- et à chaque occurrence R₄ est choisi indépendamment parmi un groupe (i) aryle, (ii) arylalkyle, (iii) cycloalkyle, (iv) cycloalkylalkyle, (v) hétérocyclique, et (vi) alkylhétérocyclique, dans lequel le groupe hétérocyclique ou la partie hétérocyclique du groupe alkylhétérocyclique peut être éventuellement substitué par un ou deux substituants choisis indépendamment parmi un groupe hydroxy, halo, oxo, alkylimino inférieur, amino, alkylamino, dialkylamino, alcoxy, alcoxyalcoxy, haloalkyle, cycloalkyle, aryle, arylalkyle, -COOH, -SO₃H et alkyle inférieur, et un hétérocycle contenant de l'azote peut être protégé pour N, et le groupe aryle ou la partie aryle du groupe arylalkyle peut être éventuellement substitué par un, deux ou trois substituants choisis indépendamment parmi un groupe alkyle inférieur, haloalkyle, alcoxy, thioalcoxy, amino, alkylamino, dialkylamino, hydroxy, halo, mercapto, nitro, carboxaldéhyde, carboxy, alcoxycarbonyle et carboxamide, ou le groupe aryle ou la partie aryle du groupe arylalkyle est le groupe tétrafluorophényle ou pentafluorophényle, et
B₁ et B₂ sont choisis indépendamment parmi -W-R₅ et -Q-C(O)-R₆ dans lesquels à chaque occurrence Q et W sont choisis indépendamment parmi une liaison covalente et un groupe alkylène, R₆ est -OR₇ dans lequel R₇ est un hydrogène ou un groupe protecteur du groupe carboxy et R₅ est le groupe 5-tétrazolyle.

6. Composé tel que défini dans la revendication 1, caractérisé en ce que A₁ et A₂ sont choisis indépendamment parmi -C(O)-G dans lequel G est -N(R₁)(R₂) dans lequel à chaque occurrence R₁ est choisi indépendamment parmi un groupe (a) alkyle inférieur (b) cycloalkyle, et (c) cycloalkylalkyle et à chaque occurrence R₂ est choisi indépendamment parmi un groupe phényle et benzyle dans lequel le groupe phényle ou le cycle phényle du groupe benzyle est substitué par -Z-R₄ dans lequel à chaque occurrence Z est choisi indépendamment parmi (i) -O-, (ii) -S- et à chaque occurrence R₄ est choisi indépendamment parmi un groupe (i) aryle, (ii) arylalkyle, (iii) hétérocyclique et (iv) alkylhétérocyclique, dans lequel le groupe hétérocyclique ou la partie hétérocyclique du groupe alkylhétérocyclique peut être éventuellement substitué par un ou deux substituants choisis indépendamment parmi un groupe hydroxy, halo, oxo, alkylimino inférieur, amino, alkylamino, dialkylamino, alcoxy, alcoxyalcoxy, haloalkyle, cycloalkyle, aryle, arylalkyle, -COOH, -SO₃H et alkyle inférieur, et un hétérocycle contenant de l'azote peut être protégé pour N, et le groupe aryle ou la partie aryle du groupe arylalkyle peut être éventuellement substitué par un, deux ou trois substituants choisis indépendamment parmi un groupe alkyle inférieur, haloalkyle, alcoxy, thioalcoxy, amino, alkylamino, dialkylamino, hydroxy, halo, mercapto, nitro, carboxaldéhyde, carboxy, alcoxycarbonyle et carboxamide, ou le groupe aryle ou la partie aryle du groupe arylalkyle est le groupe tétrafluorophényle ou pentafluorophényle, et
B₁ et B₂ sont choisis indépendamment parmi -W-R₅ et -Q-C(O)-R₆ dans lequel à chaque occurrence Q et W sont choisis indépendamment parmi une liaison covalente et un groupe alkylène, R₆ est -OR₇ dans lequel R₇ est un hydrogène ou un groupe protecteur du groupe carboxy et R₅ est le groupe 5-tétrazolyle.

7. Composé tel que défini dans la revendication 1, caractérisé en ce que A₁ et A₂ sont choisis indépendamment parmi -C(O)-G dans lequel G est -N(R₁)(R₂) dans lequel à chaque occurrence R₁ est choisi indépendamment parmi un groupe (a) alkyle inférieur (b) cycloalkyle et (c) cycloalkylalkyle et R₂ est le groupe benzyle dans lequel le cycle phényle du groupe benzyle est substitué par -Z-R₄ dans lequel à chaque occurrence Z est choisi indépendamment parmi (i) -O- et (ii) -S- et R₄ est un groupe aryle et
B₁ et B₂ sont choisis indépendamment parmi -W-R₅ et -Q-C(O)-R₆ dans lesquels à chaque occurrence Q et W sont choisis indépendamment parmi une liaison covalente et un groupe alkylène, R₆ est -OR₇ dans lequel R₇ est un hydrogène ou un groupe protecteur du groupe carboxy et R₅ est le groupe 5-tétrazolyle.

8. Composé tel que défini dans la revendication 1, caractérisé en ce que A₁ et A₂ sont choisis indépendamment parmi -C(O)-G dans lequel G est -N(R₁)(R₂) dans lequel à chaque occurrence R₁ est un groupe (a) alkyle inférieur(b) cycloalkyle et (c) cycloalkylalkyle et R₂ est le groupe benzyle dans lequel le cycle phényle du groupe benzyle est substitué par -Z-R₄ dans lequel à chaque occurrence Z est choisi indépendamment parmi (i) -O- et (ii) -S- et R₄ est un groupe hétérocyclique, dans lequel le groupe hétérocyclique peut être éventuellement substitué par un ou deux substituants choisis indépendamment parmi un groupe hydroxy, halo, oxo, alkylimino inférieur, amino, alkylamino, dialkylamino, alcoxy, alcoxyalcoxy, haloalkyle, cycloalkyle, aryle, arylalkyle, -COOH, -SO₃H et alkyle inférieur, et un hétérocycle contenant de l'azote peut être protégé pour N, et le groupe aryle ou la partie aryle du groupe arylalkyle peut être éventuellement substitué par un, deux ou trois substituants choisis indépendamment parmi un groupe alkyle inférieur, haloalkyle, alcoxy, thioalcoxy, amino, alkylamino, dialkylamino, hydroxy, halo, mercapto, nitro, carboxaldéhyde, carboxy, alcoxycarbonyle et carboxamide, ou le groupe aryle ou la partie aryle du groupe arylalkyle est le groupe tétrafluorophényle ou pentafluorophényle, et
B₁ et B₂ sont choisi indépendamment parmi -W-R₅ et -Q-C(O)-R₆ dans lesquels à chaque occurrence Q et W sont choisis indépendamment parmi une liaison covalente et un groupe alkylène, R₆ est -OR₇ dans lequel R₇ est l'hydrogène ou un groupe protecteur du groupe carboxy et R₅ est le groupe 5-térazolyle.

9. Composé de formule : 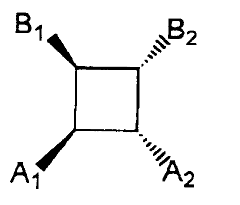 dans laquelle
A₁ et A₂ sont choisis indépendamment parmi
(1) -X-C(O)-G ou -X-C(S)-G dans lesquels à chaque occurrence X est choisi indépendamment parmi (a) une liaison covalente, (b) -CH₂-, (c) -O-, (d) -S- et (e) -N(Rₐ)- dans lequel Rₐ est l'hydrogène, un groupe alkyle inférieur, cycloalkyle ou cycloalkylalkyle et à chaque occurrence G est choisi indépendamment parmi -R₂, -N(R₁)(R₂), -OR₂ et -SR₂ dans lesquels à chaque occurrence R₁ est choisi indépendamment parmi (a) l'hydrogène, un groupe (b) alkyle inférieur, (c) alcényle, (d) alcynyle, (e) cycloalkyle, (f) cycloalkylalkyle, (g) alcoxycarbonylalkyle, (h) alcoxyalkyle, (i) thioalcoxyalkyle, (j) haloalkyle, (k) aryle, (I) hétérocyclique, (m) arylalkyle, (n) cycloalkylalkyle substitué par un aryle, (o) alkylhétérocyclique, (p) cycloalkylalkyle substitué par un hétérocycle et (q) un groupe aryle, hétérocyclique, arylalkyle, cycloalkylalkyle substitué par un aryle, alkylhétérocyclique ou cycloalkylalkyle substitué par un hétérocycle dans lequel le groupe aryle, la partie aryle du groupe arylalkyle, la partie aryle du groupe cycloalkylalkyle substitué par un aryle, le groupe hétérocyclique, la partie hétérocyclique du groupe alkylhétérocyclique ou la partie hétérocyclique du groupe cycloalkylalkyle substitué par un hétérocycle est substituée par -Y-R₃ dans lequel à chaque occurrence Y est choisi indépendamment parmi (i) une liaison covalente, (ii) -C(O)-, (iii) -CH₂-, (iv) -O-, (v) -S(O)ₘ- dans lequel m vaut 0, 1 ou 2, (vi) -N(R_{b})- dans lequel R_{b} est l'hydrogène ou un alkyle inférieur, (vii) -CH₂O-, (viii) -CH₂S(O)ₘ- dans lequel m vaut 0, 1 ou 2 et (ix) -CH₂N(R_{b})- dans lequel R_{b} est l'hydrogène ou un alkyle inférieur et à chaque occurrence R₃ est choisi indépendamment parmi un groupe (i) aryle, (ii) arylalkyle, (iii) cycloalkyle, (iv) cycloalkylalkyle, (v) hétérocyclique et (vi) alkylhétérocyclique et à chaque occurrence R₂ est choisi indépendamment parmi un groupe (i) alcényle, (ii) alcynyle, (iii) aryle, (iv) arylalkyle, (v) arylalcényle, (vi) hétérocyclique, (vii) alkylhétérocyclique et (vi) aryle, hétérocyclique, arylalkyle ou alkylhétérocyclique dans lequel le groupe aryle, la partie aryle du groupe arylalkyle, le groupe hétérocyclique ou la partie hétérocyclique du groupe alkylhétérocyclique est substitué par -Z-R₄ dans lequel à chaque occurrence Z est choisi indépendamment parmi (i) une liaison covalente, (ii) -C(O)-, (iii) -CH₂-, (iv) -O-, (v) -S(O)ₚ- dans lequel p vaut 0, 1 ou 2, (vi) -N(R_{c})- dans lequel R_{c} est l'hydrogène ou un alkyle inférieur, (vii) -CH₂O-, (viii) -CH₂S(O)ₚ- dans lequel p vaut 0, 1 ou 2 et (ix) -CH₂N(R_{c})- dans lequel R_{c} est l'hydrogène ou un alkyle inférieur et à chaque occurrence R₄ est choisi indépendamment parmi un groupe (i) aryle, (ii) arylalkyle, (iii) cycloalkyle, (iv) cycloalkylalkyle, (v) hétérocyclique et (vi) alkylhétérocyclique,
formule dans laquelle R₃ ou R₄ est un groupe hétérocyclique, alkylhétérocyclique, aryle ou arylalkyle, le groupe hétérocyclique ou la partie hétérocyclique du groupe alkylhétérocyclique peut être éventuellement substitué par un ou deux substituants choisis indépendamment parmi un groupe hydroxy, halo, oxo, alkylimino inférieur, amino, alkylamino, dialkylamino, alcoxy, alcoxyalcoxy, haloalkyle, cycloalkyle, aryle, arylalkyle, -COOH, -SO₃H et un alkyle inférieur, et un hétérocycle contenant de l'azote pouvant être protégé pour N, et le groupe aryle ou la partie aryle du groupe arylalkyle peut être éventuellement substitué par un, deux ou trois substituants choisis indépendamment parmi un groupe alkyle inférieur, haloalkyle, alcoxy, thioalcoxy, amino, alkylamino, dialkylamino, hydroxy, halo, mercapto, nitro, carboxaldéhyde, carboxy, alcoxycarbonyle et carboxamide, ou le groupe aryle ou la partie aryle du groupe arylalkyle est un groupe tétrafluorophényle ou pentafluorophényle, et
(2) -(CH₂)_{q}-N(R₁)(R₂) dans lequel q vaut 0, 1 ou 2 et à chaque occurrence R₁ et R₂ sont définis indépendamment comme ci-dessus ; et
B₁ et B₂ sont choisis indépendamment parmi
(1) -CH₂OH,
(2) -CH = NOH,
(3) -W-R₅ dans lequel à chaque occurrence W est choisi indépendamment parmi (a) une liaison covalente, un groupe (b) alkylène, (c) alcénylène, (d) -C(O)NH- et (e) -NHC(O)NH- et R₅ est choisi indépendamment parmi
(a) un groupe 5-tétrazolyle,
(b) 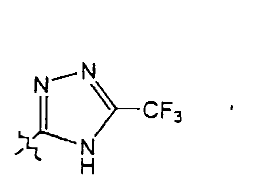
(c) 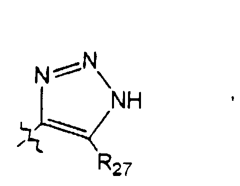 formule dans laquelle R₂₇ est -CN, -NO₂ ou -CO₂R₂₈ dans lequel R₂₈ est l'hydrogène, un groupe aryle ou alkyle inférieur, dans lequel le groupe aryle peut éventuellement être substitué par un, deux ou trois substituants choisi indépendamment parmi un groupe alkyle inférieur, haloalkyle, alcoxy, thioalcoxy, amino, alkylamino, dialkylamino, hydroxy, halo, mercapto, nitro, carboxaldéhyde, carboxy, alcoxycarbonyle et carboxamide, ou le groupe aryle peut être le groupe tétrafluorophényle ou pentafluorophényle,
(d) 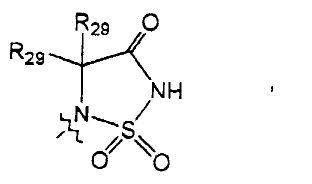 formule dans laquelle à chaque occurrence R₂₉ est choisi parmi l'hydrogène et un alkyle inférieur,
(e) 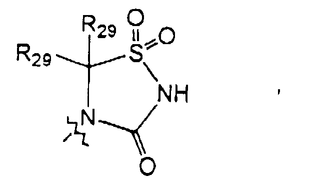 formule dans laquelle R₂₉ est tel que défini ci-dessus,
(f) 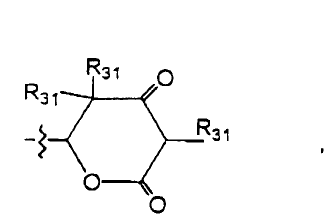 formule dans laquelle R₃₁ est à chaque occurrence choisi parmi l'hydrogène, un alkyle inférieur, alcényle, alcoxyalkyle et benzyle,
(g) 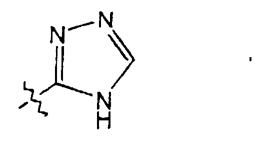
(h) 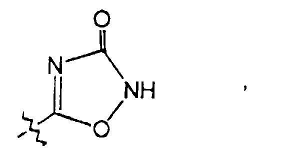
(i) 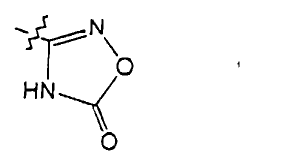
(j) 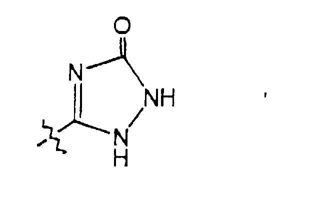
(k) 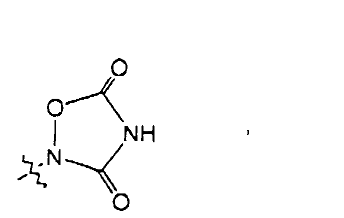
(l) 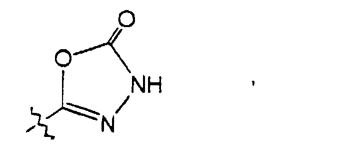
(m) 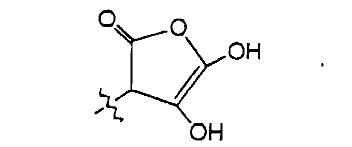 et
(n) 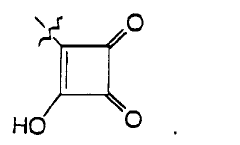
(4) -Q-C(O)R₆ dans laquelle à chaque occurrence Q est choisi indépendamment parmi (a) une liaison covalente, un groupe (b) alkylène, (c) alcénylène, (d) -CH(OH)- et (e) -NHC(O)(CH₂)ᵣ- dans lequel r vaut de 0 à 4 et à chaque occurrence R₆ est choisi indépendamment parmi (a) -OR₇ dans lequel R₇ est l'hydrogène ou un groupe protecteur du groupe carboxy, (b) -NH₂, (c) -NHOH, (d) -NHSO₂CF₃, (e) un acide alpha-aminé ou un acide béta-aminé qui est lié via le groupe alpha- ou béta-amino et (f) un di-, tri- ou tétra-peptide qui est lié via le groupe aminoterminal amino,
(5) -CH₂-N(OH)-C(O)-R₂₅ dans lequel R₂₅ est l'hydrogène, un groupe méthyle ou trifluorométhyle, et
(6) -C(O)-NH-S(O)₂-R₂₆ dans lequel R₂₆ est un groupe aryle, hétérocyclique, arylakyle, alkylhétérocyclique, cycloalkyle en C₃-C₇, alkyle en C₁-C₈ ou perfluoroalkyle en C₁-C₄,
dans lequel le groupe hétérocyclique ou la partie hétérocyclique du groupe alkylhétérocyclique peut éventuellement être substitué par un ou deux substituants choisis indépendamment parmi un groupe hydroxy, halo, oxo, alkylimino inférieur, amino, alkylamino, dialkylamino, alcoxy, alcoxyalcoxy, haloalkyle, cycloalkyle, aryle, arylalkyle, -COOH, -SO₃H et alkyle inférieur, et un hétérocycle contenant de l'azote pouvant avoir le N protégé, et le groupe aryle ou la partie aryle du groupe arylalkyle pouvant éventuellement être substitué par un, deux ou trois substituants choisis indépendamment parmi un groupe alkyle inférieur, haloalkyle, alcoxy, thioalcoxy, amino, alkylamino, dialkylamino, hydroxy, halo, mercapto, nitro, carboxaldéhyde, carboxy, alcoxycarbonyle et carboxamide, ou le groupe aryle ou la partie aryle du groupe arylalkyle est un groupe tétrafluorophényle ou pentafluorophényle;
ou son sel acceptable pharmaceutiquement.

10. Composé tel que défini dans la revendication 9, caractérisé en ce que A₁ et A₂ sont choisis indépendamment parmi -C(O)-G dans lequel G est défini comme ci-dessus et
B₁ et B₂ sont choisis indépendamment parmi
(a) -W-R₅ dans lequel W est une liaison covalente ou un groupe alkylène et R₅ est le groupe tétrazolyle et
(b) -Q-C(O)-R₆ dans lequel à chaque occurrence Q est choisi indépendamment parmi une liaison covalente et un groupe alkylène et à chaque occurrence R₆ est choisi indépendamment parmi
(1) -OR₇ dans lequel R₇ est l'hydrogène ou un groupe protecteur du groupe carboxy,
(2) un acide alpha-aminé ou un acide bêta-aminé qui est lié via le groupe alpha- ou béta-amino et
(3) un di-, tri- ou tétra-peptide qui est lié via le groupe amino terminal amino.

11. Composé tel que défini dans la revendication 9, caractérisé en ce que A₁ et A₂ sont choisis indépendamment parmi -C(O)-G dans lequel G est -N(R₁)(R₂) dans lequel R₁ et R₂ sont définis comme ci-dessus et B₁ et B₂ sont choisis indépendamment parmi
(a) ―W-R₅ dans lequel W est une liaison covalente ou un groupe alkylène et R₆ est le groupe 5-tétrazolyle et
(b) ―Q-C(O)-R₆ dans lequel à chaque occurrence Q est choisi indépendamment parmi une liaison covalente et un groupe alkylène et à chaque occurrence R₆ est choisi indépendamment parmi
(1) -OR₇ dans lequel R₇ est l'hydrogène ou un groupe protecteur du groupe carboxy,
(2) un acide alpha-aminé ou un acide béta-aminé qui est lié via le groupe alpha-amino ou béta-amino et
(3) un di-, tri- ou tétra-peptide qui est lié via le groupe amino terminal amino.

12. Composé tel que défini dans la revendication 9, caractérisé en ce que A₁ et A₂ sont indépendamment choisis parmi -C(O)-G dans lequel G est -N(R₁)(R₂) dans lequel à chaque occurrence R₁ est choisi indépendamment parmi (a) l'hydrogène, un groupe (b) alkyle inférieur, (c) cycloalkyle, (d) cycloalkylalkyle, (e) alcoxyalkyle, (f) thioalcoxyalkyle, (g) aryle, (h) hétérocyclique, (i) arylalkyle, (j) alkylhétérocyclique, (k) cycloalkylalyle substitué par un groupe aryle, (I) cycloalkylalkyle substitué par un groupe hétérocyclique et (m) un groupe aryle, hétérocyclique, arylalkyle, alkylhétérocyclique, cycloalkylalkye substitué par un groupe aryle ou cycloalkylalkyle substitué par un groupe hétérocyclique dans lequel le groupe aryle, la partie aryle du groupe arylalkyle, la partie aryle du groupe cycloalkylalkyle substitué par un groupe aryle, le groupe hétérocyclique, la partie hétérocyclique du groupe alkylhétérocyclique ou la partie hétérocyclique du groupe cycloalkylalkyle substitué par un groupe hétérocyclique est substituée par -Y-R₃ dans lequel à chaque occurrence Y est choisi indépendamment parmi (i) -O-, (ii) -S(O)ₘ- dans lequel m vaut 0, 1 ou 2 et (iii) -N(R_{b})- dans lequel R_{b} est l'hydrogène ou un groupe alkyle inférieur et à chaque occurrence R₃ est choisi indépendamment parmi un groupe (i) aryle, (ii) arylalkyle, (iii) cycloalkyle, (iv) cycloalkylalkyle, (v) hétérocyclique et (vi) alkylhétérocyclique et à chaque occurrence R₂ est choisi indépendamment parmi un groupe aryle, hétérocyclqiue, arylalkyle et alkylhétérocyclique dans lequel le groupe aryle, la partie aryle du groupe arylalkyle, le groupe hétérocyclique ou la partie hétérocyclique du groupe alkylhétérocyclique est substitué par -Z- R₄ dans lequel à chaque occurrence Z est choisi indépendamment parmi (i) -O-, (ii) -S(O)ₚ- dans lequel p vaut 0, 1 ou 2 et (iii) -N(R_{c})- dans lequel R_{c} est l'hydrogène ou un groupe alkyle inférieur et à chaque occurrence R₄ est choisi indépendamment parmi un groupe (i) aryle, (ii) arylalkyle, (iii) cycloalkyle, (iv) cycloalkylalkyle, (v) hétérocyclique, et (vi) alkylhétérocyclique, dans lequel, quand R₃ ou R₄ est un groupe hétérocyclique, alkylhétérocyclique, aryle ou arylalkyle, le groupe hétérocyclique ou la partie hétérocyclique du groupe alkylhétérocyclique peut être éventuellement substitué par un ou deux substituants choisis indépendamment parmi un groupe hydroxy, halo, oxo, alkylimino inférieur, amino, alkylamino, dialkylamino, alcoxy, alcoxyalcoxy, haloalkyle, cycloalkyle, aryle, arylalkyle, -COOH, -SO₃H et alkyle inférieur, et un hétérocycle contenant de l'azote peut être protégé pour N, et le groupe aryle ou la partie aryle du groupe arylalkyle peut être éventuellement substitué par un, deux ou trois substituants choisis indépendamment parmi un groupe alkyle inférieur, haloalkyle, alcoxy, thioalcoxy, amino, alkylamino, dialkylamino, hydroxy, halo, mercapto, nitro, carboxaldéhyde, carboxy, alcoxycarbonyle et carboxamide, ou le groupe aryle ou la partie aryle du groupe arylalkyle est le groupe tétrafluorophényle ou pentafluorophényle, et
B₁ et B₂ sont choisis indépendamment parmi
(a) -W-R₅ dans lequel W est une liaison covalente ou un groupe alkylène et R₅ est le groupe 5-tétrazolyle et
(b) -Q-C(O)-R₆ dans lequel à chaque occurrence Q est choisi indépendamment parmi une liaison covalente et un groupe alkylène et à chaque occurrence R₆ est choisi indépendamment parmi
(1) -OR₇ dans lequel R₇ est l'hydrogène ou un groupe protecteur du groupe carboxy,
(2) un acide alpha-aminé ou un acide béta-aminé qui est lié via le groupe alpha-amino ou béta-amino et
(3) un di-, tri- ou tétra-peptide qui est lié via le groupe amino terminal amino.

13. Composé tel que défini dans la revendication 9, caractérisé en ce que A₁ et A₂ sont choisis indépendamment parmi ―C(O)-G dans lequel G est -N(R₁)(R₂) dans lequel à chaque occurrence R₁ est choisi indépendamment parmi un groupe (a) alkyle inférieur (b) cycloalkyle, et (c) cycloalkylalkyle et à chaque occurrence R₂ est choisi indépendamment parmi un groupe aryle et arylalkyle dans lequel le groupe aryle ou la partie aryle du groupe arylalkyle est substitué par ―Z-R₄ dans lequel à chaque occurrence Z est choisi indépendamment parmi (i) -O-, (ii) -S- et à chaque occurrence R₄ est choisi indépendamment parmi un groupe (i) aryle, (ii) arylalkyle, (iii) cycloalkyle, (iv) cycloalkylalkyle, (v) hétérocyclique, et (vi) alkylhétérocyclique, dans lequel le groupe hétérocyclique ou la partie hétérocyclique du groupe alkylhétérocyclique peut être éventuellement substitué par un ou deux substituants choisis indépendamment parmi un groupe hydroxy, halo, oxo, alkylimino inférieur, amino, alkylamino, dialkylamino, alcoxy, alcoxyalcoxy, haloalkyle, cycloalkyle, aryle, arylalkyle, -COOH, -SO₃H et alkyle inférieur, et un hétérocycle contenant de l'azote peut être protégé pour N, et le groupe aryle ou la partie aryle du groupe arylalkyle peut être éventuellement substitué par un, deux ou trois substituants choisis indépendamment parmi un groupe alkyle inférieur, haloalkyle, alcoxy, thioalcoxy, amino, alkylamino, dialkylamino, hydroxy, halo, mercapto, nitro, carboxaldéhyde, carboxy, alcoxycarbonyle et carboxamide, ou le groupe aryle ou la partie aryle du groupe arylalkyle est le groupe tétrafluorophényle ou pentafluorophényle, et
B₁ et B₂ sont choisis indépendamment parmi -W-R₅ et -Q-C(O)-R₆ dans lesquels à chaque occurrence Q et W sont choisis indépendamment parmi une liaison covalente et un groupe alkylène, R₆ est -OR₇ dans lequel R₇ est l'hydrogène ou un groupe protecteur du groupe carboxy et R₅ est le groupe 5-tétrazolyle.

14. Composé tel que défini dans la revendication 9, caractérisé en ce que A₁ et A₂ sont choisis indépendamment parmi -C(O)-G dans lequel G est -N(R₁)(R₂) dans lequel à chaque occurrence R₁ est choisi indépendamment parmi -un groupe (a) alkyle inférieur(b) cycloalkyle, et (c) cycloalkylalkyle et à chaque occurrence R₂ est choisi indépendamment parmi un groupe phényle et benzyle dans lequel le groupe phényle ou le cycle phényle du groupe benzyle est substitué par -Z-R₄ dans lequel à chaque occurrenca Z est choisi indépendamment parmi (i) -O-, (ii) -S- et à chaque occurrence R₄ est choisi indépendamment parmi un groupe (i) aryle, (ii) arylalkyle, (iii) hétérocyclique et (iv) alkylhétérocyclique, dans lequel le groupe hétérocyclique ou la partie hétérocyclique du groupe alkylhétérocyclique peut être éventuellement substitué par un ou deux substituants choisis indépendamment parmi un groupe hydroxy, halo, oxo, alkylimino inférieur, amino, alkylamino, dialkylamino, alcoxy, alcoxyalcoxy, haloalkyle, cycloalkyle, aryle, arylalkyle, -COOH, -SO₃H et alkyle inférieur, et un hétérocycle contenant de l'azote peut être protégé pour N, et le groupe aryle ou la partie aryle du groupe arylalkyle peut être éventuellement substitué par un, deux ou trois substituants choisis indépendamment parmi un groupe alkyle inférieur, haloalkyle, alcoxy, thioalcoxy, amino, alkylamino, dialkylamino, hydroxy, halo, mercapto, nitro, carboxaldéhyde, carboxy, alcoxycarbonyle et carboxamide, ou le groupe aryle ou la partie aryle du groupe arylalkyle est le groupe tétrafluorophényle ou pentafluorophényle, et
B₁ et B₂ sont choisis indépendamment parmi -W-R₅ et -Q-C(O)-R₆ dans lesquels à chaque occurrence Q et W sont choisis indépendamment parmi une liaison covalente et un groupe alkylène, R₆ est -OR₇ dans lequel R₇ est l'hydrogène ou un groupe protecteur du groupe carboxy et R₅ est le groupe 5-tétrazolyle.

15. Composé tel que défini dans la revendication 9, caractérisé en ce que A₁ et A₂ sont choisis indépendamment parmi -C(O)-G dans lequel G est -N(R₁)(R₂) dans lequel à chaque occurrence R₁ est choisi indépendamment parmi un groupe (a) alkyle inférieur (b) cycloalkyle et (c) cycloalkylalkyle et R₂ est le groupe benzyle dans lequel le cycle phényle du groupe benzyle est substitué par -Z-R₄ dans lequel à chaque occurrence Z est choisi indépendamment parmi (i) -O- et (ii) -S- et R₄ est un groupe aryle et
B₁ et B₂ sont choisis indépendamment parmi -W-R₅ et -Q-C(O)-R₆ dans lesquels à chaque occurrence Q et W sont choisis indépendamment parmi une liaison covalente et un groupe alkylène, R₆ est -OR₇ dans lequel R₇ est un hydrogène ou un groupe protecteur du groupe carboxy et R₅ est le groupe 5-tétrazolyle.

16. Composé tel que défini dans la revendication 9, caractérisé en ce que A₁ et A₂ sont choisis indépendamment parmi -C(O)-G dans lequel G est -N(R₁)(R₂) dans lequel à chaque occurrence R₁ est un groupe (a) alkyle inférieur(b) cycloalkyle et (c) cycloalkylalkyle et R₂ est le groupe benzyle dans lequel le cycle phényle du groupe benzyle est substitué par -Z-R₄ dans lequel à chaque occurrence Z est choisi indépendamment parmi (i) -O- et (ii) -S- et R₄ est un groupe hétérocyclique, dans lequel le groupe hétérocyclique peut être éventuellement substitué par un ou deux substituants choisis indépendamment parmi un groupe hydroxy, halo, oxo, alkylimino inférieur, amino, alkylamino, dialkylamino, alcoxy, alcoxyalcoxy, haloalkyle, cycloalkyle, aryle, arylalkyle, -COOH, -SO₃H et alkyle inférieur, et un hétérocycle contenant de l'azote peut être protégé pour N, et le groupe aryle ou la partie aryle du groupe arylalkyle peut être éventuellement substitué par un, deux ou trois substituants choisis indépendamment parmi un groupe alkyle inférieur, haloalkyle, alcoxy, thioalcoxy, amino, alkylamino, dialkylamino, hydroxy, halo, mercapto, nitro, carboxaldéhyde, carboxy, alcoxycarbonyle et carboxamide, ou le groupe aryle ou la partie aryle du groupe arylalkyle est le groupe tétrafluorophényle ou pentafluorophényle, et
B₁ et B₂ sont choisis indépendamment parmi -W-R₅ et -Q-C(O)-R₆ dans lesquels à chaque occurrence Q et W sont choisis indépendamment parmi une liaison covalente et un groupe alkylène, R₆ est -OR₇ dans lequel R₇ est l'hydrogène ou un groupe protecteur du groupe carboxy et R₅ est le groupe 5-térazolyle.

17. Composé choisi dans le groupe constitué par
l'acide (1α,2β,3β,4α)-1,2-di[N-benzyl-N-(4-phénoxybenzyl)aminocarbonyl]cyclobutane-3,4-dicarboxylique ;
l'acide (1α,2β,3β,4α)-1,2-di[N-propyl-N-(4-phénoxybenzyl)aminocarbonyl]cyclobutane-3,4-dicarboxylique ;
l'acide (-)-(1α,2β,3β,4α)-1,2-di[N-propyl-N-(4-phénoxybenzyl)aminocarbonyl]cyclobutane-3,4-dicarboxylique ;
l'acide (1α,2β,3β,4α)-1,2-di[N-(2-éthylthioéthyl)-N-(4-phénoxybenzyl)aminocarbonyl]cyclobutane-3,4-dicarboxylique ;
l'acide (1α,2β,3β,4α)-1,2-di[N-(cyclopropylméthyl)-N-(4-phénoxybenzyl)aminocarbonyl]cyclobutane-3,4-dicarboxylique ;
l'acide (1α,2β,3β,4α)-1-[N-propyl-N-(4-phénoxybenzyl)aminocarbonyl]-2-[N-benzyl-N-(4-phénoxybenzyl)aminocarbonyl]cyclobutane-3,4-dicarboxylique ;
l'acide (1α,2β,3β,4α)-1,2-di[N-propyl-N-(4-phénylthiobenzyl)aminocarbonyl]cyclobutane-3,4-dicarboxylique ;
l'acide (1α,2β,3β,4α)-1,2-di[N-propyl-N-(4-phénoxybenzyl)aminocarbonyl-4-tétrazolylméthyl-cyclobutane-3-carboxylique ;
l'acide (1α,2β,3β,4α)-1,2-di[N-propyl-N-(4-phénoxybenzyl)aminocarbonyl]-4-(3-carboxypropionylamino)cyclobutane-3-carboxylique ;
l'acide (1α,2β,3β,4α)-1,2-di[N-propyl-N-(4-phénoxybenzyl)aminocarbonyl]-4-(1-carboxy-1-hydroxyméthyl)cyclobutane-3-carboxylique ;
l'acide (1α,2β,3β,4α)-1,2-di[N-propyl-N-(4-phénoxybenzyl)aminocarbonyl]cyclobutane-3-[N-(5-tétrazolyl)]carboxamide-4-carboxylique ;
l'acide (1α,2β,3β,4α)-4-(carboxyméthyl)-1,2-di[N-propyl-N-(4-phénoxybenzyl)aminocarbonyl]cyclobutane-3-carboxylique ; et
l'acide (1α,2β,3β,4α)-1,2-di[N-propyl-N-(4-phénoxybenzyl)aminocarbonyl]cyclobutane-3,4-diacétique ;
ou son sel acceptable pharmaceutiquement.

18. Composition pharmaceutique pour l'inhibition de la squalène synthétase comprenant une quantité efficace thérapeutiquement d'un composé selon la revendication 1 et un support acceptable pharmaceutiquement.

19. Composition pharmaceutique pour l'inhibition de la squalène synthétase comprenant une quantité efficace thérapeutiquement d'un composé selon la revendication 9 et un support acceptable pharmaceutiquement.

20. Utilisation d'un composé selon la revendication 1 pour la fabrication d'un médicament pour l'inhibition de la biosynthèse du cholestérol chez les humains et les mammifères inférieurs.

21. Utilisation d'un composé selon la revendication 9 pour la fabrication d'un médicament pour l'inhibition de la biosynthèse du cholestérol chez les humains et les mammifères inférieurs.

22. Utilisation d'un composé tel que défini par la revendication 1 pour la fabrication d'un médicament pour le traitement de l'hyperlipidémie ou l'artériosclérose chez un mammifère.

23. Utilisation d'un composé tel que défini par la revendication 9 pour la fabrication d'un médicament pour le traitement de l'hyperlipidémie ou l'arthériosdérose chez un mammifère.

24. Utilisation d'un composé tel que défini par la revendication 1 pour la fabrication d'un médicament pour le traitement d'une infection fongique chez un mammifère.

25. Utilisation d'un composé tel que défini par la revendication 9 pour la fabrication d'un médicament pour le traitement d'une infection fongique chez un mammifère.

26. Composé tel que défini par la revendication 1 pour l'utilisation comme agent thérapeutique.
